# EUROPEAN PATENT APPLICATION

(11) **EP 4 349 833 A1**
(43) Date of publication of application: **10.04.2024**
(21) Application number: 22815147.8
(22) Date of filing: 26.05.2022
(51) Int. Cl.: C07D 403/14, C07D 401/14, C07D 471/06, A61P 35/00, A61K 31/506, A61K 31/501

(54) **PYRIDAZINONE COMPOUND AS PARP7 INHIBITOR**

(30) Priority: 31.05.2021 CN 202110605018
(71) Applicant: Euregen Biopharma Co., Ltd., Zhongshan, Guangdong 528449 (CN); Shanghai Youli Biopharma Co., Ltd., Shanghai 201210 (CN)
(72) Inventor: LI, Jing, Shanghai 201210 (CN); CHEN, Yanhong, Shanghai 201210 (CN); CHEN, Xuxing, Shanghai 201210 (CN)
(74) Representative: dompatent von Kreisler Selting Werner - Partnerschaft von Patent- und Rechtsanwälten mbB
(86) International application number: PCT/CN2022/095271
(87) International publication number: WO 2022/253101

(57) **Abstract**

The present invention relates to a pyridazinone compound as a PARP7 inhibitor, in particular to a compound as represented by the structure of formula I, which can be used as a PARP 7 inhibitor, and can be used for preparing drugs for treating diseases comprising tumors.

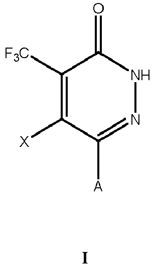

## Description

### Technical Field

The invention relates to the field of pharmaceutical chemistry. More specifically, it pertains to pyridazinone compounds as PARP7 inhibitors, which can be utilized in the preparation of pharmaceuticals for the treatment of diseases, including but not limited to cancer.

### Background

Poly(ADP-ribose) polymerases (PARPs) encompass 17 subtypes, regulating various physiological processes within organisms, including gene expression, protein degradation, and multiple cellular stress responses. Among the PARP family, PARP1 is the most extensively studied member and is associated with DNA damage repair, making it an effective target for anticancer drugs. All members of the PARP family contain a conserved catalytic domain of approximately 230 amino acid residues. Depending on the number of catalytically transferred ADP-ribose, they are categorized as poly-PARP, mono-PARP, and PARP13 (with an undefined biological function). PARP7 belongs to the mono-PARP subgroup, capable of transferring only one ADP-ribose group.

The aryl hydrocarbon receptor (AHR) is a ligand-activated transcription factor involved in the regulation of various cellular functions, including inflammatory responses and tumor metabolism. It is an important regulatory factor for the occurrence and development of cancer. AHR activation upregulates the gene expression of downstream PARP7, which can disrupt the release of interferon-beta (IFN-β) in a TBK1-dependent manner and result in an immunosuppressive tumor microenvironment. This is a significant mechanism for tumor immune escape. Inhibiting PARP7 effectively suppresses cancer cell growth, restores interferon signal transduction, and releases the "brakes" which are utilized by cancer cell to escape immune system and inhibit both innate and adaptive immune. The PARP7 gene is located in band 2, region 5 of long arm of chromosome 3 (3q25). It is often found to be amplified in various glandular cancers. PARP7 inhibitors have demonstrated persistent tumor growth inhibition, effective antiproliferative activity, and restoration of interferon signal transduction in several cancer models.

The PARP7 inhibitor RBN-2397, developed by Ribon Corporation, is currently in Phase I clinical trials, and its structure has been disclosed.

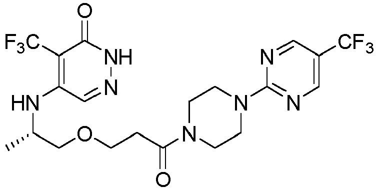

However, RBN-2397 exhibits very poor pharmacokinetic properties, therefore there are still significant unmet clinical needs. It is emergent to develop effective PARP7 inhibitor with improved drug-like characteristics in this field.

### SUMMARY

The purpose of this invention is to provide pyridazinone compounds or their pharmaceutically acceptable salts thereof.

Another objective of this invention is to provide a pharmaceutical composition comprising the pyridazinone compounds or their pharmaceutically acceptable salts thereof.

A further purpose of the present invention is to provide the use of the pyridazinone compounds or the pharmaceutically acceptable salts thereof or the compositions comprising the pyridazinone compounds or the pharmaceutically acceptable salts thereof in the preparation of anti-tumor drugs.

In the first aspect of the present invention, provided is a compound of formula I, or pharmaceutically acceptable salts thereof , enantiomers, diastereomers, tautomers, cis-trans isomers, solvates, polymorphs, deuterated compounds, or combinations thereof, 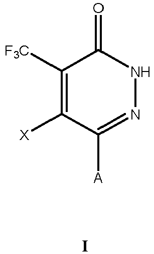 wherein,
X is selected from hydrogen, halogen, or cyano;
A is 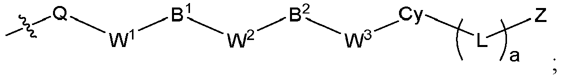
wherein,
Q is absent, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to 6-10 membered aryl, optionally substituted saturated or unsaturated 3-8 membered carbocyclyl fused to 5-10 membered heteroaryl, optionally substituted saturated or unsaturated 3-8 membered heterocyclyl fused to 6-10 membered aryl, optionally substituted saturated or unsaturated 3-8 membered heterocyclyl fused to 5-10 membered heteroaryl, optionally substituted 6-10 membered aryl, or optionally substituted 5-10 membered heteroaryl; wherein, the substitutions are defined as being replaced by one or more R groups;
W¹ is absent, -NR^{w}-, -O-, or -S-, wherein the R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups; at most one of Q and W1 is absent;
Each W² and W³ is independently absent, optionally substituted methylene group, -O-, -S-, -NR^{w'}-, -(C=O)-, -C(=O)O-, -C(=O)NR^{w'}-, -(S=O)-, -S(=O)₂-, -S(=O)₂NR^{w'}-, -S(=O)NR^{w'}-, or -NR^{w'}C(=O)NR^{w'}-, wherein, each R^{w'} is independently H, optionally substituted C1-C6 alkyl, optionally substituted saturated or unsaturated 3-8 membered carbocyclyl, or optionally substituted saturated or unsaturated 4-12 membered heterocyclyl; wherein, the substitutions are defined as being replaced by one or more R groups;
Each B¹ and B² is independently absent, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused with 6-10 membered aryl, optionally substituted saturated or unsaturated 3-8 membered carbocyclyl fused with 5-10 membered heteroaryl, optionally substituted saturated or unsaturated 3-8 membered heterocyclyl fused with 6-10 membered aryl, optionally substituted saturated or unsaturated 3-8 membered heterocyclyl fused with 5-10 membered heteroaryl, optionally substituted 6-10 membered aryl, optionally substituted 5-10 membered heteroaryl, optionally substituted C1-C4 alkyl, optionally substituted C2-C4 alkenyl, or optionally substituted C2-C4 alkynyl, and at most one of B1 and B2 is absent; wherein, the substitutions are defined as being replaced by one or more R groups;
Cy is selected from optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl ring, optionally substituted 6-10 membered aryl, and optionally substituted 5-10 membered heteroaryl; wherein, the substitutions are defined as being replaced by one or more R groups;
L is optionally substituted C1-C3 alkyl, -O-, -S-, -NR^{L}-, -(C=O)-, -C(=O)O-, -C(=O)NR^{L}-, -(S=O)-, -S(=O)₂-, -S(=O)₂NR^{L}-, -S(=O)NR^{L}-, or -NR^{L}C(=O)NR^{L}-, wherein, each R^{L} is independently H or optionally substituted C1-C4 alkyl; wherein, the substitutions are defined as being replaced by one or more R groups;
a is 0 or 1;
Z is selected from hydrogen, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, optionally substituted 5-10 membered heteroaryl, optionally substituted C1-C6 alkyl, optionally substituted C2-C6 alkenyl and optionally substituted C2-C6 alkynyl; wherein, the substitutions are defined as being replaced by one or more R groups;
Each R is independently selected from: D, halogen, -OH, oxo, thiol, cyano, -CD₃, C1-C6 alkyl, C2-C6 alkenyl, C2-C6 alkynyl, C3-C8 cycloalkyl, 6-10 membered aryl, 4-12 membered heterocyclyl, 5-10 membered heteroaryl, 6-10 membered aryl-C1-C6 alkyl, 5-10 membered heteroaryl-C1-C6 alkyl, C1-C6 haloalkyl, -OC1-C6 alkyl, -OC2-C6 alkenyl, C3-C8 cycloalkyl-O-, 4-12 membered heterocyclyl-O-, 6-10 membered aryl-O-, 5-10 membered heteroaryl-O-, -O-C1-C6 alkylphenyl, -C1-C6 alkyl-OH, -C1-C6 alkyl-SH, -C1-C6 alkyl-O-C1-C6 alkyl, -OC1-C6 haloalkyl, -NH₂, -C1-C6 alkyl-NH₂, -N(C1-C6 alkyl)₂, -NH(C1-C6 alkyl), -N(C1-C6 alkyl)(C1-C6 alkylphenyl), -NH(C1-C6 alkylphenyl), -N(C1-C6 alkyl)(6-10 membered aryl), -NH(6-10 membered aryl), nitro, -C(O)-OH, -C(O)OC1-C6 alkyl, -CONRⁱRⁱⁱ, -NHC(O)(C1-C6 alkyl), -NHC(O)(phenyl), -N(C1-C6 alkyl)C(O)(C1-C6 alkyl), -N(C1-C6 alkyl)C(O)(phenyl), -C(O)C1-C6 alkyl, 5-10 membered heteroaryl C(O), -C(O)C1-C6 alkylphenyl, -C(O)C1-C6 haloalkyl, -OC(O)C1-C6 alkyl, -S(O)₂-C1-C6 alkyl, -S(O)-C1-C6 alkyl, -S(O)₂-phenyl, -S(O)₂-C1-C6 haloalkyl, -S(O)₂NH₂, -S(O)₂NH(C1-C6 alkyl), -S(O)₂NH(phenyl), -NHS(O)₂(C1-C6 alkyl), -NHS(O)₂(phenyl), and -NHS(O)₂(C1-C6 haloalkyl); wherein each of the alkyl, alkenyl, alkynyl, cycloalkyl, phenyl, aryl, heterocyclyl, and heteroaryl is optionally further substituted by one or more substituents selected from halogen, -OH, oxo (=O), -NH₂, C3-C8 cycloalkyl, 3-8 membered heterocyclyl, C1-C4 alkyl, C1-C4 haloalkyl, -OC1-C4 alkyl, -C1-C4 alkyl-OH, -C1-C4 alkyl-O-C1-C4 alkyl, -OC1-C4 haloalkyl, cyano, nitro, -C(O)-OH, -C(O)OC1-C6 alkyl, -CON(C1-C6 alkyl)₂, -CONH(C1-C6 alkyl), -CONH₂, -NHC(O)(C1-C6 alkyl), -NH(C1-C6 alkyl)C(O)(C1-C6 alkyl), -SO₂(C1-C6 alkyl), -SO₂(phenyl), -SO₂(C1-C6 haloalkyl), -SO₂NH₂, -SO₂NH(C1-C6 alkyl), -SO₂NH(phenyl), -NHSO₂(C1-C6 alkyl), -NHSO₂(phenyl), and -NHSO₂(C1-C6 haloalkyl); each Rⁱ and Rⁱⁱ is independently H, D, or C1-6 alkyl.

In another preferred embodiment, each B¹ and B² is independently absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to 6-8 membered aryl, optionally substituted saturated or unsaturated 3-8 membered carbocyclyl fused to 5-8 membered heteroaryl, optionally substituted saturated or unsaturated 3-8 membered heterocyclyl fused to 6-8 membered aryl, optionally substituted saturated or unsaturated 3-8 membered heterocyclyl fused to 5-8 membered heteroaryl, optionally substituted 6-8 membered aryl, optionally substituted 5-8 membered heteroaryl, or optionally substituted C1-C2 alkyl; wherein, the substitutions are defined as being replaced by one or more R groups; the definition of R is as described in the previous description;

In another preferred embodiment, Cy is optionally substituted saturated or unsaturated 3-8 membered carbocyclyl, optionally substituted saturated or unsaturated 4-12 membered heterocyclyl; Preferably, Cy is optionally substituted saturated or unsaturated 4-8 membered heterocyclyl; wherein, the heterocyclyl contains 1, 2, or 3 nitrogen atoms; wherein, the substitutions are defined as being replaced by one or more R groups; the definition of R is as described in the previous description;

In another preferred embodiment, X is H or halogen;
A is 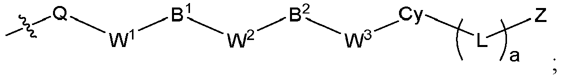 wherein,
Q is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, or optionally substituted 5-8 membered heteroaryl; wherein, the substitutions are defined as being replaced by one or more R groups;
W¹ is absent, -NR^{w}-, or -O-, wherein R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups; at most one of Q and W¹ is absent;
Each W² and W³ is independently absent, optionally substituted methylene, -O-, -S-, -NR^{w'}-, -(C=O)-, -C(=O)NR^{w'}-, -(S=O)-, -S(=O)₂-, -S(=O)₂NR^{w'}-, -S(=O)NR^{w'}-, or -NR^{w'}C(=O)NR^{w'}-, wherein each R^{w'} is independently H or optionally substituted C1-C6 alkyl; wherein, the substitutions are defined as being replaced by one or more R groups;
Each B¹ and B² is independently absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl; wherein, the substitutions are defined as being replaced by one or more R groups; at most one of B¹ and B² is absent;
Cy is optionally substituted saturated or unsaturated 3-8 membered carbocyclyl or optionally substituted saturated or unsaturated 4-12 membered heterocyclyl; wherein, the substitutions are defined as being replaced by one or more R groups;
L is optionally substituted C1-C3 alkyl, -O-, -S-, -NR^{L}-, -(C=O)-, -C(=O)NR^{L}-, -(S=O)-, -S(=O)₂-, -S(=O)₂NR^{L}-, -S(=O)NR^{L}-, or -NR^{L}C(=O)NR^{L}-, wherein, each R^{L} is independently H or optionally substituted C1-C4 alkyl; wherein, the substitutions are defined as being replaced by one or more R groups.
a is 0 or 1;
Z is optionally substituted 6-10 membered aryl, or optionally substituted 5-10 membered heteroaryl; wherein, the substitutions are defined as being replaced by one or more R groups;
The definition of R is as described in the previous description;
In another preferred embodiment, X is H;
In another preferred embodiment, Q is absent, optionally substituted saturated or unsaturated C3-C8 cycloalkyl, optionally substituted saturated or unsaturated 4-10 membered heterocyclyl, optionally substituted saturated or unsaturated 3-8 membered heterocyclyl fused to phenyl, optionally substituted saturated or unsaturated 3-8 membered heterocyclyl fused to 5-6 membered heteroaryl; preferably, Q is optionally substituted C3-C6 cycloalkyl or optionally substituted 4-10 membered heterocyclyl; wherein, the substitutions are defined as being replaced by one or more R groups; The definition of R is as described in the previous description;
In another preferred embodiment, A is either of formula A-1 or formula A-2, 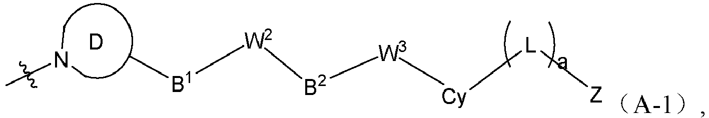 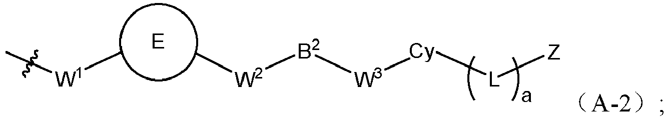 wherein,
The D ring is optionally substituted saturated or unsaturated 4-10 membered heterocyclyl, optionally substituted saturated or unsaturated 3-8 membered heterocyclyl fused to a 6-8 membered aryl, or optionally substituted saturated or unsaturated 3-8 membered heterocyclyl fused to 5-8 membered heteroaryl; wherein, the substitutions are defined as being replaced by one or more R groups;
The E ring is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to 6-8 membered aryl, optionally substituted saturated or unsaturated 3-8 membered heterocyclyl fused to 5-8 membered heteroaryl, optionally substituted 6-8 membered aryl, or optionally substituted 5-8 membered heteroaryl; wherein, the substitutions are defined as being replaced by one or more R groups;
B¹ is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl; wherein, the substitutions are defined as being replaced by one or more R groups;
W¹ is -NR^{w}- or -O-, wherein, R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups;
Each W² and W³ is independently absent, optionally substituted methylene, -O-, -S-, -NR^{w'}-, -(C=O)-, -C(=O)NR^{w'}-, -(S=O)-, -S(=O)₂-, -S(=O)₂NR^{w'}-, -S(=O)NR^{w'}-, or -NR^{w'}C(=O)NR^{w'}-; wherein, each R^{w'} is independently H, or optionally substituted C1-C6 alkyl; wherein, the substitutions are defined as being replaced by one or more R groups;
B² is optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl group; wherein, the substitutions are defined as being replaced by one or more R groups;
Cy is optionally substituted saturated or unsaturated 3-8 membered carbocyclyl or optionally substituted saturated or unsaturated 4-12 membered heterocyclyl; wherein, the substitutions are defined as being replaced by one or more R groups;
L is optionally substituted C1-C3 alkyl group, -O-, -S-, -NR^{L}-, -(C=O)-, -C(=O)NR^{L}-, -(S=O)-, -S(=O)₂-, -S(=O)₂NR^{L}-, -S(=O)NR^{L}-, or -NR^{L}C(=O)NR^{L}-; wherein, each R^{L} independently is H or optionally substituted C1-C4 alkyl group; wherein, the substitutions are defined as being replaced by one or more R groups;
a is 0 or 1;
Z is optionally substituted 6-10 membered aryl or optionally substituted 5-10 membered heteroaryl; wherein, the substitutions are defined as being replaced by one or more R groups;
The definition of the R group is as described above.

In another preferred embodiment, Z is optionally substituted phenyl, pyridyl, pyrimidyl, pyridazinyl, thiophenyl, thiazolyl, imidazolyl, pyrazolyl, oxazolyl, oxadiazole, triazole, tetrazole, indolyl, furanyl, pyrrolyl; wherein, the substitutions are defined as being replaced by one or more R groups; The definition of the R group is as described above.

In another preferred embodiment, Z is selected from: 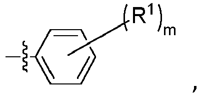 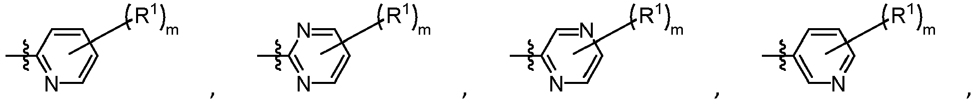 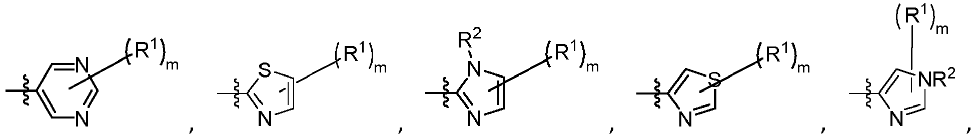 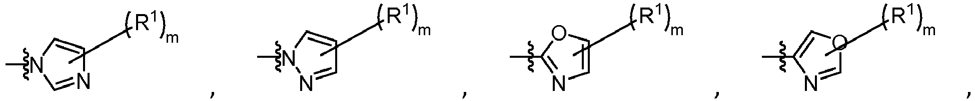 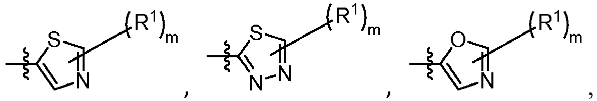 wherein, each of and R² is independently selected from: halogen, optionally substituted C1-C6 alkyl, cyano, optionally substituted C3-C16 cycloalkyl, optionally substituted 4-16 membered heterocyclyl, 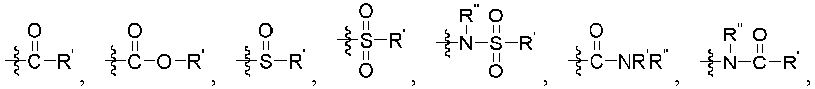 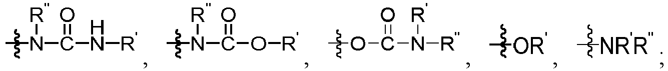 or two groups located on any adjacent atoms of ring together with their adjacent atoms form optionally substituted C3-C16 carbocyclyl, optionally substituted 4-16 membered heterocyclyl; or R² with on the adjacent atom of ring forms an optionally substituted 4-16 membered heterocyclyl; wherein, R' and R" are each independently selected from: H, optionally substituted C1-C6 alkyl, optionally substituted C3-C8 cycloalkyl, optionally substituted 4-16 membered heterocyclyl; or R' and R" together with their adjacent atoms form an optionally substituted 4-16 membered heterocyclyl; wherein, the heterocyclyl formed by R' and R" with the N atom includes 1-3 heteroatoms selected from N, O, S, P; wherein, the substitutions are defined as being replaced by one or more R groups;
m is 0, 1, 2, or 3; R is defined as previously described.

In another preferred embodiment, m is 0 or 1.

In another preferred embodiment, Z is selected from: 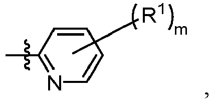 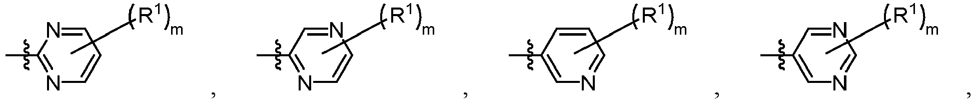 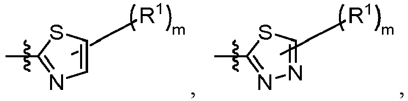 Each is independently selected from: halogen, optionally substituted C1-C6 alkyl, cyano, optionally substituted C3-C16 cycloalkyl, optionally substituted 4-16 membered heterocycloalkyl, 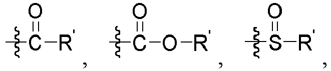 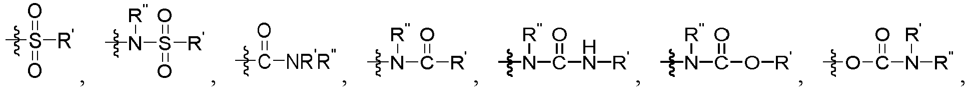 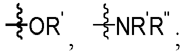 R' and R" are each independently selected from H, optionally substituted C1-C6 alkyl, optionally substituted C3-C8 cycloalkyl, optionally substituted 4-16 membered heterocycloalkyl, or optionally substituted 4-16 membered heterocycle formed by the connection of R' and R", wherein, the heterocycle formed by the connection of R' and R" contains 1-3 heteroatoms selected from N, O, S, P; Preferably, each is independently selected from halogen, optionally substituted C1-C6 alkyl, cyano, optionally substituted C3-C16 cycloalkyl, or optionally substituted 4-16 membered heterocycloalkyl; Most preferably, each is independently halogen, trifluoromethyl, difluoromethyl, fluoroethyl, cyano, methyl, ethyl, isopropyl, tert-butyl, isobutyl, cyclopropyl, or cyclopentyl; m represents the number of substituent groups R¹, and m is 0, 1, 2, or 3; m is 0 or 1. 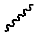 represents the point of attachment of the group.

In another preferred embodiment, and R₂ are each independently selected from halogens, optionally substituted C1-C6 alkyl, cyano, optionally substituted C3-C6 cycloalkyl, optionally substituted 4-6 membered heterocycloalkyl, 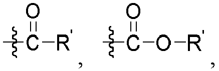 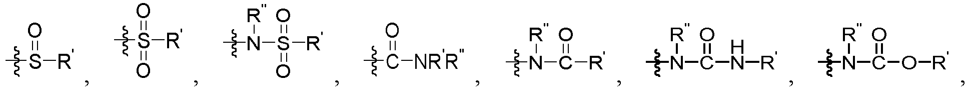 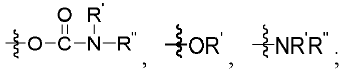 or two groups located on any adjacent atoms of ring together with their adjacent atoms form optionally substituted C3-C6 cycloalkyl or optionally substituted 4-6 membered heterocycloalkyl; wherein, the substitutions are defined as being replaced by one or more R groups;

The definitions of R, R', and R" are as described above.

In another preferred embodiment, 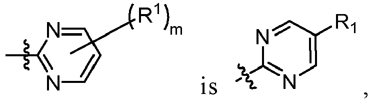 preferably is

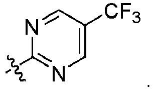

In another preferred embodiment, Cy is selected from the following group: 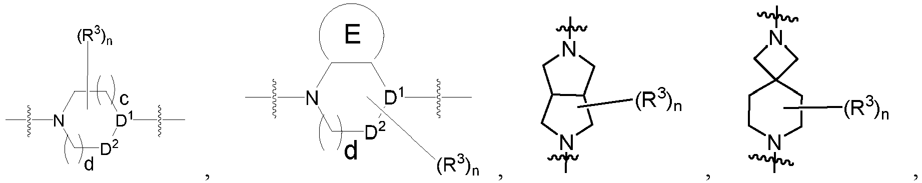 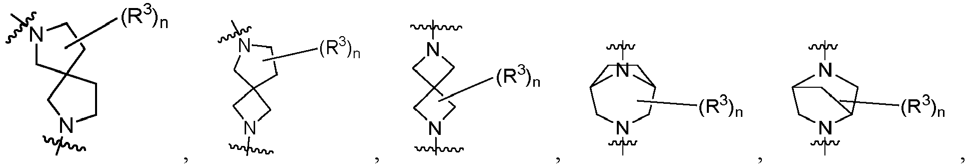 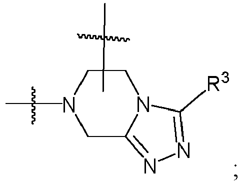
D¹ is N or CR^{D1}, wherein, R^{D1} is H, optionally substituted C1-C3 alkyl, halogen, hydroxy, optionally substituted C1-C3 alkoxy, or optionally substituted amino; wherein, the substitutions are defined as being replaced by one or more R groups;
D2 is NR^{D2}, optionally substituted methylene, O, or S; wherein, R^{D2} is H or optionally substituted C1-C4 alkyl; wherein, the substitutions are defined as being replaced by one or more R groups;
Each R³ is independently selected from: H, halogen, optionally substituted C1-C6 alkyl, optionally substituted C3-C16 cycloalkyl, optionally substituted 4-16 membered heterocyclyl, 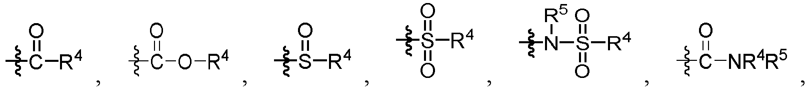 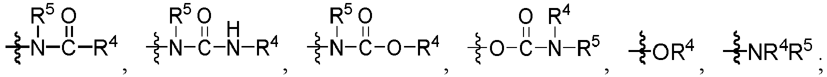 wherein, R⁴ and R⁵ are independently selected from: H, optionally substituted C1-C6 alkyl, optionally substituted C3-C8 cycloalkyl, optionally substituted 4-16 membered heterocyclyl; or R⁴ and R⁵ together with the adjacent atoms form optionally substituted 4-16 membered heterocyclyl, wherein the heterocyclyl formed by R⁴ and R⁵ with the N atom contains 1-3 heteroatoms selected from N, O, S, P, 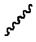 indicating the attachment position of the group; or two R³ groups located on any adjacent atoms of ring and their adjacent atoms together form optionally substituted C3-C16 cycloalkyl or optionally substituted 4-16 membered heterocyclyl; wherein, the substitutions are defined as being replaced by one or more R groups;
E-ring is optionally substituted 6-8 membered aryl or optionally substituted 5-8 membered heteroaryl; wherein, the substitutions are defined as being replaced by one or more R groups;
n represents the number of substituents R³, and n is 0, 1, 2, or 3;
c and d are independently 0, 1, 2, or 3;
The definition of R is as described above;

In another preferred embodiment, D¹ is N or CH.

In another preferred embodiment, Cy is selected from the following group:

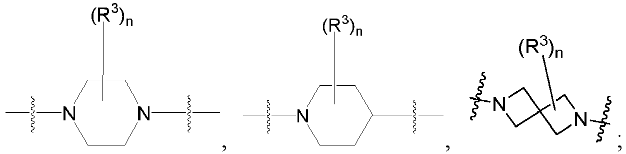

The definitions of R³ and n are as described above.

In another preferred embodiment, each R³ is independently selected from: H, halogen, optionally substituted C1-C6 alkyl, optionally substituted C3-C6 cycloalkyl, optionally substituted 4-6 membered heterocyclyl, 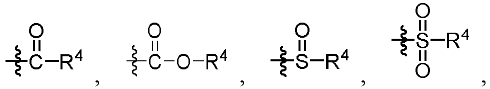 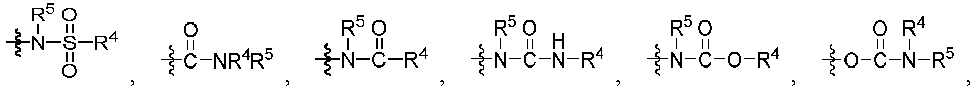 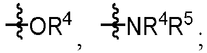 wherein, R⁴ and R⁵ are independently selected from: H, optionally substituted C1-C6 alkyl, optionally substituted C3-C6 cycloalkyl, optionally substituted 4-6 membered heterocyclyl, or R⁴ and R⁵ together with adjacent atoms form an optionally substituted 4-6 membered heterocyclyl, wherein, the heterocycle formed by R⁴ and R⁵, in conjunction with the N atom, includes 1-3 heteroatoms selected from N, O, S, P. 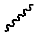 indicating the attachment position of the group; or, any two adjacent R³ groups, together with their adjacent atoms, form an optionally substituted C3-C6 cycloalkyl or an optionally substituted 4-6 membered heterocyclyl; wherein, the substitutions are defined as being replaced by one or more R groups; the definition of R remains as previously described.

In another preferred embodiment, 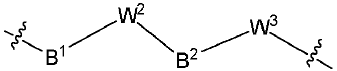 is selected from;

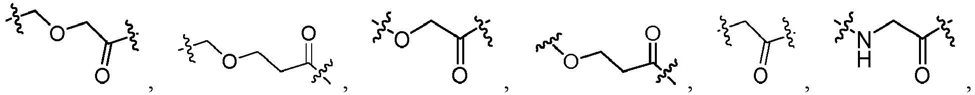

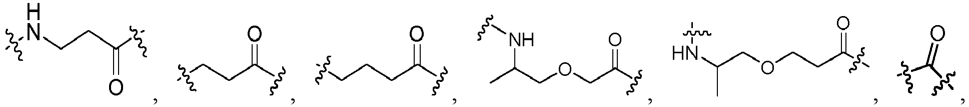

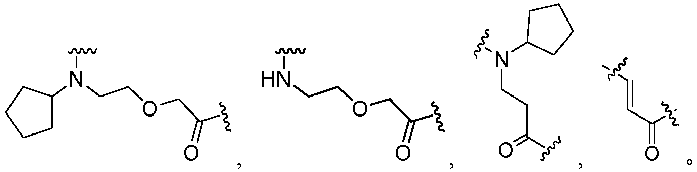

In another preferred embodiment, 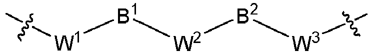 is selected from:

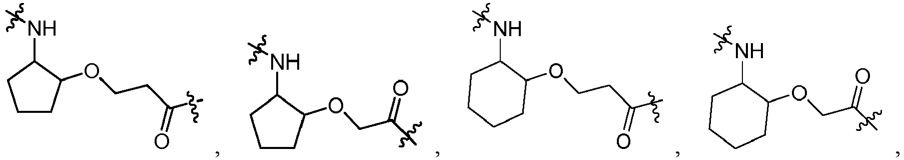

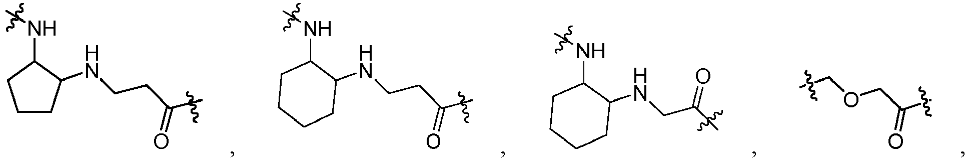

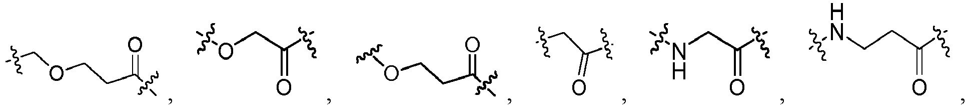

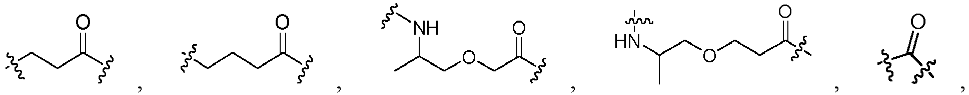

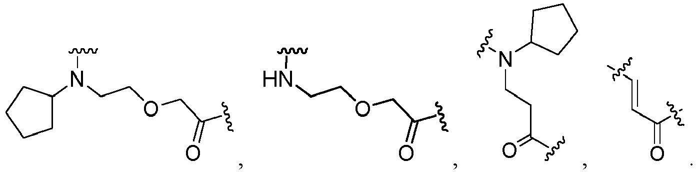

In another preferred embodiment, characterized in that,

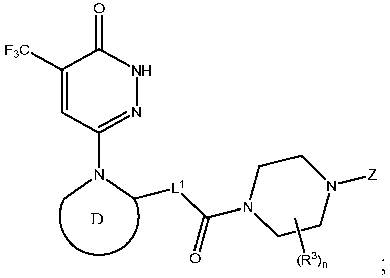

Wherein, L¹ is selected from the following groups: absent, 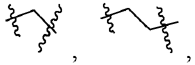 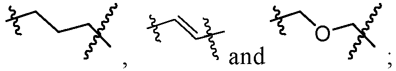 and L¹ may be optionally substituted by one or more R;

D ring is optionally substituted saturated or unsaturated 4-10 membered heterocyclyl, optionally substituted saturated or unsaturated 3-8 membered heterocyclyl fused to a 6-8 membered aryl, or optionally substituted saturated or unsaturated 3-8 membered heterocyclyl fused to a 5-8 membered heteroaryl, preferably, D ring is optionally substituted 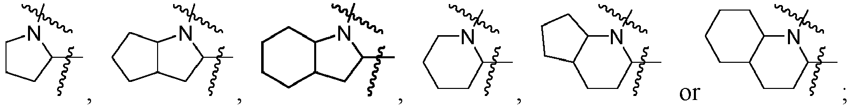 wherein, the substitutions are defined as being replaced by one or more R groups;

The definitions of Z, R³, n, and R are as previously described.

In another preferred embodiment, Q and D ring are independently 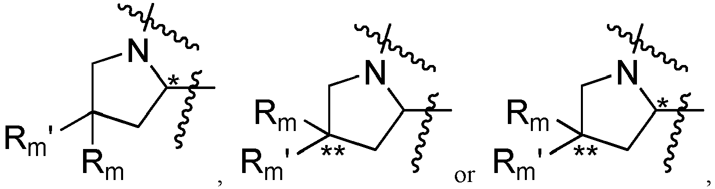 wherein * and ** are independently R or S configurations; Rm and Rm' are independently selected from H, halogens (such as F), C1-C6 alkyl (such as methyl, ethyl, propyl), C1-C6 alkoxy (such as methoxy, ethoxy, propoxy, n-butoxy, isobutoxy, tert-butoxy), phenyl, benzyl, phenoxy, halogenated phenoxy (such as. 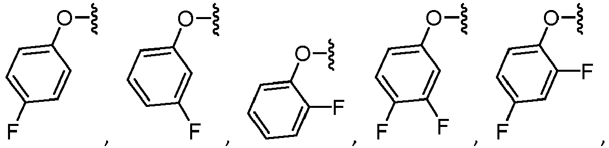 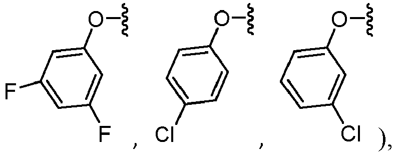 5-6 membered heteroaryl O- (such as 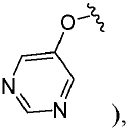 substituted 5-6 membered heteroaryl O- (such as 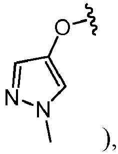 NH₂, NH(CH₃), N(CH₃)₂, OH, 3-6 membered heterocyclyl (such as pyrrolidinyl, tetrahydropyrrolyl).

In another preferred embodiment, X, A, Q, W¹, W², W³, B¹, B², L, and Cy are as defined for the specific compounds in the embodiments.

In another preferred embodiment, the compounds are selected from the following group:

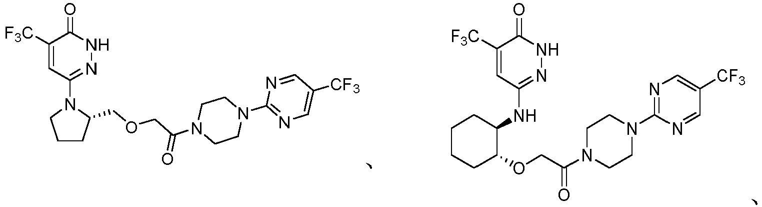

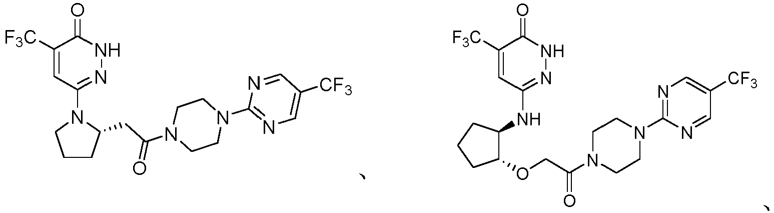

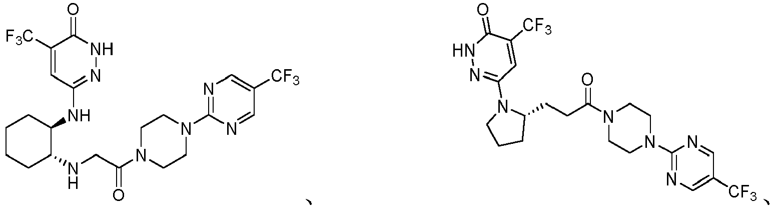

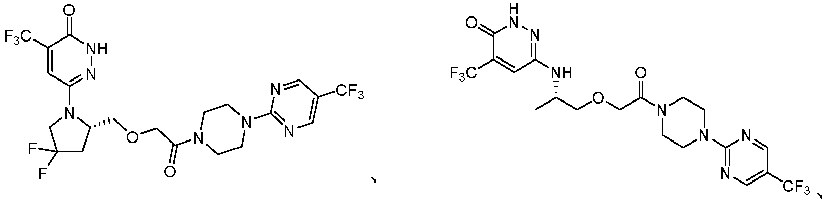

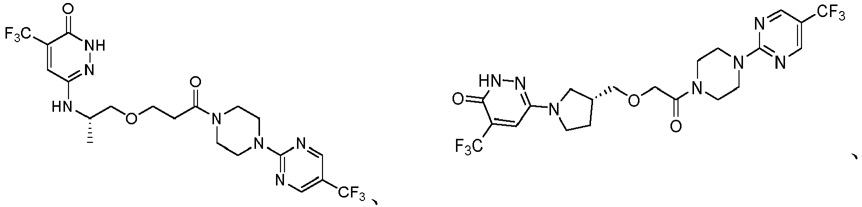

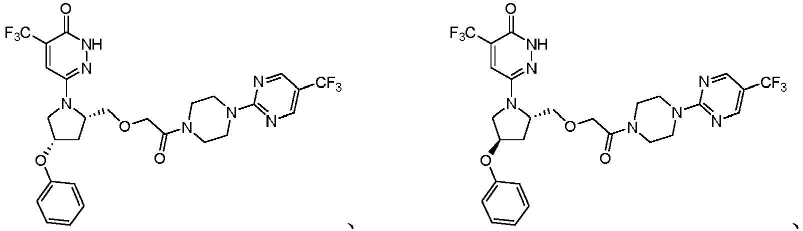

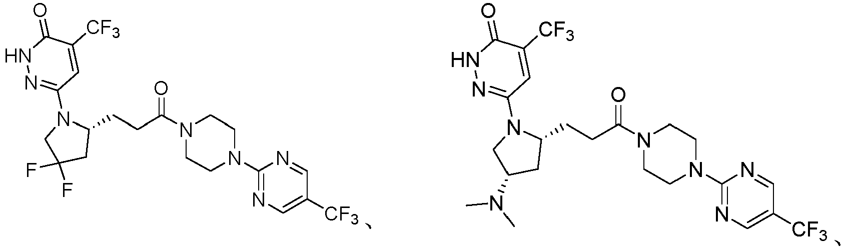

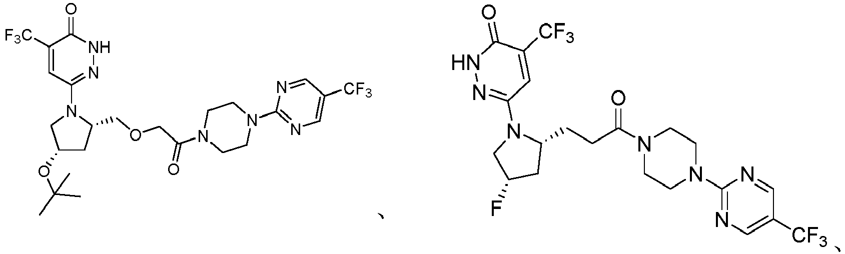

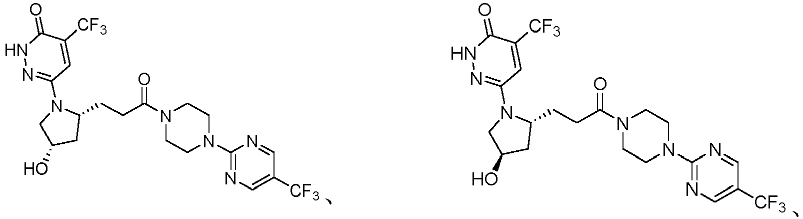

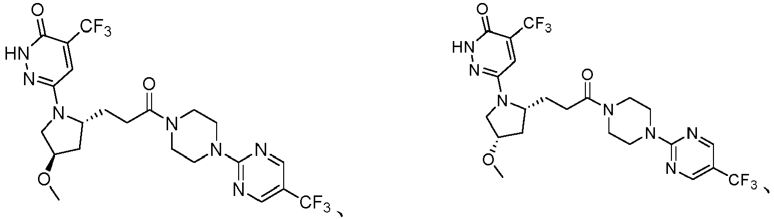

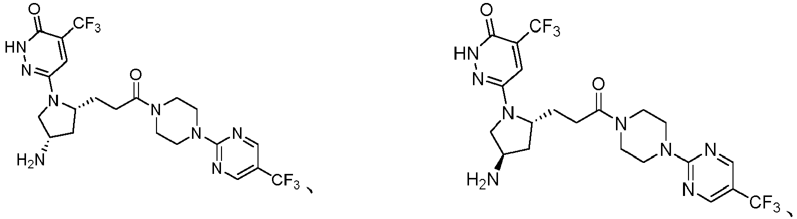

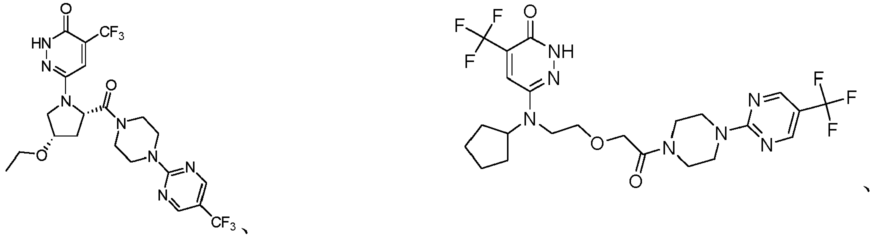

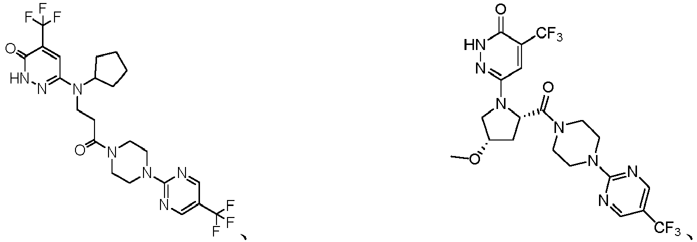

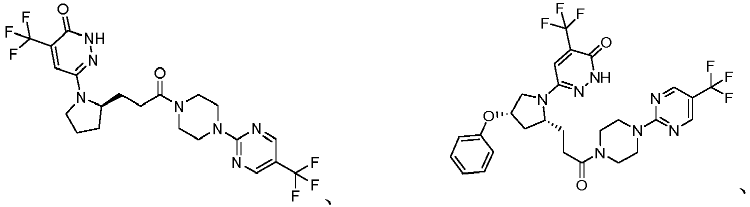

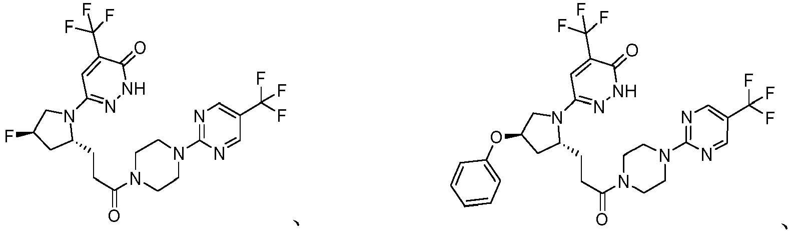

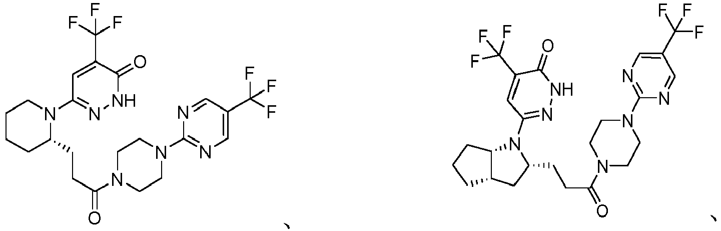

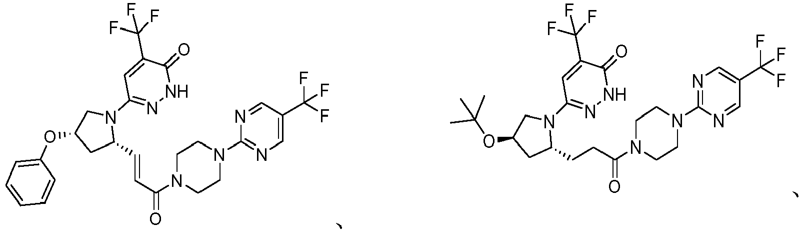

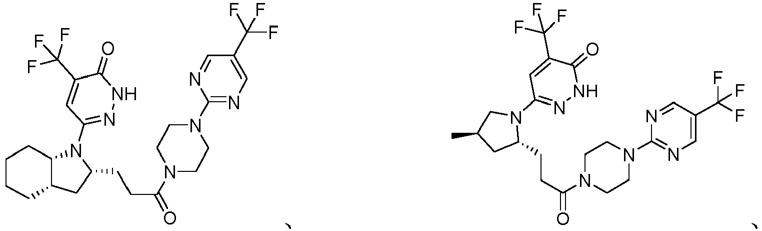

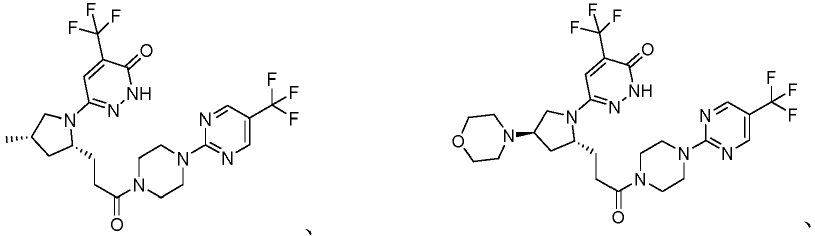

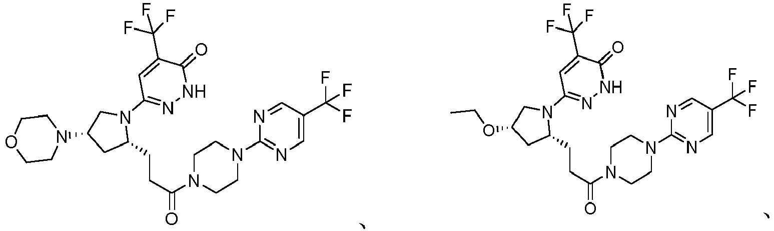

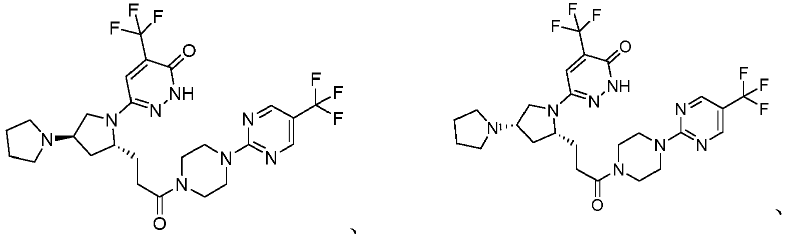

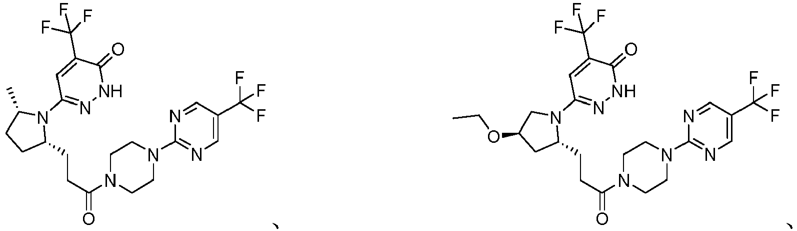

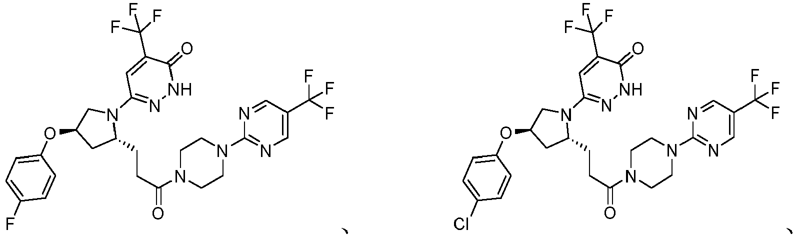

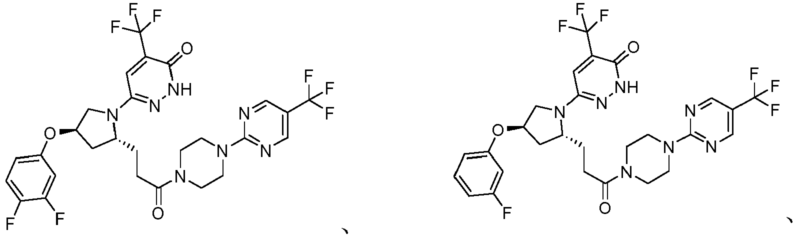

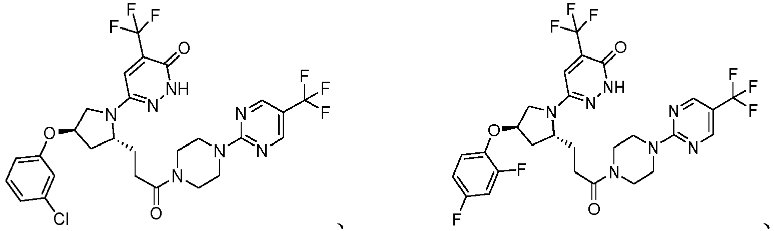

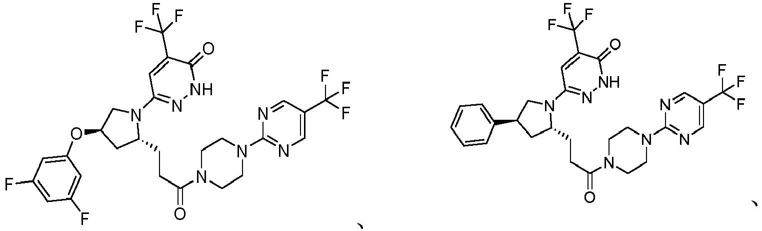

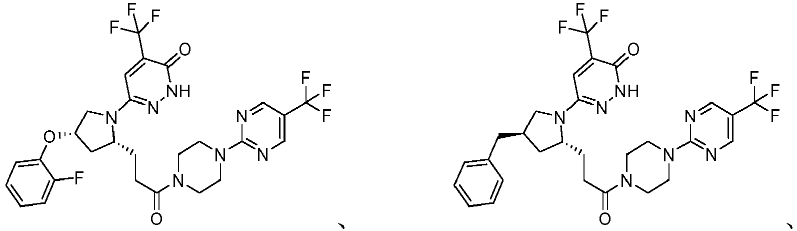

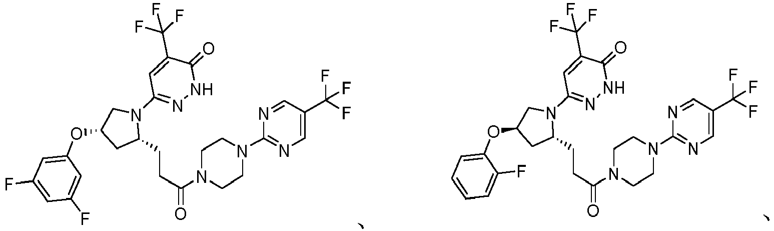

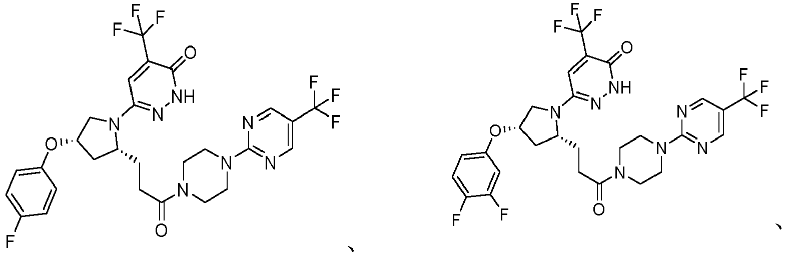

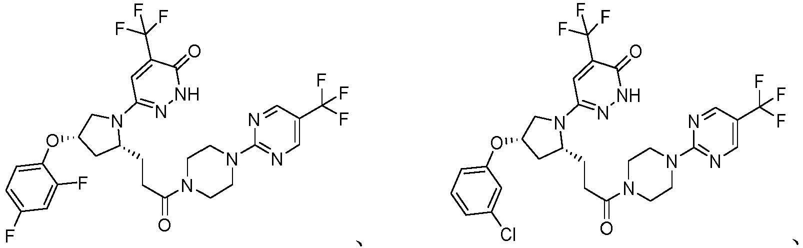

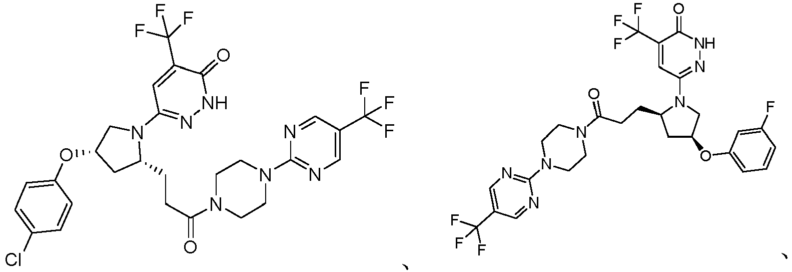

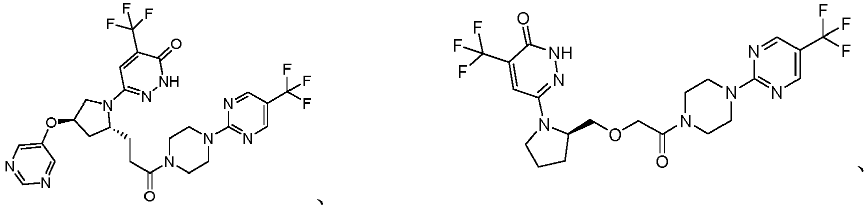

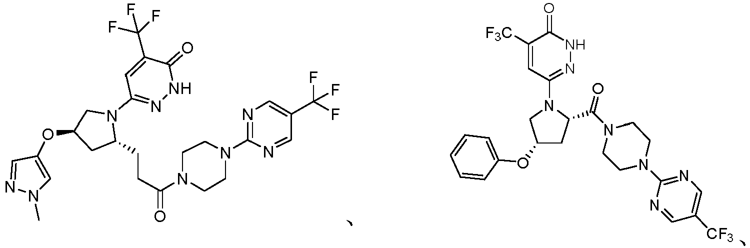

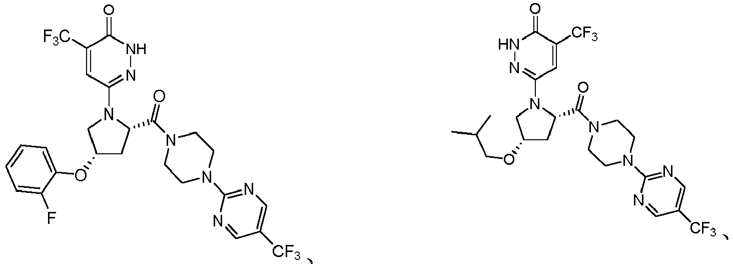

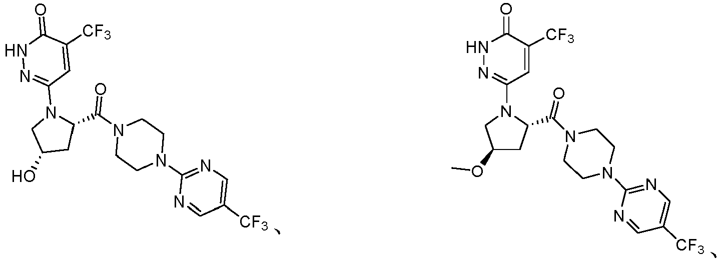

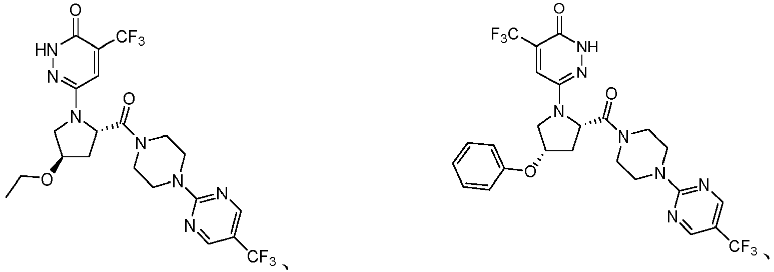

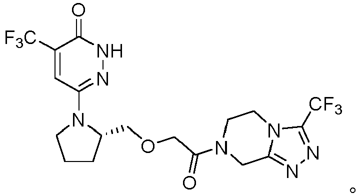

In another preferred embodiment, the compounds are selected from the compounds shown in the embodiments.

In the second aspect of the invention, provided are compounds of Formula II-1 or II-2, or pharmaceutically acceptable salts thereof, isomers, solvates, polymorphs, or deuterated forms, thereof,

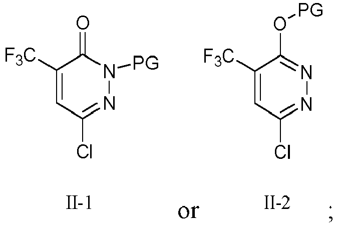

Wherein, PG is a protecting group other than a methyl group; preferably, the protecting group is 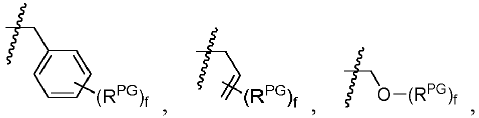 tert-butoxycarbonyl, benzyloxycarbonyl, 9-fluorenylmethyloxycarbonyl, 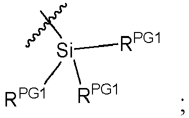 Each R^{PG} is independently C1-C4 alkyl, C1-C4 alkoxy, halogen, nitro, phenyl, benzyl, p-methoxybenzyl, trifluoromethyl, or 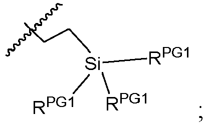 each R^{PG1} is independently C1-C4 alkyl or phenyl.

More preferably, the protecting group is 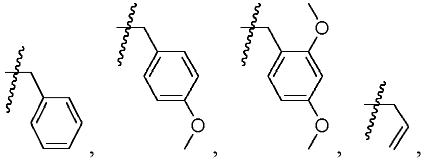 methoxymethyl, or 2-(trimethylsilyl)ethoxymethyl.

The third aspect of the present invention provides a pharmaceutical composition thereof, wherein the pharmaceutical composition comprises:
(1) An effective amount of a compound as described in the first aspect, or a pharmaceutically acceptable salt, enantiomer, diastereomer, racemate, epimer, solvate, polymorph, or deuterated form thereof, as an active ingredient; and
(2) optionally, pharmaceutically acceptable excipients.

In another preferred embodiment, the pharmaceutical composition may further include other pharmaceutically acceptable therapeutic agents or be used in combination with other pharmaceutically acceptable therapeutic agents, especially other anti-tumor drugs. The therapeutic agents include, but are not limited to: anti-tumor drugs that act on the chemical structure of DNA, such as cisplatin; anti-tumor drugs that affect nucleic acid synthesis, such as methotrexate (MTX), 5-fluorouracil (5FU), etc.; anti-tumor drugs that affect nucleic acid transcription, such as adriamycin, epirubicin, aclacinomycin, mithramycin, etc.; anti-tumor drugs that act on tubulin synthesis, such as paclitaxel, vinorelbine; aromatase inhibitors such as aminoglutethimide, lentaron, letrozole, anastrozole, etc; cell signaling pathway inhibitors such as epidermal growth factor receptor inhibitors gefitinib (Gefitinib), erlotinib (Erlotinib), lapatinib (Lapatinib), etc; mitogen-activated extracellular signal-regulated kinase (MEK) inhibitors such as trametinib (Trametinib), cobimetinib (Cobimetinib), etc.; cyclin-dependent kinase 4/6(CDK4/6) inhibitors such as Palbociclib, etc.; Src homologous tyrosine phosphatase (SHP2) inhibitor; SOS1 inhibitor; programmed death receptor -1/programmed death ligand -1(PD-1/PD-L1) inhibitors such as nivolumab (Nivolumab), pembrolizumab (Pembrolizumab), etc.

The fourth aspect of the present invention provides a use of a compound as described in the first aspect, or a pharmaceutically acceptable salt, enantiomer, diastereomer, racemate, epimer, solvate, polymorph, or deuterated form thereof, or a pharmaceutical composition as described in the second aspect for the preparation of a drug for the prevention or treatment of diseases related to PARP7.

In another preferred embodiment, the diseases are selected from the following diseases: cancer, infections, immune diseases, cardiovascular diseases, central nervous system diseases, and metabolic diseases. Specifically, cancer are selected from the following diseases: lung cancer, pancreatic cancer, colorectal cancer, leukemia, Ewing's sarcoma, breast cancer, prostate cancer, T-cell lymphoma, B-cell lymphoma, malignant rhabdomyosarcoma, synovial sarcoma, uterine fibroids, gastric cancer, liver cancer, kidney cancer, melanoma, ovarian cancer, brain glioma, bile duct cancer, nasopharyngeal cancer, cervical cancer, head and neck cancer, esophageal cancer, thyroid cancer, and bladder cancer.

It should be understood that within the scope of the present invention, the various technical features described above and the specific technical features described in the following text (such as in the examples) can be combined with each other to form new or preferred technical solutions. Due to space limitations, they are not reiterated here.

### DETAILED DESCRIPTION OF THE INVENTION

The inventors of the present invention, through extensive and in-depth research, have unexpectedly discovered a novel class of pyridazinone compounds for the first time that exhibit excellent inhibitory activity against PARP7 and possess the advantages of low toxicity and high safety. Based on this discovery, the present invention has been completed.

### Explanation of Terms

Unless otherwise defined, all technical and scientific terms used herein have the same meaning as those commonly understood by those of ordinary skill in the art of the invention.

As used herein, term "contain" or "include (comprise)" may be open, semi-closed, and closed. In other words, said term also includes "consisting essentially of" or "consisting of".

In the following sections, we will provide further details about the present invention through specific examples. It should be understood that these examples are provided for the purpose of illustrating the invention and not limiting the scope of the invention. The experimental methods without specific conditions in the following examples generally follow the conventional conditions or the conditions suggested by the manufacturer.. Unless stated otherwise, percentages and amounts are calculated by weight.

### DEFINITIONS

Definitions for standard chemical terms can be found in references including Carey and Sundberg "ADVANCED ORGANIC CHEMISTRY 4TH ED." Vols.A (2000) and B (2001), Plenum Press, New York. Unless otherwise indicated, conventional methods within the skill of the art are used, such as mass spectrometry, NMR, IR and UV/VIS spectroscopy and pharmacological methods. Unless specifically defined, the terms used herein in the relevant descriptions of analytical chemistry, organic synthetic chemistry, and pharmaceuticals and medicinal chemistry are known in the art. Standard techniques can be used in chemical synthesis, chemical analysis, pharmaceutical preparation, formulation and delivery, and treatment of patients. For example, the reaction and purification can be carried out using the manufacturer's instructions for use of the kit, or in a manner well known in the art or as described herein. The above-mentioned techniques and methods can generally be carried out in accordance with conventional methods well known in the art, or as described in the various general and more specific documents cited and discussed in this specification. In the present specification, the groups and substituents thereof can be selected by those skilled in the art to provide stable structural moieties and compounds.

When a substituent is described by a conventional formula written from left to right, the substituent likewise includes the chemically equivalent substituent obtained when the formula is written from right to left. For example, -CH₂O- is equivalent to -OCH₂-.

The section headings used herein are for the purpose of organizing the article only and should not be interpreted as limitations on the subject matter described. All documents or part of documents cited in this application, including but not limited to patents, patent applications, articles, books, manuals, and papers, are hereby incorporated by reference in their entirety.

Certain chemical groups defined herein are preceded by a simplified symbol to indicate the total number of carbon atoms present in that group. For example, C1-C6 alkyl refers to an alkyl group as defined below having 1 to 6 carbon atoms. The total number of carbon atoms in the simplified symbol does not include carbons that may be present in substituent of the group.

In addition to the foregoing, the following terms, when used in the present specification and claims, have the meanings set forth below unless otherwise specified.

As used herein, the term "halogen" refers to fluorine, chlorine, bromine or iodine.

"Hydroxy" refers to -OH group.

"Hydroxyalkyl" refers to an alkyl group, as defined below, substituted by a hydroxy (-OH) group.

"Carbonyl" refers to -C(= O)- group.

"Nitro" refers to -NO₂.

"Cyano" refers to -CN.

"Amino" refers to -NH₂.

"Substituted amino" refers to an amino group substituted by one or two substituents as defined below: alkyl, alkylcarbonyl, aryl alkyl, heteroarylalkyl group, e.g., monoalkylamino, dialkylamino, alkylamido, arylalkylamino, heteroarylalkylamino.

"Carboxyl" refers to -COOH.

As used herein, the term "alkyl" as a group or part of another group (e. g., in the case of halogen-substituted alkyl.etc.) refers to a fully saturated straight or branched hydrocarbon chain group consisting only of carbon and hydrogen atoms, having, for example, from 1 to 12 (preferably from 1 to 8, more preferably from 1 to 6) carbon atoms, and linked to the rest of the molecule by a single bond, for example including but not limited to methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl, and decyl, and the like. For the purposes of the present invention, the term "alkyl" preferably refers to an alkyl group containing 1 to 6 carbon atoms.

As used herein, the term "alkenyl", as a group or part of another group, refers to a straight or branched hydrocarbon chain group consisting only of carbon and hydrogen atoms, and at least one double bond, having, for example, from 2 to 14 (preferably from 2 to 10, more preferably from 2 to 6) carbon atoms, linked to the rest of the molecule by a single bond, for example, but not limited to, vinyl, propenyl, allyl group, but-1-enyl, but-2-enyl, pent-1-enyl, pent-1,4-dienyl, and the like.

As a group or part of another group, the term "alkynyl" refers to a straight or branched hydrocarbon chain group consisting only of carbon and hydrogen atoms, containing at least one C≡C, having, for example, from 2 to 14 (preferably from 2 to 10, more preferably from 2 to 6) carbon atoms, and linked to the rest of the molecule by a single bond, for example, but not limited to, ethynyl, 1-propynyl, 1-butynyl, heptynyl, octynyl, and the like.

As used herein,, as a group or part of another group, the term "carbocyclyl (carbocyclic group, carbocycle)" refers to a stable non-aromatic monocyclic or polycyclic hydrocarbon group consisting only of carbon and hydrogen atoms, which may include fused ring system, bridged ring system or spiro ring system, having from 3 to 15 carbon atoms, preferably from 3 to 10 carbon atoms, more preferably from 3 to 8 carbon atoms, and it is saturated or unsaturated and can be attached to the rest of the molecule by a single bond via any suitable carbon atom. Unless otherwise specifically indicated in the specification, the carbon atom in the carbocyclyl may be optionally oxidized. Examples of the carbocyclic group include, but are not limited to, cyclopropyl, cyclobutyl, cyclopentyl, cyclohexyl, cycloheptyl, cyclooctyl, adamantyl, 2,3-dihydroindenyl, octahydro-4, 7-methylene -1H-indenyl, 1,2, 3,4-tetrahydro-naphthyl, 5,6,7, 8-tetrahydro-naphthyl, cyclopentenyl, cyclohexenyl, cyclohexadienyl, 1H-indenyl, 8,9-dihydro-7H-benzocyclohepten-6-yl, 6,7,8,9-tetrahydro-5H-benzocycloheptenyl, 5,6,7,8,9, 10-hexahydro-benzocyclooctenyl, fluorenyl, bicyclo [2.2.1] heptyl, 7,7-dimethyl-bicyclo [2.2.1] heptyl, bicyclo [2.2.1] heptenyl, bicyclo [2.2.2] octyl, bicyclo [3.1.1] heptyl, bicyclo [3.2.1] octyl, bicyclo [2.2.2] octenyl, bicyclo [3.2.1] octenyl and octahydro-2, 5-methylene-cyclopentadienyl, etc.

As used herein, as a group or part of another group, the term "heterocyclyl (heterocycle)" refers to a stable 3-to 20-membered non-aromatic cyclic group consisting of 2 to 14 carbon atoms and 1 to 6 heteroatoms selected from nitrogen, phosphorus, oxygen and sulfur. Unless otherwise specifically indicated in the specification, a heterocyclyl may be a monocyclic, bicyclic, tricyclic, or more ring systems, which may include fused ring system, bridged ring system, or spiro ring system; and the nitrogen, carbon, or sulfur atom in heterocyclyl may be optionally oxidized; the nitrogen atom may be optionally quaternized; and the heterocyclyl may be partially or fully saturated. A heterocyclyl may be attached to the rest of the molecule by a single bond via a carbon atom or a heteroatom. In heterocyclyl containing fused rings, one or more of the rings may be an aryl or heteroaryl as defined below, provided that the point of attachment to the rest of the molecule is a non-aromatic ring atom. For the purposes of the present invention, the heterocyclyl is preferably a stable 4-to 11-membered non-aromatic monocyclic, bicyclic, bridged or spiro ring group containing 1 to 3 heteroatoms selected from nitrogen, oxygen and sulfur, more preferred a stable 4-to 8-membered non-aromatic monocyclic, bicyclic, bridged or spirocyclic groups comprising 1 to 3 heteroatoms selected from nitrogen, oxygen and sulfur. Examples of heterocyclyl include, but are not limited to, pyrrolidinyl, morpholinyl, piperazinyl, homopiperazinyl, piperidinyl, thiomorpholinyl, 2,7-diaza-spiro [3.5] nonan-7-yl, 2-oxa-6-aza-spiro [3.3] heptan-6-yl, 2, 5-diaza-bicyclo [2.2.1] heptan-2-yl, azetidinyl, pyranyl, tetrahydropyranyl, thiopyranyl, tetrahydrofuryl, oxazinyl, dioxocyclopentyl, tetrahydroisoquinolinyl, decahydroisoquinolinyl, imidazolinyl, imidazolidinyl, quinazinyl, thiazolidinyl, isothiazolidinyl, isoxazolidinyl, dihydroindolyl, octahydroindolyl, pyrrolidinyl, pyrazolidinyl, phthalimide, etc.

As used herein, the term "aromatic ring (aryl)", as a group or part of another group, refers to a conjugated hydrocarbocyclyl system group having from 6 to 18 carbon atoms, preferably having from 6 to 10 carbon atoms. For the purposes of the present invention, an aromatic ring (aryl) may be a monocyclic, bicyclic, tricyclic or polycyclic ring system and may also be fused with the carbocyclyl or heterocyclyl as defined above, provided that the aromatic ring (aryl) attached to the rest of the molecule by a single bond via an atom on the aromatic ring. Examples of the aromatic ring (aryl) include, but are not limited to, phenyl, naphthyl, anthryl, phenanthryl, fluorenyl, 2,3-dihydro-1H-isoindolyl, 2-benzoxazoliabsent, 2H-1, 4-benzoxazin-3 (4H)-one-7-yl, and the like.

As used herein, the term "arylalkyl" refers to an alkyl group as defined above substituted by an aryl as defined above.

As used herein, the term "heteroaromatic ring (heteroaryl)" as a group or part of another group refers to a 5-to 16-membered conjugated ring system group having from 1 to 15 carbon atoms (preferably from 1 to 10 carbon atoms) and 1 to 6 heteroatoms selected from nitrogen, oxygen and sulfur in the ring. Unless specifically indicated in the specification, the heteroaromatic ring (heteroaryl) may be a monocyclic, bicyclic, tricyclic or polycyclic ring system and may also be fused with a carbocyclyl or heterocyclyl as defined above, provided that the heteroaromatic ring (heteroaryl) is attached to the rest of the molecule by a single bond via an atom on the heteroaromatic ring. The nitrogen, carbon or sulfur atom in the heteroaromatic ring (heteroaryl) may optionally be oxidized; the nitrogen atom may optionally be quaternized. For the purposes of the present invention, the heteroaromatic ring (heteroaryl) is preferably a stable 5-to 12-membered aromatic group containing 1 to 5 heteroatoms selected from nitrogen, oxygen and sulfur, more preferred a stable 5-to 10-membered aromatic group containing 1 to 4 heteroatoms selected from nitrogen, oxygen and sulfur or 5-to 6-membered aromatic group containing 1 to 3 heteroatoms selected from nitrogen, oxygen and sulfur. Examples of heteroaromatic ring (heteroaryl) include, but are not limited to, thienyl, imidazolyl, pyrazolyl, thiazolyl, oxazolyl, oxadiazolyl, isoxazolyl, pyridinyl, pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoindolyl, indazolyl, isoindazolyl, purinyl, quinolinyl, isoquinolinyl, diazanaphthalenyl, naphthyridyl, quinoxalinyl, pteridinyl, carbazolyl, carbolinyl, phenanthridinyl, phenanthrolinyl, acridinyl, phenazinyl, isothiazolyl, benzothiazolyl, benzothiophenyl, oxatriazolyl, cinnolinyl, quinazolinyl, thiophenyl, indolizinyl, phenanthrolinyl, isoxazolyl, phenoxazinyl, phenothiazinyl, 4,5,6, 7-tetrahydrobenzo [b] thienyl, naphthopyridyl, [1,2,4] triazolo [4,3-b] pyridazine, [1,2,4] triazolo [4,3-a] pyrazine, [1,2,4] triazolo [4,3-c] pyrimidine, [1,2,4] triazolo [4,3-a] pyridine, imidazo [1,2-a] pyridine, imidazo [1,2-b] pyridazine, imidazo [1,2-a] pyrazine and the like.

As used herein, the term "heteroarylalkyl" refers to an alkyl group as defined above substituted by a heteroaryl as defined above.

In the present application, the term "absent" means that the two sides of the group defined above are directly linked by chemical bonds. For example, "B is absent in A- B-C" means "A-C".

In the present application, " 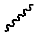 " in 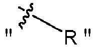 represents the connection position of group R.

In the present application, unless otherwise specified in the claims, "optionally" and "optionally substituted" mean that the subsequently described event or condition may or may not occur, and the description includes both the occurrence and non-occurrence of the event or condition. For example, "optionally substituted aryl" means that the hydrogen on the aryl is substituted or unsubstituted, and the description includes both substituted aryl and unsubstituted aryl. For example, without explicit listing of substituents, as used herein, the term "optionally substituted", "substituted" or "substituted by" refer to one or more hydrogen atoms on a given atom or group independently substituted by one or more, e.g., 1, 2, 3, or 4, substituents independently selected from: deuterium (D), halogen, -OH, sulfydryl, cyano, -CD3, -C1-C6alkyl (preferred-C1-3alkyl),C2-C6alkenyl, C2-C6alkynyl, cycloalkyl (preferably 3-8 membered cycloalkyl), aryl, heterocyclyl (preferably 3-8 membered heterocyclyl), heteroaryl, aryl-C1-C6alkyl, heteroaryl C1-C6alkyl, C1-C6haloalkyl-, -OC1-C6alkyl (preferred-OC1-C3alkyl), -OC2-C6alkenyl, -OC1-C6alkyl phenyl, -C1-C6alkyl-OH (preferred -C1-C4alkyl OH), -C1-C6alkyl SH, -C1-C6alkyl O-C1-C6alkyl, -OC1-C6haloalkyl, -NH2, C1-C6alkyl-NH2 (preferably -C1-C3alkyl-NH2), -N(C1-C6alkyl)2(preferably -N(C1-C3alkyl)2), -NH(C1-C6alkyl) (preferably -NH(C1-C3alkyl)), -N(C1-C6alkyl)(C1-C6alkyl phenyl), -NH(C1-C6alkyl phenyl), nitro, -C(O)-OH, C(O)OC1-C6alkyl (preferably -C(O)OC1-C3alkyl), -CONRiRii (wherein, Ri and Rii is H, D and C1-6alkyl, preferably C1-3alkyl), -NHC(O)(C1-C6alkyl), -NHC(O)(phenyl), -N(C1-C6alkyl) C(O)(C1-C6alkyl), -N(C1-C6alkyl) C(O)(phenyl), -C(O)C1-C6alkyl, -C(O) heteroaryl (preferably -C(O)-5-7 membered heteroaryl), -C(O)C1-C6alkyl phenyl, -C(O)C1-C6haloalkyl, -OC(O)C1-C6alkyl (preferably -OC(O)C1-C3alkyl), -S(O)₂-C1-C6alkyl, -S(O)-C1-C6alkyl, -S(O)₂-phenyl, -S(O)₂-C1-C6haloalkyl, -S(O)2NH2, -S(O)2NH(C1-C6alkyl), -S(O)₂NH (phenyl), -NHS(O)₂(C1-C6alkyl), -NHS(O)₂(phenyl) and -NHS(O)₂(C1-C6haloalkyl), wherein each of the alkyl, cycloalkyl, phenyl, aryl, heterocyclyl and heteroaryl is optionally further substituted by one or more substituents selected from halogen, -OH, -NH₂, cycloalkyl, 3-8 membered heterocyclyl, C1-C4alkyl, C1-C4haloalkyl-, -OC1-C4alkyl, -C1-C4alkyl-OH, -C1-C4alkyl O-C1-C4alkyl, -OC1-C4haloalkyl, cyano, nitro, -C(O)-OH, -C(O)OC1-C6alkyl, -CON(C1-C6alkyl)₂, -CONH(C1-C6alkyl), -CONH₂-NHC(O)(C1-C6alkyl), -NH(C1-C6alkyl) C(O)(C1-C6alkyl), -SOz(C1-C6alkyl), -SO₂(phenyl), -SOz(C1-C6haloalkyl), -SO₂NH₂, -SO₂NH(C1-C6alkyl), -SO₂NH (phenyl), -NHSO₂(C1-C6alkyl), -NHSO₂(phenyl) and -NHSO₂(C1-C6haloalkyl). When an atom or group is substituted with various substituents, the substituents may be the same or different. As used herein, the terms "moiety," "structure moiety," "chemical moiety," "group," "chemical group" refer to a specific fragment or functional group in a molecule. A chemical moiety is generally considered to be a chemical entity embedded in or attached to a molecule.

In this invention, "multiple" refers to 2, 3, or 4.

### Active ingredient.

In the context of this document, "the inventive compounds" or "active ingredient" refers to the compounds of formula I and includes their pharmaceutically acceptable salts, enantiomers, diastereomers, tautomers, cis-trans isomers, solvates, polymorphs, deuterated forms, or combinations thereof.

"Stereoisomer" refers to compound composed of the same atoms, bonded by the same bonds, but with different three-dimensional structures. The present invention is intended to encompass various stereoisomers and mixtures thereof.

When the compound of the present invention contains olefinic double bonds, the compound of the present invention is intended to include both E-and Z-geometric isomers unless otherwise specified.

The term "tautomer" refers to an isomer formed by the transfer of a proton from one atom of a molecule to another atom of the same molecule. All tautomeric forms of the compound of the invention will also be included within the scope of the invention.

The compounds of the present invention, or pharmaceutically acceptable salts thereof, may contain one or more chiral carbon atoms, and thus may give rise to enantiomers, diastereomers, and other stereoisomeric forms. Each chiral carbon atom may be defined as (R)- or (S)- based on stereochemistry. The present invention is intended to include all possible isomers, as well as racemates and optically pure forms thereof. Racemates, diastereomers or enantiomers can be selected as starting materials or intermediates in the preparation of the compounds of the present invention. Isomers with optical activity can be prepared using chiral synthons or chiral reagents, or resolved using conventional techniques, such as crystallization and chiral chromatography.

Conventional techniques for the preparation/separation of individual isomers include chiral synthesis from appropriate optically pure precursors, or resolution of the racemate (or racemate of salts or derivatives) using, for example, chiral high performance liquid chromatography, see, for example, Gerald Gübitz and Martin G. Schmid (Eds.), Chiral Separations, Methods and Protocols, Methods in Molecular Biology, Vol. 243, 2004;A.M. Stalcup, Chiral Separations. Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

As used herein, the term "pharmaceutically acceptable salts" includes pharmaceutically acceptable acid addition salts and pharmaceutically acceptable base addition salts.

The term "pharmaceutically acceptable acid addition salts" refers to salts formed with inorganic or organic acids that retain the bioavailability of the free base without exerting other adverse effects. The inorganic acid salts include but are not limited to hydrochloride, hydrobromide, sulfate, nitrate, phosphate, etc.; the organic acid salts include but are not limited to formate, acetate, 2, 2-dichloro acetate, trifluoroacetate, propionate, caproate, caprylate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, lauroleate, malate, glutamate, pyroglutamate, aspartate, benzoate, mesylate, benzene sulfonate, p-toluenesulfonate, alginate, ascorbate, salicylate, 4-aminosalicylate, naphthalene disulfonate, etc. These salts can be prepared by methods known in the art.

The term "pharmaceutically acceptable base addition salts" refers to salts formed with inorganic or organic bases that retain bioavailability of the free acid without exerting other adverse effects. Salts derived from inorganic bases include, but are not limited to, sodium salts, potassium salts, lithium salts, ammonium salts, calcium salts, magnesium salts, ferric salts, zinc salts, copper salts, manganese salts, aluminum salts, and the like. Preferred inorganic salts are ammonium salts, sodium salts, potassium salts, calcium salts and magnesium salts. Salts derived from organic bases include, but are not limited to, primary, secondary and tertiary amines; substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucamine, methylglucamine, theobromine, purine, piperazine, piperidine, N-ethylpiperidine, polyamine resin, and the like. Preferred organic bases include isopropylamine, diethylamine, ethanolamine, trimethylamine, dicyclohexylamine, choline and caffeine. These salts can be prepared by methods known in the art.

### Pharmaceutical composition and administration methods

As used herein, the term "pharmaceutical composition" refers to a formulation of a compound of the present invention in combination with a medium generally acceptable in the art for the delivery of a biologically active compound to a mammal (e. g., a human). The medium includes pharmaceutically acceptable carriers. The purpose of the pharmaceutical composition is to promote the administration of the organism, facilitate the absorption of active ingredients and thereby exert biological activity.

As used herein, the term "pharmaceutically acceptable" refers to a substance (e. g., a carrier or diluent) that does not affect the biological activity or properties of the compound of the invention, and is relatively non-toxic, i.e., the substance can be administered to an individual without causing any adverse biological reaction or interacting in an adverse manner with any of the components contained in the composition.

In the present application, "pharmaceutically acceptable excipients" include, but are not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier licensed by relevant governmental regulatory authority as acceptable for human or livestock use.

As used herein, the term "tumors" include but are not limited to lung cancer, pancreatic cancer, colorectal cancer, leukemia, Ewing's sarcoma, breast cancer, prostate cancer, T cell lymphoma, B cell lymphoma, malignant rhabdomyoma, synovial sarcoma, endometrioma, gastric cancer, liver cancer, renal cancer, melanoma, ovarian cancer, brain glioma, cholangiocarcinoma, nasopharyngeal carcinoma, cervical carcinoma, head and neck cancer, esophageal cancer, thyroid cancer and bladder cancer, etc.

As used herein, the terms "prophylactic", "preventing" and "prevention" include reducing the possibility of the occurrence or worsening of a disease or disorder in patients.

As used herein, the term "treatment" and other similar synonyms include the following meanings:
(i) Preventing the occurrence of the disease or disorder in mammals, especially when such mammals are susceptible to such diseases or disorders but have not been diagnosed as having it;
(ii) Inhibiting the disease or disorder, i.e. curbing its development;
(iii) Alleviating disease or disorder, i.e., abating the condition of the disease or disorder; or
(iv) Alleviating the symptoms caused by the disease or disorder.

As used herein, the terms "effective amount," "therapeutically effective amount," or "pharmaceutically effective amount" refer to an amount of at least one agent or compound that, upon administration, is sufficient to alleviate to some extent of one or more of the symptoms of the disease or condition being treated. The result can be the reduction and/or alleviation of signs, symptoms, or causes, or any other desired change in a biological system. For example, an "effective amount" for treatment is the amount of a composition comprising the compound disclosed herein required to provide a clinically significant condition-relieving effect. An effective amount appropriate in any individual case can be determined using techniques such as dose escalation assays.

As used herein, the terms "administration," "administed" "administering" and the like refer to methods that enable the delivery of a compound or composition to the desired site for biological action. These methods include, but are not limited to, oral routes, transduodenal routes, parenteral injection (including intravenous, subcutaneous, intraperitoneal, intramuscular, intra-arterial injection or infusion), topical administration, and rectal administration. Those skilled in the art are familiar with the application techniques of the compounds and methods described herein, such as those discussed in Goodman and Gilman, The Pharmacological of Therapeutics, current ed.; Pergamon; and Remington's, Pharmaceutical Sciences (current edition), Mack Publishing Co., Easton, Pa. In preferred embodiments, the compounds and compositions discussed herein are administered orally.

As used herein, the terms "pharmaceutical combination", "administratd in combination", "combined administration ", "administration of other treatments", "administration of other therapeutic agents" and the like refer to pharmaceutical treatments obtained by admixing or combining more than one active ingredients, which includes both fixed and non-fixed combinations of active ingredients. The term "fixed combination" refers to the simultaneous administration of at least one compound described herein and at least one synergists to a patient in the form of a single entity or single dosage form. The term "non-fixed combination" refers to that at least one compound described herein and at least one synergistic formulation are administered simultaneously, concomitantly, or sequentially at variable intervals to a patient as separate entities. These are also applied to cocktail therapies, for example the administration of three or more active ingredients.

It should also be understood by those of skill in the art that in the methods described below, the functional group of the intermediate compound may need to be protected by an appropriate protecting group. Such functional groups include hydroxyl, amino, sulfydryl, and carboxylic acid. Suitable hydroxyl protecting groups include trialkylmethanosilyl or diaromatic cyclic alkylmethanosilyl (e.g. tert-butyldimethylmethanosilyl, tert-butyl diphenylmethanosilyl or trimethylmethanosilyl), tetrahydropyranyl, benzyl, etc. Suitable protecting groups for amino, amidinyl and guanidinyl include tert-butoxycarbonyl, benzyloxycarbonyl and the like. Suitable thiol protecting groups include -C(O)-R" (wherein R" is alkyl, aryl or arylalkyl), p-methoxybenzyl, trityl, and the like. Suitable carboxy protecting groups include alkyl, aryl or aryl alkyl esters.

Protecting groups can be introduced and removed according to standard techniques known to those skilled in the art and as described herein. The use of protecting groups is described in detail in Greene, T. W. and P. G. M. Wuts, Protective Groups in Organi Synthesis, (1999), 4th Ed., in Wiley. The protecting group may also be polymer resin.

### Main advantages of this invention

(1) The compounds in this invention, with novel chemical structure, exhibits excellent inhibition against PARP7;
(2) The compounds of this invention demonstrate high selectivity;
(3) The compounds in this invention display favorable pharmacological and pharmacokinetic properties;

The present invention is further specified with specific embodiments. It should be clear to those skilled in the art that the examples are merely intended to assist in understanding the present invention and should not be considered as specific limitations to the present invention.

The experimental methods without specific conditions in the following examples generally follow the conventional conditions or the conditions suggested by the manufacturer. Unless otherwise stated, percentages and parts are caculated by weight.

Unless otherwise specified, the experimental materials and reagents used in the following examples are commercially available.

In each example, ¹H NMR was recorded on a BRUKER AVANCE NEO 400 MHz Nuclear Magnetic Resonance instrument with chemical shifts reported as *δ* (ppm); Liquid chromatography-mass spectrometry (LCMS) was recorded on Shimadzu LC-20AD, SIL-20A, CTO-20AC, SPD-M20A, CBM-20A, LCMS-2020 mass spectrometer; preparative HPLC separation used Gilson -281 liquid chromatograph.

### Examples

### Preparation of intermediates

### 1. Preparation of intermediate A

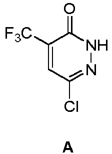

The synthetic route of intermediate A is shown below: 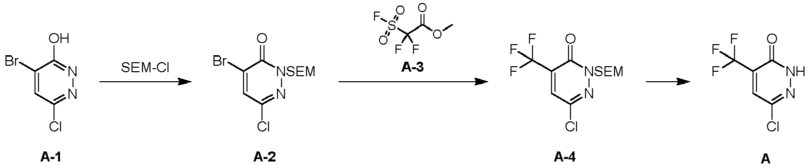
(1) At 0°C, compound **A-1** (2.58 g, 12.3 mmol) was dissolved in 40.0 mL of tetrahydrofuran, and then sodium hydride (985 mg, 24.6 mmol, 60%) was added to the solution. The mixture was stirred for 30 minutes, then 2-(trimethylsilyl)ethoxymethyl chloride (3.08 g, 18.5 mmol) was introduced into the reaction mixture. The reaction was stirred at 25°C under a nitrogen atmosphere for 3 hours. Subsequently, 50.0 mL of water was added to the reaction mixture, and it was extracted with ethyl acetate (40.0 mL × 3). The combined organic phase was washed with saturated sodium chloride solution (40.0 mL × 3), dried over anhydrous sodium sulfate, filtered, and concentrated under reduced pressure. The crude product was purified by silica gel column chromatography (petroleum ether/ethyl acetate = 1:0 to 0:1) to isolate compound **A-2.** ¹H NMR (400 MHz, CDCl₃) *δ* 7.62 (s, 1H), 5.45 (s, 2H), 3.69-3.76 (m, 2H), 0.93-1.00 (m, 2H), 0.01 (s, 9H).
(2) To a solution of compound **A-2** (1.00 g, 2.94 mmol) in N, N-dimethylformamide (20.0 mL), compound **A-3** (1.13 g, 5.89 mmol) and cuprous iodide (1.12 mg, 5.89 mmol) were added. The reaction mixture was stirred at 110°C under a nitrogen atmosphere for 5 hours. After then, 20.0 mL of water was added to quench the reaction, and the mixture was extracted with ethyl acetate (30.0 mL × 2). The combined organic phase was washed with saturated sodium chloride solution (20.0 mL × 2), dried over anhydrous sodium sulfate, filtered, and concentrated under reduced pressure. The crude product was purified by silica gel column chromatography (petroleum ether/ethyl acetate = 1:0 to 0:1) to obtain compound **A-4.** ¹H NMR (400 MHz, CDCl₃) *δ* 7.53 (s, 1H), 5.45 (s, 2H), 3.71-3.78 (m, 2H), 0.93-1.01 (m, 2H), 0.01 (s, 9H).
(3) To a solution of compound **A-4** (500 mg, 1.52 mmol) in dichloromethane (5.0 mL), trifluoroacetic acid (2.31 g, 20.2 mmol) was added, and the reaction mixture was stirred at 25°C under a nitrogen atmosphere for 1 hour. The reaction solution was then concentrated under reduced pressure, and ethanol (5.0 mL) and potassium carbonate (630 mg, 4.56 mmol) were introduced, followed by an additional 30 minutes of stirring. The reaction mixture was further concentrated under reduced pressure, and 30.0 mL of water was added. It was then extracted with ethyl acetate (30.0 mL × 2). The combined organic phase was washed with saturated sodium chloride solution (30.0 mL × 1), dried over anhydrous sodium sulfate, filtered, and concentrated under reduced pressure. Compound **A** was obtained after purification by silica gel column chromatography (petroleum ether/ethyl acetate = 1:0 to 0:1). ¹H NMR (400 MHz, CDCl₃) δ 12.06 (s, 1H), 7.60 (s, 1H).

### 2. Preparation of intermediate B

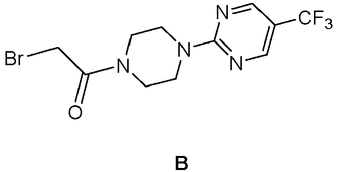

The synthetic route of intermediate B is shown below: 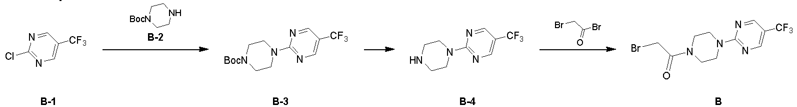
(1) To a solution of compound **B-1** (2.00 g, 10.9 mmol) in N-methylpyrrolidone (15.0 mL), potassium carbonate (3.03 g, 21.9 mmol) and compound **B-2** (2.04 g, 10.9 mmol) were added. The reaction mixture was stirred at 80°C under a nitrogen atmosphere for 1 hour. Water (60.0 mL) was then added to the reaction mixture, and it was extracted with ethyl acetate (50.0 mL × 3). The combined organic phase was washed with saturated sodium chloride solution (50.0 mL × 2), dried over anhydrous sodium sulfate, filtered, and concentrated under reduced pressure. Compound **B-3** was obtained after purification by silica gel column chromatography (petroleum ether/ethyl acetate = 1:0 to 0:1). MS-ESI [M+H]⁺, calculated: 277, found: 277. ¹H NMR (400 MHz, CDCl₃) *δ* 8.49 (s, 2H), 3.85-3.93 (m, 4H), 3.47-3.55 (m, 4H), 1.49 (s, 9H).
(2) To a solution of compound **B-3** (3.60 g, 10.8 mmol) in dichloromethane (30.0 mL), trifluoroacetic acid (12.3 g, 108 mmol) was added, and the reaction mixture was stirred at 25°C for 1 hour. The reaction mixture was then filtered, and the filtrate was concentrated under reduced pressure to obtain the crude compound of **B-4** trifluoroacetate. MS-ESI [M+H]⁺, calculated: 233, found: 233.
(3) At 0°C, to a solution of compound **B-4** trifluoroacetate (3.00 g, 8.66 mmol) in tetrahydrofuran (30.0 mL), diisopropylethylamine (5.60 g, 43.3 mmol) and bromoacetyl bromide (2.10 g, 10.4 mmol) were added. The reaction mixture was stirred at 25°C under a nitrogen atmosphere for 0.5 hours. Water (50.0 mL) was added to the reaction mixture, and it was then extracted with ethyl acetate (100 mL × 1). The combined organic phase was washed with saturated sodium chloride solution (50.0 mL × 2), dried over anhydrous sodium sulfate, filtered, and concentrated under reduced pressure. The crude product was purified by silica gel column chromatography (petroleum ether/ethyl acetate = 100:1 to 10:1) to isolate intermediate compound **B.** MS-ESI [M+H]⁺, calculated: 353, found: 353. ¹H NMR (400 MHz, CDCl₃) *δ* 8.53 (s, 2H), 4.01-4.06 (m, 2H), 3.94-3.98 (m, 2H), 3.93 (s, 2H), 3.71-3.76 (m, 2H), 3.60-3.64 (m, 2H).

### 3. Preparation of intermediate C

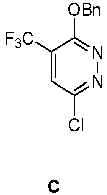

The synthetic route of intermediate C is shown below:

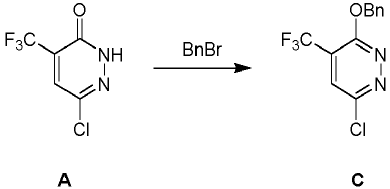

To a solution of compound **A** (1.40 g, 7.05 mmol) in toluene (30.0 mL), silver carbonate (3.50 g, 12.7 mmol) and benzyl bromide (2.42 g, 14.1 mmol) were added. The reaction mixture was stirred at 50°C under a nitrogen atmosphere for 7 hours. Water (5.0 mL) was then added to the reaction mixture, and it was extracted with ethyl acetate (20.0 mL × 1). The organic phase was dried over anhydrous sodium sulfate, filtered, and concentrated under reduced pressure. The crude product was purified by silica gel column chromatography (petroleum ether/ethyl acetate = 100:1 to 50:1) to obtain compound **C.** MS-ESI [M+H]⁺, calculated: 289, found: 289. ¹H NMR (400 MHz, CDCl₃) *δ* 7.64 (s, 1H), 7.49 (d, *J =* 7.2 Hz, 2H), 7.33-7.44 (m, 3H), 5.70 (s, 2H).

### 4. Preparation of intermediate D

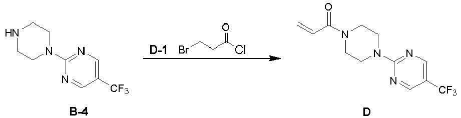

At 0°C, to a solution of compound **B-4** trifluoroacetate (500 mg, 1.44 mmol) in tetrahydrofuran (10.0 mL), diisopropylethylamine (933 mg, 7.22 mmol), and 3-bromopropionyl chloride (297 mg, 1.73 mmol) were added. The reaction mixture was stirred at 25°C under a nitrogen atmosphere for 0.5 hours. Water (10.0 mL) was added to the reaction mixture, and it was extracted with dichloromethane (50.0 mL × 3). The combined organic phase was dried over anhydrous sodium sulfate, filtered, and concentrated under reduced pressure. The crude product was purified by silica gel column chromatography (petroleum ether/ethyl acetate = 1:0 to 1:1) to obtain intermediate compound **D.** MS-ESI [M+H]⁺, calculated: 287, found: 287. ¹H NMR (400 MHz, MeOD) *δ* 8.61 (s, 2H), 6.76-6.87 (m, 1H), 6.25 (dd, *J=* 4.0, 20.0 Hz, 1H), 5.79 (dd, *J=* 4.0, 12.0 Hz, 1H), 3.92-4.01 (m, 4H), 3.71-3.80 (m, 4H).

### 5. Preparation of intermediate E

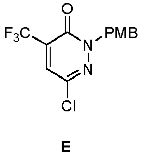

The synthetic route of intermediate E is shown below: 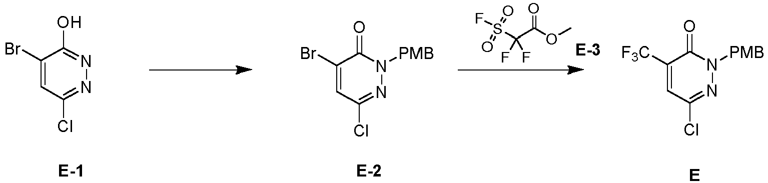
(1) At 0°C, to a solution of compound **E-1** (20.0 g, 95.5 mmol) in N,N-dimethylformamide (40.0 mL), potassium carbonate (26.4 g, 191 mmol) and p-methoxybenzyl chloride (25.4 g, 162 mmol, 22.11 mL) were added. The reaction mixture was stirred at 25°C for 2 hours. Water (200 mL) was added to the reaction mixture, and it was extracted with ethyl acetate (400 mL × 3). The combined organic phase was washed with saturated sodium chloride solution (40.0 mL × 3), dried over anhydrous sodium sulfate, filtered, and concentrated under reduced pressure. Compound **E-2** was obtained after purification by silica gel column chromatography (petroleum ether/ethyl acetate = 1:0 to 1:1). MS-ESI [M+H]⁺, calculated: 331, found: 331. ¹H NMR (400 MHz, CDCl₃) *δ* 7.55-7.59 (m, 1H), 7.41-7.46 (m, 2H), 6.85-6.91 (m, 2H), 5.23 (s, 2H), 3.80 (s, 3H)
(2) To a solution of compound **E-2** (19.7 g, 56.0 mmol) in *N,N*-dimethylformamide (200 mL), compound **E-3** (23.0 g, 120 mmol) and cuprous iodide (22.8 g, 120 mmol) were added. The reaction was conducted under a nitrogen atmosphere at 110°C for 5 hours. After then, 300 mL of water was added to quench the reaction , and the resulting mixture was extracted with ethyl acetate (300 mL × 2). The combined organic phase was washed with saturated sodium chloride solution (200 mL × 2), dried over anhydrous sodium sulfate, filtered, and concentrated under reduced pressure. Compound **E** was obtained after purification by silica gel column chromatography (petroleum ether/ethyl acetate = 1:0 to 1:1). ¹H NMR (400 MHz, CDCl₃) *δ* 7.49 (s, 1H), 7.40-7.47 (m, 2H), 6.86-6.91 (m, 2H), 5.24 (s, 2H), 3.78-3.83 (m, 3H).

### Example 1 Synthesis of Compound 1

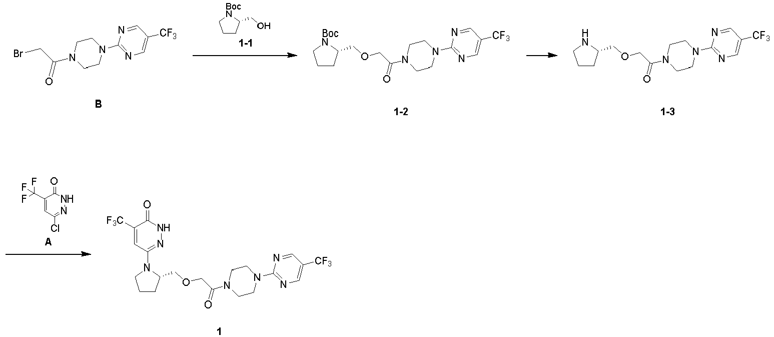

(1) To a solution of intermediate **B** (700 mg, 1.98 mmol) in dichloromethane (6.0 mL), compound **1-1** (598 mg, 2.97 mmol), sodium hydroxide (50.0 mg, 1.25 mmol), and tetrabutylammonium bisulfate (336 mg, 2.97 mmol) were added. The reaction mixture was stirred at 50°C under a nitrogen atmosphere for 10 hours. Water (50.0 mL) was added to the reaction mixture, and it was extracted with ethyl acetate (50.0 mL × 2). The combined organic phase was washed with saturated sodium chloride solution (50.0 mL × 2), dried over anhydrous sodium sulfate, filtered, and concentrated under reduced pressure. Compound **1-2** was obtained after purification by silica gel column chromatography (petroleum ether/ethyl acetate = 1:0 to 0:1). MS-ESI [M+H]⁺, calculated: 474, found: 474.
(2) To a solution of compound **1-2** (476 mg, 1.01 mmol) in dichloromethane (6.0 mL), trifluoroacetic acid (3.08 g, 27.0 mmol) was added. The reaction mixture was stirred at 25°C for 0.5 hours and then filtered. The filtrate was concentrated under reduced pressure to obtain the crude compound **1-3** trifluoroacetate. ¹H NMR (400 MHz, MeOD) *δ* 8.60 (s, 2H), 4.41 (s, 2H), 3.91-4.04 (m, 4H), 3.66-3.81 (m, 4H), 3.56-3.63 (m, 1H), 3.47-3.56 (m, 2H), 3.18-3.25 (m, 2H), 1.92-2.07 (m, 3H), 1.71-1.79 (m, 1H).
(3) To a solution of compound **1-3** trifluoroacetate (378 mg, 776 µmol) in dioxane (5.0 mL), compound **A** (140 mg, 705 µmol), cesium carbonate (689 mg, 2.12 mmol), and Methanesulfonato(2-dicyclohexylphosphino-2',6'-di-i-propoxy-1,1'-biphenyl)(2'-amino-1,1'-bip henyl-2-yl)palladium(II) (118 mg, 141 µmol) were added. The reaction was carried out under a nitrogen atmosphere at 110°C for 3 hours. After the reaction, 30.0 mL of water was added to the reaction mixture, and it was extracted with ethyl acetate (30.0 mL × 3). The organic phase was washed with saturated sodium chloride solution (30.0 mL × 2), dried over anhydrous sodium sulfate, filtered, and concentrated under reduced pressure. The crude product was purified by preparative high-performance liquid chromatography (Xtimate C18, 100 mm × 30 mm, 10 µm, solvent A: water (0.225% formic acid), solvent B: acetonitrile, gradient: 45%-65% B over 10 minutes) to isolate compound **1** formate. MS-ESI [M+H]⁺, calculated: 536, found: 536. ¹H NMR (400 MHz, CDCl₃) *δ* 8.60 (s, 2H), 7.75 (s, 1H), 4.25-4.30 (m, 2H), 4.17-4.24 (m, 1H), 3.84-4.01 (m, 4H), 3.49-3.68 (m, 7H), 3.35-3.40 (m, 1H), 1.95-2.16 (m, 4H).

### Example 2 Synthesis of Compound 2

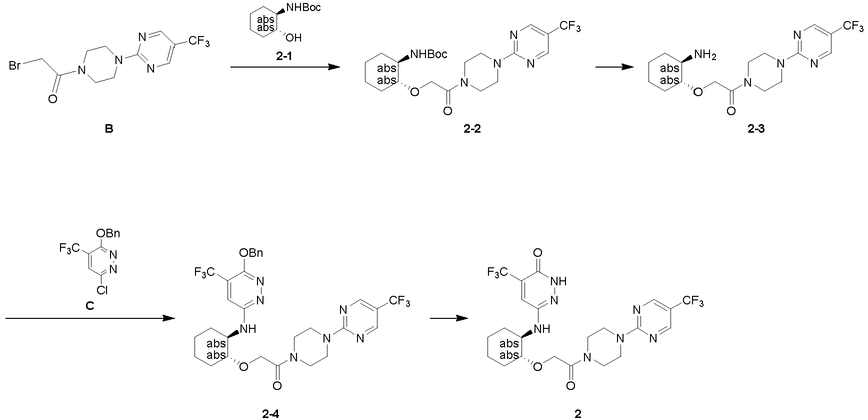

(1) To a solution of intermediate **B** (800 mg, 2.27 mmol) in dichloromethane (15.0 mL), water (4.0 mL), compound **2-1** (728 mg, 3.38 mmol), sodium hydroxide (906 mg, 22.7 mmol), and tetrabutylammonium bisulfate (385 mg, 1.13 mmol) were added. The reaction mixture was stirred at 25°C under a nitrogen atmosphere for 12 hours. Water (10.0 mL) was added to the reaction mixture, and it was then extracted with dichloromethane (60.0 mL × 1). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **2-2.** MS-ESI [M+H]⁺, calculated: 488, found: 488.
(2) To a solution of compound **2-2** (300 mg, 615 µmol) in dichloromethane (3.0 mL), trifluoroacetic acid (1.54 g, 13.5 mmol) was added. The reaction mixture was stirred at 25°C for 0.5 hours. The reaction solution was then filtered, and the filtrate was concentrated under reduced pressure to obtain the crude compound **2-3** trifluoroacetate. MS-ESI [M+H]⁺, calculated: 388, found: 388.
(3) To a solution of compound **2-3** trifluoroacetate (279 mg, 720 µmol) in toluene (5.0 mL), compound **C** (160 mg, 554 µmol), potassium tert-butoxide (120 mg, 1.07 mmol), 1,1'-binaphthyl-2,2'-diphenylphosphine (138 mg, 222 µmol), and bis(dibenzylideneacetone)palladium (63.7 mg, 111 µmol) were added. The reaction was carried out under a nitrogen atmosphere at 110°C for 2.5 hours. After the reaction, 5.0 mL of water was added to the reaction mixture, and it was extracted with ethyl acetate (50.0 mL × 1). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **2-4.** MS-ESI [M+H]⁺, calculated: 640, found: 640.
(4) To a solution of compound **2-4** (27.0 mg, 42.2 µmol) in tetrahydrofuran (2.0 mL), 10% palladium on carbon (40.0 mg) was added. The reaction mixture was stirred under a hydrogen atmosphere at 25°C for 5 hours. The reaction solution was then filtered, and the filtrate was concentrated under reduced pressure. The crude product was purified by silica gel column chromatography (petroleum ether/ethyl acetate = 100:1 to 1:1) to obtain compound **2.** MS-ESI [M+H]⁺, calculated: 550, found: 550. ¹H NMR (400 MHz, MeOD) *δ* 8.59 (s, 2H), 7.44 (s, 1H), 4.37 (d, *J =* 3.2 Hz, 2H), 3.89-4.02 (m, 4H), 3.63-3.73 (m, 2H), 3.56 (dt, *J =* 14.0, 5.2 Hz, 2H), 3.48 (td, *J =* 9.6, 4.4 Hz, 2H), 2.34 (br d, *J =* 13.6 Hz, 1H), 1.79 (br s, 1H), 1.53-1.71 (m, 2H), 1.31-1.38 (m, 4H).

### Example 3 Synthesis of Compound 3

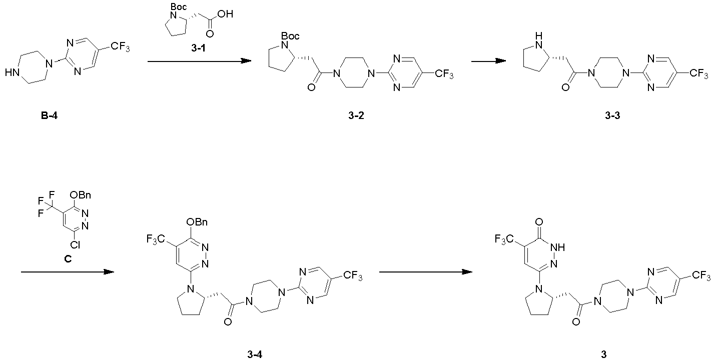

(1) To a solution of intermediate **B-4** trifluoroacetate (1.50 g, 4.33 mmol) in dichloromethane (10.0 mL), compound **3-1** (1.49 g, 6.50 mmol), triethylamine (2.19 g, 21.67 mmol), and propylphosphonic anhydride solution (5.51 g, 8.66 mmol, purity 50%) were added. The reaction mixture was stirred at 25°C under a nitrogen atmosphere for 1 hour. Water (10.0 mL) was added to the reaction mixture, and it was extracted with ethyl acetate (50.0 mL × 1). The organic phase was washed with saturated sodium chloride solution (50.0 mL × 2), dried over anhydrous sodium sulfate, filtered, and concentrated under reduced pressure. The crude product was purified by silica gel column chromatography (petroleum ether/ethyl acetate = 100:1 to 1:1) to obtain compound **3-2.** MS-ESI [M+H]⁺, calculated: 444, found: 444. ¹H NMR (400 MHz, CDCl₃) *δ* 8.52 (s, 2H), 4.06-4.18 (m, 1H), 3.87-4.02 (m, 2H), 3.69 (br d, *J =* 4.4 Hz, 4H), 3.28-3.41 (m, 4H), 2.25-2.35 (m, 1H), 2.05-2.17 (m, 1H), 1.79-1.93 (m, 4H), 1.8 (s, 9H).
(2) To a solution of compound **3-2** (700 mg, 1.58 mmol) in dichloromethane (6.0 mL), trifluoroacetic acid (3.08 g, 27.0 mmol) was added. The reaction mixture was stirred at 25°C for 0.5 hours. The reaction solution was then filtered, and the filtrate was concentrated under reduced pressure to obtain the crude compound **3-3** trifluoroacetate. MS-ESI [M+H]⁺, calculated: 344, found: 344.
(3) To a solution of compound **3-3** trifluoroacetate (460 mg, 1.34 mmol) in toluene (5.0 mL), compound **C** (300 mg, 1.04 µmol), potassium tert-butoxide (233 mg, 2.08 mmol), and bis(dibenzylideneacetone)palladium (149 mg, 260 µmol) were added. The reaction was carried out under a nitrogen atmosphere at 110°C for 3 hours. After the reaction, 20.0 mL of water was added to the reaction mixture, and it was extracted with ethyl acetate (20.0 mL × 1). The organic phase was washed with saturated sodium chloride solution (30.0 mL × 2), dried over anhydrous sodium sulfate, filtered, and concentrated under reduced pressure. The crude product was purified by silica gel column chromatography (petroleum ether/ethyl acetate = 100:1 to 3:1) to obtain compound **3-4.** MS-ESI [M+H]⁺, calculated: 596, found: 596. ¹H NMR (400 MHz, CDCl₃) *δ* 8.53 (s, 2H), 7.48 (d, *J =* 7.2 Hz, 2H), 7.39 (t, *J =* 7.6 Hz, 3H), 7.02 (s, 1H), 5.57 (s, 2H), 4.62 (br d, *J =* 6.0 Hz, 1H), 3.84-4.06 (m, 4H), 3.72-3.81 (m, 2H), 3.59-3.71 (m, 3H), 3.33-3.43 (m, 1H), 3.17 (br dd, *J =* 14.4, 3.2 Hz, 1H), 2.31-2.42 (m, 1H), 2.10-2.25 (m, 4H).
(4) To a solution of compound **3-4** (140 mg, 235 µmol) in tetrahydrofuran (5.0 mL), 10% palladium on carbon (100 mg) was added. The reaction mixture was stirred under a hydrogen atmosphere at 20°C for 5 hours. After the reaction, it was filtered, and the filtrate was concentrated under reduced pressure. The crude product was purified by preparative high-performance liquid chromatography (Phenomenex Luna C18, 100 mm × 30 mm, 3 µm, A: water (0.225% formic acid); B: acetonitrile, 41%-61% B over 8 minutes) to obtain compound **3** formate. MS-ESI [M+H]⁺, calculated: 506, found: 506. ¹H NMR (400 MHz, MeOD) *δ* 8.60 (s, 2H), 7.59 (s, 1H), 4.29-4.35 (m, 1H), 4.05-4.13 (m, 1H), 4.00 (ddd, *J=* 13.2, 6.8, 3.6 Hz, 1H), 3.88-3.95 (m, 1H), 3.83 (ddd, *J =* 13.6, 7.2, 3.6 Hz, 2H), 3.69-3.77 (m, 2H), 3.58-3.66 (m, 2H), 3.34-3.42 (m, 1H), 3.08 (dd, *J =* 14.0, 4.4 Hz, 1H), 2.35 (dd, *J =* 14.4, 9.2 Hz, 1H), 1.97-2.18 (m, 4H).

### Example 4 Synthesis of Compound 4

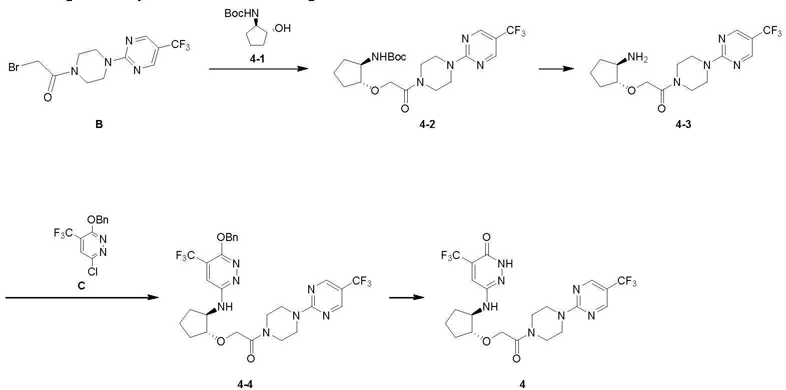

(1) To a solution of intermediate **B** (800 mg, 2.27 mmol) in dichloromethane (8.0 mL), water (2.0 mL), compound **4-1** (593 mg, 2.95 mmol), sodium hydroxide (906 mg, 22.7 mmol), and tetrabutylammonium bisulfate (385 mg, 1.13 mmol) were added. The reaction mixture was stirred at 25°C under a nitrogen atmosphere for 12 hours. Water (10.0 mL) was added to the reaction mixture, and it was extracted with dichloromethane (20.0 mL × 1). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **4-2.** MS-ESI [M+H]⁺, calculated: 474, found: 474.
(2) To a solution of compound **4-2** (600 mg, 1.27 mmol) in dichloromethane (3.0 mL), trifluoroacetic acid (3.08 g, 27.0 mmol) was added. The reaction mixture was stirred at 25°C for 0.5 hours. After the reaction, it was filtered, and the filtrate was concentrated under reduced pressure to obtain the crude compound **4-3** trifluoroacetate. MS-ESI [M+H]⁺, calculated: 374, found: 374.
(3) To a solution of compound **4-3** trifluoroacetate (437 mg, 1.17 mmol) in toluene (12.0 mL), compound **C** (260 mg, 901 µmol), potassium tert-butoxide (202 mg, 1.80 mmol), 1,1'-binaphthyl-2,2'-diphenylphosphine (280 mg, 450 µmol), and bis(dibenzylideneacetone)palladium (II) (129 mg, 225 µmol) were added. The reaction mixture was stirred at 110°C under a nitrogen atmosphere for 3 hours. Water (5.0 mL) was added to the reaction mixture, and it was extracted with ethyl acetate (50.0 mL × 1). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **4-4.** MS-ESI [M+H]⁺, calculated: 626, found: 626. ¹H NMR (400 MHz, CDCl₃) *δ* 8.50 (s, 2H), 7.28-7.47 (m, 6H), 5.52 (s, 2H), 4.44 (d, *J=* 14.4 Hz, 1H), 4.27 (d, *J =* 14.4 Hz, 1H), 4.07-4.15 (m, 1H), 3.87-4.04 (m, 5H), 3.65-3.80 (m, 2H), 3.57 (br s, 2H), 2.41-2.53 (m, 1H), 1.95-2.04 (m, 1H), 1.83-1.93 (m, 1H), 1.68-1.81 (m, 3H).
(4) Compound **4-4** (60.0 mg, 95.9 µmol) was dissolved in tetrahydrofuran (5.0 mL) and 10% palladium on carbon (100 mg) was added to the solution. The reaction mixture was stirred at 25°C under a hydrogen atmosphere for 5 hours. Afterward, the mixture was filtered, and the filtrate was concentrated under reduced pressure. The crude product was purified by high-performance liquid chromatography (Phenomenex Luna C18, 100 mm × 30 mm, 3 µm, A: water (0.225% formic acid); B: acetonitrile, 41%-61% over 8 minutes) to isolate compound **4** formate. MS-ESI [M+H]⁺, calculated: 536, found: 536. ¹H NMR (400 MHz, MeOD) *δ* 8.59 (s, 2H), 7.39 (s, 1H), 4.35-4.40 (m, 2H), 3.90-4.02 (m, 5H), 3.87 (dd, *J=* 6.0, 2.8 Hz, 1H), 3.61-3.73 (m, 4H), 2.14-2.24 (m, 1H), 1.90-1.99 (m, 1H), 1.72-1.83 (m, 3H), 1.46-1.56 (m, 1H).

### Example 5 Synthesis of Compound 5

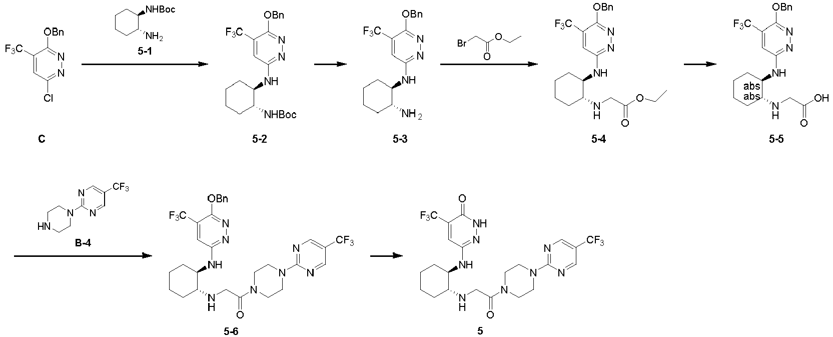

(1) To a solution of compound **5-1** (742 mg, 3.46 mmol) in toluene (15.0 mL) was added compound **C** (500 mg, 1.73 mmol), cesium carbonate (1.13 g, 3.46 mmol), 1,1'-binaphthyl-2,2'-diphenylphosphine (539 mg, 866 µmol), and bis(dibenzylideneacetone)palladium (249 mg, 433 µmol). The reaction mixture was stirred at 80°C under a nitrogen atmosphere for 3 hours. After that, the reaction mixture was treated with water (2.0 mL) and extracted with ethyl acetate (10.0 mL × 1). The organic layer was dried over anhydrous sodium sulfate, filtered, and concentrated under reduced pressure. The crude product was purified by silica gel column chromatography (petroleum ether/ethyl acetate = 100:1 to 1:1) to obtain compound **5-2.** MS-ESI [M+H]⁺, calculated: 467, found: 467.
(2) To a solution of compound **5-2** (490 mg, 1.05 mmol) in dichloromethane (3.0 mL), trifluoroacetic acid (1.54 g, 13.5 mmol) was added. The reaction mixture was stirred at 25°C for 0.5 hours. After filtration, the filtrate was concentrated under reduced pressure, affording the crude product as compound **5-3** trifluoroacetate. MS-ESI [M+H]⁺, calculated: 367, found: 367.
(3) To a solution of compound **5-3** trifluoroacetate (450 mg, 937 µmol) in acetonitrile (50.0 mL), 2-bromoethyl acetate (156 mg, 937 µmol), and sodium bicarbonate (630 mg, 7.49 mmol) were added. The reaction mixture was stirred at 25°C under a nitrogen atmosphere for 40 hours. Water (10.0 mL) was added to the reaction mixture, and it was then extracted with ethyl acetate (50.0 mL × 1). The organic phase was dried over anhydrous sodium sulfate, filtered, and concentrated under reduced pressure to obtain the crude product, which was purified by silica gel column chromatography (dichloromethane/methanol = 100:1 to 20:1), affording compound **5-4.** MS-ESI [M+H]⁺, calculated: 453, found: 453.
(4) To a solution of compound **5-4** (110 mg, 243 µmol) in ethanol (2.0 mL), water (1.0 mL), and lithium hydroxide monohydrate (102 mg, 2.43 mmol) were added. The reaction mixture was stirred at 25°C for 2 hours. Hydrochloric acid solution (1 mol/L) was added to the reaction mixture to adjust the pH to 7. The mixture was then extracted with dichloromethane (25.0 mL × 2), and the combined organic phase was dried over anhydrous sodium sulfate, filtered, and concentrated under reduced pressure to obtain the crude product, compound **5-5.** MS-ESI [M+H]⁺, calculated: 425, found: 425.
(5) To a solution of intermediate **5-5** (80.0 mg, 226 µmol) in dichloromethane (4.0 mL) was added compound **B-4** trifluoroacetate (78.3 mg, 226 µmol), diisopropylethylamine (122 mg, 942 µmol), and propylphosphoric anhydride (240 mg, 337 µmol, purity 50%). The reaction mixture was stirred at 25°C under a nitrogen atmosphere for 2 hours. The reaction mixture was then filtered, concentrated under reduced pressure, and the crude product was purified by silica gel column chromatography (dichloromethane/methanol = 100:1 to 1:1) to isolate compound **5-6.** MS-ESI [M+H]⁺, calculated: 639, found: 639.
(6) To a solution of compound **5-6** (70.0 mg, 110 µmol) in tetrahydrofuran (3.0 mL) was added 10% palladium on carbon (50.0 mg). The reaction mixture was stirred at 25°C under a hydrogen atmosphere for 5 hours. After filtration, the filtrate was concentrated under reduced pressure, and the crude product was purified by preparative high-performance liquid chromatography (Phenomenex Luna C18, 100 mm × 30 mm, 3 µm, mobile phase A: water with 0.225% formic acid, mobile phase B: acetonitrile, gradient from 20% to 50% B over 8 minutes) to obtain compound **5** formate. MS-ESI [M+H]⁺, calculated: 549, found: 549. ¹H NMR (400 MHz, MeOD) *δ* 8.61 (s, 2H), 7.46 (s, 1H), 3.91-4.04 (m, 4H), 3.82-3.89 (m, 1H), 3.65-3.79 (m, 4H), 3.57 (br t, *J =* 4.8 Hz, 2H), 2.73 (br d, *J =* 9.2 Hz, 1H), 2.11-2.22 (m, 2H), 1.74-1.87 (m, 2H), 1.22-1.44 (m, 4H).

### Example 6 Synthesis of Compound 6

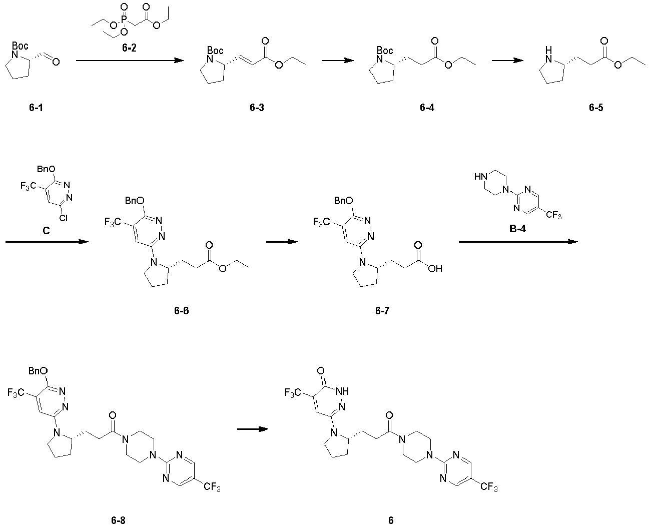

(1) To a solution of sodium hydride (3.91 g, 97.9 mmol, 60%) in tetrahydrofuran (50.0 mL) at 0°C, compound **6-2** (20.3 g, 90.3 mmol) was added. The reaction mixture was stirred at 0°C for 0.5 hours. Then, a solution of compound **6-1** (15.0 g, 75.3 mmol) in tetrahydrofuran (200 mL) was added to the reaction mixture, and stirred at 0°C under a nitrogen atmosphere for 2.5 hours. To the reaction mixture, ethyl acetate (500 mL) was added. The pH was adjusted to 7 with saturated ammonium chloride solution. The organic phase was separated and washed with water (500 mL × 1) and saturated sodium chloride solution (400 mL × 3). The organic phase was dried over anhydrous sodium sulfate, filtered, and concentrated under reduced pressure. The crude product was purified by silica gel column chromatography (petroleum ether/ethyl acetate, 1:0 to 15:1) to obtain compound **6-3.** MS-ESI [M-O^{t}Bu+H]⁺, calculated: 214, found: 214.
(2) To a solution of compound **6-3** (10.0 g, 37.1 µmol) in tetrahydrofuran (100 mL), 10% palladium on carbon (1.00 g) was added. The reaction mixture was stirred at 25°C under a hydrogen atmosphere for 2 hours. Afterward, the reaction mixture was filtered, and the solvent was removed under reduced pressure to obtain compound **6-4.** MS-ESI [M+H]⁺, calculated: 272, found: 272. ¹H NMR (400 MHz, CDCl₃) δ 4.13 (q, *J* = 7.2 Hz, 2H), 3.75-3.89 (m, 1H), 3.26-3.50 (m, 2H), 2.31 (s, 2H), 1.78-2.02 (m, 4H), 1.59-1.70 (m, 2H), 1.47 (s, 9H), 1.26 (t, *J=* 7.2 Hz, 3H).
(3) To a solution of compound **6-4** (1.00 g, 3.69 mmol) in dichloromethane (8.0 mL), trifluoroacetic acid (4.62 g, 40.5 mmol) was added. The reaction mixture was stirred at 25°C for 0.5 hours. Then, dichloromethane (20.0 mL) was added to the reaction mixture, and the pH was adjusted to 7 with saturated sodium bicarbonate solution. The organic phase was separated and washed with water (20.0 mL × 2) and saturated sodium chloride (20.0 mL × 1). The organic phase was dried over anhydrous sodium sulfate, filtered, and concentrated under reduced pressure to obtain compound **6-5.**
(4) To a solution of compound **6-5** (530 mg, 3.10 mmol) in toluene (6.0 mL), compound **C** (447 mg, 3.10 mmol), cesium carbonate (1.01 g, 3.10 mmol), 1,1'-binaphthyl-2,2'-diphenylphosphine (482 mg, 774 µmol), and bis(dibenzylideneacetone)palladium (223 mg, 387 µmol) were added. The reaction mixture was stirred at 80°C under a nitrogen atmosphere for 3 hours. Ethyl acetate (60.0 mL) was added to the reaction mixture, and it was washed with water (50.0 mL × 1) and saturated sodium chloride (30.0 mL × 3). The organic phase was dried over anhydrous sodium sulfate, filtered, and concentrated under reduced pressure to obtain compound **6-6.** MS-ESI [M+H]⁺, calculated: 424, found: 424.
(5) To a solution of compound **6-6** (344 mg, 812 µmol) in ethanol (2.0 mL), water (1.0 mL), and lithium hydroxide monohydrate (205 mg, 4.87 mmol) were added. The reaction mixture was stirred at 25°C for 2 hours. Hydrochloric acid solution (1 mol/L) was added to adjust the pH to 7. The mixture was then extracted with dichloromethane (25.0 mL × 1). The organic phase was washed with water (20.0 mL × 2) and saturated sodium chloride (20.0 mL × 1). Subsequently, it was dried over anhydrous sodium sulfate, filtered, and concentrated under reduced pressure to obtain compound **6-7.** MS-ESI [M+H]⁺, calculated: 396, found: 396. ¹H NMR (400 MHz, CDCl₃) *δ* 7.46 (d, *J =* 7.2 Hz, 2H), 7.37 (t, *J =* 7.2 Hz, 2H), 7.32 (d, *J =* 7.2 Hz, 1H), 7.08-7.17 (m, 1H), 5.52-5.57 (m, 2H), 4.26 (d, *J=* 5.6 Hz, 1H), 3.60 (s, 1H), 3.30-3.39 (m, 1H), 2.43 (s, 2H), 2.01-2.11 (m, 4H), 1.69 (s, 2H).
(6) To a solution of intermediate **6-7** (179 mg, 453 µmol) in dichloromethane (5.0 mL), compound **B-4** trifluoroacetate (188 mg, 544 µmol), diisopropylethylamine (293 mg, 2.26 mmol), and propylphosphonic anhydride solution (1.15 mg, 1.81 µmol, 50% purity in ethyl acetate) were added. The reaction mixture was stirred at 25°C under a nitrogen atmosphere for 2 hours. Dichloromethane (60.0 mL) was added to the reaction mixture, and it was washed with water (50.0 mL × 1) and saturated sodium chloride (30.0 mL × 3). Then, it was dried over anhydrous sodium sulfate, filtered, and concentrated under reduced pressure. The crude product was purified by silica gel column chromatography (dichloromethane/methanol = 1:0 to 8:1) to yield compound **6-8.** MS-ESI [M+H]⁺, calculated: 610, found: 610. ¹H NMR (400 MHz, CDCl₃) *δ* 8.51 (s, 2H), 7.46 (d, *J =* 7.2 Hz, 2H), 7.37 (t, *J =* 7.2 Hz, 2H), 7.28-7.34 (m, 2H), 5.55 (s, 2H), 4.26 (s, 1H), 3.90-3.98 (m, 4H), 3.62-3.74 (m, 3H), 3.55 (t, *J =* 5.2 Hz, 2H), 3.43 (s, 1H), 2.43-2.52 (m, 2H), 2.06-2.19 (m, 4H), 1.89-1.94 (m, 1H), 1.74 (dd, *J=* 8.8, 6.8 Hz, 1H).
(7) To a solution of compound **6-8** (160 mg, 262 µmol) in tetrahydrofuran (3.0 mL), 10% palladium on carbon (160 mg) was added. The reaction mixture was stirred at 25°C under a hydrogen atmosphere for 5 hours. Afterward, the mixture was filtered, and the filtrate was concentrated under reduced pressure. The crude product was purified using preparative high-performance liquid chromatography (Xtimate C18, 100 mm × 30 mm, 10 µm, A: water with 0.225% formic acid, B: acetonitrile, 50% to 80% B over 10 minutes) to obtain compound **6** formate. MS-ESI [M+H]⁺, calculated: 520, found: 520. ¹H NMR (400 MHz, MeOD) δ 8.74 (s, 2H), 7.98 (s, 1H), 3.87 (s, 2H), 3.77-3.86 (m, 3H), 3.56 (d, *J =* 4.4 Hz, 4H), 3.40-3.46 (m, 1H), 3.21 (d, *J=* 9.6 Hz, 1H), 2.40-2.45 (m, 2H), 1.87-1.98 (m, 3H), 1.79-1.87 (m, 2H), 1.44-1.53 (m, 1H).

### Example 7 Synthesis of Compound 7

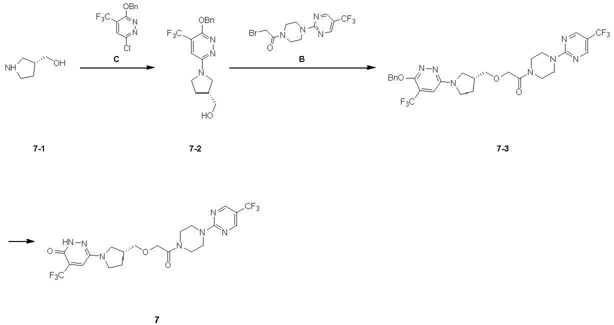

(1) To a solution of compound **7-1** (800 mg, 2.77 mmol) in toluene (15.0 mL), compound **C** (560 mg, 1.94 mmol), cesium carbonate (1.81 g, 5.54 mmol), 1,1'-binaphthyl-2,2'-diphenylphosphine (862 mg, 1.39 mmol), and tris(dibenzylideneacetone)dipalladium (634 mg, 692 µmol) were added. The reaction mixture was stirred at 80°C under a nitrogen atmosphere for 3 hours. Water (20.0 mL) was added to the reaction mixture, followed by extraction with ethyl acetate (20.0 mL × 3). The organic phase was washed with saturated sodium chloride solution (20.0 mL × 3), dried over anhydrous sodium sulfate, filtered, and concentrated under reduced pressure. The crude product was then purified by column chromatography on silica gel (dichloromethane/methanol = 20:1) to isolate compound **7-2.** MS-ESI [M+H]⁺, calculated: 354, found: 354. ¹H NMR (400 MHz, CDCl₃) *δ* 7.45-7.50 (m, 2H), 7.35-7.39 (m, 2H), 7.29-7.33 (m, 1H), 6.93-6.95 (s, 1H), 5.55-5.60 (s, 2H), 3.66-3.73 (m, 3H), 3.52-3.58 (m, 1H), 3.34-3.41 (m, 1H), 2.58-2.69 (m, 1H), 2.14-2.24 (m, 1H), 1.86-1.95 (m, 1H), 1.24-1.29 (m, 1H).
(2) Compound **7-2** (100 mg, 283 µmol) was added to a solution of intermediate **B** (150 mg, 424 µmol) in dichloromethane (4.0 mL), along with sodium hydroxide (226 mg, 5.66 mmol), and tetrabutylammonium bisulfate (48.0 mg, 141 µmol). The reaction mixture was stirred at 25°C under a nitrogen atmosphere for 12 hours. Water (10.0 mL) was added to the reaction mixture, followed by extraction with dichloromethane (10.0 mL × 3). The combined organic phase was washed with saturated sodium chloride solution (10.0 mL × 3), dried over anhydrous sodium sulfate, filtered, and concentrated under reduced pressure. The crude product was then purified by column chromatography on silica gel (dichloromethane/methanol = 20:1) to isolate compound **7-3.** MS-ESI [M+H]⁺, calculated: 626, found: 626.
(3) Compound **7-3** (100 mg, 159 µmol) was dissolved in tetrahydrofuran (5.0 mL), and 10% palladium on carbon (50.0 mg) was added to the solution. The reaction mixture was stirred under a hydrogen atmosphere at 25°C for 12 hours. After completion, the reaction mixture was filtered, and the solvent was removed under reduced pressure. The crude product was purified by preparative high-performance liquid chromatography (HPLC) using a Phenomenex Luna C18 column (100 mm × 30 mm, 3 µm) with a gradient elution of water (0.225% formic acid) and acetonitrile (38%-68%) over 7 minutes to obtain compound 7 formate. MS-ESI [M+H]⁺, calculated: 536, found: 536. ¹H NMR (400 MHz, MeOD) *δ* 8.59-8.61 (s, 2H), 7.55-7.57 (s, 1H), 4.56-4.65 (s, 1H), 4.28-4.31 (s, 2H), 3.92-3.98 (m, 4H), 3.66-3.70 (m, 2H), 3.57-3.63 (m, 4H), 3.48-3.54 (m, 2H), 3.38-3.44 (m, 1H), 2.65-2.72 (m, 1H), 2.12-2.19 (m, 1H), 1.82-1.89 (m, 1H).

### Example 8 Synthesis of Compound 8

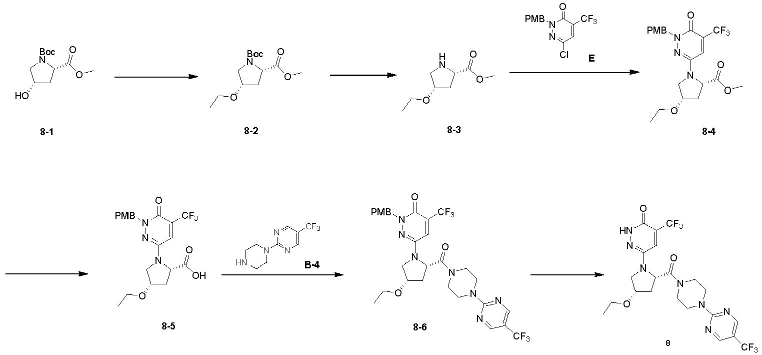

(1) To a solution of compound **8-1** (5.00 g, 20.3 mmol) in tetrahydrofuran (50.0 mL) at 0°C, sodium hydride (1.63 g, 40.7 mmol) was added, and the mixture was stirred for 30 minutes at 0°C. Then, iodoethane (15.9 g, 101 mmol) was added to the reaction mixture, and reacted at 25°C for 3 hours. The reaction mixture was quenched with hydrochloric acid (50.0 mL, 0.5 mol/L) and extracted with dichloromethane (20.0 mL × 3). The organic phase was dried over anhydrous sodium sulfate, filtered, and concentrated under reduced pressure. The crude product was purified by silica gel column chromatography (petroleum ether/ethyl acetate mixture = 25:1) to obtain compound **8-2.** MS-ESI [M-Boc+H]⁺, calculated: 174, found: 174.
(2) Compound **8-2** (2.82 g, 10.3 mmol) was dissolved in dichloromethane (6.00 mL), and then trifluoroacetic acid (1.18 g, 10.3 mmol) was added. The reaction was allowed to proceed at 25°C for 1 hour. After the reaction, the mixture was concentrated under reduced pressure. The pH of the resulting solution was adjusted to 7-8 by adding saturated sodium bicarbonate solution. The mixture was then extracted with dichloromethane (5.00 mL × 3). The organic phase was dried over anhydrous sodium sulfate, filtered, and concentrated under reduced pressure to obtain compound **8-3.**
(3) A mixture of compound **8-3** (642 mg, 3.71 mmol), compound **E** (1.42 g, 4.45 mmol), cesium carbonate (2.42 g, 3.71 mmol), tris(dibenzylideneacetone)dipalladium (339 mg, 370 µmol), and 1,1'-binaphthyl-2,2'-diphenylphosphine (461 mg, 741 µmol) in toluene (6.00 mL) was stirred at 80°C under a nitrogen atmosphere for 17 hours. After the reaction, the mixture was concentrated under reduced pressure, dried over anhydrous sodium sulfate, filtered, and concentrated under reduced pressure. The crude product was purified by silica gel column chromatography (eluent: petroleum ether/ethyl acetate = 4:1) to obtain compound **8-4.** ¹H NMR (400 MHz, CDCl₃) *δ* 7.39 (d, *J =* 8.4 Hz, 2H), 7.17 (d, *J =* 7.2 Hz, 1H), 6.81-6.88 (m, 2H), 5.03-5.18 (m, 2H), 4.38-4.48 (m, 1H), 4.15-4.28 (m, 1H), 3.79 (s, 3H), 3.68 (d, *J=* 4.8 Hz, 3H), 3.60 (d, *J=* 4.0 Hz, 1H), 3.40-3.56 (m, 3H), 2.40-2.47 (m, 1H), 2.30-2.39 (m, 1H), 2.20 (ddd, *J* = 13.4, 7.2, 5.2 Hz, 1H), 1.18 (dt, *J =* 15.6, 7.2 Hz, 3H).
(4) To a solution of compound **8-4** (376 mg, 825 µmol) in tetrahydrofuran (6.00 mL) and water (2.00 mL), lithium hydroxide monohydrate (346 mg, 8.26 mmol) was added. And the mixture was stirred at 30°C for 16 hours. The pH of the reaction mixture was adjusted to 3-4 using hydrochloric acid, and then the mixture was extracted with dichloromethane (20.0 mL × 3). The organic phase was dried over anhydrous sodium sulfate, filtered, and the crude product was purified by silica gel column chromatography (eluent: petroleum ether/ethyl acetate = 1:1) to obtain compound **8-5.**
(5) To a solution of compound **8-5** (341 mg, 772 µmol) in dichloromethane (10.0 mL), compound **B-4** trifluoroacetate (215 mg, 927 µmol), diisopropylethylamine (299 mg, 2.32 mmol), and propylphosphonic anhydride solution (1.23 g, 2.33 mmol, 50% purity) were added. The reaction mixture was stirred at 30°C for 4.5 hours under a nitrogen atmosphere. Afterward, the reaction mixture was extracted with dichloromethane (30.0 mL) and water (5.00 mL). The organic phase was dried over anhydrous sodium sulfate, filtered, and concentrated under reduced pressure to obtain compound **8-6.** MS-ESI [M+H]⁺, calculated: 656, found: 656.
(6) Compound **8-6** (324 mg, 494 µmol) was dissolved in trifluoroacetic acid (10.0 mL). Trifluoromethanesulfonic acid (1.00 mL) was added to the solution, and the mixture was stirred at 25°C for 17 hours. After concentration under reduced pressure, the crude product was purified using preparative high-performance liquid chromatography (C18-6, 100 mm × 30 mm, 5 µm; mobile phase A: water with 0.225% formic acid, mobile phase B: acetonitrile, gradient from 40% to 70% B over 15 minutes) to obtain compound **8** formate. MS-ESI [M+H]⁺, calculated: 536, found: 536. ¹H NMR (400 MHz, MeOD) *δ* 8.62 (s, 2H), 7.55-7.59 (m, 1H), 4.30 (br s, 1H), 4.05-4.17 (m, 2H), 3.98-4.04 (m, 1H), 3.85-3.97 (m, 2H), 3.76-3.85 (m, 3H), 3.69-3.76 (m, 1H), 3.65 (br d, *J =* 10.4 Hz, 1H), 3.53-3.60 (m, 3H), 2.42-2.54 (m, 1H), 2.13 (dt, *J =* 13.2, 6.4 Hz, 1H), 1.17-1.22 (m, 3H).

### Example 9 Synthesis of Compound 9

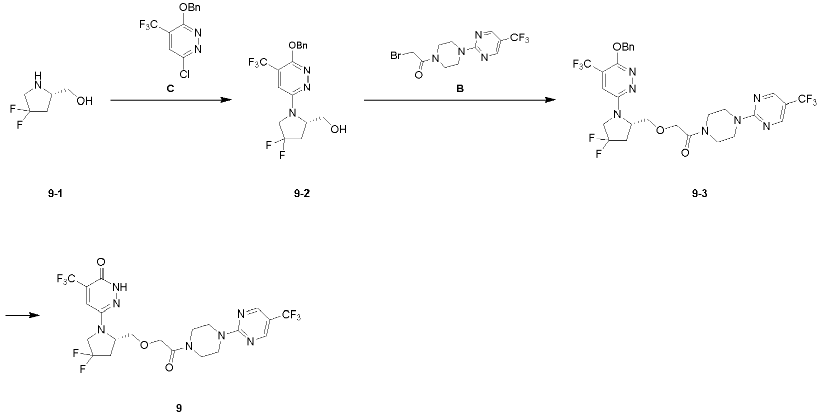

(1) To a solution of Compound **9-1** (280 mg, 2.04 mmol) in toluene (15.0 mL), compound **C** (530 mg, 1.84 mmol), cesium carbonate (1.33 g, 4.08 mmol), 1,1'-binaphthyl-2,2'-diphenylphosphine (635 mg, 1.02 mmol), and tris(dibenzylideneacetone)dipalladium (467.5 mg, 510 µmol) were added. The reaction mixture was stirred at 80°C under a nitrogen atmosphere for 2 hours. After the reaction, the mixture was diluted with water (75.0 mL × 2), and the organic phase was extracted with ethyl acetate (50.0 mL). The combined organic phase was washed with saturated sodium chloride (75.0 mL × 2), dried over anhydrous sodium sulfate, filtered, and concentrated under reduced pressure. The crude product was purified by silica gel column chromatography (petroleum ether/ethyl acetate = 1:0 to 0:1) to obtain compound **9-2.** MS-ESI [M+H]⁺, calculated: 390, found: 390. ¹H NMR (400 MHz, CDCl₃) *δ* 7.43-7.50 (m, 2H), 7.29-7.41 (m, 3H), 7.06 (s, 1H), 5.56 (s, 2H), 4.49-4.57 (m, 1H), 3.80-3.99 (m, 4H), 2.57-2.72 (m, 1H), 2.39-2.52 (m, 1H).
(2) To a solution of compound **B** (151 mg, 428 µmol) in dichloromethane (6.0 mL), compound **9-2** (200 mg, 513 µmol), sodium hydroxide (375 mg, 9.38 mmol), tetra-n-butylammonium bisulfate (72.6 mg, 214 µmol), and water (1.5 mL) were added. The reaction mixture was stirred at 25°C under a nitrogen atmosphere for 2 hours. After the reaction, the mixture was diluted with water (75.0 mL × 2), and the organic phase was extracted with dichloromethane (10.0 mL × 3). The combined organic phase was washed with saturated sodium chloride (75.0 mL × 2), dried over anhydrous sodium sulfate, filtered, and concentrated under reduced pressure. The crude product was purified by silica gel column chromatography (petroleum ether/ethyl acetate = 1:0 to 0:1) to obtain compound **9-3.** MS-ESI [M+H]⁺, calculated: 662, found: 662. ¹H NMR (400 MHz, CDCl₃) *δ* 8.50 (s, 2H), 7.42-7.47 (m, 2H), 7.37 (t, *J=* 7.2 Hz, 2H), 7.29-7.34 (m, 1H), 7.22 (s, 1H), 5.55 (s, 2H), 4.57-4.67 (m, 1H), 4.23 (s, 2H), 3.95-4.02 (m, 2H), 3.87-3.94 (m, 4H), 3.68-3.82 (m, 3H), 3.64 *(J* = 4.4 Hz, 1H), 3.38-3.55 (m, 2H), 2.58-2.71 (m, 2H).
(3) To a solution of compound **9-3** (200 mg, 302 µmol) in tetrahydrofuran (5.0 mL), 10% palladium on carbon (50.0 mg) was added, and the reaction mixture was stirred at 25°C under a hydrogen atmosphere for 3 hours. After the reaction, the mixture was filtered, and the filtrate was concentrated under reduced pressure. The crude product was purified by preparative high-performance liquid chromatography (Phenomenex Luna C18, 100 mm × 30 mm, 3 µm, using a gradient of water with 0.225% formic acid and acetonitrile from 43% to 73% over 8 minutes) to obtain compound 9 formate. MS-ESI [M+H]⁺, calculated: 572, found: 572. ¹H NMR (400 MHz, MeOD) *δ* 8.60 (s, 2H), 7.84 (s, 1H), 4.48-4.57 (m, 1H), 4.24-4.34 (m, 2H), 3.90-3.96 (m, 4H), 3.76-3.90 (m, 2H), 3.61-3.73 (m, 4H), 3.47-3.58 (m, 2H), 2.69 (*J* = 19.6 Hz, 1H), 2.39-2.56 (m, 1H).

### Example 10 Synthesis of Compound 10

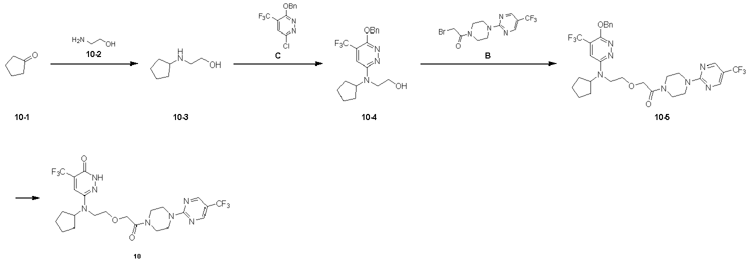

(1) Compound **10-2** (10.0 g, 164 mmol) was added to a solution of compound **10-1** (16.5 g, 196 mmol) in ethanol (150 mL). The reaction mixture was stirred at 25°C for 0.5 hours, and sodium borohydride (7.1 g, 188 mmol) was then added and stirred for 15 minutes at 0°C. The reaction solution was adjusted to pH 7 with 1 M hydrochloric acid, and then it was concentrated under reduced pressure. The pH was further adjusted to 11 using 10 M sodium hydroxide, and the mixture was extracted with ethyl acetate (200 mL). The organic phase was dried over anhydrous sodium sulfate, filtered, and concentrated under reduced pressure to obtain compound **10-3.** ¹H NMR (400 MHz, CDCl₃) *δ* 3.66-3.73 (m, 2H), 3.11-3.19 (m, 1H), 2.79-2.86 (m, 2H), 1.84-1.95 (m, 2H), 1.66-1.82 (m, 2H), 1.50-1.65 (m, 2H), 1.35-1.49 (m, 2H).
(2) To a solution of compound **C** (400 mg, 1.39 mmol) in toluene (15.0 mL), Compound **10-3** (358 mg, 2.77 mmol), cesium carbonate (903 mg, 2.77 mmol), 1,1'-binaphthyl-2,2'-diphenylphosphine (431 mg, 693 µmol), and tris(dibenzylideneacetone)dipalladium (199 mg, 346 µmol) were added. The reaction mixture was stirred at 80°C under a nitrogen atmosphere for 5 hours. After the reaction, the mixture was diluted with water (10.0 mL) and extracted with ethyl acetate (100 mL). The organic phase was dried over anhydrous sodium sulfate, filtered, and concentrated under reduced pressure. The crude product was further purified by silica gel column chromatography (eluent: petroleum ether/ethyl acetate = 100:1 to 1:1) to obtain compound **10-4.** MS-ESI [M+H]⁺, calculated: 382, found: 382.
(3) To a solution of compound **10-4** (80.0 mg, 210 µmol) in dichloromethane (2.0 mL), compound **B** (111 mg, 315 µmol), sodium hydroxide (126 mg, 3.15 mmol), tetrabutylammonium bisulfate (35.6 mg, 105 µmol) and water (0.5 mL) were added. The reaction mixture was stirred at 25°C under a nitrogen atmosphere for 12 hours. After the reaction, the mixture was extracted with ethyl acetate (50.0 mL). The organic phase was dried over anhydrous sodium sulfate, filtered, and concentrated under reduced pressure. The crude product was further purified by silica gel column chromatography (eluent: petroleum ether/ethyl acetate = 100:1 to 1:1) to obtain compound **10-5.** MS-ESI [M+H]⁺, calculated: 654, found: 654.
(4) Compound **10-5** (30.0 mg, 45.9 µmol) was dissolved in tetrahydrofuran (3.0 mL), and 10% palladium on carbon (50.0 mg) was added to the solution. The reaction mixture was stirred at 25°C in a hydrogen atmosphere for 5 hours. After the reaction, the mixture was filtered, and the filtrate was concentrated under reduced pressure. The crude product was further purified using preparative high-performance liquid chromatography (HPLC) on a C18 column (100 mm × 30 mm, 10 µm) with a gradient elution of water (0.225% formic acid) and acetonitrile (50%-80%) over 10 minutes to obtain compound **10** formate. MS-ESI [M+H]⁺, calculated: 564, found: 564. ¹H NMR (400 MHz, MeOD) *δ* 8.60 (s, 2H), 7.91 (s, 1H), 4.59 (br s, 1H), 4.29-4.35 (m, 1H), 4.28 (s, 2H), 3.88-3.96 (m, 4H), 3.66 (q, *J=* 5.6 Hz, 4H), 3.51-3.61 (m, 4H), 1.89-1.99 (m, 2H), 1.72-1.83 (m, 2H), 1.57-1.70 (m, 4H)

### Example 11 Synthesis of Compound 11

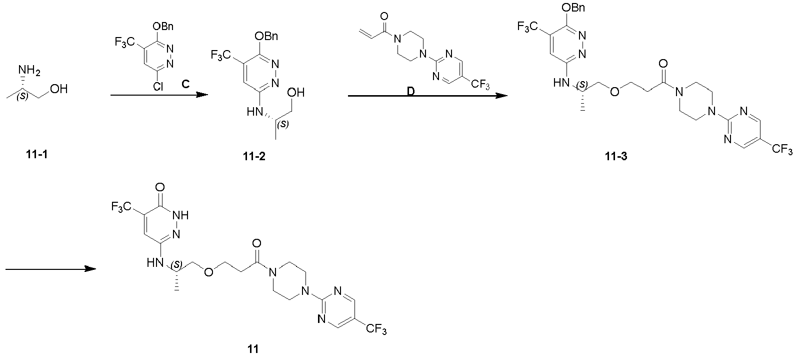

(1) To a solution of compound **11-1** (195 mg, 2.60 mmol) in toluene (20.0 mL), compound **C** (500 mg, 1.73 mmol), cesium carbonate (1.69 g, 5.20 mmol), 1,1'-binaphthyl-2,2'-diphenylphosphine (216 mg, 346 µmol), and tris(dibenzylideneacetone)dipalladium (159 mg, 173 µmol) were added. The reaction mixture was stirred at 80°C under a nitrogen atmosphere for 15 hours. Water (20.0 mL) was added to the reaction mixture, followed by extraction with dichloromethane (50.0 mL × 3). The organic phase was dried over anhydrous sodium sulfate, filtered, and concentrated under reduced pressure. The crude product was purified by silica gel column chromatography (petroleum ether/ethyl acetate = 1:0 to 1:1) to obtain compound **11-2.** MS-ESI [M+H]⁺, calculated: 328, found: 328. ¹H NMR (400 MHz, CDCl₃) *δ* 7.41-7.46 (m, 2H), 7.32-7.40 (m, 3H), 7.12 (s, 1H), 5.52 (s, 2H), 5.33-5.49 (m, 1H), 4.14-4.23 (m, 1H), 3.63-3.71 (m, 1H), 1.31 (d, *J=* 8.0 Hz, 3H)
(2) To a solution of compound **11-2** (82.3 mg, 252 µmol) and compound **D** (60.0 mg, 210 µmol) in dichloromethane (4.0 mL), sodium hydroxide (250 mg, 6.25 mmol), tetrabutylammonium bisulfate (35.6 mg, 105 µmol), and water (1.0 mL) were added. The reaction mixture was stirred at 25°C under a nitrogen atmosphere for 15 hours. The reaction mixture was diluted with dichloromethane (50.0 mL), followed by washing with water (10.0 mL × 3). The organic phase was dried over anhydrous sodium sulfate, filtered, and concentrated under reduced pressure. The crude product was purified by preparative thin-layer chromatography (dichloromethane/methanol = 10:1) to isolate compound **11-3.** MS-ESI [M+H]⁺, calculated: 614, found: 614.
(3) Compound **11-3** (110 mg, 179 µmol) was dissolved in tetrahydrofuran (10.0 mL), and 10% palladium on carbon (80.0 mg) was added to the solution. The reaction mixture was stirred at 25°C under a hydrogen atmosphere (15 psi) for 3 hours. Afterward, the reaction mixture was filtered, and the filtrate was concentrated under reduced pressure. The crude product was separated by preparative high-performance liquid chromatography (Phenomenex Luna C18, 30 mm × 30 mm, 10 µm, and YMC AQ 100 mm × 30 mm, 10 µm) using a gradient of water (10 mmol/L ammonium bicarbonate) and acetonitrile (30%-70%) over 28 minutes to obtain compound **11.** MS-ESI [M+H]⁺, calculated: 524, found: 524. ¹H NMR (400 MHz, MeOD) *δ* 8.58 (s, 2H), 7.41 (s, 1H), 3.86-3.96 (m, 5H), 3.76-3.81 (m, 2H), 3.65-3.71 (m, 4H), 3.46-3.51 (m, 2H), 2.69-2.75 (m, 2H), 1.17 (d, *J =* 8.0 Hz, 3H)

### Example 12 Synthesis of Compound 12

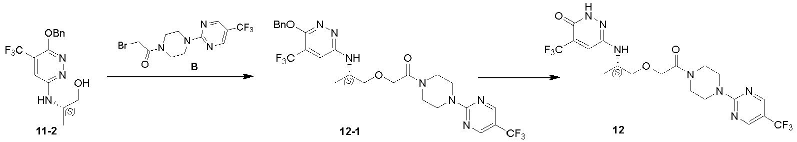

(1) Compound **11-2** (50.1 mg, 153 µmol) and compound **B** (45.0 mg, 127 µmol) were dissolved in dichloromethane (4.0 mL), and then sodium hydroxide (250 mg, 6.25 mmol), tetrabutylammonium bisulfate (21.6 mg, 63.7 µmol), and water (1.0 mL) were added to the solution. The reaction mixture was stirred at 25°C under a nitrogen atmosphere for 3 hours. The reaction mixture was diluted with dichloromethane (50.0 mL) and washed with water (10.0 mL × 3). The organic phase was dried over anhydrous sodium sulfate, filtered, and concentrated under reduced pressure. The crude product was purified by preparative thin-layer chromatography (dichloromethane/methanol = 10:1) to obtain compound **12-1.** MS-ESI [M+H]⁺, calculated: 600, found: 600. ¹H NMR (400 MHz, MeOD) *δ* 8.56 (s, 2H), 7.38-7.45 (m, 2H), 7.27-7.38 (m, 3H), 7.21 (s, 1H), 5.43 (s, 2H), 4.31 (s, 2H), 4.21-4.27 (m, 1H), 3.88-3.99 (m, 4H), 3.54-3.69 (m, 6H), 1.29 (d, *J=* 8.0 Hz, 3H).
(2) Compound **12-1** (40.0 mg, 66.7 µmol) was dissolved in tetrahydrofuran (10.0 mL), and 10% palladium on carbon (20.0 mg) was added to the solution. The reaction mixture was stirred at 25°C under a hydrogen atmosphere (15 psi) for 5 hours. After the reaction, the mixture was filtered, and the filtrate was concentrated under reduced pressure. The crude product was purified by preparative high-performance liquid chromatography (Xtimate C18, 100 mm × 30 mm, 10 µm, with a gradient of water (0.225% formic acid) and acetonitrile, 40% to 70%, over 18 minutes) to obtain compound **12** formate. MS-ESI [M+H]⁺, calculated: 510, found: 510. ¹H NMR (400 MHz, MeOD) *δ* 8.59 (s, 2H), 7.42 (s, 1H), 4.27-4.35 (m, 2H), 3.89-4.01 (m, 5H), 3.63-3.71 (m, 2H), 3.53-3.61 (m, 4H), 1.24 (d, J=8.0 Hz, 3H)

### Example 13 Synthesis of Compound 13

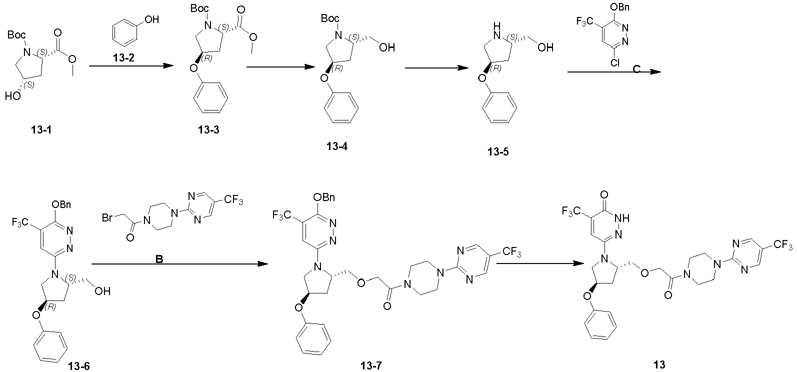

(1) Compound **13-1** (5.00 g, 20.4 mmol) and **13-2** (2.11 g, 22.4 mmol) were dissolved in tetrahydrofuran (50.0 mL) and the solution was cooled to 0°C. To this solution, triphenylphosphine (5.88 g, 22.4 mmol) and diisopropyl azodicarboxylate (4.53 g, 22.4 mmol, 4.36 mL) were added, and the reaction was allowed to proceed at 25°C for 12 hours. The reaction was quenched with water (30.0 mL), and extracted with ethyl acetate (40.0 mL × 2). The combined organic phase was washed with saturated sodium chloride (40.0 mL × 2), dried over anhydrous sodium sulfate, filtered, and concentrated under reduced pressure. The crude product was purified by silica gel column chromatography (petroleum ether/ethyl acetate = 1:0 to 5:1) to obtain compound **13-3.** MS-ESI [M+H]⁺, calculated: 322, found: 322. ¹H NMR (400 MHz, CDCl₃) *δ* 7.26 (m, 2H), 6.92-7.02 (m, 1H), 6.82-6.88 (m, 2H), 4.86-4.96 (s, 1H), 4.42 (t, *J =* 8.0 Hz, 1H), 3.77 (br s, 2H), 3.73-3.76 (m, 3H), 2.48-2.59 (m, 1H), 2.17-2.25 (m, 1H), 1.41-1.45 (m, 9H).
(2) Compound **13-3** (2.50 g, 7.78 mmol) was dissolved in tetrahydrofuran (30.0 mL) and cooled to 0°C. To this solution, lithium aluminum hydride (445 mg, 11.7 mmol) was added. The reaction mixture was stirred at 15°C for 2 hours. Water (0.5 mL), 15% sodium hydroxide solution (0.5 mL), water (1.5 mL), and sodium sulfate were added to the reaction mixture, and it was stirred for 10 minutes. The reaction mixture was then filtered. The filtrate was extracted with ethyl acetate (40.0 mL × 2). The combined organic phase was washed with saturated sodium chloride (40.0 mL × 2), dried over anhydrous sodium sulfate, filtered, and concentrated under reduced pressure. The crude product was purified by silica gel column chromatography (petroleum ether/ethyl acetate = 1:0 to 3:1) to obtain compound **13-4.** MS-ESI [M+H]⁺, calculated: 294, found: 294.
(3) Compound **13-4** (560 mg, 1.91 mmol) was dissolved in dichloromethane (5.0 mL), and a solution of hydrogen chloride in dioxane (4 M, 2.5 mL) was added. The reaction mixture was allowed to react at 25°C for 1 hour. The pH was adjusted to greater than 10 using saturated sodium hydroxide solution, and the mixture was then extracted with ethyl acetate (20.0 mL × 4). The combined organic phase was washed with saturated sodium chloride (20.0 mL × 4), dried over anhydrous sodium sulfate, filtered, and concentrated under reduced pressure to obtain crude compound **13-5,** which was used directly in the next step of the reaction.
(4) To a solution of compound **13-5** (360 mg, 1.86 mmol) in toluene (10.0 mL), compound C (485 mg, 1.68 mmol), cesium carbonate (1.21 g, 3.73 mmol), 1,1'-binaphthyl-2,2'-diphenylphosphine (232 mg, 373 µmol), and tris(dibenzylideneacetone)dipalladium (170 mg, 186 µmol) were added. The reaction mixture was stirred at 80°C under a nitrogen atmosphere for 3 hours. After the reaction, water (30.0 mL) was added, and the mixture was extracted with ethyl acetate (40.0 mL × 2). The combined organic phase was washed with saturated sodium chloride (40.0 mL × 2), dried over anhydrous sodium sulfate, filtered, and concentrated under reduced pressure. The crude product was purified by silica gel column chromatography (petroleum ether/ethyl acetate = 1:0 to 2:1) to obtain compound **13-6.** MS-ESI [M+H]⁺, calculated: 446, found: 446. ¹H NMR (400 MHz, CDCl₃) *δ* 7.47 (d, *J =* 7.2 Hz, 2H), 7.35-7.41 (m, 2H), 7.27-7.35 (m, 3H), 6.95-7.04 (m, 2H), 6.84-6.90 (m, 2H), 5.55 (s, 2H), 5.00-5.12 (m, 1H), 4.48-4.70 (m, 2H), 3.87-4.00 (m, 2H), 3.58-3.74 (m, 2H), 2.43-2.54 (m, 1H), 2.14-2.25 (m, 1H).
(5) To a solution of compound **13-6** (210 mg, 476 µmol) and compound B (140 mg, 397 µmol) in dichloromethane (4.0 mL), sodium hydroxide (250 mg, 6.25 mmol), tetrabutylammonium bisulfate (67.3 mg, 198 µmol), and water (1.0 mL) were added. The reaction mixture was stirred at 25°C under a nitrogen atmosphere for 2 hours. After the reaction, the mixture was diluted with water (30.0 mL) and extracted with dichloromethane (30.0 mL × 2). The combined organic phase was washed with saturated sodium chloride (30.0 mL × 2), dried over anhydrous sodium sulfate, filtered, and concentrated under reduced pressure. The crude product was purified by silica gel column chromatography (petroleum ether/ethyl acetate = 1:0 to 1:1) to obtain compound **13-7.** MS-ESI [M+H]⁺, calculated: 718, found: 718.
(6) Compound **13-7** (150 mg, 209 µmol) was dissolved in tetrahydrofuran (3.0 mL). To this solution, 10% palladium on carbon (30.0 mg) was added. The reaction mixture was stirred at 25°C under a hydrogen atmosphere (15 psi) for 12 hours. After the reaction, the mixture was filtered, and the filtrate was concentrated under reduced pressure. The crude product was purified by high-performance liquid chromatography (Phenomenex C18, 75 mm × 30 mm, 3 µm, A: water with 10 mmol/L ammonium bicarbonate; B: acetonitrile, 40%-70% over 10 minutes) to obtain compound **13.** MS-ESI [M+H]⁺, calculated: 628, found: 628. ¹H NMR (400 MHz, CDCl₃) *δ* 8.52 (s, 2H), 7.48 (s, 1H), 7.26-7.31 (m, 2H), 6.94-7.02 (t, *J=* 7.6 Hz, 1H), 6.87 (d, *J =* 8.0 Hz, 2H), 5.01-5.11 (m, 1H), 4.33-4.41 (m, 1H), 4.21 (s, 2H), 3.89-3.94 (m, 4H), 3.80-3.86 (m, 1H), 3.74-3.80 (m, 1H), 3.60-3.71 (m, 4H), 3.43-3.53 (m, 2H), 2.41-2.47 (m, 2H).

### Example 14 Synthesis of Compound 14

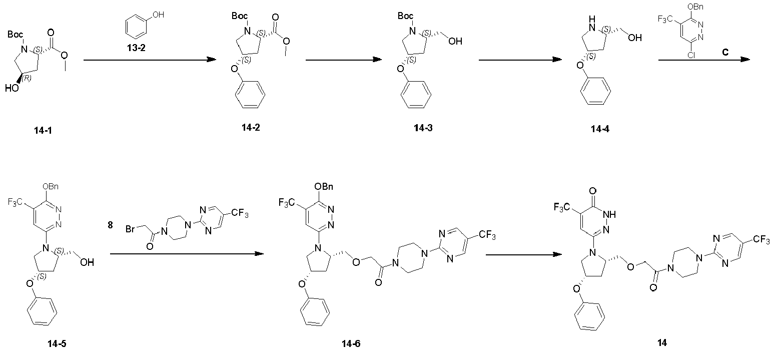

(1) Compound **14-1** (5.00 g, 20.3 mmol) and **13-2** (2.11 g, 22.4 mmol) were dissolved in tetrahydrofuran (100 mL) and cooled to 0°C. Triphenylphosphine (5.88 g, 22.4 mmol) and diisopropyl azodicarboxylate (4.53 g, 22.4 mmol, 4.36 mL) were added to the reaction mixture, and the reaction was allowed to proceed at 25°C for 12 hours. Ethyl acetate (250.0 mL) was added to the reaction mixture. It was then sequentially washed with water (250.0 mL × 2) and saturated sodium chloride solution (250.0 mL × 2). The organic phase was dried over anhydrous sodium sulfate, filtered, and concentrated under reduced pressure. The crude product was purified by silica gel column chromatography (petroleum ether/ethyl acetate = 1:0 to 0:1) to obtain compound **14-2.** MS-ESI [M+H]⁺, calculated: 322, found: 322. ¹H NMR (400 MHz, CDCl₃) *δ* 7.27-7.31 (m, 1H), 7.24 (s, 1H), 6.96 (q, *J =* 7.2 Hz, 1H), 6.79 (dd, *J =* 7.6, 4.8 Hz, 2H), 4.90 (t, *J=* 4.8, 2.0 Hz, 1H), 4.38-4.59 (m, 1H), 3.78 (d, *J=* 5.2 Hz, 1.5H), 3.72 (d, *J=* 7.2 Hz, 3H), 3.64-3.70 (m, 0.5H), 2.42-2.51 (m, 2H), 1.42-1.48 (m, 9H).
(2) Compound **14-2** (2.0 g, 6.22 mmol) was dissolved in tetrahydrofuran (25.0 mL) and cooled to 0°C. Then, lithium aluminum hydride (354 mg, 9.34 mmol) was added to the reaction mixture. The reaction was allowed to proceed at 15°C for 1 hour. After that, water (0.35 mL), 15% sodium hydroxide solution (0.35 mL), water (10.5 mL), and sodium sulfate were added to the reaction mixture and stirred for 10 minutes. The reaction mixture was filtered, and the filtrate was extracted with ethyl acetate (70.0 mL). The organic phase was successively washed with water (100.0 mL × 2) and saturated sodium chloride solution (100.0 mL × 2), dried over anhydrous sodium sulfate, filtered, and concentrated under reduced pressure. The crude product was purified by silica gel column chromatography (petroleum ether/ethyl acetate = 1:0 to 0:1) to obtain compound **14-3.** MS-ESI [M+H]⁺, calculated: 294, found: 294. ¹H NMR (400 MHz, CDCl₃) *δ* 7.30 (t, *J=* 8.0 Hz, 2H), 6.98 (t, *J=* 7.2 Hz, 1H), 6.85 (d, *J=* 8.0 Hz, 2H), 4.86 (s, 1H), 4.15 (s, 1H), 3.91 (s, 1H), 3.57-3.80 (m, 3H), 2.37 (m, 2H), 1.47 (s, 9H).
(3) Compound **14-3** (690 mg, 2.35 mmol) was dissolved in dichloromethane (7.0 mL), and a solution of hydrochloric acid in dioxane (4 M, 2.0 mL) was added to the reaction mixture. The reaction was carried out at 25°C for 1 hour. The pH of the reaction mixture was adjusted to 10 using a 4 M sodium hydroxide solution. Dichloromethane (30.0 mL) was added to dilute the mixture. It was then washed sequentially with water (75.0 mL × 2) and saturated sodium chloride solution (75.0 mL × 2), followed by drying over anhydrous sodium sulfate, filtration, and concentration under reduced pressure to obtain the crude product **14-4.** It was used directly in the next step of the reaction. MS-ESI [M+H]⁺, calculated: 194, found: 194. ¹H NMR (400 MHz, CDCl₃) *δ* 7.24-7.30 (m, 2H), 6.94 (t, *J =* 7.2 Hz, 1H), 6.82-6.90 (m, 2H), 4.76-4.94 (m, 1H), 3.57-3.69 (m, 2H), 3.38-3.47 (m, 1H), 3.22-3.28 (m, 1H), 3.14-3.21 (m, 1H), 2.24-2.35 (m, 1H), 1.72-1.82 (m, 1H).
(4) Compound **14-4** (280 mg, 1.45 mmol) was dissolved in toluene (3.0 mL), and compound **C** (376 mg, 1.30 mmol), cesium carbonate (944 mg, 2.90 mmol), 1,1'-binaphthyl-2,2'-diphenylphosphine (180 mg, 289 µmol), and tris(dibenzylideneacetone)dipalladium (132 mg, 144 µmol) were added to the reaction mixture. The reaction was stirred at 80°C under a nitrogen atmosphere for 2 hours. The reaction mixture was diluted with ethyl acetate (30.0 mL), washed sequentially with water (75.0 mL × 2) and saturated sodium chloride solution (75.0 mL × 2), dried over anhydrous sodium sulfate, filtered, and concentrated under reduced pressure. The resulting crude product was purified by silica gel column chromatography (petroleum ether/ethyl acetate = 1:0 to 0:1) to obtain compound **14-5.** MS-ESI [M+H]⁺, calculated: 446, found: 446.
(5) Compound **14-5** (180 mg, 404 µmol) and compound **B** (128.43 mg, 363 µmol) were dissolved in dichloromethane (6.0 mL). Sodium hydroxide (375 mg, 9.38 mmol), tetrabutylammonium bisulfate (68.6 mg, 202 µmol), and water (1.5 mL) were added to the solution. The reaction mixture was stirred at 25°C under a nitrogen atmosphere for 3 hours. The reaction mixture was diluted with dichloromethane (30.0 mL), washed sequentially with water (75.0 mL × 2) and saturated sodium chloride solution (75.0 mL × 2), dried over anhydrous sodium sulfate, filtered, and concentrated under reduced pressure. The resulting crude product was purified by silica gel column chromatography (petroleum ether/ethyl acetate = 1:0 to 1:1) to obtain compound **14-6.** MS-ESI [M+H]⁺, calculated: 718, found: 718.
(6) Compound **14-6** (110 mg, 153 µmol) was dissolved in tetrahydrofuran (4.0 mL), and 10% palladium on carbon (50.0 mg) was added to the solution. The reaction mixture was stirred at 25°C under a hydrogen atmosphere (15 psi) for 3 hours. Afterward, it was filtered, and the filtrate was concentrated under reduced pressure. The crude product was then purified using high-performance liquid chromatography (Phenomenex C18, 75 mm × 30 mm, 3 µm, A: water with 10 mmol/L ammonium bicarbonate; B: acetonitrile, 40%-80% over 10 minutes) to obtain compound **14.** MS-ESI [M+H]⁺, calculated: 628, found: 628. ¹H NMR (400 MHz, MeOD) *δ* 8.51 (s, 2H), 7.76 (s, 1H), 7.27-7.32 (m, 2H), 6.97 (t, *J=* 7.2 Hz, 1H), 6.86 (d, *J=* 8.0 Hz, 2H), 5.04 (s, 1H), 4.31 (q, *J =* 7.2 Hz, 1H), 4.21 (q, *J =* 13.6 Hz, 2H), 3.82-3.94 (m, 5H), 3.61-3.79 (m, 5H), 3.36-3.53 (m, 2H), 2.34-2.42 (m, 1H), 2.28-2.33 (m, 1H).

### Example 15 Synthesis of Compound 15

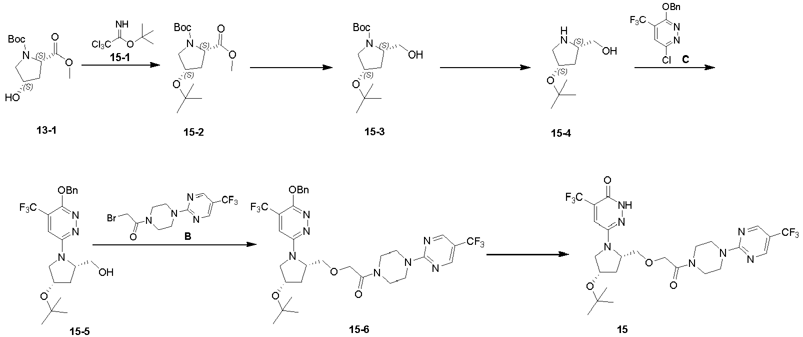

(1) To a solution of compound **13-1** (5.00 g, 20.4 mmol) in fluorobenzene (50.0 mL), 2,6-dimethylpyridine (1.09 g, 10.2 mmol) and bis (trifluoromethylsulfonyl )amine (1.43 g, 5.10 mmol) were added. Then, **15-1** (22.3 g, 101 mmol, 18.3 mL) was added dropwise to the reaction mixture at 20°C. After the dropwise addition, the reaction was allowed to proceed for 15 hours at 20°C. The reaction mixture was filtered, and the filtrate was concentrated under reduced pressure. The crude product was purified using silica gel column chromatography (eluent: petroleum ether/ethyl acetate from 1:0 to 5:1) to obtain compound **15-2.** ¹H NMR (400 MHz, CDCl₃) *δ* 4.07-4.35 (m, 2H), 3.54-3.76 (m, 4H), 3.16-3.26 (m, 1H), 2.25-2.42 (m, 1H), 1.92-2.05 (m, 1H), 1.49 (s, 3H), 1.36 (s, 6H), 1.16 (s, 9H).
(2) Compound **15-2** (2.20 g, 7.30 mmol) was dissolved in tetrahydrofuran (20.0 mL) and cooled to 0°C. To this solution, lithium aluminum hydride (305 mg, 8.03 mmol) was added. The reaction mixture was allowed to react at 0°C for 0.5 hours. Then, water (1.0 mL), 15% sodium hydroxide solution (1.0 mL), water (3.0 mL), and sodium sulfate were added to the reaction mixture, followed by stirring for 10 minutes. The reaction mixture was then filtered, and the filtrate was extracted with ethyl acetate (40.0 mL × 2). The organic phase was washed with saturated sodium chloride solution (40.0 mL × 2), dried over anhydrous sodium sulfate, filtered, and concentrated under reduced pressure. The crude product was purified by silica gel column chromatography (eluent: petroleum ether/ethyl acetate from 1:0 to 5:1) to obtain compound **15-3.** ¹H NMR (400 MHz, CDCl₃) *δ* 4.09-4.17 (m, 1H), 3.90-4.00 (m, 1H), 3.48-3.84 (m, 4H), 3.32 (m, 1H), 2.14-2.30 (m, 1H), 1.47 (s, 1H), 1.42 (s, 9H), 1.18 (s, 9H).
(3) Compound **15-3** (900 mg, 3.29 mmol) was dissolved in dichloromethane (6.0 mL), and a solution of hydrochloric acid in dioxane (4 M, 3.0 mL) was added. The reaction mixture was allowed to react at 25°C for 2 hours. After the reaction, the pH was adjusted to above 10 using saturated sodium hydroxide solution. Dichloromethane (20.0 mL × 4) was used to extract the reaction mixture. The combined organic phase was washed with saturated sodium chloride solution (20.0 mL × 4), dried over anhydrous sodium sulfate, filtered, and concentrated under reduced pressure to obtain crude compound **15-4,** which can be used directly in the next step of the reaction. ¹H NMR (400 MHz, CDCl₃) *δ* 4.08-4.16 (m, 1H), 3.55-3.62 (m, 1H), 3.48-3.54 (m, 1H), 3.29-3.36 (m, 1H), 2.88-2.95 (m, 3H), 2.82-2.87 (m, 1H), 2.05-2.13 (m, 1H), 1.43-1.49 (m, 1H), 1.18 (s, 9H).
(4) Compound **15-4** (180 mg, 1.04 mmol) was dissolved in toluene (8.0 mL), and compound **C** (200 mg, 693 µmol), cesium carbonate (452 mg, 1.39 mmol), 1,1'-binaphthyl-2,2'-diphenylphosphine (86.3 mg, 138 µmol), and tris (dibenzylideneacetone) dipalladium (63.5 mg, 69.3 µmol) were added. The reaction mixture was stirred at 80°C under a nitrogen atmosphere for 3 hours. After the reaction, the mixture was diluted with water (10.0 mL) and extracted with ethyl acetate (30.0 mL × 2). The combined organic phase was washed with saturated sodium chloride solution (40.0 mL × 2), dried over anhydrous sodium sulfate, filtered, and concentrated under reduced pressure. The crude product was purified by silica gel column chromatography (petroleum ether/ethyl acetate = 1:0 to 3:1) to obtain compound **15-5.** MS-ESI [M+H]⁺, calculated: 426, found: 426. ¹H NMR (400 MHz, CDCl₃) *δ* 7.43 (m, 2H), 7.35-7.37 (t, *J* = 7.2 Hz, 2H), 7.29 (m, 1H), 6.99 (s, 1H), 5.5 (s, 2H), 4.34-4.46 (m, 2H), 4.03-4.10 (m, 1H), 3.74-3.82 (m, 1H), 3.51-3.65 (m, 2H), 2.35-2.45 (m, 1H), 1.90-1.98 (m, 1H), 1.26 (s, 9H).
(5) To a solution of compound **15-5** (57.0 mg, 134 µmol) and compound **B** (94.6 mg, 268 µmol) in dichloromethane (2.0 mL), sodium hydroxide (125 mg, 3.13 mmol), tetrabutylammonium bisulfate (22.7 mg, 67 µmol), and water (0.5 mL) were added. The reaction mixture was stirred at 25°C under a nitrogen atmosphere for 12 hours. The reaction was quenched with water (10.0 mL) and extracted with dichloromethane (20.0 mL × 2). The combined organic phase was washed with saturated sodium chloride solution (20.0 mL × 2), dried over anhydrous sodium sulfate, filtered, and concentrated under reduced pressure. The crude product was purified by silica gel column chromatography (petroleum ether/ethyl acetate = 1:0 to 1:1) to obtain compound **15-6.** MS-ESI [M+H]⁺, calculated: 698, found: 698.¹H NMR (400 MHz, CDCl₃) *δ* 8.47-8.51 (s, 2H), 7.42-7.47 (m, 2H), 7.34-7.39 (m, 2H), 7.29 (m, 2H), 5.52 (s, 2H), 4.29-4.39 (m, 2H), 4.23 (s, 2H), 3.86-3.97 (m, 5H), 3.73-3.81 (m, 3H), 3.53-3.66 (m, 3H), 3.47-3.52 (m, 1H), 2.20-2.31 (m, 1H), 1.97-2.03 (m, 1H), 1.21 (s, 9H).
(6) Compound **15-6** (35.0 mg, 50.1 µmol) was dissolved in tetrahydrofuran (2.0 mL), and 10% palladium on carbon (10.0 mg) was added to the solution. The reaction mixture was stirred at 25°C under a hydrogen atmosphere (15 psi) for 2 hours. Afterward, the mixture was filtered, and the filtrate was concentrated under reduced pressure. The crude product was purified by preparative high-performance liquid chromatography (C18-6, 100 mm × 30 mm, 5 µm, A: water with 0.225% formic acid; B: acetonitrile, 55%-75%, 8 minutes) to obtain compound **15** formate. MS-ESI [M+H]⁺, calculated: 608, found: 608. ¹H NMR (400 MHz, CDCl₃) *δ* 8.52 (s, 2H), 7.72 (s, 1H), 4.26-4.31 (m, 1H), 4.19-4.24 (m, 2H), 4.13-4.18 (m, 1H), 3.88-3.98 (m, 4H), 3.80-3.86 (m, 1H), 3.63-3.76 (m, 3H), 3.54-3.62 (m, 2H), 3.45-3.53 (m, 1H), 3.30-3.36 (m, 1H), 2.18-2.27 (m, 1H), 1.85-1.90 (m, 1H), 1.19 (s, 9H).

### Example 16 Synthesis of Compound 16

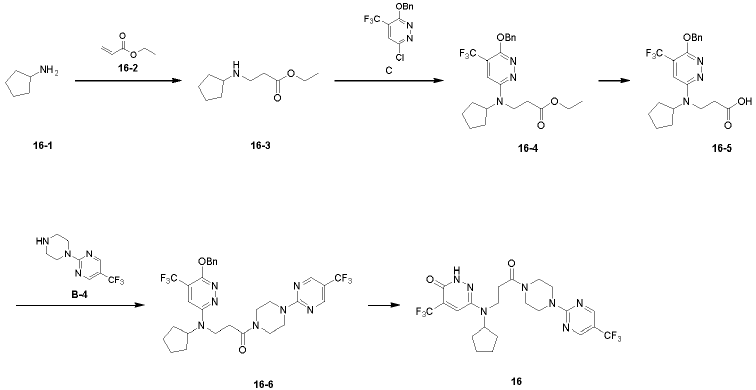

(1) Compound **16-1** (500 mg, 5.87 mmol, 579 µL) was dissolved in ethanol (10.0 mL) and then compound **16-2** (780 mg, 7.79 mmol, 846 µL) and copper oxide (467 mg, 5.87 mmol) were added to the solution. The reaction mixture was stirred at 25°C for 2 hours. Afterward, sodium hypochlorite solution (10.0 mL) was added to the reaction mixture. The mixture was then extracted with ethyl acetate (30.0 mL × 3). The organic phase was washed with water (30.0 mL × 1) and saturated sodium chloride solution (30.0 mL × 3), dried over anhydrous sodium sulfate, filtered, and concentrated under reduced pressure to obtain compound **16-3.** MS-ESI [M+H]⁺, calculated: 186, found: 186. ¹H NMR (400 MHz, CDCl₃) *δ* 4.13 (q, *J =* 7.2 Hz, 2H), 3.04-3.08 (m, 1H), 2.85 (t, *J* = 6.8 Hz, 2H), 2.50 (t, *J* = 6.8 Hz, 2H), 1.78-1.88 (m, 2H), 1.62-1.68 (m, 2H), 1.47-1.57 (m, 2H), 1.27-1.36 (m, 2H), 1.25 (t, *J=* 7.2 Hz, 3H).
(2) To the solution of compound **16-3** (154 mg, 831 µmol) in dioxane (8.0 mL), compound **C** (200 mg, 693 µmol), cesium carbonate (451 mg, 1.39 mmol), and (2-dicyclohexylphosphino-2',6'-diisopropoxy-1,1'-biphenyl)[(2'-amino-1,1'-biphenyl-2-yl)]palla dium (57.9 mg, 69.3 µmol) were added. The reaction mixture was stirred at 80°C under a nitrogen atmosphere for 12 hours. The reaction mixture was then diluted with ethyl acetate (30.0 mL) and washed with water (20.0 mL × 2) and saturated sodium chloride solution (30.0 mL × 1). The organic phase was dried over anhydrous sodium sulfate, filtered, and concentrated under reduced pressure. The crude product was purified by silica gel column chromatography (petroleum ether/ethyl acetate = 1 : 0 to 3 : 1) to obtain compound **16-4.** MS-ESI [M+H]⁺, calculated: 438, found: 438.
(3) To a solution of compound **16-4** (78.0 mg, 178 µmol) in tetrahydrofuran (2.0 mL), water (1.0 mL) and lithium hydroxide monohydrate (12.8 mg, 534 µmol) were added. The reaction mixture was stirred at 25°C for 12 hours. To the reaction mixture, 1 M hydrochloric acid solution was added to adjust the pH to 6. The mixture was then extracted with ethyl acetate (20.0 mL × 1). The organic phase was washed with water (20.0 mL × 2) and saturated sodium chloride solution (20.0 mL × 1), followed by drying over anhydrous sodium sulfate, filtration, and concentration under reduced pressure to obtain compound **16-5.** MS-ESI [M+H]⁺, calculated: 410, found: 410.
(4) To a solution of intermediate **16-5** (70.0 mg, 170 µmol) in dichloromethane (5.0 mL), compound **B-4** trifluoroacetate (47.6 mg, 205 µmol), diisopropylethylamine (66.3 mg, 513 µmol, 89.3 µL), and O-(7-Azabenzotriazol-1-yl)-N,N,N',N'-tetramethyluronium hexafluorophosphate (78.0 mg, 205 µmol) were added. The reaction mixture was stirred at 25°C under a nitrogen atmosphere for 12 hours. To the reaction mixture, dichloromethane (15.0 mL) was added, and the resulting mixture was washed with water (20.0 mL × 1) and saturated sodium chloride solution (20.0 mL × 3). Subsequently, it was dried over anhydrous sodium sulfate, filtered, and concentrated under reduced pressure. The crude product was separated by preparative thin-layer chromatography (dichloromethane/methanol = 1:0 to 10:1) to yield compound **16-6.** MS-ESI [M+H]⁺, calculated: 624, found: 624.
(5) To a solution of compound **16-6** (40.0 mg, 64.1 µmol) in tetrahydrofuran (5.0 mL), 10% palladium on carbon (10 mg) was added. The reaction mixture was stirred at 25°C under a hydrogen atmosphere for 12 hours. After filtration, the filtrate was concentrated under reduced pressure. The crude product was separated using preparative high-performance liquid chromatography (C18-6, 100 mm × 30 mm, 5 µm, A: water with 0.225% formic acid; B: acetonitrile, 54% to 74% over 8 minutes) to obtain compound **16** formate. MS-ESI [M+H]⁺, calculated: 534, found: 534.¹H NMR (400 MHz, MeOD) *δ* 8.60 (s, 2H), 7.82 (s, 1H), 4.22-4.27 (m, 1H), 3.92-3.98 (m, 4H), 3.67-3.69 (m, 4H), 3.60-3.62 (m, 2H), 2.71-2.76 (m, 2H), 1.94-1.95 (m, 2H), 1.78-1.80 (m, 2H), 1.64-1.66 (m, 4H).

### Example 17 Synthesis of Compound 17

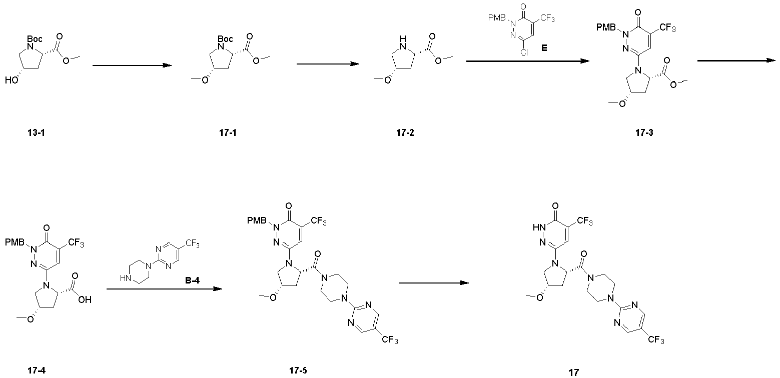

(1) At 0°C, sodium hydride (1.63 g, 40.7 mmol) was added to a solution of compound **13-1** (5.00 g, 20.3 mmol) in tetrahydrofuran (50.0 mL). The mixture was stirred for 30 minutes, and then methyl iodide (14.4 g, 101 mmol) was added and reacted at 25°C for 2.5 hours. To the reaction mixture, hydrochloric acid (50.0 mL, 0.5 M) was added, followed by extraction with ethyl acetate (100 mL × 2). The organic phase was washed with saturated sodium chloride solution (100 mL), dried over anhydrous sodium sulfate, filtered, and concentrated under reduced pressure. The crude product was purified by silica gel column chromatography (petroleum ether/ethyl acetate = 1:0 to 0:1) to obtain compound **17-1.** MS-ESI [M-Boc+H]⁺, calculated: 160, found: 160. ¹H NMR (400 MHz, CDCl₃) *δ* 4.24-4.45 (m, 1H), 3.88-4.03 (m, 1H), 3.70-3.75 (m, 3H), 3.42-3.68 (m, 2H), 3.24-3.34 (m, 3H), 2.05-2.40 (m, 2H), 1.39-1.48 (m, 9H).
(2) Compound **17-1** (2.00 g, 7.71 mmol) was dissolved in dichloromethane (20.0 mL), and trifluoroacetic acid (9.24 g, 81.0 mmol) was added. The reaction was carried out at 25°C for 2 hours. The pH was adjusted to greater than 8 by adding saturated sodium bicarbonate solution. Then, water (20.0 mL) was added, and the mixture was extracted with dichloromethane (30.0 mL × 2). After drying over anhydrous sodium sulfate, the mixture was filtered, and the solvent was removed under reduced pressure to obtain compound **17-2.** MS-ESI [M+H]⁺, calculated: 160, found: 160.
(3) To the mixture of compound **17-2** (200 mg, 1.26 mmol), compound **E** (480 mg, 1.51 mmol), cesium carbonate (1.23 g, 3.77 mmol), tris (dibenzylideneacetone) dipalladium (115 mg, 125 µmol), and 1,1'-binaphthyl-2,2'-diphenylphosphine (156 mg, 251 µmol), toluene (5.00 mL) was added. And the reaction mixture was stirred at 80°C under a nitrogen atmosphere for 3 hours. Water (10.0 mL) was added, and the mixture was extracted with ethyl acetate (20.0 mL × 2). The organic phase was washed with saturated sodium chloride solution (10.0 mL), dried over anhydrous sodium sulfate, filtered, and concentrated under reduced pressure. The crude product was purified by silica gel column chromatography (petroleum ether/ethyl acetate = 2: 1) to obtain compound **17-3.** MS-ESI [M+H]⁺, calculated: 442, found: 442.
(4) Compound **17-3** (40.0 mg, 90.6 µmol) was dissolved in tetrahydrofuran (3.0 mL) and water (1.0 mL). Then, lithium hydroxide monohydrate (22.8 mg, 543 µmol) was added, and the mixture was stirred at 25°C for 2 hours. The pH of the reaction mixture was adjusted to less than 7 by adding hydrochloric acid. Water (20.0 mL) was added, and the mixture was extracted with ethyl acetate (30.0 mL × 2). The organic phase was dried over anhydrous sodium sulfate, filtered, and separated to obtain compound **17-4.** MS-ESI [M+H]⁺, calculated: 428, found: 428.
(5) To a solution of intermediate **17-4** (35.0 mg, 81.9 µmol) in dichloromethane (2.00 mL), compound **B-4** trifluoroacetate (38.0 mg, 163 µmol), diisopropylethylamine (52.9 mg, 409 µmol), and propylphosphonic anhydride solution (208 mg, 327 µmol, purity 50% in ethyl acetate) were added. The reaction mixture was stirred at 25°C under a nitrogen atmosphere for 2 hours. It was then extracted with dichloromethane (20.0 mL × 2) and water (10.0 mL). The combined organic phase was washed with saturated sodium chloride solution (10.0 mL), dried over anhydrous sodium sulfate, filtered, and concentrated under reduced pressure to obtain compound **17-5.** MS-ESI [M+H]⁺, calculated: 642, found: 642.
(6) To a solution of compound **17-5** (30.0 mg, 46.7 µmol) in trifluoroacetic acid (1.00 mL), trifluoromethanesulfonic acid (170 mg, 1.13 mmol) was added. The mixture was stirred at 25°C for 1 hour. The pH was adjusted to greater than 8 by adding saturated sodium bicarbonate solution, and the mixture was extracted with ethyl acetate (10.0 mL × 2). The organic phase was washed with saturated sodium chloride solution (10.0 mL), concentrated under reduced pressure. The crude product was purified by preparative high-performance liquid chromatography (C18-6, 100 mm × 30 mm, 5 µm; A: water (0.225% formic acid); B: acetonitrile, 10%-65%, 15 minutes) to obtain compound **17** formate. MS-ESI [M+H]⁺, calculated: 522, found: 522. ¹H NMR (400 MHz, MeOD) *δ* 8.62 (s, 2H), 7.59 (s, 1H), 4.05-4.22 (m, 4H), 3.90-4.02 (m, 2H), 3.78-3.84 (m, 3H), 3.65-3.73 (m, 2H), 3.57 (br dd, *J =* 8.4, 4.4 Hz, 1H), 3.37 (s, 3H), 2.45-2.55 (m, 1H), 2.12 (ddd, *J =* 13.2, 7.2, 5.6 Hz, 1H).

### Example 18 Synthesis of Compound 18

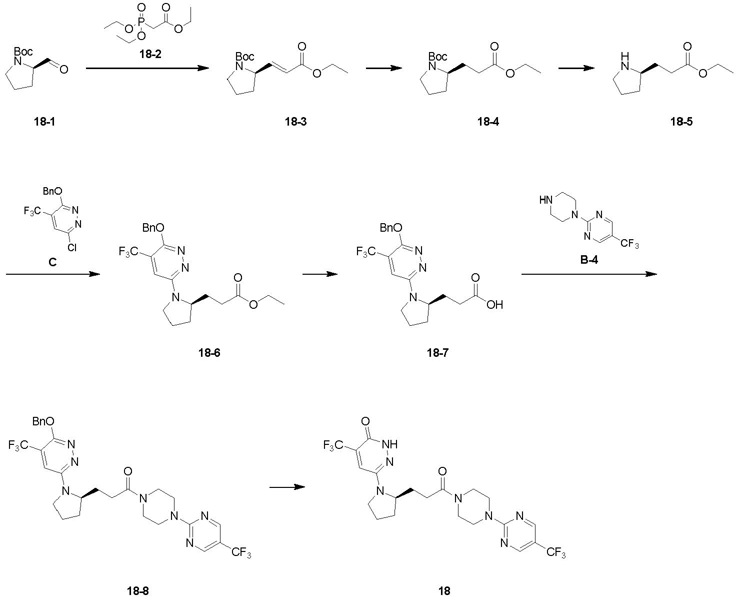

(1) At 0°C, to a solution of sodium hydride (261 mg, 6.52 mmol, 60%) in tetrahydrofuran (20.0 mL), compound **18-2** (1.35 g, 6.02 mmol, 1.19 mL) was added. The reaction mixture was stirred at 0°C for 0.5 hours. Then, a solution of compound **18-1** (1.00 g, 5.02 mmol) in tetrahydrofuran (10.0 mL) was added. The reaction mixture was stirred for 2.5 hours under a nitrogen atmosphere at 0°C. To the reaction mixture, ethyl acetate was added (50.0 mL). The pH was adjusted to 7 using saturated ammonium chloride solution. After phase separation, the organic phase was washed with water (50.0 mL × 1) and saturated sodium chloride solution (40.0 mL × 3), dried over anhydrous sodium sulfate, filtered, and concentrated under reduced pressure. The crude product was purified by silica gel column chromatography (petroleum ether/ethyl acetate = 1:0 to 10:1) to obtain Compound **18-3.** MS-ESI [M+H]⁺, calculated: 270, found: 270.
(2) To a solution of compound **18-3** (1.50 g, 5.57 mmol) in tetrahydrofuran (10.0 mL), 10% palladium on carbon (0.20 g) was added. The reaction mixture was stirred at 25°C under a hydrogen atmosphere for 2 hours. Afterward, the reaction mixture was filtered, and the filtrate was then concentrated under reduced pressure to obtain compound **18-4.** MS-ESI [M+H]⁺, calculated: 272, found: 272.¹H NMR (400 MHz, CDCl₃) *δ* 4.12 (q, *J =* 7.2 Hz, 2H), 3.73-3.86 (m, 1H), 3.27-3.47 (m, 2H), 2.25-2.41 (m, 2H), 1.83-2.02 (m, 4H), 1.66-1.73 (m, 2H), 1.46-1.52 (m, 9H), 1.24-1.28 (m, 4H).
(3) To a solution of compound **18-4** (1.50 g, 5.53 mmol) in dichloromethane (15.0 mL), trifluoroacetic acid (630 mg, 5.53 mmol, 409 µL) was added. The reaction mixture was stirred at 25°C for 0.5 hours. Then, dichloromethane (20.0 mL) was added, and the pH was adjusted to 7 with saturated sodium bicarbonate solution. After phase separation, the organic phase was washed with water (20.0 mL × 2) and saturated sodium chloride solution (20.0 mL × 1), dried over anhydrous sodium sulfate, filtered, and concentrated under reduced pressure to obtain compound **18-5.**
(4) To a solution of compound **18-5** (356 mg, 2.08 mmol) in toluene (6.0 mL), compound **C** (300 mg, 1.04 mmol), cesium carbonate (677 mg, 2.08 mmol), 1,1'-binaphthyl-2,2'-diphenylphosphine (324 mg, 520 µmol), and bis (dibenzylideneacetone) palladium (149 mg, 260 µmol) were added. The reaction mixture was stirred at 80°C under a nitrogen atmosphere for 3 hours. The reaction mixture was diluted with ethyl acetate (60.0 mL), and washed with water (50.0 mL × 1) and saturated sodium chloride solution (30.0 mL × 3). The organic phase was dried over anhydrous sodium sulfate, filtered, and concentrated under reduced pressure. The crude product was purified by silica gel column chromatography (petroleum ether/ethyl acetate = 1:0 to 12:1) to obtain compound **18-6.** MS-ESI [M+H]⁺, calculated: 424, found: 424. ¹H NMR (400 MHz, CDCl₃) *δ* 7.48 (d, *J=* 7.6 Hz, 2H), 7.38 (t, *J=* 7.6 Hz, 2H), 7.32 (d, *J =* 7.2 Hz, 1H), 7.21 (s, 1H), 5.57 (s, 2H), 4.14 (q, *J =* 7.2 Hz, 3H), 3.66 (t, *J=* 7.2 Hz, 1H), 3.38-3.48 (m, 1H), 2.40 (t, *J=* 7.2 Hz, 2H), 2.02-2.14 (m, 4H), 1.84-1.92 (m, 1H), 1.67-1.74 (m, 1H), 1.24-1.28 (m, 3H).
(5) To a solution of compound **18-6** (290 mg, 685 µmol) in ethanol (2.0 mL), water (1.0 mL) and lithium hydroxide monohydrate (172 mg, 4.11 mmol) were added. The reaction mixture was stirred at 25°C for 2 hours. The pH was adjusted to 7 by adding hydrochloric acid solution (1 M). The mixture was extracted with dichloromethane (25.0 mL × 1). The organic phase was washed with water (20.0 mL × 2) and saturated sodium chloride solution (20.0 mL × 1), dried over anhydrous sodium sulfate, filtered, and concentrated under reduced pressure to obtain compound **18-7.** MS-ESI [M+H]⁺, calculated: 396, found: 396.¹H NMR (400 MHz, CDCl₃) *δ* 7.48 (d, *J =* 7.2 Hz, 2H), 7.36-7.42 (m, 2H), 7.27-7.35 (m, 2H), 5.51-5.59 (m, 2H), 4.25-4.35 (m, 1H), 3.73 (d, *J =* 6.8 Hz, 1H), 3.38-3.49 (m, 1H), 2.49 (t, *J =* 6.8 Hz, 2H), 2.07-2.18 (m, 4H), 1.81-1.95 (m, 2H).
(6) To a solution of intermediate **18-7** (230 mg, 582 µmol) in dichloromethane (5.0 mL), compound **B-4** trifluoroacetate (242 mg, 698 µmol), diisopropylethylamine (376 mg, 2.91 mmol, 507 µL), and propylphosphonic anhydride (1.48 g, 2.33 mmol, with purity of 50% in ethyl acetate) were added. The reaction mixture was stirred at 25°C under a nitrogen atmosphere for 2 hours. The reaction mixture was diluted with dichloromethane (60.0 mL) and washed with water (50.0 mL × 1) and saturated sodium chloride solution (30.0 mL × 3). Subsequently, the organic phase was dried over anhydrous sodium sulfate, filtered, and concentrated under reduced pressure. The crude product was purified by silica gel column chromatography (elution gradient: dichloromethane/methanol = 1:0 to 8:1) to obtain compound **18-8.** MS-ESI [M+H]⁺, calculated: 610, found: 610.
(7) To a solution of compound **18-8** (300 mg, 492 µmol) in tetrahydrofuran (3.0 mL), 10% palladium on carbon (300 mg) was added. The reaction mixture was stirred at 25°C under a hydrogen atmosphere for 5 hours. The mixture was filtered, and the filtrate was concentrated under reduced pressure. The crude product was purified using high-performance liquid chromatography (Xtimate C18, 150 mm × 40 mm, 10 µm, A: water (10 mmol/L ammonium bicarbonate) and B: acetonitrile, 40% to 70% over 10 minutes) to obtain compound **18.** MS-ESI [M+H]⁺, calculated: 520, found: 520. ¹H NMR (400 MHz, DMSO-*d6*) *δ* 12.54 (s, 1H), 8.73 (s, 2H), 7.98 (s, 1H), 3.87 (d, *J* = 4.0 Hz, 3H), 3.79-3.84 (m, 2H), 3.56 (d, *J* = 4.4 Hz, 4H), 3.41-3.45 (m, 1H), 3.21 (d, *J=* 9.2 Hz, 1H), 2.41 (t, *J=* 6.8 Hz, 2H), 1.88-1.99 (m, 3H), 1.82 (d, *J* = 7.2 Hz, 2H), 1.45-1.53 (m, 1H).

### Example 19 Synthesis of Compound 19

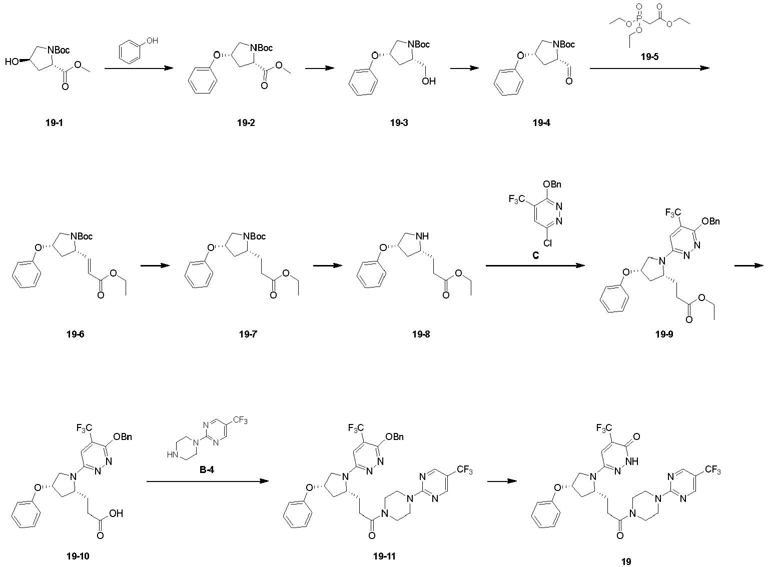

(1) To a solution of compound **19-1** (5.00 g, 20.3 mmol) in tetrahydrofuran (50.0 mL) at 0°C, phenol (2.11 g, 22.4 mmol), triphenylphosphine (5.88 g, 22.4 mmol), and diisopropyl azodicarboxylate (4.53 g, 22.4 mmol, 4.36 mL) were added. The reaction mixture was stirred at 25°C under a nitrogen atmosphere for 12 hours. Water (30.0 mL) was added to the reaction mixture, and it was extracted with ethyl acetate (100 mL × 2). The organic phase was washed with water (50.0 mL × 1) and saturated sodium chloride (100 mL × 2), dried over anhydrous sodium sulfate, filtered, and concentrated under reduced pressure. The crude product was purified by silica gel column chromatography (petroleum ether/ethyl acetate = 1:0 to 5:1) to obtain compound **19-2.** MS-ESI [M-^{t}Bu+H]⁺, calculated: 266, found: 266. ¹H NMR (400 MHz, CDCl₃) *δ* 7.26 (s, 2H), 6.92-7.00 (m, 1H), 6.76-6.83 (m, 2H), 4.85-4.95 (m, 1H), 4.38-4.58 (m, 1H), 3.75-3.84 (m, 1H), 3.68 (s, 4H), 2.38-2.51 (m, 2H), 1.45 (d, *J=* 17.6 Hz, 9H).
(2) To a solution of compound **19-2** (2.48 g, 7.72 mmol) in tetrahydrofuran (30.0 mL), lithium aluminum hydride (322 mg, 8.49 mmol) was added. The reaction mixture was stirred for 0.5 hours under a nitrogen atmosphere at 0°C. Water (2.0 mL) and 15% sodium hydroxide aqueous solution (0.5 mL) were added, and the mixture was then filtered. The filtrate was extracted with ethyl acetate (40 mL × 2). The combined organic phase was washed with saturated sodium chloride (40 mL × 2), dried over anhydrous sodium sulfate, filtered, and concentrated under reduced pressure to obtain compound **19-3.** MS-ESI [M+H]⁺, calculated: 238, found: 238.¹H NMR (400 MHz, CDCl₃) *δ* 7.29 (t, *J =* 8.0 Hz, 2H), 6.94-7.00 (m, 1H), 6.82-6.88 (m, 2H), 4.85 (s, 1H), 4.12-4.21 (m, 1H), 3.92 (t, *J =* 9.6 Hz, 1H), 3.57-3.75 (m, 3H), 2.32-2.41 (m, 1H), 1.90-2.03 (m, 1H), 1.45-1.49 (m, 9H).
(3) To a solution of oxalyl chloride (237 mg, 1.87 mmol, 164 µL) in dichloromethane (5.0 mL), dimethyl sulfoxide (266 mg, 3.41 mmol, 266 µL) was added. The reaction mixture was stirred at -78°C under a nitrogen atmosphere for 0.5 hours. A solution of compound **19-3** (500 mg, 1.70 mmol) in dichloromethane (5.0 mL) was added to the reaction mixture and continued to be stirred at -78°C for 1 hour. To the reaction mixture, triethylamine (431 mg, 4.26 mmol, 593 µL) was added, and water (30.0 mL) was then added. The mixture was extracted with dichloromethane (40.0 mL × 2). The organic phase was washed with water (20.0 mL × 2) and saturated sodium chloride (20.0 mL × 1), dried over anhydrous sodium sulfate, filtered, and concentrated under reduced pressure to obtain compound **19-4.**
(4) To a solution of sodium hydride (41.0 mg, 1.03 mmol, 60%) in tetrahydrofuran (2.0 mL) at 0°C, compound **19-5** (212 mg, 947 µmol, 187 µL) was added. The reaction mixture was stirred at 0°C for 1 hour. Then, a solution of compound **19-4** (230 mg, 789 µmol) in tetrahydrofuran (3.0 mL) was added to the reaction mixture. The reaction mixture was stirred at 0°C under a nitrogen atmosphere for 1 hour. To the reaction mixture, ethyl acetate (40.0 mL) was added. The pH was adjusted to 7 using saturated ammonium chloride solution. After separation, the organic phase was washed with water (20.0 mL × 1) and saturated sodium chloride (20.0 mL × 3), dried over anhydrous sodium sulfate, filtered, and concentrated under reduced pressure. The crude product was purified by silica gel column chromatography (petroleum ether/ethyl acetate = 1:0 to 3:1) to obtain compound **19-6.** MS-ESI [M-Boc+H]⁺, calculated: 262, found: 262.
(5) 10% palladium on carbon (0.05 g) was added to a solution of compound **19-6** (180 mg, 498 µmol) in 3.0 mL of tetrahydrofuran. The reaction mixture was stirred at 25°C under a hydrogen atmosphere for 1 hour. Afterward, it was filtered and the filtrate was concentrated under reduced pressure to obtain compound **19-7.** MS-ESI [M+H]⁺, calculated: 364, found: 364. ¹H NMR (400 MHz, CDCl₃) *δ* 7.26 (s, 2H), 6.92-7.01 (m, 1H), 6.85 (d, *J* = 8.0 Hz, 2H), 4.84-4.91 (m, 1H), 4.06-4.16 (m, 2H), 3.72-4.04 (m, 2H), 3.49-3.57 (m, 1H), 2.24-2.39 (m, 3H), 2.23 (s, 1H), 1.88-2.06 (m, 2H), 1.46 (s, 9H), 1.21-1.27 (m, 3H)
(6) 500 µL of hydrochloric acid (4 M in dioxane) was added to a solution of compound **19-7** (145 mg, 398 µmol) in dichloromethane (2.0 mL). The reaction mixture was stirred at 25°C for 0.5 hours. Then, dichloromethane (20.0 mL) was added and the pH was adjusted to 7 with saturated sodium bicarbonate solution. After phase separation, the organic phase was washed with water (20.0 mL × 2) and saturated sodium chloride (20.0 mL × 1), dried over anhydrous sodium sulfate, filtered, and concentrated under reduced pressure to obtain compound **19-8.**
(7) Compound C (75.4 mg, 261 µmol), cesium carbonate (212 mg, 653 µmol), 1,1'-binaphthyl-2,2'-diphenylphosphine (40.6 mg, 65.3 µmol), and bis (dibenzylideneacetone) palladium (29.0 mg, 32.6 µmol) were added to a solution of compound **19-8** (86.0 mg, 326 µmol) in 5.0 mL of toluene. The reaction mixture was stirred at 80°C for 3 hours under a nitrogen atmosphere. The reaction mixture was diluted with dichloromethane (30.0 mL) and washed with water (70.0 mL × 1) and saturated sodium chloride (30.0 mL × 3). The organic phase was dried over anhydrous sodium sulfate, filtered, and concentrated under reduced pressure. The crude product was purified by silica gel column chromatography (petroleum ether/ethyl acetate = 1:0 to 0:1) to obtain compound **19-9.** MS-ESI [M+H]⁺, calculated: 516, found: 516.
(8) To a solution of compound **19-9** (65.0 mg, 126.0 µmol) in ethanol (3.0 mL), water (1.0 mL) and lithium hydroxide monohydrate (31.7 mg, 756 µmol) were added. The reaction mixture was stirred at 25°C for 2 hours. The pH was adjusted to 7 by adding hydrochloric acid (1 M). The mixture was then extracted with dichloromethane (25.0 mL × 1). The organic phase was washed with water (20.0 mL × 2) and saturated sodium chloride (20.0 mL × 1), dried over anhydrous sodium sulfate, filtered, and concentrated under reduced pressure to obtain compound **19-10.** MS-ESI [M-H]⁻, calculated: 486, found: 486. ¹H NMR (400 MHz, CDCl₃) *δ* 7.47 (d, *J=* 7.2 Hz, 2H), 7.32-7.41 (m, 4H), 7.28-7.32 (m, 2H), 7.00 (t, *J=* 7.2 Hz, 1H), 6.89 (d, *J =* 8.0 Hz, 2H), 5.51-5.59 (m, 2H), 5.12 (s, 1H), 4.34 (d, *J =* 5.6 Hz, 1H), 3.84-4.01 (m, 2H), 2.26-2.32 (m, 2H), 2.02-2.11 (m, 2H), 1.27-1.32 (m, 2H).
(9) To a solution of intermediate **19-10** (50.0 mg, 102 µmol) in dichloromethane (2.0 mL), compound **B-4** trifluoroacetate (52.4 mg, 151 µmol), diisopropylethylamine (66.2 mg, 512 µmol, 89.3 µL), and propylphosphonic anhydride solution (261 mg, 410 µmol, 244 µL, with purity of 50% in ethyl acetate) were added. The reaction mixture was stirred at 25°C under a nitrogen atmosphere for 1 hour. It was then diluted with dichloromethane (30.0 mL), and washed with water (20.0 mL × 1) and saturated sodium chloride solution (30.0 mL × 3). The organic phase was dried over anhydrous sodium sulfate, filtered, concentrated under reduced pressure. The crude product was purified by silica gel column chromatography (dichloromethane/methanol = 1:0 to 0:1) to obtain compound **19-11.** MS-ESI [M+H]⁺, calculated: 702, found: 702.
(10) To a solution of compound **19-11** (36.0 mg, 51.3 µmol) in tetrahydrofuran (2.0 mL), 10% palladium on carbon (20 mg) was added. The reaction mixture was stirred at 25°C under a hydrogen atmosphere for 2 hours. Afterward, the mixture was filtered, and the filtrate was concentrated under reduced pressure. The crude product was purified using preparative high-performance liquid chromatography (Phenomenex C18, 75 mm × 30 mm, 3 µm, A: water (10 mmol/L ammonium bicarbonate) and B: acetonitrile, 36%-66% over 12 minutes) to obtain compound **19.** MS-ESI [M+H]⁺, calculated: 612, found: 612. ¹H NMR (400 MHz, MeOD) *δ* 8.59 (s, 2H), 7.93 (s, 1H), 7.22-7.34 (m, 2H), 6.88-6.98 (m, 3H), 5.13 (t, *J =* 4.8 Hz, 1H), 4.07-4.17 (m, 1H), 3.85-3.95 (m, 4H), 3.72-3.81 (m, 2H), 3.61-3.70 (m, 2H), 3.50-3.60 (m, 2H), 2.46-2.56 (m, 2H), 2.37-2.45 (m, 1H), 2.26 (d, *J=* 14.0 Hz, 1H), 2.10-2.19 (m, 1H), 1.98-2.08 (m, 1H).

### Example 20 Synthesis of Compound 20

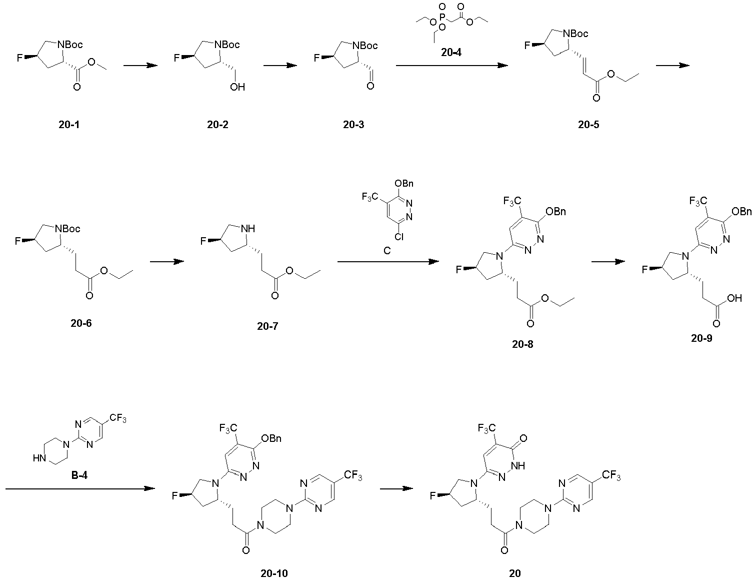

(1) To a solution of compound **20-1** (5.00 g, 20.2 mmol) in tetrahydrofuran (50.0 mL), tetrahydroaluminate lithium (844 mg, 22.2 mmol) was added. The reaction mixture was stirred for 0.5 hours under a nitrogen atmosphere at 0°C. Then, water (4.0 mL) and 15% sodium hydroxide solution (0.5 mL) were added. After filtration, the filtrate was extracted with ethyl acetate (40 mL × 2). The combined organic phase was washed with saturated sodium chloride solution (40 mL × 2), dried over anhydrous sodium sulfate, filtered, and concentrated under reduced pressure to obtain compound **20-2.**
(2) To a solution of compound **20-2** (500 mg, 2.28 mmol) in dichloromethane (10.0 mL), Dess-Martin reagent (967 mg, 2.28 mmol) was add. The reaction mixture was stirred under a nitrogen atmosphere at 25°C for 1 hour. The reaction was quenched by sodium thiosulfate solution (10.0 mL), then 30.0 mL of water was added. The mixture was extracted with dichloromethane (20.0 mL × 2). The organic phase was washed with water (20.0 mL × 2) and saturated sodium chloride solution (20.0 mL × 1), dried over anhydrous sodium sulfate, filtered, and concentrated under reduced pressure. The crude product was purified by silica gel column chromatography (petroleum ether/ethyl acetate = 1:0 to 3:1) to obtain compound **20-3.** ¹H NMR (400 MHz, CDCl₃) *δ* 9.43-9.58 (m, 1H), 5.15-5.31 (m, 1H), 4.26-4.40 (m, 1H), 3.90-3.97 (m, 1H), 3.49-3.67 (m, 1H), 2.40-2.48 (m, 1H), 1.93-2.09 (m, 1H), 1.42-1.49 (m, 9H).
(3) To a solution of sodium hydride (59.8 mg, 1.50 mmol, 60%) in tetrahydrofuran (8.0 mL) at 0°C, compound **20-4** (309 mg, 1.38 mmol, 273 µL) was added, and the reaction mixture was stirred for 1 hour at 0°C. Subsequently, compound **20-3** (250 mg, 1.15 mmol) was added, and the reaction was stirred for an additional hour at 0°C under a nitrogen atmosphere. To the reaction mixture, ethyl acetate (40.0 mL) was added, and the pH was adjusted to 7 with saturated ammonium chloride solution. After phase separation, the organic phase was washed with water (20.0 mL × 1) and saturated sodium chloride (20.0 mL × 3). The organic phase was dried over anhydrous sodium sulfate, filtered, and concentrated under reduced pressure. The crude product was purified by silica gel column chromatography (petroleum ether/ethyl acetate = 1:0 to 6:1) to yield compound **20-5.** MS-ESI [M-Boc+H]⁺, calculated: 188, found: 188.
(4) To a solution of compound **20-5** (260 mg, 904 µmol) in tetrahydrofuran (8.0 mL), 10% palladium on carbon (0.06 g) was added. The reaction mixture was stirred at 25°C under a hydrogen atmosphere for 12 hours. Afterward, it was filtered, and the reaction solution was concentrated under reduced pressure to obtain compound **20-6.** MS-ESI [M-Boc+H]⁺, calculated: 190, found: 190. ¹H NMR (400 MHz, CDCl₃) *δ* 5.04-5.17 (m, 1H), 4.12 (q, *J =* 7.2 Hz, 2H), 3.75-4.05 (m, 2H), 3.25-3.42 (m, 1H), 2.10-2.46 (m, 4H), 1.67-1.87 (m, 2H), 1.47 (s, 9H), 1.25 (t, *J* = 7.2 Hz, 3H).
(5) To a solution of compound **20-6** (180 mg, 622 µmol) in dichloromethane (4.0 mL), trifluoroacetic acid (1.54 g, 13.5 mmol, 1 mL) was added. The reaction mixture was stirred at 25°C for 1 hour. To this reaction mixture, dichloromethane (20.0 mL) was added, and the pH was adjusted to 7 with saturated sodium bicarbonate solution. After separation, the organic phase was washed with water (20.0 mL × 2) and saturated sodium chloride (20.0 mL × 1), dried over anhydrous sodium sulfate, filtered, and concentrated under reduced pressure to obtain compound **20-7.**
(6) To a solution of compound **20-7** (36.4 mg, 192 µmol) in dioxane (4.0 mL), compound **C** (50.0 mg, 173 µmol), cesium carbonate (188 mg, 577 µmol), 1,1'-binaphthyl-2,2'-diphenylphosphine (23.9 mg, 38.4 µmol), and bis (dibenzylideneacetone) palladium (17.6 mg, 19.2 µmol) were added under a nitrogen atmosphere. The reaction mixture was stirred at 80°C for 3 hours. To this reaction mixture, ethyl acetate (30.0 mL) was added, and it was washed with water (70.0 mL × 1) and saturated sodium chloride (30.0 mL × 3). The organic phase was dried over anhydrous sodium sulfate, filtered, and concentrated under reduced pressure. The crude product was purified by silica gel column chromatography (petroleum ether/ethyl acetate = 1:0 to 2:1) to obtain compound **20-8.** MS-ESI [M+H]⁺, calculated: 442, found: 442.
(7) To a solution of compound **20-8** (77.0 mg, 174 µmol) in ethanol (3.0 mL), water (1.0 mL) and lithium hydroxide monohydrate (21.9 mg, 523 µmol) were added. The reaction mixture was stirred at 25°C for 2 hours. To this reaction mixture, hydrochloric acid (1 M) was added to adjust the pH to 7, and it was extracted with dichloromethane (25.0 mL × 1). The organic phase was washed with water (20.0 mL × 2) and saturated sodium chloride solution (20.0 mL × 1). Subsequently, it was dried over anhydrous sodium sulfate, filtered, and concentrated under reduced pressure to obtain compound **20-9.** MS-ESI [M+H]⁺, calculated: 414, found: 414.
(8) To a solution of intermediate **20-9** (70.0 mg, 169 µmol) in dichloromethane (10.0 mL), compound **B-4** trifluoroacetate (47.2 mg, 203 µmol), diisopropylethylamine (65.6 mg, 508 µmol, 88.4 µL), and propylphosphonic anhydride solution (323 mg, 508 µmol, 302 µL, with 50% purity in ethyl acetate) were added. The reaction mixture was stirred at 25°C under a nitrogen atmosphere for 1 hour. To this reaction mixture, dichloromethane (30.0 mL) was added. It was washed with water (20.0 mL × 1) and saturated sodium chloride solution (30.0 mL × 3). Subsequently, the organic phase was dried over anhydrous sodium sulfate, filtered, and concentrated under reduced pressure to obtain compound **20-10.** MS-ESI [M+H]⁺, calculated: 628, found: 628.
(9) To a solution of compound **20-10** (60.0 mg, 95.6 µmol) in tetrahydrofuran (10.0 mL), 10% palladium on carbon (30 mg) was added. The reaction mixture was stirred at 25°C under a hydrogen atmosphere for 12 hours. After filtration, the reaction mixture was concentrated under reduced pressure. The crude product was then purified by preparative high-performance liquid chromatography (Xtimate C18, 100 mm × 30 mm, 10 µm, A: water (0.225% formic acid); B: acetonitrile, 50%-80% over 10 minutes) to obtain compound **20** formate. MS-ESI [M+H]⁺, calculated: 538, found: 538. ¹H NMR (400 MHz, MeOD) *δ* 8.60 (s, 2H), 7.79 (s, 1H), 5.23-5.37 (m, 1H), 4.15-4.20 (m,1H), 3.90-3.98 (m,4H), 3.66-3.68 (m,1H), 3.62-3.66 (m, 5H), 3.46-3.52 (m, 3H), 3.16-3.21 (m, 2H), 1.75-1.81 (m,1H).

### Example 21 Synthesis of Compound 21

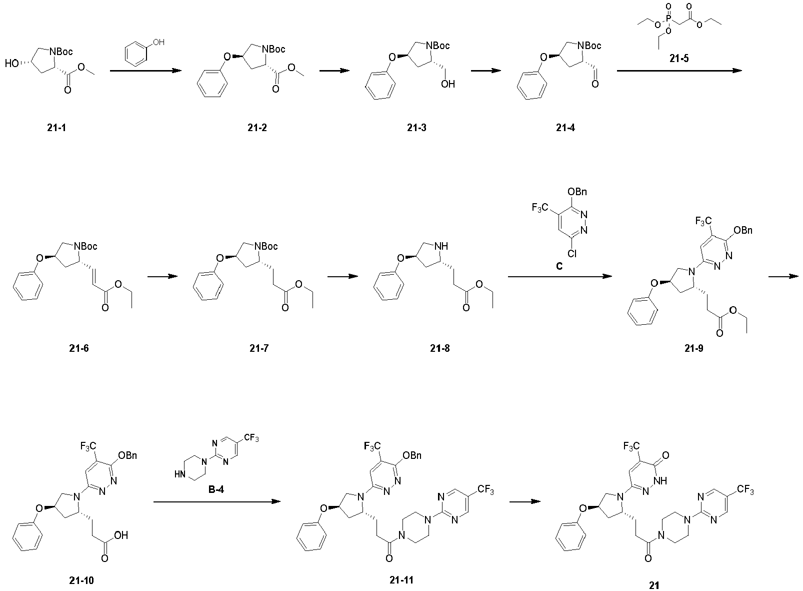

(1) At 0°C, phenol (2.11 g, 22.4 mmol, 1.97 mL), triphenylphosphine (5.88 g, 22.4 mmol), and diisopropyl azodicarboxylate (4.53 g, 22.4 mmol, 4.36 mL) were added to a solution of compound **21-1** (5.00 g, 20.3 mmol) in tetrahydrofuran (50.0 mL). The reaction mixture was stirred at 25°C for 12 hours under a nitrogen atmosphere. Water (30.0 mL) was then added, and the mixture was extracted with ethyl acetate (100 mL × 2). The organic phase was washed with water (50.0 mL × 1) and saturated sodium chloride (100 mL × 2), dried over anhydrous sodium sulfate, filtered, and concentrated under reduced pressure. The crude product was purified by silica gel column chromatography (petroleum ether/ethyl acetate = 1:0 to 5:1) to obtain compound **21-2.** MS-ESI [M-Boc+H]⁺, calculated: 222, found: 222. ¹H NMR (400 MHz, CDCl₃) *δ* 7.26-7.33 (m, 2H), 6.97-7.01 (m, 1H), 6.84-6.87 (m, 2H), 4.88-4.92 (m, 1H), 4.41-4.55 (m, 1H), 3.74-3.83 (m, 5H), 2.45-2.59 (m, 1H), 2.18-2.26 (m, 1H), 1.41-1.46 (m, 9H).
(2) To a solution of compound **21-2** (6.00 g, 18.6 mmol) in tetrahydrofuran (50.0 mL), lithium aluminum hydride (779 mg, 20.5 mmol) was added. The reaction mixture was stirred for 0.5 hours at 0°C under a nitrogen atmosphere. Water (2.0 mL) and a solution of sodium hydroxide (0.5 mL, 15%) were then added. The resulting mixture was filtered, and the filter cake was rinsed with ethyl acetate (40 mL × 2). The organic phase was washed with saturated sodium chloride (40 mL × 2), dried over anhydrous sodium sulfate, filtered and concentrated under reduced pressure to obtain compound **21-3.** MS-ESI [M-Boc+H]⁺, calculated: 238, found: 238. ¹H NMR (400 MHz, CDCl₃) *δ* 7.27-7.33 (m, 2H), 6.97-7.00 (m, 1H), 6.85-6.88 (m, 2H), 4.80 (s, 1H), 4.14-4.19 (m, 1H), 3.76-3.79 (m, 2H), 3.57-3.63 (m, 2H), 2.28-2.35 (m, 1H), 1.84-1.86 (m, 1H), 1.43-1.47 (m, 9H).
(3) To a solution of compound **21-3** (500 mg, 1.70 mmol) in dichloromethane (8.0 mL), Dess-Martin reagent (722 mg, 1.70 mmol) was added. The reaction mixture was stirred at 25°C for 1 hour under a nitrogen atmosphere. Sodium thiosulfate solution (10.0 mL) was added to quench the reaction. Subsequently, water (30.0 mL) was added, and the mixture was extracted with dichloromethane (20.0 mL × 2). The organic phase was then washed with water (20.0 mL × 2) and saturated sodium chloride (20.0 mL × 1), dried over anhydrous sodium sulfate, filtered and concentrated under reduced pressure to obtain compound **21-4.** MS-ESI [M-Boc+H]⁺, calculated: 192, found: 192.
(4) Compound **21-5** (323 mg, 1.44 mmol, 286 µL) was added into a solution of sodium hydride (62.4 mg, 1.56 mmol, 60%) in tetrahydrofuran (8.0 mL) at 0°C. The reaction mixture was stirred for 0.5 hours at 0°C. Compound **21-4** (350 mg, 1.20 mmol) was then added. And the reaction mixture was stirred at 0°C under a nitrogen atmosphere for 1 hour. Ethyl acetate (40.0 mL) was added to the reaction mixture. The pH was adjusted to 7 using saturated ammonium chloride solution. After phase separation, the organic phase was washed with water (20.0 mL × 1) and saturated sodium chloride (20.0 mL × 3), dried over anhydrous sodium sulfate, filtered, and concentrated under reduced pressure to obtain compound **21-6.** MS-ESI [M-Boc+H]⁺, calculated: 262, found: 262. ¹H NMR (400 MHz, CDCl₃) *δ* 7.28-7.31 (m, 2H), 6.97-7.01 (m, 1H), 6.84-6.87 (m, 3H), 5.88-5.93 (m, 1H), 4.82-4.85 (m, 1H), 4.54-4.68 (m, 1H), 4.17-4.23 (m, 2H), 3.74-3.84 (m, 1H), 3.62-3.67 (m, 1H), 2.40-2.46 (m, 1H), 2.00-2.06 (m, 1H), 1.43 (s, 9H), 1.28 (t, *J* = 7.2 Hz, 3H).
(5) To a solution of compound **21-6** (350 mg, 968 µmol) in tetrahydrofuran (3.0 mL), 10% palladium on carbon (0.1 g) was added. The reaction mixture was stirred under a hydrogen atmosphere at 25°C for 1 hour. Then, the mixture was filtered, and the filtrate was concentrated under reduced pressure to obtain compound **21-7.** MS-ESI [M+H]⁺, calculated: 364, found: 364. ¹H NMR (400 MHz, CDCl₃) *δ* 7.27-7.31 (m, 1.5H), 7.24-7.25 (m, 0.5H), 6.94-6.99 (m, 1H), 6.83-6.86 (m, 2H), 4.82 (s, 1H), 4.13 (q, *J =* 7.2 Hz, 2H), 4.00-4.07 (s, 1H), 3.75-3.90 (m, 1H), 3.48-3.53 (m, 1H), 2.27-2.32 (m, 3H), 2.08-2.19 (m, 1H), 1.87-1.96 (m, 1H), 1.77-1.86 (m, 1H), 1.39-1.52 (m, 9H), 1.25 (t, *J=* 7.2 Hz, 3H).
(6) To a solution of compound **21-7** (300 mg, 825 µmol) in dichloromethane (2.0 mL), trifluoroacetic acid (3.08 g, 27.0 mmol, 2.00 mL) was added. And the reaction mixture was stirred at 25°C for 1 hour. Then, dichloromethane (20.0 mL) was added, and the pH was adjusted to 7 using saturated sodium bicarbonate solution. After phase separation, the organic phase was washed with water (20.0 mL × 2) and saturated sodium chloride (20.0 mL × 1), dried over anhydrous sodium sulfate, filtered, and concentrated under reduced pressure to obtain compound **21-8.** MS-ESI [M+H]⁺, calculated: 264, found: 264.
(7) To a solution of compound **21-8** (70.0 mg, 265 µmol) in toluene (3.0 mL), compound **C** (69.0 mg, 239 µmol), cesium carbonate (173 mg, 531 µmol), 1,1'-binaphthyl-2,2'-diphenylphosphine (24.3 mg, 26.5 µmol), and bis (dibenzylideneacetone) palladium (33.1 mg, 53.1 µmol) were added. The reaction mixture was stirred at 80°C under a nitrogen atmosphere for 3 hours. It was then diluted with ethyl acetate (30.0 mL) and washed with water (70.0 mL × 1) and saturated sodium chloride solution (30.0 mL × 3). The organic phase was dried over anhydrous sodium sulfate, filtered, concentrated under reduced pressure. The crude product was purified by silica gel column chromatography (dichloromethane/methanol = 1:0 to 10:1) to obtain compound **21-9.** MS-ESI [M+H]⁺, calculated: 516, found: 516. ¹H NMR (400 MHz, CDCl₃) *δ* 7.45-7.48 (m, 2H), 7.35-7.38 (m, 2H), 7.28-7.33 (m, 4H), 6.96-6.99 (m, 1H), 6.84-6.87 (m, 2H), 5.56 (s, 2H), 5.05 (s, 1H), 4.37-4.40 (m, 1H), 4.13-4.20 (m, 0.5H), 4.08-4.10 (m, 0.5H), 3.98-4.00 (m, 1H), 3.89-3.93 (m, 1H), 2.50-2.54 (m, 1 H), 2.40-2.48 (m, 2H), 2.36-2.39 (m, 1H), 2.14-2.17 (m, 1H), 1.74-1.81 (m, 2H), 1.23 (t, *J* = 6.4 Hz, 3H).
(8) To a solution of compound **21-9** (130 mg, 252 µmol) in ethanol (3.0 mL), water (1.0 mL) and lithium hydroxide monohydrate (52.9 mg, 1.26 mmol) were added. The reaction mixture was stirred at 25°C for 2 hours. The pH was adjusted to 7 by adding hydrochloric acid solution (1 M). The mixture was then extracted with dichloromethane (25.0 mL × 1). The organic phase was separated, washed with water (20.0 mL × 2) and saturated sodium chloride solution (20.0 mL × 1), dried over anhydrous sodium sulfate, filtered, and concentrated under reduced pressure to obtain compound **21-10.** MS-ESI [M+H]⁺, calculated: 488, found: 488.
(9) To a solution of intermediate **21-10** (120 mg, 246 µmol) in dichloromethane (10.0 mL), compound **B-4** trifluoroacetate (68.6 mg, 295 µmol), diisopropylethylamine (95.4 mg, 738 µmol, 129 µL), and propylphosphonic anhydride solution (938 mg, 1.48 mmol, 878 µL, with purity 50% in ethyl acetate) were added. The reaction mixture was stirred at 25°C under a nitrogen atmosphere for 1 hour. It was then diluted with dichloromethane (30.0 mL) and washed with water (20.0 mL × 1) and saturated sodium chloride solution (30.0 mL × 3). The organic phase was dried over anhydrous sodium sulfate, filtered, and concentrated under reduced pressure. The crude product was purified by silica gel column chromatography (petroleum ether/methanol/ethyl acetate = 1:0 to 2:1) to obtain compound **21-11.** MS-ESI [M+H]⁺, calculated: 702, found: 702.
(10) To a solution of compound **21-11** (100 mg, 142 µmol) in tetrahydrofuran (8.0 mL), 10% palladium on carbon (30 mg) was added. The reaction mixture was stirred at 25°C under a hydrogen atmosphere for 2 hours. The mixture was then filtered, and the filtrate was concentrated under reduced pressure. The crude product was purified by preparative high-performance liquid chromatography (Xtimate C18 column, 100 mm × 30 mm, 10 µm, A: water (with 0.225% formic acid) and B: acetonitrile, 58% - 88% over 10 minutes) to obtain compound **21** formate. MS-ESI [M+H]⁺, calculated: 612, found: 612. ¹H NMR (400 MHz, MeOD) *δ* 8.60 (s, 2H), 7.81 (s, 1H), 7.22-7.31 (m, 2H), 6.88-6.96 (m, 3H), 5.09-5.11 (m, 1H), 4.16-4.23 (m, 1H), 3.84-3.97 (m, 5H), 3.61-3.70 (m, 5H), 2.50-2.53 (m, 2H), 2.43-2.49 (m, 1H), 2.14-2.24 (m, 2H), 1.79-1.83 (m, 1H).

### Example 22 Synthesis of Compound 22

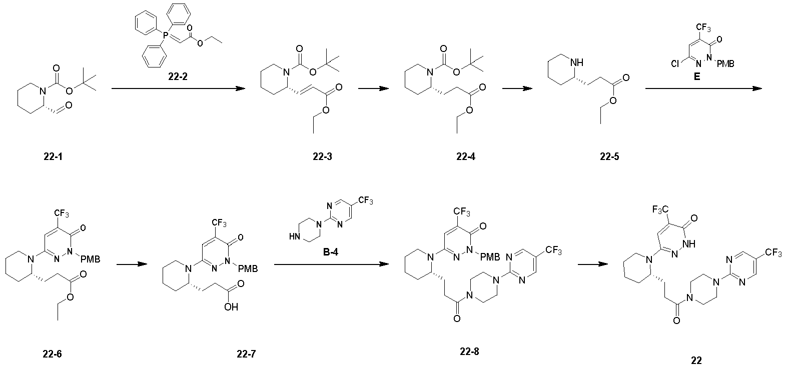

(1) To a solution of compound **22-1** (2.0 g, 9.38 mmol) in 70.0 mL of toluene, compound **22-2** (3.59 g, 10.3 mmol) was added. The reaction mixture was stirred at 50°C for 12 hours. Water (70.0 mL) was added to the reaction mixture, and it was then extracted with ethyl acetate (70.0 mL × 2). The combined organic phase was washed with saturated saltwater (70.0 mL × 2), dried over anhydrous sodium sulfate, filtered, and concentrated under reduced pressure. The crude product was purified by silica gel column chromatography ( petroleum ether/ethyl acetate = 1:0 to 5:1) to obtain compound **22-3.** ¹H NMR (400 MHz, CDCl₃) *δ* 6.87 (dd, *J* = 16.0, 4.0 Hz, 1H), 5.80 (dd, *J =* 16.0, 2.0 Hz, 1H), 4.19 (q, *J =* 7.2 Hz, 2H), 3.98 (d, *J =* 12.4 Hz, 1H), 2.50-3.06 (m, 2H), 1.58-1.84 (m, 6H), 1.45 (s, 9H), 1.29 (t, *J=* 7.2 Hz, 3H).
(2) To a solution of compound **22-3** (2.43 g, 8.58 µmol) in tetrahydrofuran (25.0 mL), 10% palladium on carbon (500 mg) was added. The reaction mixture was stirred at 25°C under a hydrogen atmosphere for 8 hours. After filtration, the filtrate was concentrated under reduced pressure. The crude product was purified by silica gel column chromatography ( petroleum ether/ethyl acetate = 1:0 to 5:1) to obtain compound **22-4.**
(3) To a solution of compound **22-4** (1.95 g, 6.83 mmol) in dichloromethane (20.0 mL), trifluoroacetic acid (7.70 g, 67.5 mmol) was added. The reaction mixture was stirred at 25°C for 1 hour. After filtration and concentration under reduced pressure, compound **22-5** was obtained. ¹H NMR (400 MHz, CDCl₃) *δ* 4.12 (q, *J =* 7.2 Hz, 2H), 3.38 (d, *J =* 12.4 Hz, 1H), 2.98-3.07 (m, 1H), 2.84 (td, *J* = 12.0, 4.8 Hz, 1H), 2.36-2.54 (m, 2H), 2.04 (dq, *J* = 14.8, 6.8 Hz, 1H), 1.87-1.95 (m, 3H), 1.73-1.83 (m, 2H), 1.43-1.63 (m, 2H), 1.24 (t, *J=* 7.2 Hz, 3H).
(4) To a solution of compound **22-5** (104 mg, 564 µmol) in toluene (10.0 mL), compound **E** (150 mg, 470 µmol), cesium carbonate (460 mg, 1.41 mmol), 1,1'-binaphthyl-2,2'-diphenylphosphine (58.6 mg, 94.1 µmol), and tris (dibenzylideneacetone) dipalladium (43.1 mg, 47.1 µmol) were added. The reaction mixture was stirred at 80°C under a nitrogen atmosphere for 8 hours. After the reaction, the mixture was quenched with 10.0 mL of water and extracted with ethyl acetate (10.0 mL × 2). The combined organic phase was washed with saturated sodium chloride (10.0 mL × 2), dried over anhydrous sodium sulfate, filtered, and concentrated under reduced pressure. The crude product was purified by silica gel column chromatography ( petroleum ether/ethyl acetate = 1:0 to 5:1) to obtain compound **22-6.** MS-ESI [M+H]⁺, calculated: 469, found: 469.
(5) To a solution of compound **22-6** (30.0 mg, 64.1 µmol) in tetrahydrofuran (3.0 mL), water (1.0 mL) and lithium hydroxide monohydrate (13.5 mg, 320 µmol) were added. The reaction mixture was stirred at 25°C for 8 hours. The pH of the reaction mixture was adjusted to 7 by adding 1 M hydrochloric acid solution. The mixture was then extracted with ethyl acetate (20.0 mL × 2). The organic phases were washed with saturated sodium chloride (20.0 mL × 2), dried over anhydrous sodium sulfate, filtered, and concentrated under reduced pressure to obtain compound **22-7.**
(6) To a solution of compound **22-7** (20.0 mg, 45.5 µmol) in dichloromethane (1.0 mL), compound **B-4** trifluoroacetate (21.1 mg, 91.0 µmol), triethylamine (4.61 mg, 45.5 µmol), and a solution of propylphosphonic anhydride in ethyl acetate (115 mg, 182 µmol, 50% purity) were added. The reaction mixture was stirred at 25°C under a nitrogen atmosphere for 1 hour. It was then extracted with ethyl acetate (10.0 mL × 2). The organic phases were washed with saturated sodium chloride (10.0 mL × 2), dried over anhydrous sodium sulfate, filtered, and concentrated under reduced pressure. The crude product was purified by silica gel column chromatography ( petroleum ether/ethyl acetate = 3:1) to obtain compound **22-8.** MS-ESI [M+H]⁺, calculated: 654, found: 654.
(7) To a solution of compound **22-8** (5.0 mg, 7.65 µmol) in trifluoroacetic acid (2.0 mL), trifluoromethanesulfonic acid (0.2 mL) was added. The reaction mixture was stirred at 25°C for 0.5 hours. The reaction mixture was then diluted with water (10.0 mL) and extracted with ethyl acetate (10.0 mL × 3). The organic phases were washed with saturated sodium chloride (10.0 mL × 3), dried over anhydrous sodium sulfate, filtered, and concentrated under reduced pressure. The crude product was purified using high-performance liquid chromatography ( Xtimate C18 column,100 mm × 30 mm × 10 µm, A: water (0.225% formic acid) and B: acetonitrile, 50%-80% over 10 minutes) to obtain compound **22** formate. MS-ESI [M+H]⁺, calculated: 534, found: 534. ¹H NMR (400 MHz, MeOD) *δ* 8.48-8.69 (m, 2H), 7.83 (s, 1H), 4.14-4.25 (m, 1H), 3.85-3.94 (m, 4H), 3.57-3.70 (m, 4H), 2.97-3.16 (m, 2H), 2.41-2.51 (m, 2H), 2.03-2.12 (m, 1H), 1.86-1.97 (m, 1H), 1.60-1.80 (m, 6H).

### Example 23 Synthesis of Compound 23

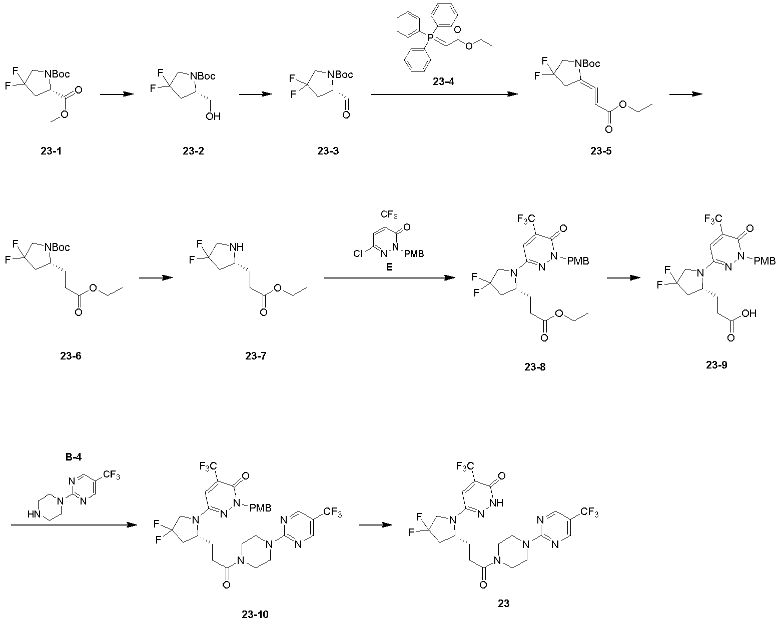

(1)To a solution of compound **23-1** (5.0 g, 18.8 mmol) in tetrahydrofuran (50.0 mL), lithium aluminum hydride (786 mg, 20.7 mmol) was added. The mixture was stirred for 0.5 hours at 0°C under a nitrogen atmosphere. At 0°C, water (1.0 mL) was added to the reaction mixture, followed by 15% sodium hydroxide solution (1.0 mL). The reaction mixture was dried over anhydrous sodium sulfate, filtered, and concentrated under reduced pressure. The crude product was purified by silica gel column chromatography ( petroleum ether/ethyl acetate = 1:0 to 0:1) to obtain compound **23-2.** ¹H NMR (400 MHz, CDCl₃) *δ* 4.15-4.35 (m, 1H), 3.77-3.93 (m, 1H), 3.50-3.75 (m, 3H), 2.40-2.57 (m, 1H), 2.07-2.31 (m, 1H), 1.43-1.52 (m, 9H).
(2) To a solution of compound **23-2** (3.0 g, 12.6 mmol) in dichloromethane (30.0 mL), Dess-Martin reagent (5.36 g, 12.6 mmol) was added. The reaction mixture was stirred at 25°C for 5 hours. To the reaction mixture, 50.0 mL of sodium sulfite solution was added, followed by 30.0 mL of water. The mixture was extracted with DCM (50.0 mL × 2). The organic layer was washed with sodium bicarbonate solution (50.0 mL), dried over anhydrous sodium sulfate, filtered, and concentrated under reduced pressure. The crude product was purified by silica gel column chromatography ( petroleum ether /ethyl acetate = 1:0 to 0:1) to obtain compound **23-3.** ¹H NMR (400 MHz, CDCl₃) *δ* 9.45-9.69 (m, 1H), 4.24-4.48 (m, 1H), 3.75-3.93 (m, 2H), 2.40-2.65 (m, 2H), 1.45-1.50 (m, 9H).
(3) To a solution of compound **23-3** (1.30 g, 5.53 mmol) in dichloromethane (10.0 mL), compound **23-4** (2.31 g, 6.63 mmol) was added. The reaction mixture was stirred at 25°C for 12 hours. Afterward, the reaction mixture was filtered, and the filtrate was concentrated under reduced pressure. The crude product was purified by silica gel column chromatography ( petroleum ether /ethyl acetate = 1:0 to 0:1) to obtain compound **23-5.** ¹H NMR (400 MHz, CDCl₃) *δ* 6.75-6.89 (m, 1H), 5.84-5.96 (m, 1H), 4.52-4.79 (m, 1H), 4.18-4.25 (m, 2H), 4.13-4.16 (m, 1H), 3.79-3.93 (m, 1H), 3.65-3.77 (m, 1H), 2.56-2.71 (m, 1H), 2.18-2.33 (m, 1H), 1.42-1.47 (m, 9H), 1.29 (d, *J=* 6.4 Hz, 3H).
(4) To a solution of compound **23-5** (900 mg, 2.95 mmol) in tetrahydrofuran (10.0 mL), 10% palladium on carbon (500 mg) was added. The reaction mixture was stirred at 25°C under a hydrogen atmosphere for 6 hours. After filtration, the reaction mixture was concentrated under reduced pressure to obtain compound **23-6.** ¹H NMR (400 MHz, CDCl₃) *δ* 3.70-4.18 (m, 4H), 3.52-3.67 (m, 1H), 2.41-2.59 (m, 1H), 2.27-2.38 (m, 2H), 2.02-2.19 (m, 2H), 1.78-1.90 (m, 1H), 1.44-1.49 (m, 9H), 1.23-1.29 (m, 3H).
(5) To a solution of compound **23-6** (400 mg, 1.3 mmol) in dichloromethane (8.0 mL), trifluoroacetic acid (3.08 g, 27 mmol) was added. The reaction mixture was stirred at 25°C for 1 hour. The reaction mixture was adjusted to pH > 8 using sodium bicarbonate, diluted with water (20.0 mL), and extracted with dichloromethane (30.0 mL×2). The combined organic phase was washed with saturated sodium chloride solution (30.0 mL), dried over anhydrous sodium sulfate, filtered, and concentrated under reduced pressure to obtain compound **23-7.** ¹H NMR (400 MHz, CDCl₃) *δ* 4.11-4.19 (m, 2H), 3.27-3.41 (m, 2H), 3.10-3.22 (m, 1H), 2.39-2.46 (m, 2H), 2.08-2.26 (m, 2H), 1.86-1.91 (m, 2H), 1.27 (t, *J=* 7.2 Hz, 3H).
(6) To a solution of compound **23-7** (250 mg, 1.21 mmol) in toluene (10.0 mL), compound **E** (346 mg, 1.09 mmol), cesium carbonate (1.18 g, 3.62 mmol), 1,1'-binaphthyl-2,2'-diphenylphosphine (150 mg, 241 µmol), and tris (dibenzylideneacetone) dipalladium (110 mg, 120 µmol) were added. The reaction mixture was stirred at 80°C under a nitrogen atmosphere for 2 hours. The reaction mixture was then diluted with water (50.0 mL) and extracted with ethyl acetate (50.0 mL×2). The combined organic phase was washed with saturated sodium chloride solution (50.0 mL), dried over anhydrous sodium sulfate, filtered, and concentrated under reduced pressure to obtain compound **23-8.** MS-ESI [M+H]⁺, calculated: 490, found: 490. ¹H NMR (400 MHz, CDCl₃) *δ* 7.50-7.53 (m, 1H), 7.42-7.46 (d, *J =* 6.8 Hz, 2H), 6.83-6.90 (m, *J =* 2.0 Hz, 2H), 5.07-5.25 (m, 2H), 4.14-4.18 (m, 2H), 3.79-3.81 (m, 3H), 2.51-2.66 (m, 1H), 2.36-2.45 (m, 1H), 2.22-2.33 (m, 2H), 2.06-2.13 (m, 1H), 1.73-1.85 (m, 1H), 1.60-1.64 (m, 3H), 1.25-1.29 (t, *J=* 6.8 Hz, 3H)
(7) Compound **23-8** (150 mg, 306 µmol) was dissolved in tetrahydrofuran (3.0 mL), and then water (1.0 mL ) and lithium hydroxide monohydrate (77.1 mg, 1.84 mmol) were added. The reaction mixture was stirred at 25°C under a nitrogen atmosphere for 1 hour. The pH was adjusted to less than 7 by adding 1 M hydrochloric acid (HCl) solution. The reaction mixture was then diluted with 20.0 mL of water and extracted with dichloromethane (30.0 mL×2). The organic phase was washed with saturated sodium chloride solution (30.0 mL), dried over anhydrous sodium sulfate, filtered, and concentrated under reduced pressure to obtain compound **23-9.** MS-ESI [M+H]⁺, calculated: 462, found: 462.
(8) Compound **23-9** (70.0 mg, 151 µmol) was dissolved in dichloromethane (5.0 mL), and then compound **B-4** trifluoroacetate (105 mg, 303 µmol), diisopropylethylamine (98.0 mg, 758 µmol), and a solution of propylphosphonic anhydride in ethyl acetate (386 mg, 606 µmol, 50% purity) were added. The reaction mixture was stirred at 25°C under a nitrogen atmosphere for 1 hour. Water (50.0 mL) was added to the reaction mixture, and it was then extracted with dichloromethane (50.0 mL × 2). The organic phase was washed with saturated sodium chloride solution (50.0 mL), dried over anhydrous sodium sulfate, filtered, and concentrated under reduced pressure. The crude product was purified by silica gel column chromatography ( petroleum ether/ethyl acetate = 1:0 to 0:1) to obtain compound **23-10.** MS-ESI [M+H]⁺, calculated: 676, found: 676.
(9) Compound **23-10** (50.0 mg, 74.0 µmol) was dissolved in trifluoroacetic acid (3.0 mL), and trifluoromethanesulfonic acid (0.3 mL) was added. The reaction mixture was stirred at 25°C for 1 hour. Sodium bicarbonate solution (50.0 mL) was added to the reaction mixture, and it was then extracted with ethyl acetate (50.0 mL × 2). The organic phase was washed with saturated sodium chloride solution (50.0 mL), dried over anhydrous sodium sulfate, filtered, and concentrated under reduced pressure. The crude product was purified by preparative high-performance liquid chromatography (Xtimate C18, 100 mm × 30 mm × 10 µm, A: water (0.225% formic acid); B: acetonitrile, 50%-80% over 10 minutes) to obtain compound **23** formate. MS-ESI [M+H]⁺, calculated: 556, found: 556. ¹H NMR (400 MHz, CD₃OD) *δ* 8.56-8.63 (m, 2H), 7.97-7.99 (m, 1H), 4.25-4.32 (m, 1H), 3.96-4.00 (m, 2H), 3.91-3.95 (m, 2H), 3.81-3.89 (m, 2H), 3.67-3.72 (m, 2H), 3.61-3.65 (m, 2H), 2.37-2.68 (m, 4H), 2.08-2.18 (m, 1H), 1.80-1.90 (m, 1H).

### Example 24 Synthesis of Compound 24

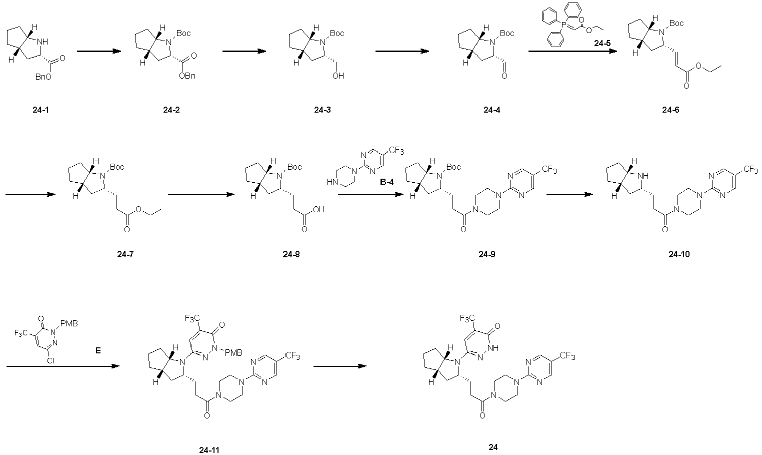

(1) Compound **24-1** (4.5 g, 16.0 mmol) was dissolved in 72.0 mL of dichloromethane, and di-tert-butyl dicarbonate (5.23 g, 24.0 mmol) and diisopropylethylamine (5.16 g, 39.9 mmol) were added. The reaction mixture was stirred at 25°C for 2 hours. After that, it was concentrated under reduced pressure. The crude product was purified by silica gel column chromatography ( petroleum ether/ethyl acetate = 100:1 to 1:1) to obtain compound **24-2.** MS-ESI [M+H]⁺, calculated: 346, found: 346. ¹H NMR (400 MHz, CDCl₃) *δ* 7.36 (s, 5H), 5.05-5.19 (m, 2H), 4.29 (br d, *J =* 5.6 Hz, 1H), 4.04-4.12 (m, 1H), 2.59-2.70 (m, 1H), 2.29-2.46 (m, 1H), 1.59-1.87 (m, 5H), 1.47 (s, 1H), 1.39 (s, 5H), 1.26 (s, 5H).
(2) At 0°C, to a solution of compound **24-2** (500 mg, 1.45 mmol) in 6.0 mL of tetrahydrofuran, lithium aluminum hydride (66.0 mg, 1.74 mmol) was added. The reaction mixture was stirred at 0°C for 1 hour. Then, 0.1 mL of water, 0.1 mL of 15% sodium hydroxide solution and 0.3 mL of water were added to the reaction mixture. It was dried over anhydrous sodium sulfate. After filtration and concentration under reduced pressure, compound **24-3** was obtained. ¹H NMR (400 MHz, CDCl₃) *δ* 4.06-4.21 (m, 1H), 3.87-4.02 (m, 1H), 3.56-3.69 (m, 2H), 2.57-2.69 (m, 2H), 2.56-2.62 (m, 1H), 2.14-2.25 (m, 1H), 1.54-1.76 (m, 4H), 1.45-1.49 (m, 9H), 1.21-1.30 (m, 1H).
(3) To a solution of compound **24-3** (690 mg, 2.86 mmol) in 10.0 mL of dichloromethane, Dess-Martin reagent (1.82 g, 4.29 mmol) was added. The reaction mixture was stirred at 25°C for 12 hours. Then, 15.0 mL of sodium sulfite solution was added to the reaction mixture, followed by extraction with 120 mL of ethyl acetate. The organic layer was dried over anhydrous sodium sulfate, filtered, and concentrated under reduced pressure. The crude product was purified by silica gel column chromatography ( petroleum ether/ethyl acetate = 100:1 to 1:1) to obtain compound **24-4.** ¹H NMR (400 MHz, CDCl₃) *δ* 9.43-9.59 (m, 1H), 4.05-4.25 (m, 2H), 2.67-2.74 (m, 1H), 2.22 (br dd, *J =* 12.8, 7.2 Hz, 1H), 1.83-1.98 (m, 2H), 1.60-1.80 (m, 5H), 1.42-1.49 (m, 9H).
(4) To a solution of compound **24-4** (410 mg, 1.71 mmol) in 60.0 mL of dichloromethane, compound **24-5** (657 mg, 1.88 mmol) was added. The reaction mixture was stirred at 25°C under a nitrogen atmosphere for 12 hours. Then, 20.0 mL of water was added to the reaction mixture, followed by extraction with 100 mL of dichloromethane. The organic layer was dried over anhydrous sodium sulfate, filtered, and concentrated under reduced pressure. The crude product was purified by silica gel column chromatography ( petroleum ether/ethyl acetate = 100:1 to 1:1) to obtain compound **24-6.** MS-ESI [M-Boc+H]⁺, calculated: 210, found: 210. ¹H NMR (400 MHz, CDCl₃) *δ* 6.89 (br dd, *J =* 15.6, 6.4 Hz, 1H), 5.84 (d, *J=* 15.6 Hz, 1H), 4.19 (q, *J =* 7.2 Hz, 3H), 2.56-2.72 (m, 1H), 2.26-2.35 (m, 1H), 1.87-2.00 (m, 1H), 1.69-1.79 (m, 4H), 1.49-1.58 (m, 2H), 1.41-1.46 (m, 10H), 1.27-1.32 (m, 3H).
(5) To a solution of compound **24-6** (300 mg, 970 µmol) in 5.0 mL of tetrahydrofuran, 10% palladium on carbon (140 mg) was added. The reaction mixture was stirred at 25°C under a hydrogen atmosphere for 2 hours. Afterward, the reaction mixture was filtered, concentrated under reduced pressure, and compound **24-7** was isolated. MS-ESI [M+H]⁺, calculated: 312, found: 312.¹H NMR (400 MHz, CDCl₃) *δ* 4.13 (q, *J* = 7.2 Hz, 2H), 3.73-3.84 (m, 1H), 2.54-2.67 (m, 1H), 2.26-2.36 (m, 2H), 2.13-2.25 (m, 2H), 1.86-1.97 (m, 1H), 1.71-1.82 (m, 3H), 1.58-1.69 (m, 5H), 1.47 (s, 9H), 1.26 (t, *J=* 7.2 Hz, 3H).
(6) To a solution of compound **24-7** (300 mg, 963 µmol) in 6.0 mL of tetrahydrofuran, water (2.0 mL) and lithium hydroxide monohydrate (500 mg, 11.9 mmol) were added. The reaction mixture was stirred at 25°C under a nitrogen atmosphere for 12 hours. The pH was adjusted to 7 with 1 M hydrochloric acid solution. Then, the mixture was extracted with 90.0 mL of ethyl acetate. The organic layer was dried over anhydrous sodium sulfate, filtered, concentrated under reduced pressure, and compound **24-8** was obtained.
(7) To a solution of compound **24-8** (270 mg, 953 µmol) in 3.0 mL of dichloromethane, compound **B-4** trifluoroacetate (384 mg, 1.43 mmol), diisopropylethylamine (616 mg, 4.76 mmol), and a solution of propylphosphonic anhydride in ethyl acetate (1.82 g, 2.86 mmol, 50% purity) were added. The reaction mixture was stirred at 25°C under a nitrogen atmosphere for 4 hours. Water (20.0 mL) was added to the reaction mixture, and it was then extracted with 80.0 mL of dichloromethane. The organic layer was dried over anhydrous sodium sulfate, filtered, concentrated under reduced pressure. The crude product was purified by silica gel column chromatography (petroleum ether/ethyl acetate = 100:1 to 1:1) to obtain compound **24-9.** MS-ESI [M+H]⁺, calculated: 498, found: 498. ¹H NMR (400 MHz, CDCl₃) *δ* 8.51 (s, 2H), 3.81-3.99 (m, 6H), 3.71 (br d, *J =* 4.4 Hz, 2H), 3.57 (br s, 2H), 2.61 (tt, *J =* 8.8, 4.0 Hz, 1H), 2.36-2.43 (m, 2H), 2.23 (dt, *J =* 13.2, 8.8 Hz, 1H), 2.08-2.17 (m, 1H), 1.85-1.94 (m, 2H), 1.72 (s, 4H), 1.60-1.66 (m, 1H), 1.50-1.53 (m, 1H), 1.46 (s, 9H).
(8) To a solution of compound **24-9** (460 mg, 925 µmol) in 3.0 mL of dichloromethane, 1.0 mL of trifluoroacetic acid was added. The reaction mixture was stirred at 25°C for 0.5 hours and adjusted to pH 7 with sodium carbonate, then extracted with 50.0 mL of dichloromethane. The organic layer was dried over anhydrous sodium sulfate, filtered, concentrated under reduced pressure, and compound **24-10** was obtained. MS-ESI [M+H]⁺, calculated: 398, found: 398.
(9) To a solution of compound **24-10** (49.9 mg, 125 µmol) in 1.5 mL of dioxane, compound **E** (40.0 mg, 125 µmol), cesium carbonate (81.8 mg, 251 µmol), and methanesulfonic (2-dicyclohexylphosphino-2',6'-diisopropoxy-1,1'-biphenyl)[(2'-amino-1,1'-biphenyl-2-yl)]palla dium (15.8 mg, 18.8 µmol) were added. The reaction mixture was stirred at 60°C for 12 hours under a nitrogen atmosphere. The reaction mixture was dried over anhydrous sodium sulfate, filtered, concentrated under reduced pressure, and the crude product was purified by silica gel column chromatography (petroleum ether/ethyl acetate = 100:1 to 1:1) to obtain compound **24-11.** MS-ESI [M+H]⁺, calculated: 680, found: 680. ¹H NMR (400 MHz, CDCl₃) *δ* 8.53 (s, 2H), 7.54 (s, 1H), 7.43 (d, *J=* 8.8 Hz, 2H), 6.84 (d, *J=* 8.8 Hz, 2H), 5.14-5.20 (m, 2H), 4.82 (s, 1H), 4.08 (s, 1H), 3.88-3.96 (m, 6H), 3.78 (s, 3H), 3.73 (br s, 2H), 3.50 (br s, 2H), 2.74 (br dd, *J* = 8.0, 4.4 Hz, 1H), 2.30-2.40 (m, 3H), 1.91-2.01 (m, 1H), 1.74-1.87 (m, 3H), 1.67 (br dd, *J* = 8.8, 4.8 Hz, 3H).
(10) To a solution of compound **24-11** (60.0 mg, 88.3 µmol) in 1.0 mL of trifluoroacetic acid, trifluoromethanesulfonic acid (0.1 mL) was added. The reaction mixture was stirred at 25°C for 1 hour. Sodium bicarbonate was added to adjust the pH to 7. The mixture was extracted with ethyl acetate (100 mL). The organic phase was dried over anhydrous sodium sulfate, filtered, concentrated under reduced pressure, and the crude product was purified by preparative high-performance liquid chromatography ( Xtimate C18 column, 100 mm × 30 mm, 10 µm, A: water (0.225% formic acid) and B: acetonitrile (65%-95%) over 10 minutes ) to obtain compound **24** formate. MS-ESI [M+H]⁺, calculated: 560, found: 560. ¹H NMR (400 MHz, MeOD) *δ* 8.60 (s, 2H), 7.74 (s, 1H), 4.17 (br d, *J=* 3.2 Hz, 1H), 3.96-4.02 (m, 2H), 3.93 (br dd, *J =* 6.0, 3.2 Hz, 3H), 3.67 (dt, *J =* 15.2, 5.2 Hz, 4H), 2.72-2.86 (m, 1H), 2.50 (t, *J =* 7.6 Hz, 2H), 2.37 (dt, *J =* 13.2, 8.8 Hz, 1H), 2.22 (dtd, *J =* 13.2, 8.0, 2.8 Hz, 1H), 1.96-2.06 (m, 1H), 1.75-1.90 (m, 3H), 1.60-1.73 (m, 4H).

### Example 25 Synthesis of Compound 25

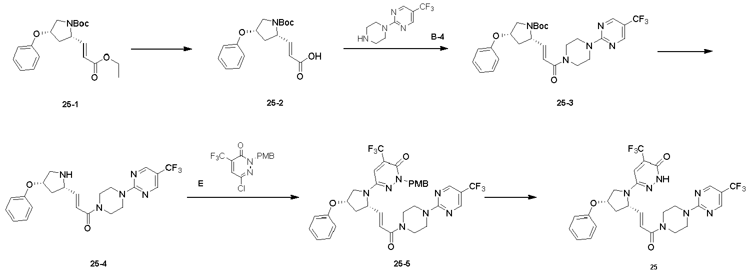

(1) To a solution of compound **25-1** (569 mg, 1.57 mmol) in 6.0 mL of tetrahydrofuran, water (2.0 mL) and lithium hydroxide monohydrate (330 mg, 7.87 mmol) were added. The reaction mixture was stirred at 25°C under a nitrogen atmosphere for 12 hours. Hydrochloric acid solution (1 M) was added to adjust the pH to approximately 3-4. The mixture was extracted with ethyl acetate (20.0 mL). The combined organic phase was washed with water (50.0 mL × 2) and saturated sodium chloride (50.0 mL × 2), dried over anhydrous sodium sulfate, filtered, and concentrated under reduced pressure to obtain compound **25-2.** MS-ESI [M-^{t}Bu+H]⁺, calculated: 278, found: 278. ¹H NMR (400 MHz, CDCl₃) *δ* 7.72 (dd, *J =* 5.6, 3.4 Hz, 1H), 7.54 (dd, *J =* 5.8, 3.2 Hz, 1H), 7.27-7.33 (m, 2H), 6.97-7.14 (m, 1H), 6.85 (d, *J =* 7.9 Hz, 1H), 5.90 ( s, 1H), 4.21-4.34 (m, 2H), 3.71-3.83 (m, 1H), 3.14 (dd, *J=* 7.3, 4.7 Hz, 2H), 2.19 (s, 1H), 1.42-1.51 (m, 9H).
(2) At 0°C, to a solution of compound **25-2** (142 mg, 425 µmol) in 8.0 mL of dichloromethane, compound **B-4** trifluoroacetate (247 mg, 1.06 mmol), triethylamine (129 mg, 1.28 mmol), and a solution of propylphosphonic anhydride in ethyl acetate (677 mg, 1.06 mmol, 50% purity ) were added. The reaction mixture was stirred at 25°C under a nitrogen atmosphere for 1 hour. The reaction mixture was then extracted with dichloromethane (30.0 mL). The organic phase was washed with saturated sodium chloride (75.0 mL × 2), dried over anhydrous sodium sulfate, filtered, and concentrated under reduced pressure. The crude product was purified by silica gel column chromatography ( petroleum ether /ethyl acetate = 1:0 to 0:1) to obtain compound **25-3.** MS-ESI [M+H]⁺, calculated: 548, found: 548.
(3) To a solution of compound **25-3** (50.0 mg, 91.3 µmol) in 2.0 mL of dichloromethane, trifluoroacetic acid (0.5 mL) was added. The reaction mixture was stirred at 25°C for 0.5 hours and adjusted to pH 7-8 with sodium bicarbonate, then extracted with dichloromethane (20.0 mL). The combined organic phase was washed with saturated sodium chloride (25.0 mL × 2), dried over anhydrous sodium sulfate, filtered, and concentrated under reduced pressure to obtain compound **25-4.** MS-ESI [M+H]⁺, calculated: 448, found: 448.
(4) To a solution of compound **25-4** (70.0 mg, 156 µmol) in 3.0 mL of toluene, compound **E** (44.8 mg, 141 µmol), cesium carbonate (101 mg, 312 µmol), 1,1'-binaphthyl-2,2'-diphenylphosphine (19.4 mg, 31.2 µmol), and tris (dibenzylideneacetone) dipalladium (14.3 mg, 15.6 µmol) were added. The reaction mixture was stirred at 80°C under a nitrogen atmosphere for 2 hours. The reaction mixture was then diluted with water (75.0 mL × 2) and extracted with ethyl acetate (30.0 mL). The combined organic phase was washed with saturated sodium chloride (75.0 mL × 2), dried over anhydrous sodium sulfate, filtered, and concentrated under reduced pressure. The crude product was purified by silica gel column chromatography (petroleum ether/ethyl acetate = 1:1) to obtain compound **25-5.** MS-ESI [M+H]⁺, calculated: 730, found: 730.
(5) To a solution of compound **25-5** (30.0 mg, 41.1 µmol) in 1.0 mL of trifluoroacetic acid, trifluoromethanesulfonic acid (0.1 mL) was added. The reaction mixture was stirred at 25°C for 0.5 hours, then adjusted to pH 7-8 with sodium bicarbonate and extracted with ethyl acetate (20.0 mL). The organic phase was dried over anhydrous sodium sulfate, filtered, and concentrated under reduced pressure. The crude product was purified by preparative high-performance liquid chromatography (Phenomenex C18, 75 mm × 30 mm, 3 µm, A: water (10 mmol/L ammonium bicarbonate) and B: acetonitrile (35%-65% ) over 12 minutes) to obtain compound **25.** MS-ESI [M+H]⁺, calculated: 610, found: 610.¹H NMR (400 MHz, MeOD) *δ* 8.47-8.72 (m, 2H), 7.57 (s, 1H), 7.29 (t, *J=* 8.0 Hz, 2H), 6.91-7.01 (m, 3H), 6.84-6.91 (m, 1H), 6.62 (d, *J =* 15.2 Hz, 1H), 5.18 ( s, 1H), 4.76-4.79 (m, 1H), 3.88-3.97 (m, 4H), 3.80-3.88 (m, 2H), 3.65-3.76 (m, 4H), 2.58-2.71 (m, 1H), 2.31 ( d, *J=* 14.4 Hz, 1H).

### Example 26 Synthesis of Compound 26

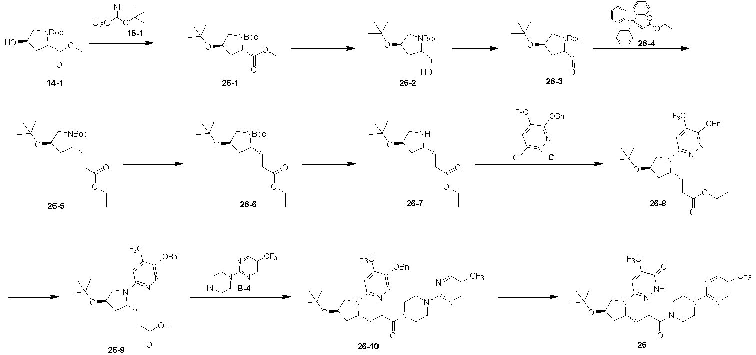

(1) To a solution of compound **14-1** (5.00 g, 20.4 mmol) in fluorobenzene (50.0 mL), 2,6-dimethylpyridine (1.09 g, 10.2 mmol), and bis (trifluoromethanesulfonyl) amine (1.43 g, 5.10 mmol) were added. Then, compound **15-1** (22.3 g, 101 mmol, 18.3 mL) was slowly added at 20°C. After the addition was complete, the reaction mixture was allowed to react at 25°C for 3 hours. The reaction mixture was filtered, and the solvent was removed under reduced pressure. The crude product was purified by silica gel column chromatography (petroleum ether/ethyl acetate = 1:0 to 5:1) to obtain compound **26-1.** ¹H NMR (400 MHz, CDCl₃) *δ* 4.20-4.41 (m, 2H), 3.64-3.76 (m, 4H), 3.11-3.28 (m, 1H), 2.01-2.21 (m, 2H), 1.47(s, 3H),1.39 (s, 6H), 1.17 (s, 9H).
(2) A solution of lithium aluminum hydride (484 mg, 12.7 mmol) in tetrahydrofuran (50.0 mL) was cooled to 0°C, and a solution of compound **26-1** (3.2 g, 10.6 mmol) in tetrahydrofuran (32.0 mL) was slowly added. The reaction mixture was allowed to react at 0°C for 0.5 hours. Water (1.0 mL), 15% sodium hydroxide solution (1.0 mL), water (3.0 mL), and sodium sulfate were added to the reaction mixture, and it was stirred for 10 minutes. The mixture was then filtered. The filtrate was extracted with ethyl acetate (50.0 mL × 2). The combined organic phase was washed with saturated sodium chloride (50.0 mL × 2), dried over anhydrous sodium sulfate, filtered, and concentrated under reduced pressure. The crude product was purified by silica gel column chromatography (petroleum ether/ethyl acetate = 1:0 to 5:1) to obtain compound **26-2.**
(3) Compound **26-2** (2.59 g, 9.47 mmol) was dissolved in dichloromethane (25.0 mL), and Dess-Martin oxidizing agent (4.02 g, 9.47 mmol, 2.93 mL) was added. The reaction mixture was allowed to react at 25°C for 8 hours. The reaction mixture was then poured into water (20.0 mL), stirred for 10 minutes, and extracted with ethyl acetate (20.0 mL × 3). The combined organic phase was washed with saturated sodium chloride (20.0 mL × 3), dried over anhydrous sodium sulfate, filtered, and concentrated under reduced pressure. The crude product was purified by silica gel column chromatography (petroleum ether/ethyl acetate = 1:0 to 5:1) to obtain compound **26-3.** ¹H NMR (400 MHz, CDCl₃) *δ* 9.38-9.58 (m, 1H), 4.09-4.34 (m, 2H), 3.56-3.65 (m, 1H), 3.16-3.40 (m, 1H), 1.94-2.07 (m, 2H), 1.47 (s, 9H), 1.40 (m, 6H), 1.16 (s, 9H).
(4) Compound **26-3** (1.15 g, 4.24 mmol) was dissolved in dichloromethane (10.0 mL), and compound **26-4** (1.62 g, 4.66 mmol) was added. The reaction was allowed to proceed at 25°C for 12 hours. The reaction mixture was then poured into ice water (10.0 mL), and extracted with ethyl acetate (20.0 mL). The organic phase was washed with saturated sodium chloride (20.0 mL × 3), dried over anhydrous sodium sulfate, filtered, and concentrated under reduced pressure. The crude product was purified by silica gel column chromatography (petroleum ether/ethyl acetate = 1:0 to 5:1) to obtain compound **26-5.**
(5) Compound **26-5** (1.3 g, 3.81 mmol) was dissolved in tetrahydrofuran (10.0 mL), and 10% palladium on carbon (0.5 g) was added to the solution. The reaction mixture was stirred at 25°C under a hydrogen atmosphere (15 psi) for 12 hours. Afterward, the reaction mixture was filtered, and the filtrate was concentrated under reduced pressure to obtain compound **26-6.**
(6) Compound **26-6** (0.2 g, 582 µmol) was dissolved in dichloromethane (2.0 mL), and a solution of trifluoroacetic acid (616 mg, 5.40 mmol) in dichloromethane (0.8 mL) was added to the reaction mixture. The reaction was stirred at 25°C for 0.5 hours. To the reaction mixture, dichloromethane (20.0 mL) was added. The pH was adjusted to 7 using a saturated sodium bicarbonate aqueous solution. The organic phase was separated, washed with water (20.0 mL × 2) and saturated sodium chloride (20.0 mL), dried over anhydrous sodium sulfate, filtered, and concentrated under reduced pressure to obtain compound **26-7.** MS-ESI [M+H]⁺, calculated: 244, found: 244.
(7) Compound **26-7** (120 mg, 493 µmol) was dissolved in toluene (3.0 mL), and compound **C** (121 mg, 419 µmol), cesium carbonate (482 mg, 1.48 mmol), 1,1'-binaphthyl-2,2'-diphenylphosphine (61.4 mg, 98.6 µmol), and tris (dibenzylideneacetone) dipalladium (45.2 mg, 49.3 µmol) were added to the solution. The reaction mixture was stirred at 80°C under a nitrogen atmosphere for 12 hours. The reaction mixture was poured into water (10.0 mL), and extracted with ethyl acetate (10.0 mL × 2). The combined organic phase was washed with saturated sodium chloride (10.0 mL × 2), dried over anhydrous sodium sulfate, filtered, and concentrated under reduced pressure. The crude product was separated using silica gel column chromatography (petroleum ether/ethyl acetate = 1:0 to 5:1) to obtain compound **26-8.** ¹H NMR (400 MHz, CDCl₃) *δ* 7.46-7.49 (m, 2H), 7.36-7.40 (m, 2H), 7.31-7.35 (m, 1H), 7.10-7.19 (m, 1H), 5.55-5.61 (m, 2H), 4.44-4.51 (m, 1H), 4.23 (t, *J=* 8.4 Hz, 1H), 4.09-4.17 (m, 3H), 3.76-3.85 (m, 1H), 2.35-2.40 (m, 2H), 2.04-2.07 (m, 2H), 1.68-1.75 (m, 2H), 1.25-1.27 (m, 3H), 1.22-1.24 (m, 9H).
(8) Compound **26-8** (50 mg, 100 µmol) was dissolved in tetrahydrofuran (6.0 mL) and water (2.0 mL). Then, lithium hydroxide monohydrate (21.6 mg, 504 µmol) was added, and the reaction was carried out at 25°C for 8 hours. The reaction mixture was adjusted to pH 8 with 1 M hydrochloric acid solution, stirred for 5 minutes, and extracted with ethyl acetate (20.0 mL × 2). The organic phases were combined, washed with saturated sodium chloride (20.0 mL × 2), dried over anhydrous sodium sulfate, filtered, and concentrated under reduced pressure to obtain compound **26-9.**
(9) Compound **26-9** (25 mg, 53.5 µmol) and compound **B-4** (24.8 mg, 106 µmol) were dissolved in N, N-dimethylformamide (2.0 mL), and then triethylamine (5.41 mg, 53.5 µmol, 7.44 µL) was added. The reaction mixture was cooled to 0°C, and propylphosphonic anhydride solution (136 mg, 214 µmol, 127 µL) was added. The reaction was carried out at 25°C for 0.5 hours. The reaction mixture was poured into water (10.0 mL), and extracted with ethyl acetate (10.0 mL × 2). The combined organic phase was washed with saturated sodium chloride (10.0 mL × 2), dried over anhydrous sodium sulfate, filtered, and concentrated under reduced pressure. The crude product was separated using silica gel column chromatography (petroleum ether/ethyl acetate = 1:0 to 5:1) to obtain compound **26-10.** MS-ESI [M+H]⁺, calculated: 682, found: 682.
(10) Compound **26-10** (25.0 mg, 36.7 µmol) was dissolved in tetrahydrofuran (5.0 mL), and 10% palladium on carbon (10.0 mg) was added. The reaction was stirred at 25°C under a hydrogen atmosphere (15 psi) for 8 hours. After the reaction was completed, it was filtered, and the filtrate was concentrated under reduced pressure. The crude product was separated using a reverse-phase high-performance liquid chromatography (Xtimate C18, 100 mm × 30 mm, 10 µm, A: water (0.225% formic acid); B: acetonitrile (53%-83%) over 8 minutes) to obtain compound **26** formate. MS-ESI [M+H]⁺, calculated: 592, found: 592..¹H NMR (400 MHz, CDCl₃) *δ* 8.51 (s, 2H), 7.72 (s, 1H), 4.39-4.45 (m, 1H), 3.98-4.09 (m, 2H), 3.89-3.97 (m, 4H), 3.68-3.77 (m, 3H), 3.56 (d, *J=* 4.0 Hz, 1H), 3.16 (dd, *J=* 10.0, 6.0 Hz, 1H), 2.41 (t, *J =* 7.2 Hz, 2H), 1.99-2.13 (m, 4H), 1.22 (s, 9H).

### Example 27 Synthesis of Compound 27

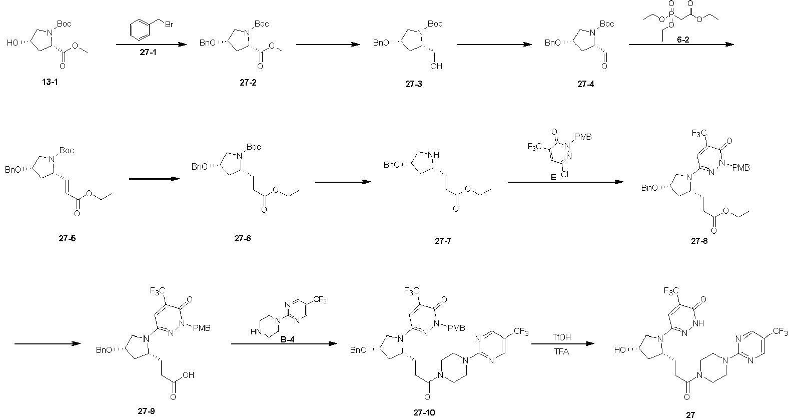

(1) At 0°C, 60% sodium hydride (978 mg, 24.4 mmol) was added to a solution of compound **13-1** (5.00 g, 20.3 mmol) in tetrahydrofuran (50.0 mL), and the mixture was stirred for 10 minutes. Then, at 0°C, a solution of compound **27-1** (3.84 g, 22.4 mmol) in tetrahydrofuran (30.0 mL) was added to the reaction mixture, and the reaction was allowed to proceed at 25°C for 1 hour. The reaction mixture was poured into saturated ammonium chloride solution (30.0 mL) and extracted with ethyl acetate (50.0 mL). The organic layer was sequentially washed with water (375 mL × 2) and saturated sodium chloride solution (375 mL × 2), dried over anhydrous sodium sulfate, filtered, and concentrated under reduced pressure. The crude product was then purified by silica gel column chromatography (petroleum ether/ethyl acetate = 1:0 to 0:1) to isolate compound **27-2.** MS-ESI [M+H]⁺, calculated: 336, found: 336.
(2) The compound **27-2** (500 mg, 1.49 mmol) was dissolved in 10.0 mL of tetrahydrofuran and cooled to 0°C. Then, lithium aluminum hydride (84.8 mg, 2.24 mmol) was slowly added to the solution. The reaction mixture was allowed to react at 0°C for 1.5 hours. Water (0.1 mL), 15% sodium hydroxide solution (0.1 mL), water (0.3 mL), and sodium sulfate were added to the reaction mixture, and it was stirred for 10 minutes. The mixture was then filtered. The filtrate was diluted with ethyl acetate (30.0 mL) and sequentially washed with water (50.0 mL × 2) and saturated sodium chloride (50.0 mL × 2). The organic layer was dried over anhydrous sodium sulfate, filtered, and concentrated under reduced pressure. The crude product was purified by silica gel column chromatography (petroleum ether/ethyl acetate = 1:0 to 0:1) to isolate compound **27-3.** MS-ESI [M-^{t}Bu+H]⁺, calculated: 252, found: 252. ¹H NMR (400 MHz, CDCl₃) *δ* 7.27-7.39 (m, 5H), 4.46-4.56 (m, 2H), 4.08 (s, 1H), 3.66-3.82 (m, 2H), 3.46-3.62 (m, 2H), 2.13-2.27 (m, 2H), 2.07 ( d, *J =* 6.4 Hz, 2H), 1.47 (s, 9H).
(3) Compound **27-3** (3.00 g, 9.76 mmol) was dissolved in 70.0 mL of dichloromethane, and then Dess-Martin oxidizing reagent (6.21 g, 14.6 mmol, 4.53 mL) was added. The reaction mixture was allowed to react at 25°C for 1 hour. The reaction mixture was poured into saturated sodium bisulfite solution (50.0 mL), stirred for 10 minutes, and extracted with ethyl acetate (150.0 mL). The organic phase was successively washed with water (150 mL × 2) and saturated sodium chloride solution (150 mL × 2), dried over anhydrous sodium sulfate, filtered, and concentrated under reduced pressure. The crude product was purified by silica gel column chromatography (petroleum ether/ethyl acetate = 1:0 to 0:1) to isolate compound **27-4.**
(4) At 0°C, compound **6-2** (158 mg, 707 µmol, 140 µL) was added to a solution of 60% sodium hydride (30.6 mg, 766 µmol) in tetrahydrofuran (5.0 mL) and stirred for 30 minutes. Then, a solution of compound **27-4** (180 mg, 589 µmol) in tetrahydrofuran (2.0 mL) was added to the reaction mixture, and the reaction continued at 0°C for an additional 30 minutes. The reaction mixture was poured into cold water (10.0 mL), and extracted with ethyl acetate (30.0 mL). The organic phase was successively washed with water (75.0 mL × 2) and saturated sodium chloride solution (75.0 mL × 2), dried over anhydrous sodium sulfate, filtered, and concentrated under reduced pressure. The crude product was purified by silica gel column chromatography (petroleum ether/ethyl acetate = 1:0 to 0:1) to isolate compound **27-5.** ¹H NMR (400 MHz, CDCl₃) *δ* 7.27-7.40 (m, 5H), 6.85-7.09 (m, 1H), 5.77-5.96 (m, 1H), 4.45-4.55 (m, 2H), 4.05-4.26 (m, 3H), 3.56-3.63 (m, 1H), 2.25 (s, 1H), 1.94-2.11 (m, 1H), 1.62 (s, 2H), 1.40-1.46 (m, 9H), 1.23-1.30 (m, 3H).
(5) Compound **27-5** (120 mg, 319 µmol) was dissolved in 3.0 mL of tetrahydrofuran, and 10% palladium on carbon (30.0 mg) was added. The reaction mixture was stirred for 2 hours under a hydrogen atmosphere (15 psi) at 25°C. After the reaction, the mixture was filtered, and the solvent was removed under reduced pressure to obtain compound **27-6.** MS-ESI [M+H]⁺, calculated: 378, found: 378.
(6) Compound **27-6** (500 mg, 1.32 mmol) was dissolved in 6.0 mL of dichloromethane, and trifluoroacetic acid (3.08 g, 27.0 mmol) was added to the solution. The reaction mixture was stirred for 1 hour at 25°C. The pH of the reaction mixture was adjusted to above 7 using saturated sodium bicarbonate solution. The mixture was diluted with dichloromethane (20.0 mL), and washed with water (75.0 mL × 2) and saturated sodium chloride solution (75.0 mL × 2). The organic phase was dried over anhydrous sodium sulfate, filtered, concentrated under reduced pressure, resulting in the compound **26-7.** MS-ESI [M+H]⁺, calculated: 278, found: 278.¹H NMR (400 MHz, CDCl₃) *δ* 7.26-7.40 (m, 5H), 4.49 (d, *J=* 2.4 Hz, 2H), 4.12 (d, *J =* 6.8 Hz, 3H), 3.37-3.47 (m, 2H), 3.32 ( d, *J =* 12.4 Hz, 1H), 3.09 (dd, *J =* 12.4, 5.2 Hz, 1H), 2.50 (t, *J=* 7.2 Hz, 2H), 2.29 (s, 1H), 1.93-2.13 (m, 2H), 1.60-1.75 (m, 1H), 1.24 (t, *J=* 6.8 Hz, 3H).
(7) Compound **26-7** (150 mg, 540 µmol) was dissolved in 5.0 mL of toluene. To this solution, compound **E** (155 mg, 486 µmol), cesium carbonate (352 mg, 1.08 mmol), 1,1'-binaphthyl-2,2'-diphenylphosphine (67.3 mg, 108 µmol), and tris (dibenzylideneacetone) dipalladium (49.5 mg, 54.0 µmol) were added. The reaction mixture was stirred at 80°C under a nitrogen atmosphere for 2 hours. After the reaction, the mixture was filtered and concentrated under reduced pressure. The residue was dissolved in ethyl acetate (30.0 mL) and then washed with water (75.0 mL × 2) and saturated sodium chloride solution (75.0 mL × 2). The organic phase was dried over anhydrous sodium sulfate, filtered, and concentrated under reduced pressure. The crude product was purified by silica gel column chromatography (petroleum ether/ethyl acetate = 1 : 0 to 0 : 1) to obtain compound **27-8.** MS-ESI [M+H]⁺, calculated: 560, found: 560. ¹H NMR (400 MHz, CDCl₃) *δ* 7.44 (d, *J =* 8.8 Hz, 3H), 7.28-7.39 (m, 5H), 6.84 (d, *J =* 8.4 Hz, 2H), 5.06-5.21 (m, 2H), 4.55 (s, 2H), 4.23 ( s, 1H), 4.11-4.18 (m, 2H), 3.92 (t, *J* = 8.8 Hz, 1H), 3.78 (s, 3H), 3.59-3.64 (m, 1H), 3.50 (dd, *J =* 11.6, 5.3 Hz, 1H), 2.35-2.45 (m, 1H), 2.25-2.35 (m, 1H), 2.04-2.19 (m, 3H), 1.90-2.01 (m, 1H), 1.25 (t, *J =* 7.2 Hz, 3H).
(8) Compound **27-8** (70.0 mg, 125 µmol) was dissolved in 3.0 mL of tetrahydrofuran and 2.0 mL of water. To this solution, lithium hydroxide monohydrate (15.7 mg, 375 µmol) was added. The reaction mixture was stirred at 25°C for 12 hours. Hydrochloric acid solution (1 mol/L) was then added to adjust the pH to around 7. The mixture was further diluted with dichloromethane (30.0 mL), and then washed with water (75.0 mL × 2). The organic phase was dried over anhydrous sodium sulfate, filtered, and concentrated under reduced pressure to obtain compound **27-9.** MS-ESI [M+H]⁺, calculated: 532, found: 532.
(9) Compound **27-9** (92.0 mg, 173 µmol) and compound **B-4** (80.3 mg, 346 µmol) were dissolved in 5.0 mL of dichloromethane. To this solution, diisopropylethylamine (67.1 mg, 519 µmol, 90.4 µL) was added, and the mixture was cooled to 0°C. Then, propylphosphonic anhydride solution (330 mg, 519 µmol, 308 µL) was added. The reaction mixture was stirred at 25°C for 1 hour. The reaction mixture was diluted with dichloromethane (30.0 mL) and then washed with water (75.0 mL × 2) and saturated sodium chloride (75.0 mL × 2). The organic phase was dried over anhydrous sodium sulfate, filtered, and concentrated under reduced pressure. The crude product was purified by silica gel column chromatography (petroleum ether/ethyl acetate = 1 : 0 to 0 : 1) to obtain compound **27-10.** MS-ESI [M+H]⁺, calculated: 746, found: 746.
(10) Compound **27-10** (14.0 mg, 18.7 µmol) was dissolved in 1.0 mL of trifluoroacetic acid, and trifluoromethanesulfonic acid (1.13 mmol, 0.1 mL) was added to the solution. The mixture was stirred at 25°C for 30 minutes. The reaction mixture was then adjusted to a pH of 7-8 with saturated sodium bicarbonate and extracted with ethyl acetate (20.0 mL). The organic phase was washed sequentially with water (30.0 mL × 2) and saturated sodium chloride (30.0 mL × 2), dried over anhydrous sodium sulfate, filtered, and concentrated under reduced pressure. The crude product was further purified by preparative high-performance liquid chromatography (C18-6, 100 mm × 30 mm, 5 µm, A: water (0.225% formic acid); B: acetonitrile (10%-70%) over 15 minutes) to obtain compound **27** formate. MS-ESI [M+H]⁺, calculated: 536, found: 536. ¹H NMR (400 MHz, CDCl₃) *δ* 8.60 (s, 2H), 7.87 (s, 1H), 4.48-4.52 (m, 1H), 3.96-4.03 (m, 3H), 3.90-3.95 (m, 2H), 3.63-3.72 (m, 4H), 3.56-3.61 (m, 1H), 3.46-3.51 (m, 1H), 2.46-2.57 (m, 2H), 2.21-2.30 (m, 1H), 2.11-2.19 (m, 1H), 2.01-2.09 (m, 1H), 1.93-2.00 (m, 1H).

### Example 28 Synthesis of Compound 28

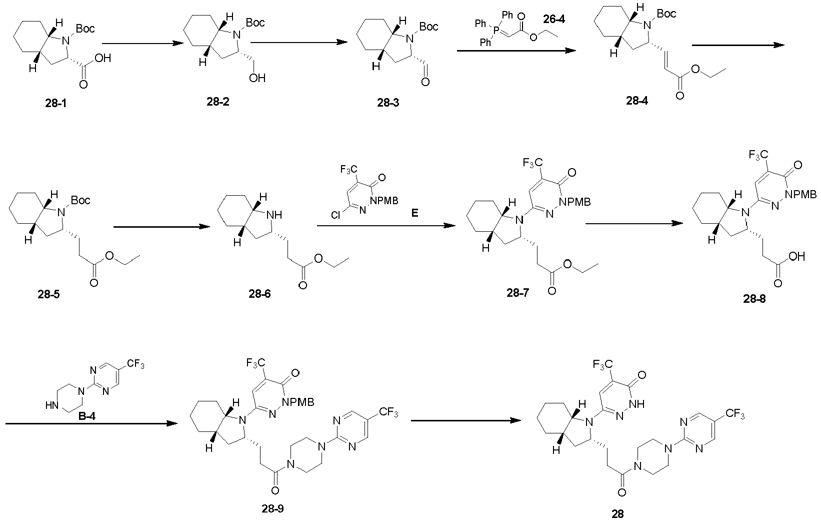

(1) Compound **28-1** (4.00 g, 14.9 mmol) was dissolved in 40.0 mL of tetrahydrofuran and cooled to 0°C. A solution of borane in THF (1 M, 44.6 mL) was added slowly, and the reaction was allowed to proceed at 25°C for 2 hours. After the reaction was complete, the reaction mixture was cooled to 0°C and quenched with methanol (50.0 mL). Water (50.0 mL) and ethyl acetate (50.0 mL × 3) were added for extraction. The combined organic phase was washed with water (150 mL), dried over anhydrous sodium sulfate, filtered, and concentrated under reduced pressure to obtain compound **28-2.** MS-ESI [M+H]⁺, calculated: 256, found: 256.
(2) Compound **28-2** (3.30 g, 11.6 mmol) was dissolved in 40.0 mL of dichloromethane, and Dess-Martin reagent (7.40 g, 17.5 mmol) and sodium bicarbonate (2.93 g, 34.9 mmol) were added. The reaction mixture was allowed to react at 25°C for 12 hours. After the reaction, it was quenched with saturated sodium sulfite solution (5.0 mL), and then extracted with dichloromethane (15.0 mL × 3). The combined organic phase was dried over anhydrous sodium sulfate, filtered, and concentrated under reduced pressure. The crude product was purified by silica gel column chromatography ( petroleum ether/ethyl acetate = 20:1 to 2:1) to obtain compound **28-3.**
(3) To a solution of compound **28-3** (1.00 g, 3.95 mmol) in 10.0 mL of dichloromethane, compound **26-4** (1.51 g, 4.34 mmol) was added. The reaction proceeded at 25°C for 12 hours. Water (50.0 mL) was added to the reaction mixture, and then it was extracted with dichloromethane (15.0 mL × 3). The combined organic phase was dried over anhydrous sodium sulfate, filtered, and concentrated under reduced pressure. The crude product was purified by silica gel column chromatography ( petroleum ether/ethyl acetate = 20:1 to 2:1) to obtain compound **28-4.**
(4) Compound **28-4** (580 mg, 1.79 mmol) was dissolved in 5.0 mL of tetrahydrofuran, and 10% palladium on carbon (100 mg) was added. The reaction mixture was stirred at 25°C under a hydrogen atmosphere (15 psi) for 2 hours. It was then filtered, and the filtrate was concentrated under reduced pressure to obtain compound **28-5.** MS-ESI [M+H]⁺, calculated: 326, found: 326.
(5) Compound **28-5** (300 mg, 963 µmol) was dissolved in 2.0 mL of dichloromethane, and trifluoroacetic acid (0.75 mL, 10.1 mmol) was added to the solution. The reaction mixture was stirred at 25°C for 1 hour. It was then adjusted to a pH of 7 using saturated sodium bicarbonate solution and extracted with dichloromethane (15.0 mL × 3). The combined organic phase was dried over anhydrous sodium sulfate, filtered, and concentrated under reduced pressure. The crude product was purified by silica gel column chromatography ( petroleum ether/ethyl acetate = 20:1 to 2:1) to obtain compound **28-6.** ¹H NMR (400 MHz, CDCl₃) *δ* 4.12 (q, *J=* 7.2 Hz, 2H), 3.02-3.22 (m, 2H), 2.36-2.44 (m, 2H), 1.93-2.05 (m, 2H), 1.79-1.91 (m, 2H), 1.59-1.71 (m, 2H), 1.43-1.58 (m, 4H), 1.29-1.38 (m, 2H), 1.25 (t, *J=* 7.2 Hz, 5H).
(6) To a mixture of compound **E** (70.7 mg, 222 µmol), compound **28-6** (50.0 mg, 222 µmol), cesium carbonate (145 mg, 444 µmol), and (2-dicyclohexylphosphino-2',6'-diisopropoxy-1,1'-biphenyl)[(2'-amino-1,1'-biphenyl-2-yl)]palla dium methanesulfonate (37.1 mg, 44.4 µmol) was added 1,2-dimethoxyethane (1.5 mL). The reaction mixture was stirred at 100°C for 12 hours under nitrogen protection. The reaction mixture was filtered, and the solvent was removed under reduced pressure. The crude product was purified by silica gel column chromatography ( petroleum ether/ethyl acetate =1:0 to 4:3) to obtain compound **28-7.** MS-ESI [M+H]⁺, calculated: 508, found: 508.
(7) Compound **28-7** (30.0 mg, 59.1 µmol) was dissolved in tetrahydrofuran (0.6 mL) and water (0.2 mL), and then lithium hydroxide monohydrate (50.0 mg, 1.19 mmol) was added. The reaction proceeded at 25°C for 12 hours. Hydrochloric acid solution (1 mol/L) was added to the reaction mixture to adjust the pH to 6. The mixture was then extracted with dichloromethane (10.0 mL × 3). The combined organic phase was washed with saturated sodium chloride (20.0 mL), dried over anhydrous sodium sulfate, filtered, and concentrated under reduced pressure to obtain compound **28-8.** MS-ESI [M+H]⁺, calculated: 480, found: 480.
(8) To a solution of compound **28-8** (20.0 mg, 41.7 µmol) and compound **B-4** (13.0 mg, 55.9 µmol) in dichloromethane (3.0 mL), diisopropylethylamine (0.03 mL, 172 µmol) was added. The reaction mixture was cooled to 0°C, and 50% propylphosphonic anhydride solution (0.03 mL, 50.4 µmol) was added. The reaction proceeded at 25°C for 2 hours. Water (10.0 mL) was added to the reaction mixture, and it was extracted with dichloromethane (10.0 mL × 3). The combined organic phase was dried over anhydrous sodium sulfate, filtered, and concentrated under reduced pressure. The crude product was separated by silica gel column chromatography (petroleum ether/ethyl acetate = 1:0 to 67:33) to obtain compound **28-9.** MS-ESI [M+H]⁺, calculated: 694, found: 694.
(9) Compound **28-9** (15.0 mg, 21.6 µmol) was dissolved in dichloromethane (1.0 mL), and trifluoromethanesulfonic acid (1.13 mmol, 0.1 mL) was added. The reaction mixture was stirred at 25°C for 1 hour. The reaction mixture was then concentrated under reduced pressure. The crude product was separated using preparative high-performance liquid chromatography (Xtimate C18, 100 mm × 30 mm 10 µm, A: water (0.225% formic acid) and B: acetonitrile (60%-90% ) over 10 minutes) to obtain compound **28** formate. MS-ESI [M+H]⁺, calculated: 574, found: 574. ¹H NMR (400 MHz, MeOD) *δ* 8.60 (s, 2H), 7.66 (s, 1H), 3.87-4.05 (m, 5H), 3.77-3.85 (m, 1H), 3.62-3.74 (m, 4H), 2.46-2.58 (m, 2H), 2.25-2.40 (m, 2H), 2.10-2.20 (m, 1H), 1.90-2.05 (m, 2H), 1.80-1.88 (m, 1H), 1.60-1.78 (m, 3H), 1.39-1.56 (m, 2H), 1.27-1.37 (m, 2H).

### Example 29 Synthesis of Compound 29

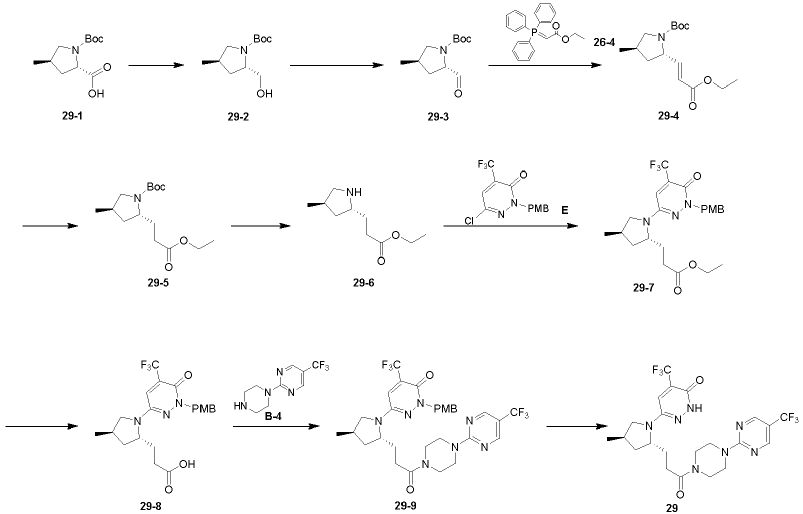

(1) Compound **29-1** (1.00 g, 4.36 mmol ) was dissolved in tetrahydrofuran (15.0 mL). The solution was cooled to 0°C. A solution of borane in tetrahydrofuran (1M, 19.6 mL) was added slowly. The reaction mixture was stirred at 25°C for 5 hours. After the reaction was completed, the mixture was cooled to 0°C and quenched with methanol (25.0 mL). It was extracted with water (50.0 mL) and dichloromethane (300.0 mL). The organic phase was dried over anhydrous sodium sulfate, filtered, and concentrated under reduced pressure. The crude product was purified by silica gel column chromatography (petroleum ether/ethyl acetate = 1 : 0 to 4 : 1) to obtain compound **29-2.** ¹H NMR (400 MHz, CDCl₃) *δ* 4.00-4.08 (m, 1H), 3.62 (d, *J =* 5.6 Hz, 2H), 3.49 (dd, *J =* 10.8, 7.2 Hz, 1H), 2.97 (dd, *J =* 10.8, 8.4 Hz, 1H), 2.22-2.34 (m, 1H), 1.58-1.81 (m, 2H), 1.48 (s, 9H), 1.02 (d, *J=* 6.4 Hz, 3H).
(2) Compound **29-2** (938 mg, 4.36 mmol) was dissolved in dichloromethane (20.0 mL), and Dess-Martin reagent (2.22 g, 5.23 mmol) was added. The reaction mixture was allowed to react at 25°C for 12 hours. The reaction was quenched with saturated sodium bisulfite solution (30.0 mL), and then extracted with dichloromethane (100 mL). The organic layer was dried over anhydrous sodium sulfate, filtered, and concentrated under reduced pressure. The crude product was purified by silica gel column chromatography ( petroleum ether/ethyl acetate = 1 : 0 to 9 : 1) to obtain compound **29-3.** ¹H NMR (400 MHz, CDCl₃) *δ* 9.51-9.64 (m, 1H), 4.06-4.21 (m, 1H), 3.60-3.72 (m, 1H), 2.92-3.10 (m, 1H), 2.19-2.30 (m, 1H), 2.07-2.17 (m, 1H), 1.66-1.83 (m, 1H), 1.48 (s, 3H), 1.43 (s, 6H), 1.02-1.10 (m, 3H).
(3) Compound **29-3** (620 mg, 2.91 mmol) was dissolved in dichloromethane (12.0 mL), and compound **26-4** (1.22 g, 3.49 mmol) was added. The reaction mixture was allowed to react at 25°C for 12 hours. The reaction mixture was then concentrated under reduced pressure. The crude product was purified by silica gel column chromatography (petroleum ether/ethyl acetate = 1 : 0 to 9 : 1) to obtain compound **29-4.** ¹H NMR (400 MHz, CDCl₃) *δ* 6.84 (br d, *J =* 13.6 Hz, 1H), 5.82 (br d, *J =* 15.6 Hz, 1H), 4.34-4.57 (m, 1H), 4.12-4.23 (m, 2H), 3.50-3.63 (m, 1H), 2.82-3.05 (m, 1H), 2.22-2.36 (m, 1H), 1.80-1.90 (m, 1H), 1.75 (br s, 1H), 1.43 (s, 9H), 1.29 (t, *J* = 7.2 Hz, 3H), 1.04 (d, *J =* 6.8 Hz, 3H).
(4) Compound **29-4** (630 mg, 2.22 mmol) was dissolved in tetrahydrofuran (6.0 mL), and 10% palladium on carbon (200 mg) was added. The reaction mixture was stirred at 25°C under a hydrogen atmosphere (15 psi) for 2 hours. The mixture was filtered, and the filtrate was concentrated under reduced pressure to obtain compound **29-5.** ¹H NMR (400 MHz, CDCl₃) *δ* 4.12 (q, *J =* 7.2 Hz, 2H), 3.78-3.87 (m, 1H), 3.45 (dd, *J* = 10.4, 7.6 Hz, 1H), 2.90 (t, *J =* 9.6 Hz, 1H), 2.27-2.36 (m, 3H), 1.94-2.04 (m, 1H), 1.65-1.77 (m, 2H), 1.53-1.65 (m, 1H), 1.46 (s, 9H), 1.26 (t, *J=* 7.2 Hz, 3H), 1.00-1.05 (m, 3H).
(5) Compound **29-5** (620 mg, 2.17 mmol) was dissolved in dichloromethane (6.0 mL), and trifluoroacetic acid (3.08 g, 27.0 mmol, 2.0 mL) was added. The reaction mixture was stirred at 25°C for 0.5 hours. It was concentrated under reduced pressure, and the residue was dissolved in dichloromethane (5.0 mL). The pH was adjusted to 10 using saturated sodium bicarbonate solution, and it was extracted with dichloromethane (50.0 mL). The organic phase was dried over anhydrous sodium sulfate, filtered, and concentrated under reduced pressure to obtain compound **29-6.**
(6) Compound **E** (250 mg, 785 µmol) and **29-6** (132 mg, 713 µmol) were dissolved in toluene (4.0 mL), and cesium carbonate (465 mg, 1.43 mmol), 1,1'-binaphthyl-2,2'-diphenylphosphine (88.8 mg, 143 µmol), and tris(dibenzylideneacetone)dipalladium (65.3 mg, 71.3 µmol) were added. The reaction was carried out at 80°C under a nitrogen atmosphere for 4 hours. The reaction mixture was filtered and concentrated under reduced pressure. The residue was dissolved in ethyl acetate (50.0 mL) and washed with water (50.0 mL). The organic phase was dried over anhydrous sodium sulfate, filtered, and concentrated under reduced pressure to obtain compound **29-7.** MS-ESI [M+H]⁺, calculated: 468, found: 468.¹H NMR (400 MHz, CDCl₃) *δ* 7.41-7.50 (m, 2H), 7.37 (s, 1H), 6.82-6.91 (m, 2H), 5.15 (d, *J=* 2.4 Hz, 2H), 4.09-4.17 (m, 2H), 3.87-3.96 (m, 1H), 3.79 (s, 3H), 3.60 (dd, *J =* 9.6, 7.6 Hz, 1H), 2.82 (t, *J =* 9.2 Hz, 1H), 2.42-2.54 (m, 1H), 2.27-2.39 (m, 2H), 1.93-2.03 (m, 1H), 1.89 (dd, *J =* 12.4, 6.4 Hz, 1H), 1.62-1.71 (m, 2H), 1.23-1.30 (m, 3H), 1.12 (d, *J* = 6.8 Hz, 3H).
(7) Compound **29-7** (50.0 mg, 107 µmol) was dissolved in tetrahydrofuran (2.0 mL) and water (0.5 mL), and then lithium hydroxide monohydrate (25.0 mg, 1.04 mmol) was added. The reaction was allowed to proceed at 25°C for 12 hours. To the reaction mixture, 1 M hydrochloric acid solution was added to adjust the pH to 5. The mixture was extracted with dichloromethane (50.0 mL). The organic phase was dried over anhydrous sodium sulfate, filtered, and concentrated under reduced pressure to obtain compound **29-8.** MS-ESI [M+H]⁺, calculated: 440, found: 440.¹H NMR (400 MHz, CDCl₃) *δ* 7.38-7.47 (m, 3H), 6.85 (d, *J=* 8.4 Hz, 2H), 5.15 (d, *J =* 5.6 Hz, 2H), 3.78 (s, 3H), 3.60 (dd, *J =* 9.6, 7.2 Hz, 1H), 2.77-2.87 (m, 1H), 2.34-2.53 (m, 3H), 1.89 (br dd, *J =* 12.8, 6.0 Hz, 2H), 1.57-1.71 (m, 3H), 1.12 (d, *J =* 6.4 Hz, 3H).
(8) Compound **29-8** (45.0 mg, 102 µmol) and compound **B-4** (33.0 mg, 123 µmol) were dissolved in dichloromethane (2.0 mL), diisopropylethylamine (66.2 mg, 512 µmol) and 50% propylphosphonic anhydride solution (196 mg, 307 µmol, 183 µL) were added. The reaction was allowed to proceed at 25°C for 2 hours. To the reaction mixture, water (10.0 mL) was added, and the mixture was extracted with ethyl acetate (50.0 mL). The organic phase was dried over anhydrous sodium sulfate, filtered, and concentrated under reduced pressure. The crude product was purified by silica gel column chromatography (petroleum ether/ethyl acetate = 1 : 0 to 7 : 3) to obtain compound **29-9.** MS-ESI [M+H]⁺, calculated: 654, found: 654. ¹H NMR (400 MHz, CDCl₃) *δ* 8.53 (s, 2H), 7.65 (s, 1H), 7.44 (d, *J* = 8.8 Hz, 2H), 6.81-6.89 (m, 2H), 5.12-5.19 (m, 2H), 3.88-3.96 (m, 5H), 3.77-3.79 (m, 3H), 3.72 (br s, 2H), 3.61 (dd, *J=* 10.0, 7.6 Hz, 1H), 3.45-3.54 (m, 2H), 2.84 (t, *J =* 9.6 Hz, 1H), 2.45-2.58 (m, 1H), 2.31-2.43 (m, 2H), 1.98-2.05 (m, 1H), 1.91 (dd, J=12.4, 6.0 Hz, 1H), 1.63-1.77 (m, 2H), 1.13 (d, *J* = 6.4 Hz, 3H).
(9) Compound **29-9** (38.0 mg, 58.1 µmol) was dissolved in trifluoroacetic acid (1.0 mL), and trifluoromethanesulfonic acid (1.13 mmol, 0.1 mL) was added. The reaction mixture was stirred at 25°C for 1 hour. It was then adjusted to a pH of 7 using saturated sodium bicarbonate solution and extracted with ethyl acetate (50.0 mL). The organic phase was dried over anhydrous sodium sulfate, filtered, and concentrated under reduced pressure. The crude product was purified using preparative high-performance liquid chromatography (Xtimate C18, 100 mm × 30 mm, 10 µm, A: water (0.225% formic acid), B: acetonitrile (58% to 78%) over 10 minutes) to obtain compound **29** formate. MS-ESI [M+H]⁺, calculated: 534, found: 534. ¹H NMR (400 MHz, MeOD) *δ* 8.60 (s, 2H), 7.88 (s, 1H), 3.89-4.03 (m, 5H), 3.61-3.72 (m, 5H), 2.88 (t, *J =* 9.6 Hz, 1H), 2.46-2.58 (m, 3H), 1.96-2.04 (m, 2H), 1.63-1.75 (m, 2H), 1.13 (d, *J=* 6.4 Hz, 3H).

### Example 30 Synthesis of Compound 30

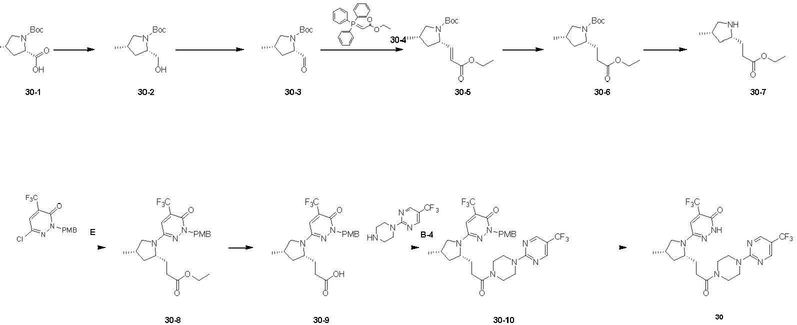

(1) Compound **30-1** (500 mg, 2.18 mmol) was dissolved in 6.0 mL of tetrahydrofuran at 0°C, and a solution of borane THF complex (1 M, 10.9 mL) was added. The reaction mixture was stirred at 25°C for 12 hours. Afterward, the reaction mixture was cooled to 0°C, and 10.0 mL of methanol was added. The mixture was concentrated under reduced pressure. The crude product was purified by silica gel column chromatography (petroleum ether/ethyl acetate = 100:1 to 3:1) to obtain compound **30-2.** ¹H NMR (400 MHz, CDCl₃) *δ* 3.89-3.99 (m, 1H), 3.64-3.75 (m, 2H), 3.54-3.63 (m, 1H), 2.89 (br s, 1H), 2.77 (t, *J=* 10.4 Hz, 1H), 2.05-2.18 (m, 2H), 1.47 (s, 9H), 1.02 (d, *J=* 6.0 Hz, 3H).
(2) Compound **30-2** (520 mg, 2.42 mmol) was dissolved in 8.0 mL of dichloromethane, and Dess-Martin reagent (1.23 g, 2.90 mmol) was added. The reaction mixture was stirred at 25°C for 12 hours. Sodium sulfite solution (20.0 mL) was added to the reaction mixture, and it was then extracted with 100 mL of dichloromethane. The organic layer was dried over anhydrous sodium sulfate, filtered, and concentrated under reduced pressure. The crude product was further purified by silica gel column chromatography (petroleum ether/ethyl acetate = 100:1 to 1:1) to obtain compound **30-3.** MS-ESI [M-^{t}Bu+H]⁺, calculated: 158, found: 158. ¹H NMR (400 MHz, CDCl₃) *δ* 9.37-9.52 (m, 1H), 3.99-4.14 (m, 1H), 3.66-3.82 (m, 1H), 2.92-3.03 (m, 1H), 2.19-2.31 (m, 2H), 1.47-1.48 (m, 3H), 1.43 (s, 7H), 1.05-1.10 (m, 3H).
(3) Compound **30-3** (350 mg, 1.64 mmol) was dissolved in 6.0 mL of dichloromethane, and compound **30-4** (629 mg, 1.81 mmol) was added. The reaction mixture was stirred at 25°C for 12 hours. Water (20.0 mL) was added to the reaction mixture, and it was then extracted with 100 mL of dichloromethane. The organic layer was dried over anhydrous sodium sulfate, filtered, and concentrated under reduced pressure. The crude product was further purified by silica gel column chromatography (petroleum ether/ethyl acetate = 100:1 to 1:1) to obtain compound **30-5.** MS-ESI [M-^{t}Bu+H]⁺, calculated: 228, found: 228. ¹H NMR (400 MHz, CDCl₃) *δ* 6.84 (br d, *J =* 9.2 Hz, 1H), 5.85 (br d, *J =* 15.2 Hz, 1H), 4.20 (q, *J =* 7.2 Hz, 2H), 3.68-3.83 (m, 1H), 2.86 (t, *J=* 10.4 Hz, 1H), 2.12-2.30 (m, 2H), 1.42 (br s, 11H), 1.29 (t, *J=* 7.2 Hz, 3H), 1.05 (d, *J* = 6.4 Hz, 3H).
(4) Compound **30-5** (340 mg, 1.20 mmol) was dissolved in 5.0 mL of tetrahydrofuran, and 10% palladium on carbon (100 mg) was added to the solution. The reaction mixture was stirred at 25°C under a hydrogen atmosphere for 2 hours. Afterward, the reaction mixture was filtered, and the solvent was removed under reduced pressure to obtain compound **30-6.** ¹H NMR (400 MHz, CDCl₃) *δ* 3.71-3.81 (m, 2H), 2.64-2.72 (m, 1H), 2.26-2.31 (m, 2H), 2.15-2.24 (m, 2H), 2.06-2.11 (m, 1H), 1.71-1.82 (m, 2H), 1.46 (s, 9H), 1.40-1.43 (m, 1H), 1.23-1.26 (m, 3H), 1.06-1.20 (m, 1H), 1.01 (d, *J=* 6.4 Hz, 3H)
(5) Compound **30-6** (320 mg, 1.12 mmol) was dissolved in 3.0 mL of dichloromethane, and trifluoroacetic acid (1.45 g, 12.7 mmol) was added to the solution. The reaction mixture was stirred at 25°C for 0.5 hours, adjusted to pH 10 using sodium carbonate, and then extracted with 100 mL of dichloromethane. The organic phase was dried over anhydrous sodium sulfate, filtered, and concentrated under reduced pressure to obtain compound **30-7.**
(6) Compound **30-7** (100 mg, 540 µmol) was dissolved in 2.0 mL of toluene, and compound **E** (190 mg, 594 µmol), cesium carbonate (352 mg, 1.08 mmol), 1,1'-binaphthyl-2,2'-diphenylphosphine (67.2 mg, 108 µmol), and tris (dibenzylideneacetone) dipalladium (49.4 mg, 54.0 µmol) were added to the solution. The reaction mixture was stirred at 80°C for 5 hours under a nitrogen atmosphere. Water (20.0 mL) was added to the reaction mixture, and the mixture was extracted with ethyl acetate (100 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **30-8.** MS-ESI [M+H]⁺, calculated: 469, found: 469. ¹H NMR (400 MHz, CDCl₃) *δ* 7.43-7.47 (m, 2H), 7.26 (s, 1H), 6.83-6.87 (m, 2H), 5.18-5.25 (m, 1H), 5.04-5.11 (m, 1H), 4.08-4.19 (m, 2H), 3.83-3.91 (m, 1H), 3.77-3.80 (m, 3H), 3.54 (dd, *J =* 9.2, 7.8 Hz, 1H), 3.02 (t, *J=* 9.6 Hz, 1H), 2.27-2.38 (m, 3H), 2.16-2.26 (m, 2H), 1.63-1.73 (m, 1H), 1.32-1.41 (m, 1H), 1.23-1.27 (m, 3H), 1.12 (d, *J=* 6.5 Hz, 3H)
(7) Compound **30-8** (59.0 mg, 126 µmol) was dissolved in 1.2 mL of tetrahydrofuran. Water (0.4 mL) and lithium hydroxide monohydrate (53.0 mg, 1.26 mmol) were added. The reaction mixture was stirred at 25°C under a nitrogen atmosphere for 3 hours. The pH was adjusted to less than 7 by adding 1 M hydrochloric acid solution. The mixture was then extracted with ethyl acetate (60.0 mL). The organic phase was dried over anhydrous sodium sulfate, filtered, and concentrated under reduced pressure to obtain compound **30-9.** MS-ESI [M+H]⁺, calculated: 440, found: 440.
(8) To a solution of compound **30-9** (70.0 mg, 159 µmol) in 1.0 mL of dichloromethane, compound **B-4** trifluoroacetate (59.9 mg, 223 µmol), diisopropylethylamine (103 mg, 796 µmol), and a solution of propylphosphonic anhydride in ethyl acetate (304 mg, 478 µmol, 50% purity) were added. The reaction mixture was stirred at 25°C under a nitrogen atmosphere for 4 hours. The pH was adjusted to 7 by adding 1 M hydrochloric acid solution. The mixture was then extracted with dichloromethane (50.0 mL). The organic phase was dried over anhydrous sodium sulfate, filtered, and concentrated under reduced pressure. The crude product was purified by silica gel column chromatography ( petroleum ether/ethyl acetate = 100:1 to 1:1) to obtain compound **30-10.** MS-ESI [M+H]⁺, calculated: 654, found: 654. ¹H NMR (400 MHz, CDCl₃) *δ* 8.54 (s, 2H), 7.41-7.48 (m, 3H), 6.81-6.87 (m, 2H), 5.17-5.23 (m, 1H), 5.06-5.12 (m, 1H), 3.92 (br s, 5H), 3.77-3.79 (m, 3H), 3.66-3.75 (m, 2H), 3.56-3.61 (m, 1H), 3.43-3.49 (m, 1H), 3.04 (t, *J =* 9.6 Hz, 1H), 2.32-2.41 (m, 3H), 2.17-2.28 (m, 2H), 1.66-1.77 (m, 1H), 1.33-1.45 (m, 2H), 1.13 (d, *J =* 6.4 Hz, 3H).
(9) To a solution of compound **30-10** in trifluoroacetic acid (1.0 mL), trifluoromethanesulfonic acid (0.10 mL) was added. The reaction mixture was stirred at 25°C for 1 hour. The pH of the reaction mixture was adjusted to 7 using sodium bicarbonate. The mixture was then extracted with ethyl acetate (50.0 mL). The organic phase was dried over anhydrous sodium sulfate, filtered, and concentrated under reduced pressure. The crude product was purified by preparative high-performance liquid chromatography (Xtimate C18,100 mm × 30 mm 10 µm, A: water (0.225% formic acid) and B: acetonitrile (50%-80%) over 10 minutes) to obtain compound **30** formate. MS-ESI [M+H]⁺, calculated: 534, found: 534. ¹H NMR (400 MHz, MeOD) *δ* 8.60 (s, 2H), 7.71 (s, 1H), 3.85-3.99 (m, 5H), 3.59-3.69 (m, 5H), 3.07 (t, *J =* 9.6 Hz, 1H), 2.47 (t, *J =* 7.6 Hz, 2H), 2.35-2.42 (m, 1H), 2.15-2.27 (m, 2H), 1.74 (br dd, *J =* 13.6, 8.4 Hz, 1H), 1.45 (dt, *J =* 10.4, 1.8 Hz, 1H), 1.14 (d, *J =* 6.4 Hz, 3H).

### Example 31 Synthesis of Compound 31

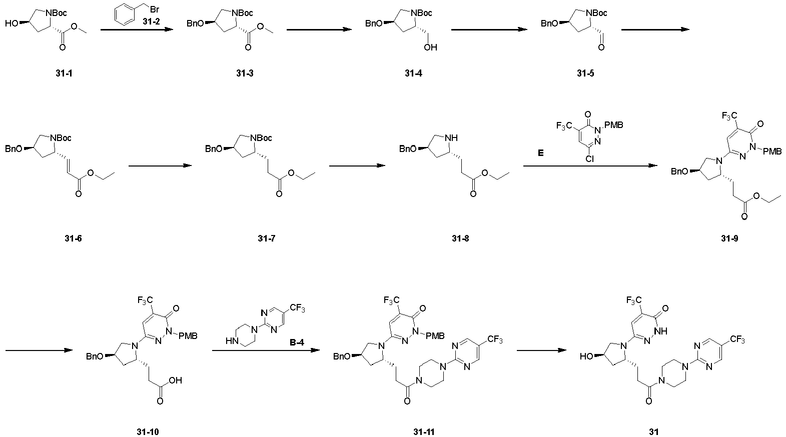

(1) At 0°C, sodium hydride (3.91 g, 97.9 mmol) was added to a solution of compound **31-1** (20.0 g, 81.5 mmol) in N,N-dimethylformamide (150 mL). The reaction mixture was stirred at 0°C for 0.5 hours. Subsequently, a solution of compound **31-2** (16.7 g, 97.9 mmol) in N,N-dimethylformamide (150 mL) was added to the reaction mixture. The reaction mixture was stirred at 25°C under a nitrogen atmosphere for 15.5 hours. It was diluted with ethyl acetate (500 mL), adjusted the pH to below 7 by saturating with ammonium chloride, and washed with water (500 mL) and saturated sodium chloride solution (400 mL × 3). The organic phase was dried over anhydrous sodium sulfate, filtered, and concentrated under reduced pressure. The crude product was separated by silica gel column chromatography (petroleum ether/ethyl acetate = 1:0 to 6:1) to obtain compound **31-3.** MS-ESI [M-Boc+H]⁺, calculated: 236, found: 236.
(2) At 0°C, lithium aluminum hydride (747 mg, 19.7 mmol) was added to a solution of compound **31-3** (6.0 g, 17.9 mmol) in tetrahydrofuran (50.0 mL). The reaction mixture was stirred at 0°C for 0.5 hours. At 0°C, water (1.0 mL), 15% sodium hydroxide solution (1.0 mL) and water (3.0 mL) were added to the reaction mixture. The mixture was filtered, and the filtrate was concentrated under reduced pressure. The crude product was separated by silica gel column chromatography (petroleum ether/ethyl acetate = 1:0 to 15:1) to obtain compound **31-4.** MS-ESI [M+H]⁺, calculated: 252, found: 252. ¹H NMR (400 MHz, CDCl₃) *δ* 7.27-7.39 (m, 5H), 4.48-4.54 (m, 2H), 4.04-4.09 (m, 1H), 3.65-3.80 (m, 2H), 3.56 (dd, *J* = 11.2, 7.2 Hz, 1H), 3.40 (dd, *J=* 12.0, 4.4 Hz, 1H), 2.40 (s, 2H), 2.15-2.28 (m, 1H), 1.48 (s, 9H).
(3) Dess-Martin reagent (10.6 g, 24.9 mmol) was added to a solution of compound **31-4** (5.1 g, 16.6 mmol) in dichloromethane (50.0 mL). The reaction mixture was stirred at 25°C for 2 hours. The reaction mixture was quenched with sodium thiosulfate (10.0 mL) and extracted with ethyl acetate (200 mL).The organic phase was washed with water (100 mL × 3) and saturated sodium chloride (100 mL × 3), dried over anhydrous sodium sulfate, filtered, concentrated under reduced pressure. And the crude product was purified by silica gel column chromatography (petroleum ether/ethyl acetate = 1:0 to 13:1) to yield compound **31-5.** ¹H NMR (400 MHz, CDCl₃) *δ* 9.42-9.57 (m, 1H), 7.28-7.40 (m, 5H), 4.47-4.58 (m, 2H), 4.21-4.40 (m, 1H), 4.15 (dd, *J =* 4.0, 2.0 Hz, 1H), 3.52-3.82 (m, 2H), 2.22-2.34 (m, 1H), 1.91-2.02 (m, 1H), 1.42-1.49 (m, 9H).
(4) To a solution of sodium hydride (235 mg, 5.87 mmol, 60%) in tetrahydrofuran (12.0 mL), triethyl phosphonoacetate (1.22 g, 5.42 mmol) was added at 0°C, and the reaction mixture was stirred for 0.5 hours. Then, a solution of compound **31-5** (1.38 g, 4.52 mmol) in tetrahydrofuran (13.0 mL) was added, and the reaction was stirred for an additional 1.5 hours at 0°C under a nitrogen atmosphere. Ethyl acetate (500 mL) was added to the reaction mixture. The pH was adjusted to less than 7 with saturated ammonium chloride solution. After phase separation, the organic phase was washed with water (50.0 mL) and saturated sodium chloride (40.0 mL × 3), dried over anhydrous sodium sulfate, filtered, concentrated under reduced pressure to obtain compound **31-6.** MS-ESI [M-Boc+H]⁺, calculated: 276, found: 276. ¹H NMR (400 MHz, CDCl₃) *δ* 7.29-7.38 (m, 5H), 6.81 (s, 1H), 5.79-5.92 (m, 1H), 4.48-4.56 (m, 2H), 4.17-4.25 (m, 2H), 3.72-3.82 (m, 1H), 3.48 (d, *J=* 8.4 Hz, 1H), 2.28 (s, 1H), 1.87-1.93 (m, 1H), 1.68 (s, 2H), 1.43 (d, *J=* 4.4 Hz, 9H), 1.28-1.32 (m, 3H).
(5) To a solution of compound **31-6** (1.68 g, 4.47 mmol) in tetrahydrofuran (20.0 mL) was added 10% palladium on carbon (150 mg), and the reaction mixture was stirred at 25°C under a hydrogen atmosphere for 3 hours. After filtration, the filtrate was concentrated under reduced pressure to isolate compound **31-7.** MS-ESI [M+H]⁺, calculated: 378, found: 378. ¹H NMR (400 MHz, DMSO-*d6*) *δ* 7.28-7.39 (m, 5H), 4.47-4.58 (m, 2H), 4.19 (d, *J* = 7.2 Hz, 1H), 4.10-4.16 (m, 2H), 3.78-4.03 (m, 1H), 3.32-3.77 (m, 2H), 2.17-2.38 (m, 2H), 1.84-1.94 (m, 1H), 1.78 (td, *J=* 12.4, 5.6 Hz, 1H), 1.42-1.55 (m, 9H), 1.35-1.42 (m, 2H), 1.27-1.32 (m, 3H).
(6) To a solution of compound **31-7** (1.8 g, 4.77 mmol) in dichloromethane (20.0 mL) was added trifluoroacetic acid (7.66 g, 67.2 mmol), and the reaction mixture was stirred at 25°C for 1 hour. The organic phase was neutralized with sodium bicarbonate solution (20.0 mL × 2) and extracted with ethyl acetate (20.0 mL). The organic phase was then washed with saturated sodium chloride (20.0 mL × 2), dried over anhydrous sodium sulfate, filtered, and concentrated under reduced pressure to obtain compound **31-8.** MS-ESI [M+H]⁺, calculated: 278, found: 278.
(7)To a solution of compound **E** (689 mg, 2.16 mmol) in toluene (20.0 mL), compound **31-8** (1.20 g, 4.33 mmol), cesium carbonate (1.41 g, 4.33 mmol), 1,1'-binaphthyl-2,2'-diphenylphosphine (674 mg, 1.08 mmol), and tris(dibenzylideneacetone)dipalladium (311 mg, 541 µmol) was added. The reaction mixture was stirred under a nitrogen atmosphere at 80°C for 3 hours. It was then extracted with ethyl acetate (50.0 mL). The organic phase was washed with saturated sodium chloride (30.0 mL × 3), dried over anhydrous sodium sulfate, filtered, concentrated under reduced pressure. The crude product was purified by silica gel column chromatography (petroleum ether/ethyl acetate = 1:0 to 9:1) to yield compound **31-9.** ¹H NMR (400 MHz, CDCl₃) *δ* 7.40 (d, *J* = 8.4 Hz, 2H), 7.29-7.35 (m, 4H), 7.07 (s, 1H), 6.80-6.87 (m, 3H), 5.89 (d, *J =* 15.6 Hz, 1H), 5.15-5.23 (m, 1H), 5.01-5.08 (m, 1H), 4.54-4.64 (m, 2H), 4.47-4.52 (m, 1H), 4.25-4.29 (m, 1H), 4.15-4.24 (m, 2H), 3.78 (s, 3H), 3.70 (dd, *J* = 10.8, 4.8 Hz, 1H), 3.52-3.59 (m, 1H), 2.36-2.44 (m, 1H), 2.21-2.36 (m, 1H), 2.02-2.14 (m, 1H), 1.74-2.02 (m, 1H), 1.60-1.66 (m, 1H), 1.30 (t, *J =* 7.2 Hz, 3H)
(8) To a solution of compound **31-9** (500 mg, 894 µmol) in tetrahydrofuran (8.0 mL), lithium hydroxide monohydrate (225 mg, 5.36 mmol) was added. The reaction mixture was stirred at 25°C under a nitrogen atmosphere for 3 hours. The pH was adjusted to 7 by adding 1 M hydrochloric acid solution, and then the mixture was extracted with ethyl acetate (50.0 mL). The organic phase was washed with saturated sodium chloride (30.0 mL × 3), dried over anhydrous sodium sulfate, filtered, and concentrated under reduced pressure to obtain compound **31-10.** MS-ESI [M+H]⁺, calculated: 532, found: 532.
(9) To a solution of compound **31-10** (377 mg, 709 µmol) in dichloromethane (6.0 mL), compound **B-4** trifluoroacetate (247 mg, 1.06 mmol), diisopropylethylamine (458 mg, 3.55 mmol), and a solution of propylphosphonic anhydride in ethyl acetate (1.81 g, 2.84 mmol, 50% purity) were added. The reaction mixture was stirred at 25°C under a nitrogen atmosphere for 3 hours. The reaction mixture was extracted with ethyl acetate (50.0 mL), washed with saturated sodium chloride (30.0 mL × 3), dried over anhydrous sodium sulfate, filtered, and concentrated under reduced pressure. The crude product was purified by silica gel column chromatography (petroleum ether/ethyl acetate = 1:0 to 19:1) to obtain compound **31-11.** MS-ESI [M+H]⁺, calculated: 746, found: 746.
(10)To a solution of compound **31-11** (54.0 mg, 72.4 µmol) in trifluoroacetic acid (3.0 mL), trifluoromethanesulfonic acid (432 µL) was added. The reaction mixture was stirred at 25°C for 0.5 hours. The reaction mixture was concentrated under reduced pressure. The crude product was purified by preparative high-performance liquid chromatography (Phenomenex C18, 75 mm × 30 mm, 3 µm, A: 10 mmol/L ammonium bicarbonate in water; B: acetonitrile, 23% to 53% over 11 minutes) to obtain compound 31. MS-ESI [M+H]⁺, calculated: 536, found: 536. ¹H NMR (400 MHz, MeOD) *δ* 8.60 (s, 2H), 7.80 (s, 1H), 4.45-4.54 (m, 1H), 4.08-4.16 (m, 1H), 3.95-4.00 (m, 2H), 3.92 (t, *J* = 5.2 Hz, 2H), 3.67-3.71 (m, 2H), 3.64 (dd, *J =* 10.4, 4.8 Hz, 3H), 3.38 (dd, *J=* 11.2, 2.0 Hz, 1H), 2.48 (t, *J=* 7.2 Hz, 2H), 2.11-2.18 (m, 2H),1.98-2.05 (m, 1H), 1.69-1.79 (m, 1H).

### Example 32 Synthesis of Compound 32

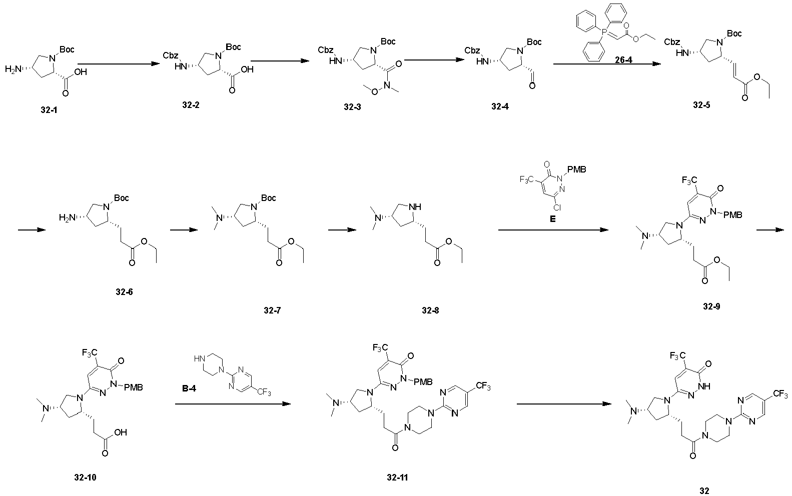

(1) To a solution of compound **32-1** (4.25 g, 18.5 mmol) in water (55.0 mL) was added sodium hydroxide (0.37 g, 9.25 mmol) and N-benzyloxycarbonyl succinimide (3.46 g, 20.3 mmol, 2.90 mL). The reaction was carried out at 25°C for 6 hours. After completion of the reaction, water (30.0 mL) was added, and the mixture was extracted with ethyl acetate (200 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **32-2.** ¹H NMR (400 MHz, CDCl₃) *δ* 7.30-7.43 (m, 5H), 5.11 (s, 2H), 4.74-4.86 (m, 1H), 4.25-4.42 (m, 2H), 3.74 (s, 4H), 3.23-3.43 (m, 1H), 2.11-2.30 (m, 2H), 1.46 (s, 3H), 1.40(s, 6H).
(2) Compound **32-2** (4.40 g, 12.0 mmol) was dissolved in tetrahydrofuran (40.0 mL) and cooled to 0°C. Borane-tetrahydrofuran complex (1 M, 30.2 mL) was added. The reaction mixture was allowed to react at 25°C for 2 hours. Methanol (25.0 mL) was added, and the reaction was continued at 25°C for an additional 2 hours. Water (100 mL) was added, and the mixture was extracted with ethyl acetate (200 mL). The organic phase was dried over anhydrous sodium sulfate, filtered, and concentrated under reduced pressure to obtain compound **32-3.** MS-ESI [M-Boc+H]⁺, calculated: 351, found: 351.
(3) Compound **32-3** (3.10 g, 8.85 mmol) was dissolved in dichloromethane (40.0 mL), and Dess-Martin reagent (7.50 g, 17.7 mmol) was added. The reaction mixture was allowed to react at 25°C for 5 hours. The reaction was quenched with saturated sodium sulfite solution (20.0 mL) and extracted with dichloromethane (100 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **32-4.** ¹H NMR (400 MHz, CDCl₃) *δ* 9.40-9.62 (m, 1H), 7.32-7.37 (m, 5H), 5.10 (s, 2H), 4.86 (br d, *J =* 2.4 Hz, 1H), 4.27 (br s, 1H), 4.12-4.19 (m, 1H), 3.72 (br dd, *J* = 11.2, 5.6 Hz, 1H), 3.26-3.48 (m, 1H), 2.05-2.29 (m, 2H), 1.46 (s, 3H), 1.42(s, 6H).
(4) Compound **32-4** (330 mg, 947 µmol) was dissolved in tetrahydrofuran (4.0 mL), and compound **26-4** (363 mg, 1.04 mmol) was added. The reaction proceeded at 25°C for 12 hours. The reaction mixture was then concentrated under reduced pressure. The crude product was purified by silica gel column chromatography (petroleum ether/ethyl acetate = 100:1 to 1:1) to obtain compound **32-5.** ¹H NMR (400 MHz, CDCl₃) *δ* 7.31-7.32 (m, 5H), 6.79 (br d, *J =* 5.2 Hz, 1H), 5.85 (br d, *J =* 15.6 Hz, 1H), 5.11 (s, 2H), 4.80 (br s, 1H), 4.39-4.60 (m, 1H), 4.28 (br d, *J* = 4.8 Hz, 1H), 4.20 (br d, *J =* 6.8 Hz, 2H), 3.70 (br s, 1H), 3.16-3.39 (m, 1H), 2.04-2.0.8 (m, 1H), 1.43 (br s, 9H), 1.27-1.34 (m, 3H).
(5) Compound **32-5** (295 mg, 705 µmol) was dissolved in tetrahydrofuran (5.0 mL), and 10% palladium on carbon (30 mg) was added. The reaction was stirred at 25°C under a hydrogen atmosphere (15 psi) for 1 hour. The reaction mixture was then filtered, and the filtrate was concentrated under reduced pressure to obtain compound **32-6.** MS-ESI [M-Boc+H]⁺, calculated: 287, found: 287.
(6) Compound **32-6** (150 mg, 524 µmol) was dissolved in ethanol (5.0 mL), and paraformaldehyde (157 mg, 5.24 mmol) and sodium cyanoborohydride (198 mg, 3.14 mmol) were added. The reaction was carried out at 25°C for 12 hours. After completion of the reaction, water (30.0 mL) was added, and the mixture was extracted with ethyl acetate (200 mL). The organic phase was dried over anhydrous sodium sulfate, filtered, and concentrated under reduced pressure. The crude product was purified by silica gel column chromatography (dichloromethane/methanol = 100:1 to 20:1) to obtain compound **32-7.** MS-ESI [M+H]⁺, calculated: 315, found: 315.¹H NMR (400 MHz, CDCl₃) *δ* 4.12 (q, *J =* 7.2 Hz, 2H), 3.78-4.03 (m, 2H), 2.99-3.09 (m, 1H), 2.52-2.63 (m, 1H), 2.32-2.37 (m, 2H), 2.31 (s, 6H), 1.80-1.94 (m, 2H), 1.48-1.63 (m, 2H), 1.47 (s, 9H), 1.25 (t, *J=* 7.2 Hz, 3H).
(7) Compound **32-7** (60.0 mg, 191 µmol) was dissolved in dichloromethane (1.5 mL), and trifluoroacetic acid (790 mg, 6.95 mmol, 514 µmol) was added. The reaction mixture was stirred at 25°C for 1 hour. The reaction was then adjusted to pH 9 using saturated sodium carbonate solution, and the mixture was extracted with ethyl acetate (50.0 mL). The organic phase was dried over anhydrous sodium sulfate, filtered, and concentrated under reduced pressure to obtain compound **32-8.** MS-ESI [M+H]⁺, calculated: 215, found: 215.
(8) To a solution of compound **E** (40.2 mg, 126 µmol) and compound **32-8** (30.0 mg, 140 µmol) in dioxane (1.0 mL) was added cesium carbonate (91.2 mg, 280 µmol) and Methanesulfonato(2-dicyclohexylphosphino-2',6'-di-i-propoxy-1,1'-biphenyl)(2'-amino-1,1'-bip henyl-2-yl)palladium(II) (23.4 mg, 28.0 µmol). The reaction mixture was stirred at 60°C under a nitrogen atmosphere for 5 hours. The reaction mixture was filtered, and the filtrate was concentrated under reduced pressure. The resulting residue was dissolved in dichloromethane (50.0 mL) and washed with water (30.0 mL). The organic phase was dried over anhydrous sodium sulfate, filtered, and concentrated under reduced pressure. The crude product was purified by silica gel column chromatography (dichloromethane/methanol = 100:1 to 10:1) to obtain compound **32-9.** MS-ESI [M+H]⁺, calculated: 497, found: 497.
(9) Compound **32-9** (50.0 mg, 101 µmol) was dissolved in tetrahydrofuran (0.9 mL) and water (0.3 mL), and then lithium hydroxide monohydrate (21.1 mg, 504 µmol) was added. The reaction was carried out at 55°C for 8 hours. To the reaction mixture, 1 M hydrochloric acid solution was added to adjust the pH to less than 7. The mixture was then extracted with ethyl acetate (50.0 mL). The organic phase was dried over anhydrous sodium sulfate, filtered, and concentrated under reduced pressure to obtain compound **32-10.** MS-ESI [M+H]⁺, calculated: 469, found: 469.
(10) Compound **32-10** (30.0 mg, 64.0 µmol) and compound **B-4** (9.3 mg, 71.9 µmol) was dissolved in dichloromethane (1.0 mL), and then diisopropylethylamine (33.1 mg, 256 µmol) and 50% propylphosphonic anhydride solution (48.9 mg, 76.9 µmol) were added. The reaction was carried out at 25°C for 4 hours. Water (5.0 mL) was added to the reaction mixture, and it was then extracted with ethyl acetate (50.0 mL). The organic phase was dried over anhydrous sodium sulfate, filtered, and concentrated under reduced pressure. The crude product was separated using preparative thin-layer chromatography (dichloromethane/methanol = 10:1) to obtain compound **32-11.** MS-ESI [M+H]⁺, calculated: 683, found: 683.
(11) Compound **32-11** (20.0 mg, 29.3 µmol) was dissolved in trifluoroacetic acid (1.0 mL), and then trifluoromethanesulfonic acid (340 mg, 2.27 mmol, 200 µL) was added. The reaction mixture was stirred at 25°C for 30 minutes. The reaction mixture was then adjusted to pH > 7 using saturated sodium carbonate solution and extracted with ethyl acetate (50.0 mL). The organic phase was dried over anhydrous sodium sulfate, filtered, and concentrated under reduced pressure. The crude product was separated using preparative high-performance liquid chromatography (Phenomenex C18, 75 mm × 30 mm 3 µm, A: water (10 mmol/L ammonium bicarbonate); B: acetonitrile, 25%-65%: 14 minutes) to obtain compound **32.** MS-ESI [M+H]⁺, calculated: 563, found: 563. ¹H NMR (400 MHz, MeOD) *δ* 8.60 (s, 2H), 7.82 (s, 1H), 4.02-4.17 (m, 2H), 3.95-4.00 (m, 2H), 3.92 (t, *J=* 5.2 Hz, 2H), 3.60-3.72 (m, 5H), 3.50-3.56 (m, 1H), 2.89 (s, 6H), 2.73-2.82 (m, 1H), 2.53 (t, *J=* 7.2 Hz, 2H), 2.18-2.27 (m, 1H), 1.79-1.96 (m, 2H).

### Example 33 Synthesis of Compound 33

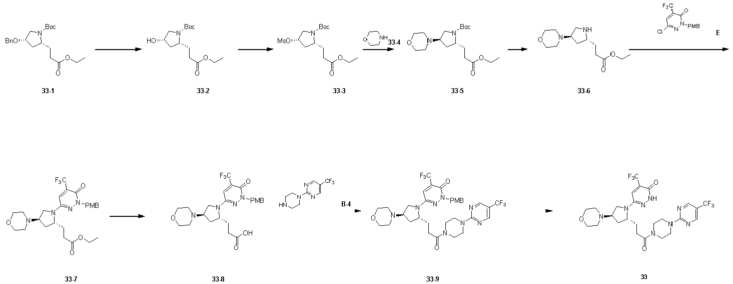

(1) To a solution of compound **33-1** (1.30 g, 3.44 mmol) in ethanol (30.0 mL), 10% palladium on carbon (500 mg) was added. The reaction mixture was stirred at 40°C under a hydrogen atmosphere for 14 hours. After filtration, the filtrate was concentrated under reduced pressure to obtain compound **33-2.** ¹H NMR (400 MHz, DMSO-*d6*) *δ* 4.96 (br s, 1H), 4.18 (br s, 1H), 4.04 (q, *J=* 7.2 Hz, 2H), 3.64-3.73 (m, 1H), 3.45 (br s, 1H), 3.04 (br s, 1H), 2.21-2.29 (m, 2H), 1.97-2.13 (m, 2H), 1.71-1.92 (m, 1H), 1.53-1.67 (m, 1H), 1.39 (s, 9H), 1.18 (t, *J =* 7.2 Hz, 3H)
(2) To a solution of compound **33-2** (600 mg, 2.09 mmol) in dichloromethane (6.0 mL), triethylamine (1.27 g, 12.5 mmol) and methanesulfonyl chloride (1.22 g, 10.7 mmol) were added at 0°C under a nitrogen atmosphere. The reaction mixture was stirred at 25°C for 12 hours. Afterward, water (20.0 mL) and dichloromethane (100 mL) were used for extraction. The organic phase was dried over anhydrous sodium sulfate, filtered, and then concentrated under reduced pressure to obtain compound **33-3.**
(3) To a solution of compound **33-3** (300 mg, 821 µmol) in acetonitrile (1.0 mL), compound **33-4** (1.14 g, 13.1 mmol) was added. The reaction mixture was stirred at 80°C for 24 hours, followed by drying over anhydrous sodium sulfate, filtration, and concentration under reduced pressure. The crude product was purified by silica gel column chromatography (petroleum ether/ethyl acetate = 100:1 to 1:1) to yield compound **33-5.** MS-ESI [M+H]⁺, calculated: 357, found: 357. ¹H NMR (400 MHz, CDCl₃) *δ* 4.13 (q, *J =* 7.2 Hz, 2H), 3.88-4.05 (m, 1H), 3.79 (br s, 4H), 3.57 (br dd, *J* = 10.0, 7.6 Hz, 1H), 3.30-3.44 (m, 1H), 2.93-3.10 (m, 1H), 2.44-2.64 (m, 3H), 2.26-2.41 (m, 2H), 1.90 (br d, *J=* 5.2 Hz, 2H), 1.58-1.77 (m, 3H), 1.46 (s, 9H), 1.26 (t, *J=* 7.2 Hz, 3H).
(4) To a solution of compound **33-5** (150 mg, 421 µmol) in dichloromethane (3.0 mL), trifluoroacetic acid (1.54 g, 13.51 mmol) was added. The reaction mixture was stirred at 25°C for 0.5 hours. It was adjusted to pH 12 using sodium carbonate and extracted with dichloromethane (100 mL). The organic phase was dried over anhydrous sodium sulfate, filtered, and then concentrated under reduced pressure to obtain compound **33-6.**
(5) To a solution of compound **E** (54.7 mg, 172 µmol) in toluene (1.0 mL), compound **33-6** (40.0 mg, 156 µmol), cesium carbonate (102 mg, 312 µmol), 1,1'-binaphthyl-2,2'-diphenylphosphine (19.4 mg, 31.2 µmol), and tris (dibenzylideneacetone) dipalladium (14.3 mg, 15.6 µmol) were added. The reaction mixture was stirred at 80°C under a nitrogen atmosphere for 5 hours. After the reaction was complete, water (5.0 mL) was added, and the mixture was extracted with ethyl acetate (50.0 mL × 2). The organic phase was dried over anhydrous sodium sulfate, filtered, and concentrated under reduced pressure. The crude product was purified by silica gel column chromatography (petroleum ether/ethyl acetate = 0:1) to obtain compound **33-7.** MS-ESI [M+H]⁺, calculated: 539, found: 539.
(6) To a solution of compound **33-7** (38.0 mg, 70.6 µmol) in tetrahydrofuran (1.2 mL), water (0.4 mL) and lithium hydroxide monohydrate (29.6 mg, 706 µmol) were added. The reaction mixture was stirred at 25°C under a nitrogen atmosphere for 8 hours. To the reaction mixture, hydrochloric acid solution (1 mol/L) was added to adjust the pH to less than 7, and then it was extracted with ethyl acetate (50.0 mL). The organic phase was dried over anhydrous sodium sulfate, filtered, and concentrated under reduced pressure to obtain compound **33-8.** MS-ESI [M+H]⁺, calculated: 511, found: 511.
(7) To a solution of compound **33-8** (30.0 mg, 58.8 µmol) in dichloromethane (1.0 mL), compound **B-4** trifluoroacetate (15.0 mg, 64.7 µmol), diisopropylethylamine (38.0 mg, 294 µmol), and a solution of propylphosphonic anhydride in ethyl acetate (112 mg, 176 µmol, 50% purity) were added. The reaction mixture was stirred at 25°C under a nitrogen atmosphere for 4 hours. To the reaction mixture, water (10.0 mL) was added, and then it was extracted with dichloromethane (50.0 mL). The organic phase was dried over anhydrous sodium sulfate, filtered, and concentrated under reduced pressure. The crude product was purified by silica gel column chromatography ( petroleum ether/ethyl acetate mixture = 2:1) to obtain compound **33-9.** MS-ESI [M+H]⁺, calculated: 725, found: 725.
(8) To a solution of compound **33-9** (11.0 mg, 15.2 µmol) in trifluoroacetic acid (1.0 mL), trifluoromethanesulfonic acid (0.1 mL) was added, and the reaction mixture was stirred at 25°C for 0.5 hours. The reaction mixture was then diluted with ethyl acetate (20.0 mL), and the pH was adjusted to 7 with sodium bicarbonate. After extraction with ethyl acetate (50.0 mL), the organic phase was dried over anhydrous sodium sulfate, filtered, and concentrated under reduced pressure. The crude product was purified by preparative high-performance liquid chromatography (Xtimate C18, 100 mm × 30 mm 10 µm, A: water (0.225% formic acid); B: acetonitrile, 20%-50%: 10 minutes) to obtain compound **33** formate. MS-ESI [M+H]⁺, calculated: 606, found: 606. ¹H NMR (400 MHz, MeOD) *δ* 8.60 (s, 2H), 8.04 (s, 1H), 4.15-4.32 (m, 3H), 4.11 (br dd, *J =* 10.0, 1.5 Hz, 1H), 3.87-4.06 (m, 7H), 3.70 (q, *J =* 5.2 Hz, 3H), 3.62-3.66 (m, 2H), 3.58 (br dd, *J =* 11.2, 7.6 Hz, 2H), 3.40-3.52 (m, 2H), 2.44-2.58 (m, 3H), 2.20-2.29 (m, 1H), 1.98-2.05 (m, 1H), 1.65-1.76 (m, 1H).

### Example 34 Synthesis of Compound 34

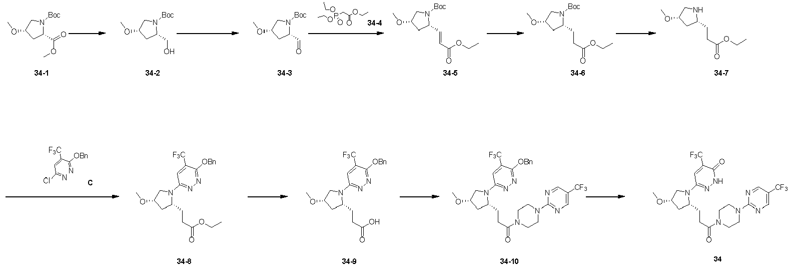

(1) At 0°C, lithium aluminum hydride (500 mg, 13.2 mmol) was added to a solution of compound **34-1** (1.50 g, 5.78 mmol) in tetrahydrofuran (20.0 mL). The reaction mixture was stirred for 1 hour at 0°C. Then, at 0°C, water (0.5 mL), 15% sodium hydroxide (0.5 mL) and additional water (1.5 mL) were added to the reaction mixture. After that, the reaction mixture was dried over anhydrous sodium sulfate, filtered, and concentrated under reduced pressure to obtain compound **34-2.** ¹H NMR (400 MHz, CDCl₃) *δ* 4.44 (br d, *J =* 6.4 Hz, 1H), 4.06 (br s, 1H), 3.88 (br s, 1H), 3.72 (br s, 1H), 3.50-3.56 (m, 1H), 3.29-3.36 (m, 3H), 2.20 (br s, 1H), 1.62-1.85 (m, 2H), 1.47 (s, 9H).
(2) To the solution of compound **34-2** (1.0 g, 4.32 mmol) in dichloromethane (15.0 mL), Dess-Martin reagent (2.2 g, 5.19 mmol) was added. The reaction mixture was stirred at 25°C for 12 hours. Sodium bisulfite solution (30.0 mL) was added to the reaction mixture, and it was then extracted with dichloromethane (100 mL). The organic layer was dried over anhydrous sodium sulfate, filtered, and concentrated under reduced pressure. The crude product was purified by silica gel column chromatography ( petroleum ether/ethyl acetate = 100:1 to 1:1) to obtain compound **34-3.** MS-ESI [M-^{t}Bu+H]⁺, calculated: 174, found: 174.¹H NMR (400 MHz, CDCl₃) *δ* 9.47-9.60 (m, 1H), 4.08 (br d, *J =* 9.6 Hz, 1H), 3.92 (t, *J =* 3.6 Hz, 1H), 3.71 (d, *J* = 12.0 Hz, 1H), 3.37-3.50 (m, 1H), 3.21-3.26 (m, 3H), 2.28-2.42 (m, 1H), 2.05-2.22 (m, 1H), 1.44-1.51 (m, 9H).
(3) To a solution of sodium hydride (45.4 mg, 1.13 mmol, 60%) in tetrahydrofuran (4.0 mL) at 0°C, compound **34-4** (254 mg, 1.13 mmol) was added. The reaction mixture was stirred at 0°C for 0.5 hours. Then, a solution of compound **34-3** (200 mg, 872 µmol) in tetrahydrofuran (2.0 mL) was added to the reaction mixture, and the reaction continued at 0°C under a nitrogen atmosphere for 0.5 hours. Ice-cold water (10.0 mL) was added to the reaction mixture, followed by extraction with ethyl acetate (50.0 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **34-5.** MS-ESI [M-Boc+H]⁺, calculated: 200, found: 200. ¹H NMR (400 MHz, CDCl₃) *δ* 6.91 (br dd, *J =* 14.4, 7.6 Hz, 1H), 5.82 (br d, *J =* 15.2 Hz, 1H), 4.31-4.57 (m, 1H), 4.20 (br d, *J =* 6.8 Hz, 2H), 3.94 (tt, *J =* 5.2, 2.4 Hz, 1H), 3.47-3.63 (m, 2H), 3.30 (s, 3H), 2.17-2.32 (m, 1H), 1.96 (br dd, *J =* 13.6, 0.9 Hz, 1H), 1.40-1.46 (m, 9H), 1.25-1.30 (m, 3H).
(4) To a solution of compound **34-5** (250 mg, 835 µmol) in tetrahydrofuran (3.0 mL), 10% palladium on carbon (50.0 mg) was added. The reaction mixture was stirred at 25°C in a hydrogen atmosphere for 2 hours. After the reaction, the mixture was filtered, and the filtrate was concentrated under reduced pressure to obtain compound **34-6.** MS-ESI [M+H]⁺, calculated: 302, found: 302.¹H NMR (400 MHz, CDCl₃) *δ* 4.13 (q, *J =* 7.2 Hz, 2H), 3.82-3.94 (m, 2H), 3.55-3.75 (m, 1H), 3.32-3.36 (m, 1H), 3.31 (s, 3H), 2.28-2.37 (m, 2H), 2.03-2.19 (m, 2H), 1.78-1.91 (m, 2H), 1.47 (s, 9H), 1.26 (t, *J=* 7.2 Hz, 3H).
(5) To a solution of compound **34-6** (200 mg, 664 µmol) in dichloromethane (3.0 mL), trifluoroacetic acid (1.54 g, 13.5 mmol) was added. The reaction mixture was stirred at 25°C for 0.5 hours. It was adjusted to pH = 11 using sodium carbonate and then extracted with dichloromethane (100 mL). After extraction, the organic phase was dried over anhydrous sodium sulfate, filtered, and concentrated under reduced pressure to obtain compound **34-7.** ¹H NMR (400 MHz, CDCl₃) *δ* 4.14 (q, *J=* 7.2 Hz, 2H), 3.27-3.30 (m, 3H), 3.15-3.19 (m, 1H), 2.89 (dd, *J =* 12.4, 4.8 Hz, 1H), 2.46 (td, *J =* 7.2, 1.6 Hz, 1H), 2.16-2.27 (m, 5H), 1.92 (qd, *J =* 7.2, 2.8 Hz, 1H), 1.41-1.50 (m, 1H), 1.24-1.28 (m, 3H).
(6) To a solution of compound **34-7** (65.0 mg, 323 µmol) in toluene (2.0 mL), compound **C** (103 mg, 355 µmol), cesium carbonate (210 mg, 646 µmol), 1,1'-binaphthyl-2,2'-diphenylphosphine (40.2 mg, 64.6 µmol), and tris (dibenzylideneacetone) dipalladium (29.6 mg, 32.3 µmol) were added. The reaction mixture was stirred at 80°C under a nitrogen atmosphere for 5 hours. After the reaction, water (10.0 mL) was added, and the mixture was extracted with ethyl acetate (100 mL). The organic phase was dried over anhydrous sodium sulfate, filtered, concentrated under reduced pressure. The crude product was purified by silica gel column chromatography (petroleum ether/ethyl acetate = 3:1) to obtain compound **34-8.** MS-ESI [M+H]⁺, calculated: 454, found: 454.
(7) To a solution of compound **34-8** (89.0 mg, 196 µmol) in tetrahydrofuran (1.2 mL), water (0.4 mL) and lithium hydroxide monohydrate (82.4 mg, 1.96 mmol) were added. The reaction mixture was stirred at 25°C under a nitrogen atmosphere for 24 hours. Then, hydrochloric acid solution (1 M) was added to adjust the pH to 6. The mixture was extracted with ethyl acetate (50.0 mL). The organic phase was dried over anhydrous sodium sulfate, filtered, and concentrated under reduced pressure to obtain compound **34-9.** MS-ESI [M+H]⁺, calculated: 426, found: 426. ¹H NMR (400 MHz, CDCl₃) *δ* 7.44-7.48 (m, 2H), 7.30-7.42 (m, 4H), 5.53 (s, 2H), 4.23-4.30 (m, 1H), 4.08-4.17 (m, 1H), 3.89 (br d, *J=* 11.6 Hz, 1H), 3.73 (br s, 1H), 3.37 (s, 3H), 2.43-2.62 (m, 2H), 2.06 (s, 2H), 1.20-1.30 (m, 2H).
(8) To a solution of compound **34-9** (60.0 mg, 141 µmol) in dichloromethane (2.0 mL), compound **B-4** trifluoroacetate (39.3 mg, 169 µmol), diisopropylethylamine (91.1 mg, 705 µmol), and a solution of propylphosphonic anhydride in ethyl acetate (269 mg, 423 µmol, 50% purity) were added. The reaction mixture was stirred at 25°C under a nitrogen atmosphere for 4 hours. The reaction mixture was then extracted with dichloromethane (50.0 mL), and the organic phase was dried over anhydrous sodium sulfate, filtered, and concentrated under reduced pressure. The crude product was purified by silica gel column chromatography (petroleum ether/ethyl acetate = 100:1 to 1:1) to obtain compound **34-10.** MS-ESI [M+H]⁺, calculated: 640, found: 640.
(9) To a solution of compound **34-10** (50.0 mg, 78.2 µmol) in tetrahydrofuran (1.0 mL), 10% palladium on carbon (70.0 mg) was added. The reaction mixture was stirred at 25°C under a hydrogen atmosphere for 4 hours. After filtration, the filtrate was concentrated under reduced pressure. The crude product was purified by preparative high-performance liquid chromatography (Xtimate C18, 100 mm × 30 mm × 10 µm, A: water (0.225% formic acid); B: acetonitrile, 42%-72% over 10 minutes) to obtain compound **34** formate. MS-ESI [M+H]⁺, calculated: 550, found: 550.¹H NMR (400 MHz, MeOD) *δ* 8.60 (s, 2H), 7.90 (s, 1H), 4.62 (s, 2H), 4.09 (br t, *J* = 4.8 Hz, 1H), 3.96-4.00 (m, 2H), 3.92 (t, *J =* 5.2 Hz, 2H), 3.63-3.70 (m, 4H), 3.51-3.57 (m, 1H), 3.37 (s, 3H), 2.46-2.57 (m, 2H), 2.12-2.24 (m, 2H), 2.03-2.11 (m, 1H), 1.95 (br s, 1H).

### Example 35 Synthesis of Compound 35

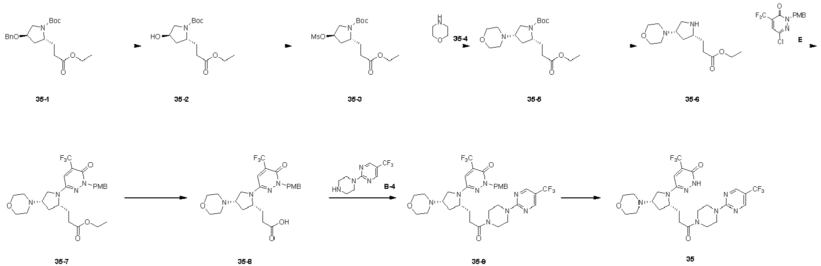

(1) To a solution of compound **35-1** (1.0 g, 2.65 mmol) in ethanol (20.0 mL), 10% palladium on carbon (500 mg) was added. The reaction mixture was stirred at 40°C under a hydrogen atmosphere for 12 hours. After the reaction, the mixture was filtered, and the solvent was concentrated under reduced pressure to yield compound **35-2.** ¹H NMR (400 MHz, CDCl₃) *δ* 4.39-4.47 (m, 1H), 4.09-4.17 (m, 2H), 3.83-4.03 (m, 1H), 3.63-3.77 (m, 1H), 3.24-3.43 (m, 1H), 1.81-2.44 (m, 6H), 1.45-1.49 (m, 9H), 1.24-1.29 (m, 3H).
(2) To a solution of compound **35-2** (300 mg, 1.04 mmol) in dichloromethane (5.0 mL), triethylamine (211 mg, 2.09 mmol) and methanesulfonyl chloride (179 mg, 1.57 mmol) were added at 0°C. The reaction mixture was stirred at 25°C under a nitrogen atmosphere for 2 hours. To the mixture, sodium bicarbonate solution (2.0 mL) was added, followed by extraction with dichloromethane (5.0 mL × 3). The organic layer was washed by saturated sodium chloride solution (5.0 mL), dried over anhydrous sodium sulfate, filtered, and concentrated under reduced pressure to yield compound **35-3.** MS-ESI [M-Boc+H]⁺ , calculated: 266, found: 266.
(3) To a solution of compound **35-3** (275 mg, 752 µmol) in acetonitrile (3.0 mL), compound **35-4** (1.05 g, 12.0 mmol) was added. The reaction mixture was stirred at 80°C for 48 hours. After the reaction, water (10.0 mL) was added, and the mixture was extracted with ethyl acetate (5.0 mL × 3). The organic layer was washed by saturated sodium chloride solution (5.0 mL), dried over anhydrous sodium sulfate, filtered, and concentrated under reduced pressure. The crude product was purified by silica gel column chromatography (petroleum ether/ethyl acetate = 100:0 to 2:1) to yield compound **35-5.** MS-ESI [M+H]⁺, calculated: 357, found: 357.
(4) To a solution of compound **35-5** (70.0 mg, 196 µmol) in dichloromethane (1.0 mL), trifluoroacetic acid (462 mg, 4.05 mmol) was added. The reaction mixture was stirred at 25°C for 2 hours. The organic phase was adjusted to pH 7-8 using sodium bicarbonate, and water (0.50 mL) was added. It was then extracted with dichloromethane (1.50 mL × 4). The organic layer was dried over anhydrous magnesium sulfate, filtered, and concentrated under reduced pressure to yield compound **35-6.** MS-ESI [M+H]⁺ , calculated: 257, found: 257.
(5) To a solution of compound **35-6** (35.0 mg, 136 µmol) in dioxolane (1.0 mL), compound **E** (43.5 mg, 136 µmol), cesium carbonate (89.0 mg, 273 µmol), and Methanesulfonato(2-dicyclohexylphosphino-2',6'-di-i-propoxy-1,1'-biphenyl) (2'-amino-1,1'-biphenyl-2-yl) palladium(II) (22.8 mg, 27.3 µmol) were added. The reaction mixture was stirred at 60°C under a nitrogen atmosphere. Water (6.0 mL) was added to dilute the mixture, and it was then extracted with dichloromethane (5.0 mL × 3). The organic phase was washed by saturated sodium chloride solution (5.0 mL), dried over anhydrous sodium sulfate, filtered, and concentrated under reduced pressure. The crude product was purified by silica gel column chromatography (dichloromethane/methanol = 20:1) to yield compound **35-7.** MS-ESI [M+H]⁺, calculated: 539, found: 539.
(6) To a solution of compound **35-7** (33.0 mg, 61.3 µmol) in tetrahydrofuran (1.0 mL), water (0.25 mL) and lithium hydroxide monohydrate (12.9 mg, 306 µmol) were added. The reaction mixture was stirred at 30°C under a nitrogen atmosphere for 12 hours. The pH was adjusted to 6-7 by adding 20% citric acid solution. The mixture was then extracted with dichloromethane (3.0 mL × 3). The organic phase was dried over anhydrous sodium sulfate, filtered, and concentrated under reduced pressure to obtain compound **35-8.** MS-ESI[M+H]⁺, calculated: 511, found: 511.
(7) To a solution of compound **35-8** (30.0 mg, 58.8 µmol) in dichloromethane (1.0 mL), compound **B-4** trifluoroacetate (15.8 mg, 58.8 µmol), diisopropylethylamine (30.4 mg, 235 µmol), and a solution of propylphosphonic anhydride in ethyl acetate (44.9 mg, 70.5 µmol, 50% purity) were added. The reaction mixture was stirred at 25°C under a nitrogen atmosphere for 2 hours. Water (3.0 mL × 2) was added to the reaction mixture, followed by extraction with dichloromethane (5.0 mL). The organic phase was dried over anhydrous sodium sulfate, filtered, and concentrated under reduced pressure. The crude product was purified by silica gel column chromatography (dichloromethane /methanol = 20:1) to obtain compound **35-9.** MS-ESI [M+H]⁺, calculated: 725, found: 725.
(8) To a solution of compound **35-9** (23.0 mg, 31.74 µmol) in dichloromethane (1.0 mL), trifluoromethanesulfonic acid (170.0 mg, 1.13 mmol) was added. The reaction mixture was stirred at 25°C for 1 hour. The reaction was quenched by adding sodium bicarbonate (1.0 mL), followed by extraction with dichloromethane (2.0 mL × 3). The organic phase was washed with saturated sodium chloride (2.0 mL), dried over anhydrous sodium sulfate, filtered, and concentrated under reduced pressure. The crude product was purified by preparative high-performance liquid chromatography (Phenomenex C18 column, 75 mm × 30 mm × 3 µm, A: water (10 mmol/L ammonium bicarbonate) and B: acetonitrile (25%-65% ) over 14 minutes) to obtain compound **35.** MS-ESI [M+H]⁺, calculated: 605, found: 605. ¹H NMR (400 MHz, MeOD) *δ* 8.60 (s, 2H), 7.93 (s, 0.7H), 7.75 (s, 0.3H), 4.02-4.14 (m, 1H), 3.90-4.00 (m, 4H), 3.76-3.87 (m, 1H), 3.62-3.74 (m, 8H), 3.10-3.24 (m, 2H), 2.48-2.63 (m, 6H), 2.18 (dd, *J=* 12.4, 5.2 Hz, 1H), 1.82-2.07 (m, 2H), 1.65-1.76 (m, 1H).

### Example 36 Synthesis of Compound 36

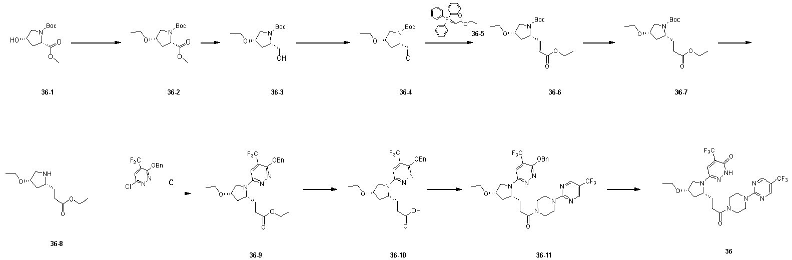

(1) To a solution of compound **36-1** (10.0 g, 40.8 mmol) in N, N-dimethylformamide (100 mL), sodium hydride (3.26 g, 81.5 mmol) was added at 0°C. The mixture was stirred at 25°C for 0.5 hours. Then, ethyl iodide (12.7 g, 81.5 mmol) was added, and the reaction mixture was stirred at 25°C for 2 hours. At 0°C, a solution of hydrochloric acid (0.5 mol/L, 100 mL) was added to the reaction mixture. The resulting mixture was extracted with ethyl acetate (200 mL). The organic phase was washed with saturated sodium chloride solution (100 mL × 3), dried over anhydrous sodium sulfate, filtered, and concentrated under reduced pressure. The crude product was purified by silica gel column chromatography ( petroleum ether/ethyl acetate from 1:0 to 3:1) to obtain compound **36-2.** MS-ESI [M+H]⁺, calculated: 274, found: 274.
(2) To a solution of compound **36-2** (9.5 g, 34.8 mmol) in tetrahydrofuran (100 mL) under nitrogen protection at 0°C, lithium aluminum hydride (2.5 g, 65.9 mmol) was added. The reaction mixture was stirred at 0°C for 1 hour. Water (2.5 mL) was then added to the reaction mixture, followed by 15% sodium hydroxide (2.5 mL) and additional water (7.5 mL). The mixture was dried over anhydrous sodium sulfate, filtered, and concentrated under reduced pressure to obtain compound **36-3.** ¹H NMR (400 MHz, CDCl₃) *δ* 3.91-4.09 (m, 2H), 3.69-3.76 (m, 1H), 3.36-3.58 (m, 5H), 2.16-2.32 (m, 1H), 2.05-2.15 (m, 1H), 1.46 (s, 9H), 1.15-1.22 (m, 3H).
(3) To a solution of compound **36-3** (1.0 g, 4.08 mmol) in dichloromethane (40.0 mL), Dess-Martin reagent (2.25 g, 5.30 mmol) was added. The reaction mixture was stirred at 25°C for 12 hours. Sodium sulfite (30.0 mL) was then added, and the mixture was extracted with dichloromethane (100 mL). After extraction, the organic layer was dried over anhydrous sodium sulfate, filtered, and concentrated under reduced pressure. The crude product was then purified by silica gel column chromatography to obtain compound **36-4.** MS-ESI [M-^{t}Bu+H]⁺, calculated: 188, found: 188. ¹H NMR (400 MHz, CDCl₃) *δ* 9.49-9.61 (m, 1H), 3.98-4.21 (m, 2H), 3.53-3.71 (m, 1H), 3.32-3.48 (m, 3H), 2.24-2.37 (m, 1H), 2.02-2.21 (m, 1H), 1.43-1.52 (m, 9H), 1.13 (t, *J* = 6.8 Hz, 3H).
(4) To a solution of compound **36-4** (400 mg, 1.64 mmol) in dichloromethane (7.0 mL), compound **36-5** (687 mg, 1.97 mmol) was added. The reaction mixture was stirred at 25°C under a nitrogen atmosphere for 12 hours. It was then concentrated under reduced pressure. The crude product was purified by silica gel column chromatography (petroleum ether/ethyl acetate = 1:0 to 5:1) to isolate compound **36-6.** MS-ESI [M-Boc+H]⁺, calculated: 214, found: 214.¹H NMR (400 MHz, CDCl₃) *δ* 6.88-7.02 (m, 1H), 5.76-5.91 (m, 1H), 4.29-4.53 (m, 1H), 4.14-4.27 (m, 2H), 3.98-4.08 (m, 1H), 3.60 (br s, 1H), 3.42-3.53 (m, 3H), 2.23 (br s, 1H), 1.90-1.99 (m, 1H), 1.39-1.52 (m, 9H), 1.23-1.34 (m, 3H), 1.19 (t, *J =* 7.2 Hz, 3H).
(5) To a solution of compound **36-6** (340 mg, 1.08 mmol) in tetrahydrofuran (5.0 mL), 10% palladium on carbon (100 mg) was added. The reaction mixture was stirred at 25°C under a hydrogen atmosphere for 2 hours. The reaction mixture was then filtered, concentrated under reduced pressure, and compound **36-7** was isolated. MS-ESI [M+H]⁺, calculated: 316, found: 316.¹H NMR (400 MHz, CDCl₃) *δ* 4.13 (q, *J=* 7.2 Hz, 2H), 3.94-4.02 (m, 1H), 3.85 (br s, 1H), 3.56-3.77 (m, 1H), 3.40-3.51 (m, 2H), 3.30 (dd, *J =* 11.6, 3.2 Hz, 1H), 2.27-2.37 (m, 2H), 2.04-2.21 (m, 2H), 1.88 (dq, *J=* 13.6, 8.0 Hz, 1H), 1.75-1.83 (m, 1H), 1.46 (s, 9H), 1.26(t, *J=* 7.2 Hz, 3H), 1.20 (t, *J=* 6.8 Hz, 3H).
(6) To a solution of compound **36-7** (300 mg, 951 µmol) in dichloromethane (3.0 mL), trifluoroacetic acid (1.0 mL) was added. The reaction mixture was stirred at 25°C for 0.5 hours. It was adjusted to pH 10 using sodium carbonate, and then extracted with dichloromethane (50.0 mL). The organic phase was dried over anhydrous sodium sulfate, filtered, and concentrated under reduced pressure to obtain compound **36-8.** ¹H NMR (400 MHz, CDCl₃) *δ* 4.13 (q, *J =* 7.2 Hz, 2H), 3.98-4.07 (m, 1H), 3.43 (q, *J =* 7.2 Hz, 2H), 3.08-3.21 (m, 2H), 2.90 (br dd, *J=* 12.4, 5.2 Hz, 3H), 2.40-2.49 (m, 2H), 2.17-2.28 (m, 1H), 1.89-1.93 (m, 1H), 1.26 (t, *J* = 7.2 Hz, 3H), 1.18 (t, *J* = 7.2 Hz, 3H).
(7) To a solution of compound **36-8** (98.5 mg, 457 µmol) in toluene (4.0 mL), compound **C** (120 mg, 416 µmol), cesium carbonate (271 mg, 831 µmol), 1,1'-binaphthyl-2,2'-diphenylphosphine (51.8 mg, 83.1 µmol), and tris (dibenzylideneacetone) dipalladium (38.1 mg, 41.6 µmol) were added. The reaction was carried out under a nitrogen atmosphere at 80°C for 5 hours. Afterward, water (10.0 mL) was added to the reaction mixture, followed by extraction with ethyl acetate (50.0 mL). The organic layer was dried over anhydrous sodium sulfate, filtered, concentrated under reduced pressure, and the crude product was purified by silica gel column chromatography (petroleum ether/ethyl acetate = 1:0 to 2:1) to yield compound **36-9.** MS-ESI [M+H]⁺, calculated: 468, found: 468.¹H NMR (400 MHz, CDCl₃) *δ* 7.48 (d, *J =* 8.0 Hz, 2H), 7.38 (t, *J =* 8.0 Hz, 3H), 7.29-7.35 (m, 1H), 5.57 (s, 2H), 4.05-4.23 (m, 4H), 3.69-3.87 (m, 2H), 3.47-3.60 (m, 2H), 2.31-2.50 (m, 2H), 1.95-2.24 (m, 4H), 1.27 (t, *J=* 6.8 Hz, 3H), 1.22 (t, *J=* 7.2 Hz, 3H).
(8) To a solution of compound **36-9** (74.0 mg, 158 µmol) in tetrahydrofuran (3.0 mL), water (1.0 mL) and lithium hydroxide monohydrate (90.0 mg, 3.76 mmol) were added. The reaction was stirred at 30°C under a nitrogen atmosphere for 12 hours. Then, hydrochloric acid solution (1 M) was added to adjust the pH to 6. The mixture was extracted with dichloromethane (50.0 mL). The organic layer was dried over anhydrous sodium sulfate, filtered, and concentrated under reduced pressure to yield compound **36-10.** MS-ESI [M+H]⁺, calculated: 440, found: 440. ¹H NMR (400 MHz, CDCl₃) *δ* 7.45-7.50 (m, 2H), 7.29-7.41 (m, 4H), 5.55 (s, 2H), 4.24 (br s, 2H), 3.84 (br s, 1H), 3.74 (br s, 1H), 3.48-3.57 (m,3H), 2.48-2.56 (m, 3H), 2.17-2.29 (m, 2H), 1.19-1.22 (m, 3H).
(9) To a solution of compound **36-10** (68.0 mg, 155 µmol) in dichloromethane (3.0 mL), compound **B-4** trifluoroacetate (62.4 mg, 269 µmol), diisopropylethylamine (111 mg, 861 µmol), and propylphosphonic anhydride solution in ethyl acetate (214 mg, 336 µmol, 50% purity) were added to the reaction mixture. The reaction was stirred at 25°C under a nitrogen atmosphere for 1 hour. Water (20.0 mL) was added to the reaction mixture, followed by extraction with ethyl acetate (50.0 mL). The organic phase was dried over anhydrous sodium sulfate, filtered, concentrated under reduced pressure. The crude product was purified by silica gel column chromatography ( petroleum ether/ethyl acetate = 1:0 to 1:1) to yield compound **36-11.** MS-ESI [M+H]⁺, calculated: 654, found: 654.
(10) To a solution of compound **36-11** (70.0 mg, 107 µmol) in tetrahydrofuran (3.0 mL), 10% palladium on carbon (50.0 mg) was added. The reaction was stirred under a hydrogen atmosphere at 25°C for 3 hours. After the reaction, the mixture was filtered, and the filtrate was concentrated under reduced pressure. The crude product was then purified by preparative high-performance liquid chromatography (Xtimate C18, 100 mm × 30 mm 10 µm, A: water (0.225% formic acid) and B: acetonitrile (50% to 80%) to isolate compound **36** formate. MS-ESI [M+H]⁺, calculated: 564, found: 564. ¹H NMR (400 MHz, MeOD) *δ* 8.60 (s, 2H), 7.89 (s, 1H), 4.16-4.22 (m, 1H), 3.96-4.06 (m, 3H), 3.93 (t, 2H), 3.63-3.74 (m, 4H), 3.49-3.61 (m, 4H), 2.46-2.58 (m, 2H), 2.16-2.25 (m, 1H), 2.04-2.14 (m, 2H), 1.92-2.03 (m, 1H), 1.20 (t, *J* = 7.2 Hz, 3H).

### Example 37 Synthesis of Compound 37

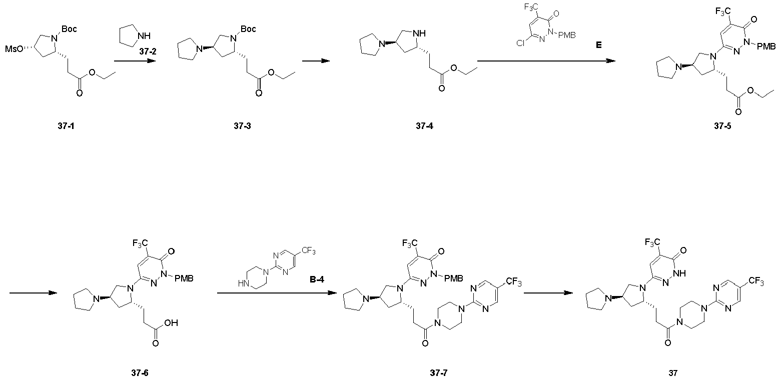

(1) To a solution of compound **37-1** (450 mg, 1.23 mmol) in acetonitrile (5.0 mL), compound **37-2** (876 mg, 12.3 mmol) was added. The reaction mixture was stirred at 80°C for 12 hours. It was filtered, and the filtrate was concentrated under reduced pressure. The crude product was then purified by silica gel column chromatography using ( petroleum ether and ethyl acetate = 100: 1 to 1:1) to isolate compound **37-3.** ¹H NMR (400 MHz, CDCl₃) *δ* 4.13 (q, *J* = 7.2 Hz, 2H), 3.44 (dt, *J =* 16.4, 6.8 Hz, 4H), 2.89-3.06 (m, 1H), 2.31 (br d, *J =* 5.2 Hz, 2H), 2.04-2.06 (m, 3H), 1.81-1.90 (m, 7H), 1.71 (br dd, *J =* 13.6, 7.6 Hz, 1H), 1.46 (s, 9H), 1.26 (t, *J* = 7.2 Hz, 3H).
(2) To a solution of compound **37-3** (240 mg, 705 µmol) in dichloromethane (3.0 mL), trifluoroacetic acid (1.54 g, 13.5 mmol) was added. The reaction was stirred at 25°C for 0.5 hours. The mixture was then adjusted to pH greater than 7 using sodium bicarbonate, and extracted with dichloromethane (100 mL). The organic phase was dried over anhydrous sodium sulfate, filtered, and concentrated under reduced pressure to obtain compound **37-4.** ¹H NMR (400 MHz, CDCl₃) *δ* 4.12 (q, *J* = 7.2 Hz, 2H), 3.40-3.47 (m, 4H), 3.24-3.30 (m, 1H), 2.84-3.05 (m, 3H), 2.34-2.45 (m, 2H), 2.07-2.15 (m, 1H), 1.93-1.99 (m, 2H), 1.85-1.90 (m, 4H), 1.61-1.72 (m, 1H), 1.25 (t, *J =* 7.2 Hz, 3H).
(3) To a solution of compound **37-4** (198.15 mg, 621.8 µmol) in toluene (3.0 mL), compound **E** (110.0 mg, 518.16 µmol), cesium carbonate (337.66 mg, 1.04 mmol), tris(dibenzylideneacetone)dipalladium (47.45 mg, 51.82 µmol), and 1,1'-binaphthyl-2,2'-diphenylphosphine (64.53 mg, 103.63 µmol) were added. The reaction was carried out at 80°C under a nitrogen atmosphere for 5 hours. After the reaction, the mixture was diluted with water (10.0 mL) and extracted with ethyl acetate (100 mL). The organic phase was dried over anhydrous sodium sulfate, filtered, and concentrated under reduced pressure. The crude product was purified by silica gel column chromatography ( petroleum ether/ethyl acetate = 100:1 to 1:1) to obtain compound **37-5.** MS-ESI [M+H]⁺, calculated: 523, found: 523. ¹H NMR (400 MHz, CDCl₃) *δ* 7.42 (br d, *J =* 8.8 Hz, 3H), 6.82-6.89 (m, 2H), 4.06-4.18 (m, 3H), 4.04 (br d, *J =* 3.2 Hz, 1H), 3.79 (s, 3H), 3.44 (dt, *J =* 16.4, 6.8 Hz, 5H), 2.36-2.50 (m, 2H), 2.27-2.36 (m, 2H), 1.92-2.01 (m, 5H), 1.82-1.90 (m, 3H), 1.27-1.29 (m, 1H), 1.20-1.26 (m, 3H).
(4) To a solution of compound **37-5** (125 mg, 239 µmol) in tetrahydrofuran (1.5 mL), water (0.50 mL) and lithium hydroxide monohydrate (100 mg, 2.39 mmol) were added. The reaction mixture was stirred at 25°C under a nitrogen atmosphere for 12 hours. To the reaction mixture, hydrochloric acid solution (1 M, 100 mL) was added to adjust the pH to less than 7, and then it was extracted with ethyl acetate (100 mL). The organic phase was dried over anhydrous sodium sulfate, filtered, and concentrated under reduced pressure to obtain compound **37-6.** MS-ESI [M+H]⁺, calculated: 495, found: 495. ¹H NMR (400 MHz, CDCl₃) *δ* 7.34-7.46 (m, 3H), 6.86 (br d, *J=* 8.4 Hz, 2H), 3.89 (s, 1H), 3.77-80 (m, 3H), 3.43-3.51 (m, 4H), 2.36 (br s, 1H), 2.26-2.31 (m, 1H), 2.12-2.15 (m, 3H), 1.94 (br dd, *J=* 5.6, 3.2 Hz, 5H), 1.46 (br s, 4H), 0.89 (br s, 2H).
(5) To a solution of compound **37-6** (100 mg, 202 µmol) in dichloromethane (2.0 mL), compound **B-4** trifluoroacetate (51.7 mg, 222 µmol), diisopropylethylamine (131 mg, 1.01 mmol), and a solution of propylphosphonic anhydride in ethyl acetate (386.0 mg, 607 µmol, 50% purity) were added. The reaction mixture was stirred at 25°C under a nitrogen atmosphere for 4 hours. It was then quenched with water (5.0 mL) and extracted with ethyl acetate (50.0 mL). The organic phase was dried over anhydrous sodium sulfate, filtered, and concentrated under reduced pressure. The crude product was purified by silica gel column chromatography (dichloromethane/methanol = 100:1 to 90:1) to obtain compound **37-7.** MS-ESI [M+H]⁺, calculated: 709, found: 709. ¹H NMR (400 MHz, CDCl₃) *δ* 8.48-8.54 (m, 2H), 7.70 (s, 1H), 7.33-7.42 (m, 2H), 6.86 (d, *J =* 8.8 Hz, 2H), 5.10-5.20 (m, 2H), 3.86-3.92 (m, 4H), 3.85 (br d, *J* = 7.2 Hz, 1H), 3.77-3.80 (m, 4H), 3.62-3.70 (m, 2H), 3.48-3.58 (m, 1H), 3.32-3.47 (m, 2H), 2.89 (s, 2H), 2.66-2.79 (m, 1H), 2.20-2.40 (m, 4H), 1.85-2.15 (m, 3H), 1.50 (br d, *J =* 2.8 Hz, 2H), 1.11-1.37 (m, 3H).
(6) To a solution of compound **37-7** (100 mg, 141 µmol) in trifluoroacetic acid (1.0 mL), trifluoromethanesulfonic acid (0.1 mL) was added. The reaction mixture was stirred at 25°C for 0.5 hours. After the reaction, the mixture was quenched with water (3.0 mL) and the pH was adjusted to 7 using sodium bicarbonate. The mixture was then extracted with ethyl acetate (50.0 mL). The organic phase was dried over anhydrous sodium sulfate, filtered, and concentrated under reduced pressure. The crude product was purified by preparative high-performance liquid chromatography (Xtimate C18, 100 mm × 30 mm 10 µm, A: water with 0.225% formic acid and B: acetonitrile (22% to 42% over 10 minutes ) to obtain compound **37** formate. MS-ESI [M+H]⁺, calculated: 589, found: 589. ¹H NMR (400 MHz, MeOD) *δ* 8.60 (s, 2H), 7.95 (s, 1H), 4.09-4.14 (m, 1H), 3.96-4.00 (m, 2H), 3.93 (br dd, *J =* 6.4, 4.1 Hz, 2H), 3.77-3.83 (m, 1H), 3.67-3.73 (m, 2H), 3.61-3.66 (m, 2H), 3.48 (s, 1H), 3.13 (br s, 1H), 2.74-2.83 (m, 3H), 2.52 (br t, *J =* 6.8 Hz, 2H), 2.18-2.23 (m, 1H), 1.98-2.07 (m, 2H), 1.91 (br s, 4H), 1.67-1.74 (m, 1H), 1.29 (br s, 1H).

### Example 38 Synthesis of Compound 38

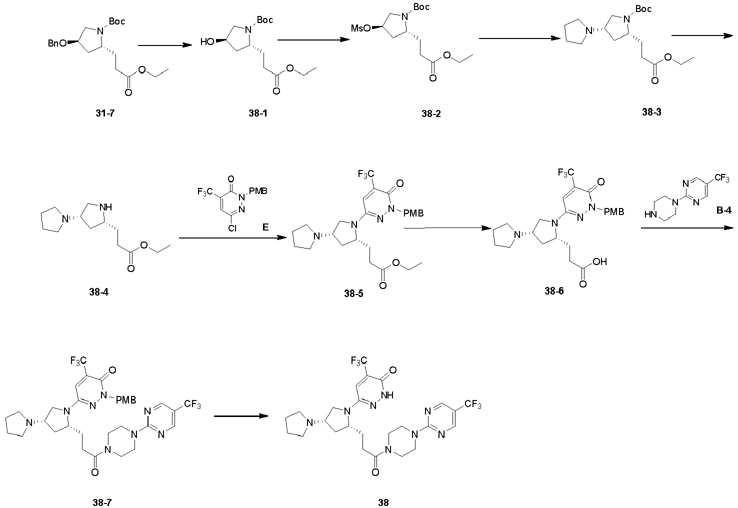

(1) To a solution of compound **31-7** (1 g, 2.65 mmol) in 30.0 mL of ethanol, 10% palladium on carbon (1 g) was added. The reaction mixture was stirred at 40°C under a hydrogen atmosphere (50 psi) for 12 hours. The reaction mixture was filtered, and the filtrate was concentrated under reduced pressure to obtain compound **38-1.** MS-ESI [M+Na]⁺, calculated: 310, found: 310.
(2) Compound **38-1** (300 mg, 1.04 mmol) and triethylamine (317 mg, 3.13 mmol) were dissolved in 3.0 mL of dichloromethane. The mixture was cooled to 0°C. A solution of methylsulfonyl chloride (179 mg, 1.57 mmol) in 2.0 mL of dichloromethane was added. The reaction mixture was reacted at 25°C for 2 hours and then quenched by saturated sodium bicarbonate (2.0 mL). It was extracted with dichloromethane (5.0 mL×3). The combined organic phases was washed with saturated sodium chloride solution (5.0 mL), dried over anhydrous sodium sulfate, filtered, and concentrated under reduced pressure to obtain compound **38-2.** MS-ESI [M+Na]⁺, calculated: 388, found: 388.
(3) To the solution of compound **38-2** (300 mg, 821 µmol) in 3.0 mL of acetonitrile, pyrrolidine (876 mg, 12.3 mmol) was added. The mixture was reacted at 80°C for 12 hours. After completion of the reaction, water (6.0 mL) was added to the reaction mixture, followed by extraction with dichloromethane (3.0 mL×3). The combined organic phase was washed sequentially with water (25.0 mL × 2) and saturated sodium chloride solution (5.0 mL), dried over anhydrous sodium sulfate, filtered, and concentrated under reduced pressure. The crude product was purified by silica gel column chromatography (petroleum ether/ethyl acetate = 1:0 to 1:1) to obtain compound **38-3.** MS-ESI [M+H]⁺, calculated: 341, found: 341. ¹H NMR (400 MHz, CDCl₃) *δ* 4.08-4.19 (m, 2H), 3.81-4.04 (m, 1H), 3.37-3.65 (m, 2H), 3.01-3.25 (m, 1H), 2.53-2.93 (m, 3H), 2.24-2.43 (m, 2H), 1.83-2.05 (m, 5H), 1.53-1.78 (m, 4H), 1.46 (s, 9H), 1.26 (t, *J* = 7.2 Hz, 3H).
(4) To a solution of compound **38-3** (100 mg, 293 µmol) in 1.0 mL of dichloromethane, trifluoroacetic acid (462 mg, 4.05 mmol, 300 µL) was added. The reaction mixture was stirred at 25°C for 2 hours and concentrated under reduced pressure. The resulting residue was dissolved in water (0.5 mL) and the pH was adjusted to 7-8 using sodium carbonate, followed by extraction with dichloromethane (1.5 mL× 4). The organic phase was dried with anhydrous magnesium sulfate, filtered, and concentrated under reduced pressure to obtain compound **38-4.**
(5) To a solution of compound **E** (53.0 mg, 166 µmol), compound **38-4** (40.0 mg, 166 µmol), and cesium carbonate (108 mg, 333 µmol) in 1.0 mL of dioxane, Methanesulfonato(2-dicyclohexylphosphino-2',6'-di-i-propoxy-1,1'-biphenyl)(2'-amino-1,1'-bip henyl-2-yl)palladium(II) (27.8 mg, 33.3 µmol) was added. The reaction mixture was stirred at 60°C under a nitrogen atmosphere for 3 hours. Upon completion of the reaction, water (10.0 mL) was added to the reaction mixture, followed by extraction with dichloromethane (5.0 mL× 4). The combined organic phase was washed sequentially with water (5.0 mL) and saturated sodium chloride (5.0 mL), dried over anhydrous sodium sulfate, filtered, and concentrated under reduced pressure. The crude product was purified by preparative TLC (dichloromethane/methanol = 20:1) to obtain compound **38-5.** MS-ESI [M+H]⁺, calculated: 523, found: 523.
(6) To a solution of compound **38-5** (50.0 mg, 95.7 µmol) in tetrahydrofuran (1.0 mL) and water (0.25 mL), lithium hydroxide monohydrate (20.1 mg, 478 µmol) was added. The reaction proceeded at 25°C for 12 hours. The reaction mixture was concentrated under reduced pressure to remove tetrahydrofuran and water (0.5 mL) was added. The pH was adjusted to 6-7 using a 20% citric acid solution, followed by extraction with dichloromethane (3.0 mL × 3). The organic phase was dried over anhydrous sodium sulfate, filtered, and concentrated under reduced pressure to obtain compound **38-6.** MS-ESI [M+H]⁺, calculated: 495, found: 495.
(7) To a solution of compound **B-4** (14.1 mg, 60.6 µmol) and diisopropylethylamine (31.4 mg, 243 µmol, 42 µL) in dichloromethane (1.0 mL), a solution of compound **38-6** (30.0 mg, 60.6 µmol) in dichloromethane (0.5 mL) and 50% propylphosphonic anhydride solution (46.3 mg, 72.8 µmol, 43.3 µL) were added. The reaction proceeded at 25°C for 2 hours. The reaction mixture was diluted with dichloromethane (5.0 mL) and washed sequentially with water (3.0 mL) and saturated sodium chloride solution (3.0 mL). The organic phase was dried over anhydrous sodium sulfate, filtered, and concentrated under reduced pressure. The crude product was purified by preparative thin-layer chromatography (dichloromethane/methanol = 1:0 to 7:3) to obtain compound **38-7.** MS-ESI [M+H]⁺, calculated: 709, found: 709.
(8) Trifluoromethanesulfonic acid (1.13 mmol, 0.1 mL) was added to a solution of compound **38-7** (27.0 mg, 38.1 µmol) in dichloromethane (1.0 mL) and stirred at 25°C for 1 hour. The pH of the reaction mixture was adjusted with saturated sodium bicarbonate solution to 7, followed by extraction with dichloromethane (2.0 mL × 3). The combined organic phase was washed with saturated sodium chloride solution (2.0 mL), dried over anhydrous sodium sulfate, filtered, and concentrated under reduced pressure. The crude product was purified by high-performance liquid chromatography (Phenomenex C18, 75 mm × 30 mm, 3 µm, A: water (10 mmol/L ammonium bicarbonate); B: acetonitrile, 30%-70% over 11 minutes) to obtain compound **38.** MS-ESI [M+H]⁺, calculated: 589, found: 589. ¹H NMR (400 MHz, MeOD) *δ* 8.60 (s, 2H), 7.92 (s, 0.8H), 4.03-4.11 (m, 1H), 3.90-4.01 (m, 4H), 3.76 (dd, *J =* 10.0, 6.8 Hz, 1H), 3.67 (dt, *J =* 19.2, 5.2 Hz, 4H), 3.19-3.25 (m, 1H), 3.08-3.18 (m, 1H), 2.61-2.71 (m, 4H), 2.46-2.56 (m, 2H), 2.16 (dd, *J=* 12.0, 6.0 Hz, 1H), 1.97-2.08 (m, 2H), 1.85 (s, 4H), 1.72 (ddd, *J* = 13.6, 9.2, 6.4 Hz, 1H).

### Example 39 Synthesis of Compound 39

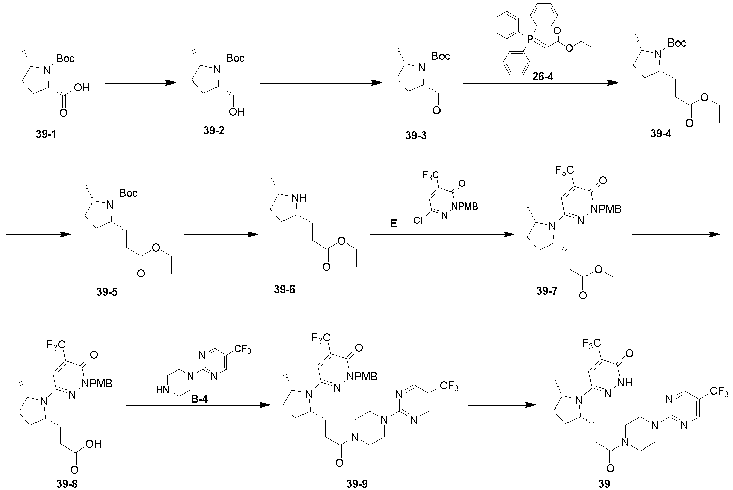

(1) A solution of compound **39-1** (4.50 g, 19.6 mmol) in tetrahydrofuran (50.0 mL) was cooled to 5°C, and 1 M borane-tetrahydrofuran solution (39.5 mL) was slowly added. The mixture was reacted at 25°C for 2 hours. After the reaction is complete, the reaction mixture was cooled to 5°C and methanol (50.0 mL) was slowly added. After then, the mixture was stirred at 25°C for 30 minutes. The quenched reaction mixture was concentrated under reduced pressure to obtain compound **39-2.** ¹H NMR (400 MHz, CDCl₃) *δ* 3.89-4.00 (m, 2H), 3.69 (dd, *J =* 2.8, 11.2 Hz, 1H), 3.50-3.58 (m, 1H), 2.68-2.75 (m, 1H), 1.88-2.03 (m, 2H), 1.59-1.71 (m, 1H), 1.50-1.58 (m, 1H), 1.47 (s, 9H), 1.16 (d, *J=* 6.4 Hz, 3H).
(2) To a solution of compound **39-2** (4.50 g, 20.9 mmol) in dichloromethane (60.0 mL), sodium bicarbonate (7.02 g, 83.6 mmol) and Dess-Martin oxidizing agent (17.7 g, 41.8 mmol) were added. The reaction was allowed to proceed at 25°C for 3 hours, quenched with saturated sodium bisulfite solution (20.0 mL), and followed by extraction with dichloromethane (20.0 mL × 3). The combined organic phase was dried over anhydrous sodium sulfate, filtered, and concentrated under reduced pressure to obtain compound **39-3.** ¹H NMR (400 MHz, CDCl₃) *δ* 9.31-9.57 (m, 1H), 3.89-4.09 (m, 1H), 1.87-2.11 (m, 3H), 1.54-1.69 (m, 2H), 1.37-1.53 (m, 9H), 1.20-1.28 (m, 3H).
(3) To a solution of compound **39-3** (3.00 g, 14.1 mmol) in tetrahydrofuran (40.0 mL), compound **26-4** (4.90 g, 14.1 mmol) was added. The reaction was allowed to proceed at 25°C for 12 hours. The reaction mixture was concentrated under reduced pressure. The crude product was purified by silica gel column chromatography (petroleum ether/ethyl acetate = 1:0 to 17:3) to obtain compound **39-4.** ¹H NMR (400 MHz, CDCl₃) *δ* 6.84 (dd, *J =* 6.0, 15.6 Hz, 1H), 5.87 (d, *J=* 15.2 Hz, 1H), 4.29-4.57 (m, 1H), 4.19 (q, *J=* 7.2 Hz, 2H), 3.91 (brs, 1H), 1.94-2.09 (m, 2H), 1.72-1.83 (m, 1H), 1.50-1.61 (m, 1H), 1.43 (s, 9H), 1.24-1.32 (m, 6H).
(4) To a solution of compound **39-4** (2.00 g, 7.06 mmol) in tetrahydrofuran (20.0 mL), 10% palladium on carbon (200 mg) was added. The reaction mixture was stirred at 25°C under a hydrogen atmosphere (15 psi) for 16 hours. Then, another portion of 10% palladium on carbon (850 mg) was add and continued stirring for an additional 3 hours under the same hydrogen atmosphere. The reaction mixture was filtered, and then the filtrate was concentrated under reduced pressure to obtain compound **39-5.** ¹H NMR (400 MHz, CDCl₃) *δ* 4.12 (q, *J =* 7.2 Hz, 2H), 3.81 (brs, 2H), 2.32 (t, *J =* 8.4 Hz, 2H), 1.94-2.08 (m, 2H), 1.82-1.93 (m, 1H), 1.52-1.72 (m, 3H), 1.45 (s, 9H), 1.18-1.28 (m, 6H).
(5) To a solution of compound **39-5** (1.00 g, 3.50 mmol) in dichloromethane (10.0 mL), 4 M hydrochloric acid in ethyl acetate solution (5.0 mL) was added. The reaction mixture was stirred at 25°C for 1 hour and concentrated under reduced pressure. The residue was then dissolved in water (20.0 mL). The pH was adjusted to 10 using saturated sodium carbonate solution, followed by extraction with dichloromethane (20.0 mL × 3). The combined organic phase was dried over anhydrous sodium sulfate, filtered, and then concentrated under reduced pressure to obtain compound **39-6.** ¹H NMR (400 MHz, CDCl₃) *δ* 4.12 (q, *J* = 7.2 Hz, 2H), 3.10-3.21 (m, 1H), 3.00-3.10 (m, 1H), 2.31-2.46 (m, 2H), 1.84-1.91 (m, 2H), 1.77-1.84 (m, 2H), 1.28-1.43 (m, 2H), 1.25 (t, *J=* 7.2 Hz, 3H), 1.18 (d, *J=* 6.4 Hz, 3H)
(6) To a solution of compound **E** (173 mg, 543 µmol) and compound **39-6** (100 mg, 540 µmol) in dioxane (5.0 mL), cesium carbonate (352 mg, 1.08 mmol) and Methanesulfonato(2-dicyclohexylphosphino-2',6'-di-i-propoxy-1,1'-biphenyl)(2'-amino-1,1'-bip henyl-2-yl)palladium(II) (46.0 mg, 55.0 µmol) were added. The reaction mixture was stirred at 80°C for 3 hours under a nitrogen atmosphere. The reaction mixture was filtered and the filtrate was concentrated under reduced pressure. The resulting residue was dissolved in ethyl acetate (20.0 mL), washed with saturated sodium chloride solution (10.0 mL × 3). The organic phase was dried over anhydrous sodium sulfate, filtered, and then concentrated under reduced pressure. The crude product was purified by silica gel column chromatography (petroleum ether/ethyl acetate = 1 : 0 to 9 : 1) to obtain compound **39-7.** MS-ESI [M+H]⁺, calculated: 468, found: 468. ¹H NMR (400 MHz, CDCl₃) *δ* 7.41-7.48 (m, 2H), 7.39 (s, 1H), 6.80-6.90 (m, 2H), 5.09-5.20 (m, 2H), 4.15 (q, *J=* 7.2 Hz, 2H), 3.79-3.90 (m, 2H), 3.78 (s, 3H), 2.24-2.43 (m, 2H), 1.92-2.15 (m, 3H), 1.62-1.82 (m, 3H), 1.22-1.29 (m, 6H).
(7) To a solution of compound **39-7** (110 mg, 235 µmol) in tetrahydrofuran (3.0 ml) and water (1.0 ml), lithium hydroxide monohydrate (50.0 mg, 1.19 mmol) was added. The reaction was allowed to proceed at 25°C for 16 hours. The pH was adjusted to 6 by adding 1 M hydrochloric acid solution, followed by extraction with dichloromethane (20.0 ml × 3). The combined organic phases was dried over anhydrous sodium sulfate, filtered, and then concentrated under reduced pressure to obtain compound **39-8.** MS-ESI [M+H]⁺, calculated: 440, found: 440.
(8) To a solution of compound **B-4** (70.0 mg, 261 µmol) in dichloromethane (5.0 ml), diisopropylethylamine (0.12 ml, 689 µmol), compound **39-8** (90.0 mg, 205 µmol), and 50% propylphosphonic anhydride solution (0.15 ml, 252 µmol) were added. The reaction was allowed to proceed at 25°C for 3 hours. Water (10.0 ml) was added to the reaction mixture, followed by extraction with dichloromethane (10.0 ml × 3). The combined organic phase was washed with saturated sodium chloride solution (20.0 ml), dried over anhydrous sodium sulfate, filtered, and then concentrated under reduced pressure. The crude product was purified by silica gel column chromatography ( petroleum ether/ethyl acetate = 1:0 to 63:37) to obtain compound **39-9.** MS-ESI [M+H]⁺, calculated: 654, found: 654.
(9) To the solution of compound **39-9** (50.0 mg, 76.5 µmol) in dichloromethane (1.5 ml), trifluoromethanesulfonic acid (1.70 mmol, 0.15 ml) was added. The mixture was stirred at 25°C for 30 minutes. The pH was adjusted with saturated sodium carbonate solution to pH 7, followed by extraction with dichloromethane (10.0 ml × 3). The combined organic phase was washed with saturated sodium chloride solution (20.0 ml), dried over anhydrous sodium sulfate, filtered, and then concentrated under reduced pressure. The crude product was purified by preparative high-performance liquid chromatography (C18-6, 100 mm × 30 mm, 5 µm, A: water (0.225% formic acid) and B: acetonitrile (52%-82%) over 15 minutes) to obtain compound **39** formate. MS-ESI [M+H]⁺, calculated: 534, found: 534. ¹H NMR (400 MHz, MeOD) *δ* 8.51 (s, 2H), 7.78 (s, 1H), 3.87-3.98 (m, 5H), 3.66-3.80 (m, 2H), 3.50-3.62 (m, 2H), 2.33-2.51 (m, 2H), 2.08-2.20 (m, 2H), 1.98-2.07 (m, 1H), 1.64-1.85 (m, 4H), 1.28 (d, *J* = 6.0 Hz, 3H).

### Example 40 Synthesis of Compound 40

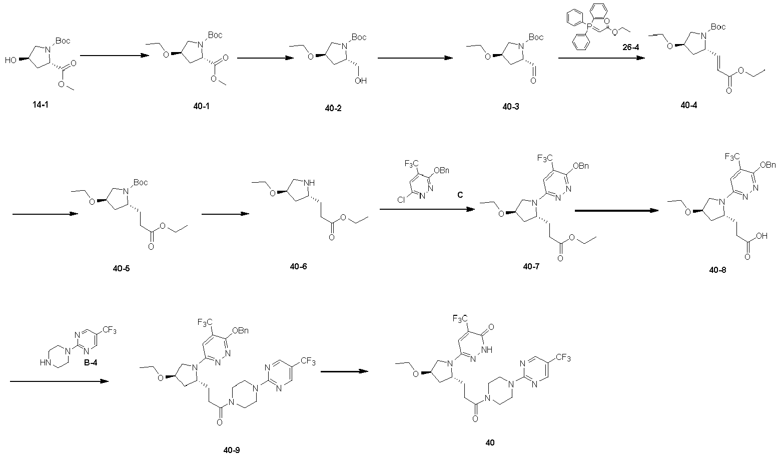

(1) The solution of compound **14-1** (1.00 g, 4.08 mmol) in tetrahydrofuran (10.0 mL) was cooled to 0°C, and 60% sodium hydride (326 mg, 8.16 mmol) was added. The mixture was reacted at 25°C for 1 hour. It was then cooled to 0°C and iodomethane (1.27 g, 8.16 mmol) was added. The mixture was reacted at 25°C for 2 hours. It was cooled to 0°C and quenched by adding 1 M hydrochloric acid (100.0 mL), followed by extraction with ethyl acetate (200.0 mL). The combined organic phase was washed with saturated sodium chloride solution (200.0 mL), dried over anhydrous sodium sulfate, filtered, and then concentrated under reduced pressure. The crude product was purified by silica gel column chromatography (petroleum ether/ethyl acetate = 1:0 to 5:1) to obtain compound **40-1.** ¹H NMR (400 MHz, CDCl₃) *δ* 4.29-4.58 (m, 2H), 4.01-4.16 (m, 1H), 3.39-3.80 (m, 6H), 2.09-2.52 (m, 2H), 1.39-1.45 (m, 9H), 1.17-1.29 (m, 3H).
(2) Compound **40-1** (800 mg, 2.78 mmol) was dissolved in tetrahydrofuran (10.0 mL) and cool to 0°C. Lithium aluminum hydride (211 mg, 5.57 mmol) was added. The reaction was allowed to proceed at 0°C for 1 hour. Water (2.5 mL), 15% sodium hydroxide solution (7.5 mL), and sodium sulfate were added. The mixture was stirred for 10 minutes, then filtered and concentrated to obtain compound **40-2.**
(3) Compound **40-2** (690 mg, 2.81 mmol) was dissolved in dichloromethane (20.0 mL) and cool to 0°C. Dess-Martin reagent (1.55 g, 3.66 mmol) was added. The reaction was allowed to proceed at 25°C for 12 hours. Saturated sodium bisulfite solution (30.0 mL) was added. The reaction mixture was stirred for 10 minutes and extracted with dichloromethane (100.0 mL). The organic phase was dried with anhydrous sodium sulfate, filtered, and concentrated under reduced pressure. The crude product was purified by silica gel column chromatography (petroleum ether/ethyl acetate = 1:0 to 4:1) to obtain compound **40-3.** ¹H NMR (400 MHz, CDCl₃) *δ* 9.39-9.62 (m, 1H), 4.15-3.98 (m, 2H), 3.65-3.47 (m, 4H), 2.13-2.28 (m, 1H), 1.88-2.09 (m, 1H), 1.42-1.46 (m, 9H), 1.20 (t, *J* = 7.2 Hz, 3H).
(4) To a solution of compound **40-3** (200 mg, 822 µmol) in dichloromethane (10.0 mL) added compound **26-4** (315 mg, 904 µmol). The reaction was allowed to proceed at 25°C for 10 hours. The reaction mixture was concentrated under reduced pressure. The crude product was purified by silica gel column chromatography (petroleum ether/ethyl acetate = 1:0 to 3:1) to obtain compound **40-4.** MS-ESI [M-Boc+H]⁺, calculated: 214, found: 214.
(5) To a solution of compound **40-4** (190 mg, 606 µmol) in tetrahydrofuran (3.0 mL) added 10% palladium on carbon (100 mg). The reaction mixture was stirred at 25°C under a hydrogen atmosphere (15 psi) for 10 hours. The reaction mixture was filtered, and the filtrate was then concentrated under reduced pressure to obtain compound **40-5.** MS-ESI [M+H]⁺, calculated: 316, found: 316.
(6) Trifluoroacetic acid (61.5 mg, 539 µmol) was added to a solution of compound **40-5** (170 mg, 539 µmol) in dichloromethane (3.0 mL). The reaction mixture was stirred at 25°C for 1 hour. Then, the reaction mixture was concentrated under reduced pressure to obtain compound **40-6.**
(7) Cesium carbonate (242 mg, 743 µmol), 1,1'-binaphthyl-2,2'-diphenylphosphine (68.1 mg, 74.3 µmol), and tris(dibenzylideneacetone)dipalladium (46.3 mg, 74.3 µmol) were added to a solution of compound **C** (118 mg, 409 µmol) and **40-6** (80.0 mg, 372 µmol) in toluene (2.0 mL). The reaction mixture was stirred at 80°C for 2 hours under a nitrogen atmosphere. After the reaction, the mixture was diluted with water (30.0 mL) and extracted with ethyl acetate (50.0 mL × 2). The combined organic phase was dried over anhydrous sodium sulfate, filtered, and concentrated under reduced pressure. The crude product was purified by silica gel column chromatography (petroleum ether/ethyl acetate = 1:0 to 3:1) to obtain compound **40-7.** MS-ESI [M+H]⁺, calculated: 468, found: 468.
(8) To a solution of compound **40-7** (40.0 mg, 85.6 µmol) in tetrahydrofuran (3.0 mL) and water (1.0 mL), lithium hydroxide monohydrate (17.9 mg, 428 µmol) was then added. The reaction mixture was stirred at 25°C for 2 hours. The reaction mixture was concentrated under reduced pressure. The resulting residue was dissolved in water (50.0 mL), and extracted with ethyl acetate (50.0 mL × 2). The pH of the aqueous phase was adjusted to 5 with 1 M hydrochloric acid, and extracted with ethyl acetate (50.0 mL × 2). The combined organic phase was dried over anhydrous sodium sulfate, filtered, and concentrated under reduced pressure to obtain compound **40-8.** MS-ESI [M+H]⁺, calculated: 440, found: 440.
(9) To a solution of compound **40-8** (30.0 mg, 68.3 µmol) and compound **B-4** (15.9 mg, 68.3 µmol) in dichloromethane (3.0 mL), diisopropylethylamine (61.8 mg, 478 µmol) and 50% propylphosphonic anhydride solution (109 mg, 171 µmol) were added. The reaction proceeded at 25°C for 1 hour. Saturated sodium bicarbonate solution (30.0 mL) was added. The mixture was extracted with ethyl acetate (50.0 mL × 2). The combined organic phase was dried over anhydrous sodium sulfate, filtered, and concentrated under reduced pressure. The crude product was purified by silica gel column chromatography (petroleum ether/ethyl acetate = 1:0 to 3:1) to obtain compound **40-9.** MS-ESI [M+H]⁺, calculated: 654, found: 654.
(10) To a solution of compound **40-9** (25.0 mg, 38.3 µmol) in tetrahydrofuran (3.0 mL) was added 10% palladium on carbon (50.0 mg). The reaction mixture was stirred at 25°C under a hydrogen atmosphere (15 psi) for 2 hours and filtered. The filtrate was concentrated under reduced pressure. The crude product was purified by high-performance liquid chromatography (Welch Xtimate C18 column, 150 mm × 30 mm, 5 µm, A: water (10 mmol/L ammonium bicarbonate) and B: acetonitrile (25% to 70%) over 12 minutes) to obtain compound **40.** MS-ESI [M+H]⁺, calculated: 564, found: 564. ¹H NMR (400 MHz, MeOD) *δ* 8.60 (s, 2H), 7.82 (s, 1H), 4.24 (q, *J* = 4.4 Hz, 1H), 4.04-4.13 (m, 1H), 3.88-4.02 (m, 4H), 3.62-3.73 (m, 4H), 3.43-3.58 (m, 4H) , 2.49 (t, *J* = 7.2 Hz, 2H), 1.99-2.28 (m, 4H), 1.17 (t, *J* = 7.2 Hz, 3H).

### Example 41 Synthesis of Compound 41

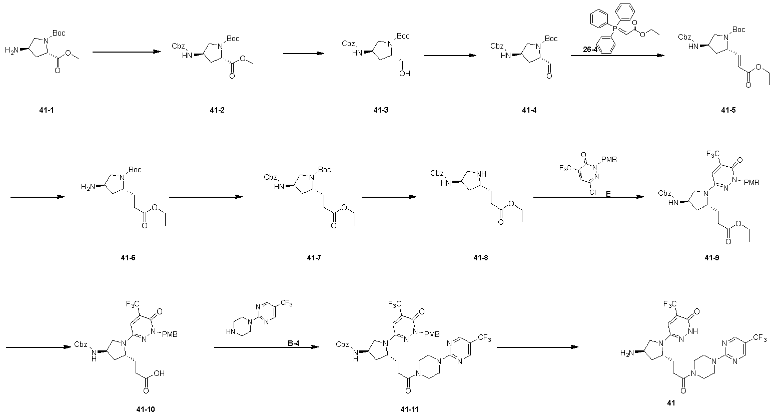

(1) To a solution of compound **41-1** (500 mg, 2.05 mmol) in tetrahydrofuran (10.0 mL) and water (10.0 mL), sodium carbonate (868 mg, 8.19 mmol) and N-benzyloxycarbonyl succinimide (1.02 g, 4.09 mmol) were added. The reaction was allowed to proceed at 25°C for 6 hours. Once the reaction was complete, water (30.0 mL) was added, followed by extraction with ethyl acetate (200.0 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **41-2.** MS-ESI [M-Boc+H]⁺, calculated: 279, found: 279. ¹H NMR (400 MHz, CDCl₃) *δ* 7.30-7.43 (m, 5H), 5.11 (s, 2H), 4.74-4.86 (m, 1H), 4.25-4.42 (m, 2H), 3.74 (s, 4H), 3.23-3.43 (m, 1H), 2.11-2.30 (m, 2H), 1.46 (s, 3H), 1.40(s, 6H).
(2) Compound **41-2** (750 mg, 1.98 mmol) was dissolved in tetrahydrofuran (8.0 mL) and cooled to 0°C. Lithium aluminum hydride (150 mg, 3.96 mmol) was added. The reaction was allowed to proceed at 0°C for 2 hours. Then, water (0.15 mL), 15% sodium hydroxide solution (0.15 mL), and water (0.45 mL) were added. The mixture was extracted with ethyl acetate (100 mL). The organic phase was dried over anhydrous sodium sulfate, filtered, and concentrated under reduced pressure to obtain compound **41-3.** MS-ESI [M+Na]⁺, calculated: 373, found: 373.
(3) Compound **41-3** (420 mg, 1.20 mmol) was dissolved in dichloromethane (5.0 mL), and Dess-Martin reagent (610 mg, 1.44 mmol, 445 µL) was added. The reaction was allowed to proceed at 25°C for 5 hours. The reaction mixture was quenched with saturated sodium bisulfite solution (20.0 mL) and extracted with dichloromethane (100.0 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **41-4.** ¹H NMR (400 MHz, CDCl₃) *δ* 9.40-9.62 (m, 1H), 7.32-7.37 (m, 5H), 5.10 (s, 2H), 4.86 (br d, *J* = 2.4 Hz, 1H), 4.27 (br s, 1H), 4.12-4.19 (m, 1H), 3.72 (br dd, *J* = 11.2, 5.6 Hz, 1H), 3.26-3.48 (m, 1H), 2.05-2.29 (m, 2H), 1.46 (s, 3H), 1.42(s, 6H).
(4) To the solution of compound **41-4** (299 mg, 858 µmol) in dichloromethane (4.0 mL), compound **26-4** (329 mg, 944 µmol) was added. The reaction was allowed to proceed at 25°C for 12 hours. The reaction mixture was then concentrated under reduced pressure. The crude product was purified by silica gel column chromatography (petroleum ether/ethyl acetate = 100:1 to 1:1) to yield compound **41-5.** ¹H NMR (400 MHz, CDCl₃) *δ* 7.31-7.41 (m, 5H), 6.79 (br d, *J* = 5.2 Hz, 1H), 5.85 (br d, *J* = 15.6 Hz, 1H), 5.11 (s, 2H), 4.80 (br s, 1H), 4.39-4.60 (m, 1H), 4.28 (br d, *J* = 4.8 Hz, 1H), 4.20 (br d, *J* = 6.8 Hz, 2H), 3.70 (br s, 1H), 3.16-3.39 (m, 1H), 2.04-2.0.8 (m, 1H), 1.43 (br s, 9H), 1.27-1.34 (m, 3H).
(5) To a solution of compound **41-5** (260 mg, 629 µmol) in tetrahydrofuran (3.0 mL) added 10% palladium on carbon (50 mg). The reaction mixture was stirred under a hydrogen atmosphere (15 psi) at 25°C for 1 hour. After completion, the reaction mixture was filtered, and the filtrate was concentrated under reduced pressure to obtain compound **41-6.** MS-ESI [M-Boc+H]⁺, calculated: 287, found: 287.
(6) Compound **41-6** (160 mg, 559 µmol) was dissolved in a mixture of tetrahydrofuran (3.0 mL) and water (3.0 mL). To this solution, sodium carbonate (237 mg, 2.23 mmol) and N-benzyloxycarbonyl succinimide (278 mg, 1.12 mmol) were added. The reaction was carried out at 25°C for 6 hours. After completion, water (30.0 mL) and ethyl acetate (200.0 mL) were added for extraction. The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **41-7.** MS-ESI [M+H]⁺, calculated: 421, found: 421.¹H NMR (400 MHz, CDCl₃) *δ* 7.41 (s, 2H), 7.35-7.37 (m, 3H), 5.10 (s, 2H), 4.76 (br s, 1H), 4.30 (br d, *J* = 6.0 Hz, 1H), 3.90 (br s, 1H), 3.57 (br dd, *J* = 11.2, 6.0 Hz, 1H), 3.23-3.42 (m, 1H), 2.85 (s, 2H), 2.30 (br t, *J* = 7.2 Hz, 2H), 2.03-2.13 (m, 1H), 1.94 (br s, 2H), 1.75 (br dd, *J* = 13.6, 7.6 Hz, 1H), 1.46 (s, 9H), 1.23-1.28 (m, 3H).
(7) Compound **41-7** (190 mg, 452 µmol) was dissolved in dichloromethane (1.5 mL), and trifluoroacetic acid (770 mg, 6.75 mmol, 0.5 mL) was added. The reaction mixture was stirred at 25°C for 1 hour. The reaction mixture was then adjusted to pH 9 using saturated sodium carbonate solution, and extracted with ethyl acetate (50.0 mL). The organic phase was dried over anhydrous sodium sulfate, filtered, and concentrated under reduced pressure to obtain compound **41-8.** MS-ESI [M+H]⁺, calculated: 321, found: 321.
(8) To the solution of compound **E** (199 mg, 624 µmol) and compound **41-8** (100 mg, 312 µmol) in toluene (2.0 mL), cesium carbonate (203 mg, 624 µmol), 1,1'-binaphthyl-2,2'-diphenylphosphine (38.9 mg, 62.4 µmol), and tris (dibenzylideneacetone) dipalladium (28.6 mg, 31.2 µmol) were added. The reaction mixture was stirred at 80°C under a nitrogen atmosphere for 5 hours. After the reaction, the mixture was filtered, and the filtrate was concentrated under reduced pressure. The resulting residue was dissolved in ethyl acetate (50.0 mL) and washed with water (30.0 mL). The organic phase was dried over anhydrous sodium sulfate, filtered, and concentrated under reduced pressure. The crude product was purified by silica gel column chromatography (petroleum ether/ethyl acetate = 100:1 to 1:1) to obtain compound **41-9.** MS-ESI [M+H]⁺, calculated: 603, found: 603.
(9) Compound **41-9** (24.0 mg, 39.8 µmol) was dissolved in tetrahydrofuran (0.9 mL) and water (0.3 mL), and then lithium hydroxide monohydrate (16.7 mg, 398 µmol) was added. The reaction was carried out at 25°C for 8 hours. To the reaction mixture, 1 M hydrochloric acid solution was added to adjust the pH to less than 7, and the mixture was then extracted with ethyl acetate (50.0 mL). The organic phase was dried over anhydrous sodium sulfate, filtered, and concentrated under reduced pressure to obtain compound **41-10.** MS-ESI [M+H]⁺, calculated: 575, found: 575.
(10) Compound **41-10** (20.0 mg, 34.8 µmol) and compound **B-4** (12.1 mg, 52.2 µmol) was dissolved in dichloromethane (1.0 mL), and then diisopropylethylamine (22.5 mg, 174 µmol, 30.3 µL) and 50% propylphosphonic anhydride solution (66.5 mg, 104 µmol, 62.1 µL) were added. The reaction was carried out at 25°C for 4 hours. To the reaction mixture, water (5.0 mL) was added, and it was then extracted with ethyl acetate (50.0 mL). The organic phase was dried over anhydrous sodium sulfate, filtered, and concentrated under reduced pressure. The crude product was separated by preparative TLC (dichloromethane/methanol = 10:1) to obtain compound **41-11.** MS-ESI [M+H]⁺, calculated: 789, found: 789.
(11) Compound **41-11** (20.0 mg, 25.4 µmol) was dissolved in trifluoroacetic acid (1.0 mL), and then trifluoromethanesulfonic acid (340 mg, 2.27 mmol, 200 µL) was added. The mixture was stirred at 25°C for 30 minutes. To the reaction mixture, saturated sodium carbonate solution was added to adjust the pH to >7, followed by extraction with ethyl acetate (50.0 mL). The organic phase was dried over anhydrous sodium sulfate, filtered, and concentrated under reduced pressure. The crude product was separated by preparative high-performance liquid chromatography ( Phenomenex C18 column, 75 mm × 30 mm, 3 µm, A: water (10 mmol/L ammonium bicarbonate) and B: acetonitrile (24%-54%) in 14 minutes) to obtain compound **41.** MS-ESI [M+H]⁺, calculated: 535, found: 535. ¹H NMR (400 MHz, MeOD) *δ*8.60 (s, 2H), 7.86 (s, 1H), 4.04-4.13 (m, 1H), 3.88-4.00 (m, 4H), 3.61-3.74 (m, 6H), 3.10-3.17 (m, 1H), 2.50 (t, *J* = 7.2 Hz, 2H), 2.01-2.15 (m, 2H), 1.94 (dt, *J* = 12.4, 7.6 Hz, 1H), 1.67-1.77 (m, 1H).

### Example 42 Synthesis of Compound 42

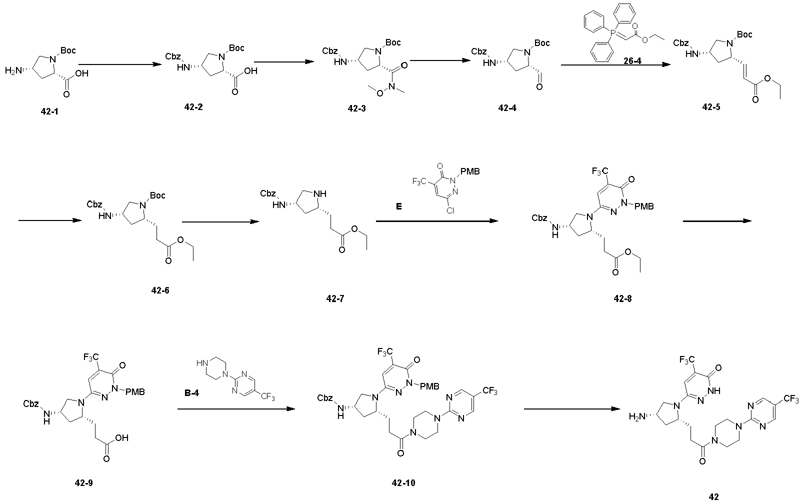

(1) Compound **42-1** (2.00 g, 8.69 mmol) was dissolved in tetrahydrofuran (20.0 mL) and water (20.0 mL). To this solution, sodium carbonate (3.68 g, 34.7 mmol) and N-benzyloxycarbonyl succinimide (4.33 g, 17.3 mmol) were added. The reaction was carried out at 25°C for 12 hours. Upon completion of the reaction, water (100 mL) and ethyl acetate (200 mL × 2) were used for extraction. The combined organic phase was washed with saturated sodium chloride solution (100 mL), dried over anhydrous sodium sulfate, filtered, and then concentrated under reduced pressure. The crude product was purified by silica gel column chromatography ( dichloromethane/methanol = 10:1) to obtain compound **42-2.** MS-ESI [M-Boc+H]⁺, calculated: 265, found: 265.
(2) Compound **42-2** (2.90 g, 7.96 mmol), N-methoxymethylamine hydrochloride (1.55 g, 15.9 mmol), 50% propylphosphonic anhydride solution (10.1 g, 15.9 mmol, 9.47 mL ) and diisopropylethylamine (5.14 g, 39.7 mmol, 6.93 mL) were dissolved in dichloromethane (30.0 mL). The reaction was carried out at 25°C for 1 hour under a nitrogen atmosphere. Water (100 mL) was added to the reaction mixture, followed by extraction with dichloromethane (200 mL × 2). The combined organic phase was washed with saturated sodium chloride solution (100 mL), dried over anhydrous sodium sulfate, filtered, and then concentrated under reduced pressure. The crude product was purified by silica gel column chromatography ( dichloromethane/methanol = 10:1) to obtain compound **42-3.** MS-ESI [M+H]⁺, calculated: 408, found: 408. ¹H NMR (400 MHz, CDCl₃) *δ* 7.28-7.40 (m, 5H), 5.09 (s, 2H), 4.70-4.86 (m, 1H), 4.44 (br s, 1H), 3.49-3.83 (m, 5H), 3.21 (s, 3H), 2.36-2.52 (m, 1H), 1.86 (br t, *J* = 14.4 Hz, 1H), 1.44 (d, *J* = 14.8 Hz, 9H).
(3) Compound **42-3** (1.50 g, 3.68 mmol) was dissolved in dichloromethane (30.0 mL) under a nitrogen atmosphere, cooled to -78°C, and 1 M diisobutylaluminum hydride (11.0 mL) was added dropwise. The reaction mixture was stirred at -78°C for 2 hours. After the reaction was complete, it was slowly quenched with methanol (10.0 mL). Hydrochloric acid (1 M) was added to adjust the pH to 6, and the mixture was extracted with ethyl acetate (100 mL). The organic phase was dried over anhydrous sodium sulfate, filtered, and then concentrated under reduced pressure to obtain compound **42-4.**
(4) Compound **42-4** (1.28 g, 3.67 mmol) was dissolved in dichloromethane (50.0 mL), and compound **26-4** (1.54 g, 4.41 mmol) was added to the solution. The reaction was carried out at 25°C for 2 hours. The reaction mixture was then concentrated under reduced pressure. The crude product was separated by silica gel column chromatography (petroleum ether/ethyl acetate = 1:0 to 0:1) to obtain compound **42-5.** MS-ESI [M-Boc+H]⁺, calculated: 319, found: 319.
(5) Compound **42-5** (800 mg, 1.91 mmol) was dissolved in tetrahydrofuran (5.0 mL), and 10% palladium on carbon (100 mg) was added to the solution. The reaction mixture was stirred at 25°C under a hydrogen atmosphere (15 psi) for 6 hours. After completion of the reaction, the reaction mixture was filtered, and the filtrate was concentrated under reduced pressure to obtain compound **42-6.** MS-ESI [M-Boc+H]⁺, calculated: 421, found: 421. ¹H NMR (400 MHz, CDCl₃) *δ* 7.28-7.40 (m, 5H), 5.11 (s, 2H), 4.13 (q, *J* = 7.2 Hz, 3H), 3.85 (br s, 2H), 3.04 (br s, 2H), 2.06-2.42 (m, 4H), 1.76-1.86 (m, 1H), 1.51-1.60 (m, 1H), 1.46 (s, 9H), 1.25 (t, *J* = 7.2 Hz, 3H).
(6) Compound **42-6** (370 mg, 879 µmol) was dissolved in dichloromethane (8.0 mL), and trifluoroacetic acid (3.08 g, 27.0 mmol) was added to the solution. The reaction mixture was stirred at 25°C for 1 hour. After the reaction, the mixture was adjusted to a pH greater than 8 using saturated sodium bicarbonate solution, and then extracted with dichloromethane (30.0 mL × 2). The combined organic phase was washed with saturated sodium chloride (30.0 mL), dried over anhydrous sodium sulfate, filtered, and concentrated under reduced pressure to obtain compound **42-7.** MS-ESI [M+H]⁺, calculated: 321, found: 321.
(7) Compound **E** (179 mg, 561 µmol) and compound **42-7** (150 mg, 468 µmol) were dissolved in toluene (10.0 mL), and cesium carbonate (457 mg, 1.40 mmol), 1,1'-binaphthyl-2,2'-diphenylphosphine (58.3 mg, 93.6 µmol), and tris (dibenzylideneacetone) dipalladium (42.8 mg, 46.8 µmol) were added. The reaction mixture was stirred at 80°C for 6 hours under nitrogen protection. Water (10.0 mL) was added to the reaction mixture, and it was then extracted with ethyl acetate (50.0 mL × 2). The combined organic phase was washed with saturated sodium chloride (10.0 mL), dried over anhydrous sodium sulfate, filtered, and concentrated under reduced pressure. The crude product was purified by silica gel column chromatography (petroleum ether/ethyl acetate = 2:1) to obtain compound **42-8.** MS-ESI [M+H]⁺, calculated: 603, found: 603.
(8) Compound **42-8** (10.0 mg, 16.5 µmol) was dissolved in tetrahydrofuran (3.0 mL) and water (1.0 mL). To this solution, lithium hydroxide monohydrate (4.18 mg, 99.5 µmol) was added, and the reaction was carried out at 25°C for 2 hours. 1 M hydrochloric acid solution was added to adjust the pH to less than 7. The mixture was diluted with water (20.0 mL) and extracted with dichloromethane (30.0 mL × 2). The combined organic phase was washed with saturated sodium chloride (30.0 mL), dried over anhydrous sodium sulfate, filtered, and concentrated under reduced pressure to obtain compound **42-9.** MS-ESI [M+H]⁺, calculated: 575, found: 575.
(9) Compound **42-9** (9.00 mg, 15.6 µmol) and **B-4** (7.27 mg, 31.3 µmol) were dissolved in dichloromethane (2.0 mL). To this solution, diisopropylethylamine (10.1 mg, 78.3 µmol) and 50% propylphosphonic anhydride (39.8 mg, 62.6 µmol) were added. The reaction was carried out at 25°C for 1 hour. To the reaction mixture, water (50.0 mL) was added, and it was extracted with dichloromethane (50.0 mL × 2). The combined organic phase was washed with saturated sodium chloride (50.0 mL), dried over anhydrous sodium sulfate, filtered, and concentrated under reduced pressure. The crude product was purified by silica gel column chromatography (dichloromethane/methanol = 1:1 to 10:1) to obtain compound **42-10.** MS-ESI [M+H]⁺, calculated: 789, found: 789. ¹H NMR (400 MHz, CDCl₃) *δ* 8.49-8.55 (m, 3H), 7.41-7.43 (m, 2H), 7.36-7.39 (m, 5H), 6.84-6.87 (m, 2H), 5.13-5.14 (m, 2H), 3.94-4.04 (m, 2H), 3.86-3.89 (m, 4H), 3.79 (s, 4H), 3.65-3.77 (m, 6H), 3.38-3.48 (m, 4H), 2.33-2.37 (m, 2H), 2.13-2.23 (m, 2H).
(10) Compound **42-10** (6.00 mg, 7.61 µmol) was dissolved in trifluoroacetic acid (1.0 mL). To this solution, trifluoromethanesulfonic acid (1.14 mg, 7.61 µmol) was added, and the mixture was stirred at 25°C for 1 hour. The reaction mixture was then adjusted to pH > 7 with saturated sodium carbonate solution and extracted with ethyl acetate (20.0 mL × 2). The combined organic phase was washed with saturated sodium chloride (50.0 mL), dried over anhydrous sodium sulfate, filtered, and concentrated under reduced pressure. The crude product was purified by preparative high-performance liquid chromatography (Welch Xtimate C18 column, 150 mm × 30 mm, 5 µm, A: water (10 mmol/L ammonium bicarbonate) and B: acetonitrile (25%-65%) over 12 minutes) to obtain compound **42.** MS-ESI [M+H]⁺, calculated: 535, found: 535.

### Example 43 Synthesis of Compound 43

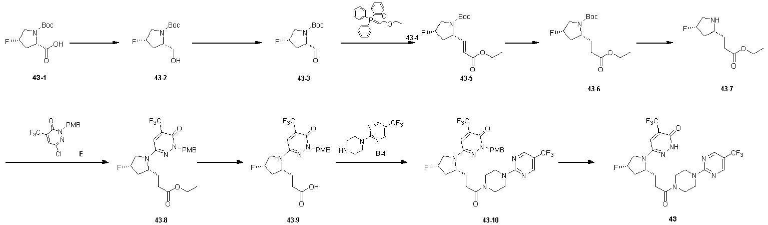

(1) To a solution of Compound **43-1** (2.0 g, 8.58 mmol) in tetrahydrofuran (20.0 mL), 1 M borane tetrahydrofuran complex (25.7 mL) was added at 0°C. The reaction was stirred for 1 hour at 0°C. Subsequently, 100 mL of methanol was introduced into the reaction mixture at 0°C. The mixture was concentrated under reduced pressure. The crude product was then purified by silica gel column chromatography ( petroleum ether/ethyl acetate = 1:0 to 4:1) to isolate compound **43-2.** MS-ESI [M-^{t}Bu+H]⁺, calculated: 164, found: 164.
(2) Compound **43-2** (1.16 g, 5.29 mmol) was dissolved in 20.0 mL of dichloromethane. To this solution, Dess-Martin reagent (2.69 g, 6.35 mmol) was added, and the reaction mixture was stirred at 25°C for 8 hours. Sodium sulfite (10.0 mL) was added to the reaction mixture, followed by extraction with dichloromethane (50.0 mL). The organic layer was separated, dried over anhydrous sodium sulfate, filtered, and then concentrated under reduced pressure. The crude product was further purified by silica gel column chromatography (petroleum ether/ethyl acetate = 1:0 to 2:1) to obtain compound **43-3.** MS-ESI [M-^{t}Bu+H]⁺, calculated: 162, found: 162. ¹H NMR (400 MHz, CDCl₃) *δ* 9.56-9.65 (m, 1H), 5.13-5.33 (m, 1H), 4.17-4.36 (m, 1H), 3.77-3.99 (m, 1H), 3.52-3.68 (m, 1H), 2.26-2.50 (m, 2H), 1.46-1.51 (m, 9H).
(3) Compound **43-3** (400 mg, 1.84 mmol) was dissolved in 5.0 mL of dichloromethane. To this solution, compound **43-4** (770 mg, 2.21 mmol) was added, and the reaction mixture was stirred at 25°C for 10 hours. The reaction mixture was then concentrated under reduced pressure. The resulting crude product was further purified by silica gel column chromatography (petroleum ether/ethyl acetate = 1:0 to 2:1) to obtain compound **43-5.** MS-ESI [M-Boc+H]⁺, calculated: 188, found: 188. ¹H NMR (400 MHz, CDCl₃) *δ* 6.81-7.00 (m, 1H), 5.82-5.97 (m, 1H), 5.14-5.34 (m, 1H), 4.41-4.68 (m, 1H), 4.21 (br d, *J* = 6.4 Hz, 2H), 3.53-3.88 (m, 2H), 2.15-2.4 (m, 2H), 1.41-1.51 (m, 9H), 1.30 (br t, *J* = 6.8 Hz, 3H).
(4) Compound **43-5** (410 mg, 1.43 mmol) was dissolved in 5.0 mL of tetrahydrofuran. To this solution, 10% palladium on carbon (120 mg) was added, and the reaction mixture was stirred under a hydrogen atmosphere at 40°C for 6 hours. The reaction mixture was then filtered, and the filtrate was concentrated under reduced pressure to obtain compound **43-6.** MS-ESI [M+H]⁺, calculated: 290, found: 290.
(5) Compound **43-6** (400 mg, 1.38 mmol) was dissolved in 6.0 mL of dichloromethane. To this solution, trifluoroacetic acid (3.08 g, 27.0 mmol) was added, and the reaction mixture was stirred at 25°C for 0.5 hours. It was adjusted to a pH greater than 7 using sodium carbonate. The mixture was then extracted with dichloromethane (100 mL). The organic layer was separated, dried over anhydrous sodium sulfate, filtered, and concentrated under reduced pressure to obtain compound **43-7.** ¹H NMR (400 MHz, CDCl₃) *δ* 4.14 (q, *J* = 7.2 Hz, 2H), 3.33 (ddd, *J* = 19.6, 13.2, 1.6 Hz, 1H), 3.07-3.16 (m, 1H), 2.77-2.93 (m, 1H), 2.45 (td, *J* = 7.6, 1.2 Hz, 2H), 2.15-2.38 (m, 2H), 1.92 (q, *J* = 7.2 Hz, 2H), 1.57-1.74 (m, 1H), 1.26 (t, *J* = 7.2 Hz, 3H).
(6) Compound **43-7** (200 mg, 1.06 mmol) was dissolved in 10.0 mL of toluene. To this solution, compound **E** (505 mg, 1.59 mmol), cesium carbonate (689 mg, 2.11 mmol), 1,1'-binaphthyl-2,2'-diphenylphosphine (132 mg, 211 µmol), and tris (dibenzylideneacetone) dipalladium (96.8 mg, 106 µmol) were added. The reaction mixture was stirred at 80°C under a nitrogen atmosphere for 6 hours. Water (5.0 mL) was added to the reaction mixture, followed by extraction with ethyl acetate (50.0 mL). The organic layer was separated, dried over anhydrous sodium sulfate, filtered, and concentrated under reduced pressure. The crude product was further purified by silica gel column chromatography (petroleum ether/ethyl acetate = 4:1) to obtain compound **43-8.** MS-ESI [M+H]⁺, calculated: 472, found: 472.
(7) Compound **43-8** (340 mg, 721 µmol) was dissolved in 3.0 mL of tetrahydrofuran. To this solution, 1.0 mL of water and lithium hydroxide monohydrate (454 mg, 10.8 mmol) were added. The reaction mixture was stirred at 25°C under a nitrogen atmosphere for 12 hours. The pH of the reaction mixture was adjusted to less than 7 by adding 1 M hydrochloric acid solution. The mixture was then extracted with ethyl acetate (100 mL). The organic layer was separated, dried over anhydrous sodium sulfate, filtered, and concentrated under reduced pressure to obtain compound **43-9.** MS-ESI [M+H]⁺, calculated: 444, found: 444.
(8) Compound **43-9** (300 mg, 677 µmol) was dissolved in 5.0 mL of dichloromethane. To this solution, compound **B-4** trifluoroacetate (189 mg, 812 µmol), diisopropylethylamine (437 mg, 3.38 mmol), and a solution of propylphosphonic anhydride in ethyl acetate (1.29 g, 2.03 mmol, 50% purity ) were added. The reaction mixture was stirred at 25°C under a nitrogen atmosphere for 4 hours. Water (15.0 mL) was added to the reaction mixture, followed by extraction with dichloromethane (100 mL). The organic layer was separated, dried over anhydrous sodium sulfate, filtered, and concentrated under reduced pressure. The crude product was further purified by silica gel column chromatography ( petroleum ether/ethyl acetate = 1:1) to obtain compound **43-10.** MS-ESI [M+H]⁺, calculated: 658, found: 658.
(9) Compound **43-10** (120 mg, 182 µmol) was dissolved in 2.0 mL of trifluoroacetic acid. To this solution, trifluoromethanesulfonic acid (0.2 mL) was added, and the reaction mixture was stirred at 25°C for 0.5 hours. The pH of the reaction mixture was adjusted to greater than 7 by adding sodium carbonate. The mixture was then extracted with ethyl acetate (100 mL). The organic layer was separated, dried over anhydrous sodium sulfate, filtered, and concentrated under reduced pressure. The crude product was further purified by preparative high-performance liquid chromatography (C18, 100 mm × 30 mm × 5 µm, A: water (0.225% formic acid) and B: acetonitrile, (40% to 70%) over 15 minutes) to obtain compound **43** formate. MS-ESI [M+H]⁺, calculated: 538, found: 538. ¹H NMR (400 MHz, MeOD) *δ* 8.60 (s, 2H), 8.00 (s, 1H), 5.27-5.47 (m, 1H), 4.07-4.18 (m, 1H), 3.90-4.00 (m, 4H), 3.74-3.84 (m, 1H), 3.59-3.72 (m, 5H), 2.49-2.60 (m, 2H), 2.22-2.34 (m, 2H), 2.08-2.16 (m, 1H), 1.83-1.95 (m, 1H).

### Example 44 Synthesis of Compound 44

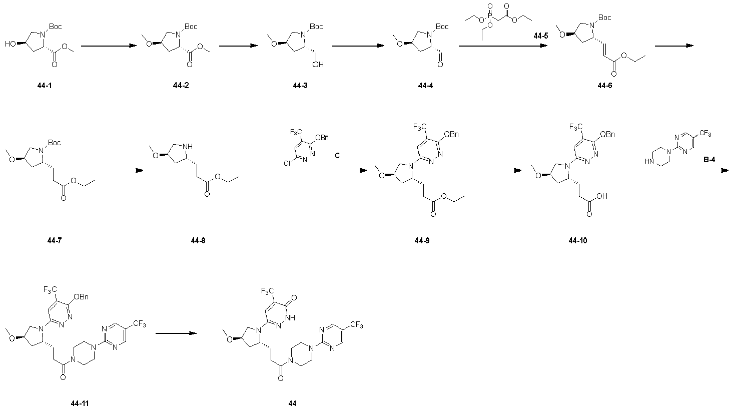

(1) To the solution of compound **44-1** (5.0 g, 20.3 mmol) in 50.0 mL of *N, N*-dimethylformamide, sodium hydride (1.63 g, 40.7 mmol) was added at 0°C. The reaction mixture was stirred at 25°C for 0.5 hours. Then, methyl iodide (14.4 g, 101 mmol) was added to the reaction mixture, and the mixture was stirred at 25°C for 2 hours under a nitrogen atmosphere. The reaction mixture was quenched with hydrochloric acid (50.0 mL, 0.5 mol/L) and extracted with ethyl acetate (150 mL). The organic phase was washed with saturated sodium chloride (500 mL × 3), dried over anhydrous sodium sulfate, filtered, and concentrated under reduced pressure. The crude product was further purified by silica gel column chromatography (petroleum ether/ethyl acetate =1:0 to 0:1) to obtain compound **44-2.** MS-ESI [M-^{t}Bu+H]⁺, calculated: 204, found: 204.
(2) At 0°C, compound **44-2** (3.6 g, 13.8 mmol) was dissolved in 40.0 mL of tetrahydrofuran. To this solution, lithium aluminum hydride (1.0 g, 26.3 mmol) was added, and the reaction mixture was stirred at 0°C for 1 hour. At 0°C, water (1.0 mL) and 15% sodium hydroxide (1.0 mL), and water (3.0 mL) were added to the reaction mixture. The mixture was then extracted with water (50.0 mL) and ethyl acetate (50.0 mL). The organic layer was separated, and further washed with saturated sodium chloride (50.0 mL × 2). The combined organic layer was dried over anhydrous sodium sulfate, filtered, and concentrated under reduced pressure to obtain compound **44-3.** MS-ESI [M-^{t}Bu+H]⁺, calculated: 176, found: 176. ¹H NMR (400 MHz, CDCl₃) *δ* 3.95-4.09 (m, 1H), 3.66-3.86 (m, 2H), 3.44-3.63 (m, 2H), 3.26-3.39 (m, 4H), 2.09-2.20 (m, 1H), 1.71-1.86 (m, 0.5H), 1.54-1.65 (m, 0.5H), 1.46 (s, 9H).
(3) To a solution of compound **44-3** (1.02 g, 4.41 mmol) in 15.0 mL of dichloromethane, Dess-Martin reagent (2.81 g, 6.62 mmol) was added. The reaction mixture was stirred at 25°C for 12 hours. Sodium sulfite solution (30.0 mL) was added to the reaction mixture, and the mixture was diluted with 20.0 mL of dichloromethane. The resulting mixture was then extracted with dichloromethane (50.0 mL × 2). The organic layer was separated, dried over anhydrous sodium sulfate, filtered, and concentrated under reduced pressure. The crude product was further purified by silica gel column chromatography (petroleum ether/ethyl acetate =1:1 to 0:1) to obtain compound **44-4.** ¹H NMR (400 MHz, CDCl₃) *δ* 9.44-9.64 (m, 1H), 4.02-4.23 (m, 1H), 3.85-3.94 (m, 1H), 3.55-3.74 (m, 1H), 3.38-3.48 (m, 1H), 3.18-3.28 (m, 3H), 2.24-2.41 (m, 1H), 2.00-2.20 (m, 1H), 1.42-1.49 (m, 9H).
(4) At 0°C, compound **44-5** (469 mg, 2.09 mmol) was added to a solution of 60% sodium hydride (104 mg, 2.62 mmol) in 6.0 mL of tetrahydrofuran. The reaction mixture was stirred at 0°C for 0.5 hours. Subsequently, a solution of compound **44-4** (400 mg, 1.74 mmol) in 6.0 mL of tetrahydrofuran was added, and the mixture was stirred at 0°C for an additional 0.5 hours under a nitrogen atmosphere. Ice-cold water (20.0 mL) was added, followed by extraction with ethyl acetate (30.0 mL). The organic layer was washed with water (75.0 mL × 2) and saturated sodium chloride (75.0 mL × 2), dried over anhydrous sodium sulfate, filtered and concentrated under reduced pressure to obtain compound **44-6.** MS-ESI [M-Boc+H]⁺, calculated: 276, found: 276. ¹H NMR (400 MHz, CDCl₃) *δ* 6.66-7.07 (m, 1H), 5.69-5.98 (m, 1H), 4.27-4.61 (m, 1H), 4.11-4.25 (m, 2H), 3.86-3.97 (m, 1H), 3.41-3.75 (m, 2H), 3.30 (d, *J* = 7.6 Hz, 3H), 2.22 (s, 1H), 1.82-2.01 (m, 1H), 1.41 (s, 9H), 1.22-1.33 (m, 3H).
(5) To a solution of compound **44-6** (300 mg, 1.0 mmol) in 6.0 mL of tetrahydrofuran, 10% palladium on carbon (150 mg) was added. The reaction mixture was stirred under a hydrogen atmosphere at 25°C for 2 hours. Afterward, the mixture was filtered, and the filtrate was concentrated under reduced pressure to obtain compound **44-7.** MS-ESI [M+H]⁺, calculated: 302, found: 302. ¹H NMR (400 MHz, CDCl₃) *δ* 4.12 (q, *J* = 7.2 Hz, 2H), 3.76-3.96 (m, 2H), 3.50-3.76 (m, 1H), 3.50-3.76 (m, 1H), 3.30 (d, *J* = 1.6 Hz, 3H), 2.23-2.37 (m, 2H), 2.06 (d, *J* = 16.8 Hz, 2H), 1.71-1.90 (m, 2H), 1.46 (s, 9H), 1.25 (t, *J* = 7.2 Hz, 3H).
(6) To a solution of compound **44-7** (300 mg, 995 µmol) in 8.0 mL of dichloromethane, trifluoroacetic acid (2.31 g, 20.2 mmol) was added. The reaction mixture was stirred at 25°C for 1 hour. The mixture was adjusted to pH greater than 7 using sodium bicarbonate, diluted with 20.0 mL of dichloromethane and washed with water (40.0 mL × 2). The organic layer was separated, dried over anhydrous sodium sulfate, filtered, and concentrated under reduced pressure to obtain compound **44-8.** MS-ESI [M+H]⁺, calculated: 202, found: 202.¹H NMR (400 MHz, CDCl₃) δ 4.13-4.17 (m, 1H), 4.08 (dd, *J* = 4.8, 2.4 Hz, 1H), 3.75-3.90 (m, 1H), 3.40-3.57 (m, 1H), 3.31 (d, *J* = 8.4 Hz, 3H), 2.44-2.58 (m, 2H), 2.26-2.43 (m, 3H), 2.05-2.22 (m, 2H), 1.80-1.92 (m, 1H), 1.23-1.30 (m, 3H).
(7) To a solution of compound **44-8** (344 mg, 1.19 mmol) in toluene (3.0 mL), compound **C** (344 mg, 1.19 mmol), cesium carbonate (647 mg, 1.99 mmol), 1,1'-binaphthyl-2,2'-diphenylphosphine (123 mg, 198 µmol), and tris (dibenzylideneacetone) dipalladium (91.0 mg, 99.3 µmol) were added. The reaction mixture was stirred at 80°C under a nitrogen atmosphere for 5 hours. The reaction mixture was then diluted with ethyl acetate (20.0 mL), followed by washing with water (25.0 mL × 2) and saturated sodium chloride (50.0 mL × 2). After drying over anhydrous sodium sulfate, the mixture was filtered and concentrated under reduced pressure. The crude product was further purified by silica gel column chromatography (petroleum ether/ethyl acetate =1:0 to 0:1) to obtain compound **44-9.** MS-ESI [M+H]⁺, calculated: 454, found: 454. ¹H NMR (400 MHz, CDCl₃) *δ* 7.47 (d, *J* = 8.0 Hz, 2H), 7.36-7.42 (m, 2H), 7.28-7.34 (m, 1H), 7.19-7.26 (m, 1H), 5.51-5.61 (m, 2H), 4.12-4.21 (m, 2H), 3.65-3.93 (m, 2H), 3.30-3.39 (m, 3H), 2.38-2.50 (m, 1H), 2.31-2.37 (m, 1H), 2.12-2.30 (m, 2H), 2.05-2.11 (m, 1H), 1.94-2.03 (m, 1H), 1.62-1.76 (m, 1H), 1.58 (s, 1H), 1.24-1.29 (m, 3H).
(8) To a solution of compound **44-9** (145 mg, 319 µmol) in 3.0 mL of tetrahydrofuran, lithium hydroxide monohydrate (201 mg, 4.80 mmol) was added. The reaction mixture was stirred at 25°C under a nitrogen atmosphere for 24 hours. The pH of the reaction mixture was adjusted to greater than 7 by adding 1 M hydrochloric acid solution. The mixture was then extracted with ethyl acetate (10.0 mL), followed by washing with water (15.0 mL × 2). After drying over anhydrous sodium sulfate, the mixture was filtered and concentrated under reduced pressure to obtain compound **44-10.** MS-ESI [M+H]⁺, calculated: 426, found: 426. ¹H NMR (400 MHz, CDCl₃) *δ* 7.46 (d, *J* = 7.6 Hz, 2H), 7.27-7.41 (m, 4H), 5.48-5.61 (m, 2H), 4.18-4.32 (m, 1H), 4.10-4.16 (m, 1H), 3.77-3.90 (m, 1H), 3.63-3.73 (m, 1H), 3.35 (d, *J* = 11.6 Hz, 3H), 2.38-2.59 (m, 2H), 2.14-2.33 (m, 2H), 2.10-2.13 (m, 1H), 1.93-2.03 (m, 1H).
(9) To a solution of compound **44-10** (60.0 mg, 141 µmol) in 3.0 mL of dichloromethane, compound **B-4** trifluoroacetate (42.5 mg, 183 µmol), diisopropylethylamine (54.6 mg, 423 µmol), and propylphosphonic anhydride solution (269 mg, 423 µmol, 50% purity in ethyl acetate) were added. The reaction mixture was stirred at 25°C for 1 hour under a nitrogen atmosphere. It was then diluted with dichloromethane (10.0 mL), followed by washing with water (25.0 mL × 2) and saturated sodium chloride (25.0 mL × 2). After drying over anhydrous sodium sulfate, the mixture was filtered and concentrated under reduced pressure. The crude product was further purified by silica gel column chromatography (petroleum ether/ethyl acetate =1:0 to 5:1) to yield compound **44-11.** MS-ESI [M+H]⁺, calculated: 640, found: 640.
(10) To a solution of compound **44-11** (70.0 mg, 109 µmol) in 3.0 mL of tetrahydrofuran, 10% palladium on carbon (20.0 mg) was added. The reaction mixture was stirred at 25°C under a hydrogen atmosphere for 12 hours. After filtering, the reaction mixture was concentrated under reduced pressure. The crude product was further purified using a preparative high-performance liquid chromatography method (Phenomenex C18 column, 75 mm × 30 mm, 3 µm, A: water (10 mM ammonium bicarbonate) and B: acetonitrile (31% to 62.5%) over 11 minutes) to obtain compound **44.** MS-ESI [M+H]⁺, calculated: 550, found: 550. ¹H NMR (400 MHz, MeOD) *δ* 8.60 (s, 2H), 7.81 (s, 1H), 4.04-4.15 (m, 2H), 3.95-4.00 (m, 2H), 3.88-3.94 (m, 2H), 3.60-3.71 (m, 5H), 3.50 (dd, *J* = 11.2, 2.4 Hz, 1H), 3.33 (s, 3H), 2.48 (t, *J* = 7.2 Hz, 2H), 2.20-2.28 (m, 1H), 2.07-2.15 (m, 1H), 1.99-2.06 (m, 1H), 1.67-1.79 (m, 1H).

### Example 45 Synthesis of Compound 45

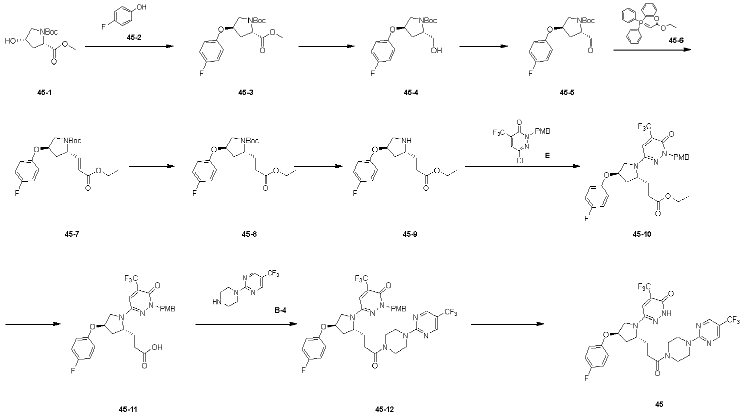

(1) At 0°C, compound **45-2** (2.29 g, 20.4 mmol), triphenylphosphine (5.88 g, 22.2 mmol), and diisopropyl azodicarboxylate (4.12 g, 20.4 mmol) were added to a solution of compound **45-1** (5.0 g, 20.4 mmol) in 75.0 mL of tetrahydrofuran. The reaction mixture was stirred at 25°C for 12 hours. After the reaction, 400 mL of ethyl acetate and 50.0 mL of water were added to the mixture. The organic layer was separated, and dried over anhydrous sodium sulfate. After filtering, the organic solution was concentrated under reduced pressure. The crude product was further purified by silica gel column chromatography ( petroleum ether/ethyl acetate = 1:0 to 5:1) to obtain compound **45-3.** MS-ESI [M-^{t}Bu+H]⁺, calculated: 284, found: 284.
(2) At 0°C, to a solution of compound **45-3** (3.6 g, 10.6 mmol) in 40.0 mL of tetrahydrofuran, lithium aluminum hydride (805 mg, 21.2 mmol) was added. The reaction mixture was stirred at 0°C for 1 hour under a nitrogen atmosphere. At 0°C, water (1.0 mL), 15% sodium hydroxide (1.0 mL) and water (3.0 mL) were added to the reaction mixture. The mixture was then dried over anhydrous sodium sulfate, filtered, and concentrated under reduced pressure to obtain compound **45-4.** MS-ESI [M-^{t}Bu+H]⁺, calculated: 256, found: 256.
(3) To a solution of compound **45-4** (1.5 g, 4.82 mmol) in 15.0 mL of dichloromethane, Dess-Martin reagent (2.45 g, 5.78 mmol) was added, and the reaction mixture was stirred at 25°C for 12 hours. After the reaction, 80.0 mL of sodium sulfite solution was added, and the mixture was extracted with 300 mL of dichloromethane. The organic phase was dried over anhydrous sodium sulfate, filtered, and concentrated under reduced pressure. The crude product was purified by silica gel column chromatography ( petroleum ether/ethyl acetate 1:0 to 4:1) to obtain compound **45-5.** MS-ESI [M-Boc+H]⁺, calculated: 210, found: 210.
(4) Compound **45-5** (1.05 g, 3.39 mmol) was dissolved in 12.0 mL of dichloromethane, and compound **45-6** (1.3 g, 3.73 mmol) was added to this solution. The reaction mixture was stirred at 25°C for 12 hours. After completion of the reaction, the mixture was dried over anhydrous sodium sulfate, filtered, and concentrated under reduced pressure. The crude product was further purified by silica gel column chromatography to obtain compound 45-7. MS-ESI [M-Boc+H]⁺, calculated: 280, found: 280.
(5) Compound **45-7** (1.08 g, 2.85 mmol) was dissolved in 10.0 mL of tetrahydrofuran, and 10% palladium on carbon (300 mg) was added to the solution. The reaction mixture was stirred at 25°C in a hydrogen atmosphere for 1 hour. Following the reaction, the mixture was filtered, and the filtrate was concentrated under reduced pressure to obtain compound **45-8.** MS-ESI [M-Boc+H]⁺, calculated: 282, found: 282. ¹H NMR (400 MHz, CDCl₃) *δ* 6.85-6.95 (m, 2H), 6.65-6.76 (m, 2H), 4.63-4.69 (m, 1H), 4.02-4.10 (m, 2H), 3.97 (br dd, *J* = 7.2, 5.2 Hz, 1H), 3.67-3.77 (m, 1H), 3.39 (dd, *J* = 12.4, 4.4 Hz, 1H), 2.20-2.29 (m, 3H), 2.00-2.11 (m, 1H), 1.70-1.86 (m, 2H), 1.39 (s, 9H), 1.16-1.20 (m, 3H).
(6) Compound **45-8** (1.04 g, 2.73 mmol) was dissolved in 9.0 mL of dichloromethane, and trifluoroacetic acid (4.62 g, 40.5 mmol) was added to the solution. The reaction mixture was stirred at 25°C for 0.5 hours. The organic phase was adjusted to a pH greater than 7 using sodium carbonate and then extracted with 200 mL of dichloromethane. The organic layer was separated, dried over anhydrous sodium sulfate, filtered, and concentrated under reduced pressure to obtain compound **45-9.** MS-ESI [M+H]⁺, calculated: 282, found: 282. ¹H NMR (400 MHz, CDCl₃) *δ* 6.96-7.02 (m, 2H), 6.79-6.84 (m, 2H), 4.95 (t, *J* = 4.8 Hz, 1H), 4.11 (q, *J* = 7.2 Hz, 2H), 3.75-3.84 (m, 1H), 3.68 (dd, *J* = 12.8, 5.2 Hz, 1H), 3.40 (d, *J* = 12.8 Hz, 1H), 2.45-2.53 (m, 2H), 2.38 (dd, *J* = 13.6, 5.6 Hz, 1H), 2.09 (br dd, *J* = 12.8, 6.8 Hz, 2H), 1.95 (ddd, *J* = 13.6, 11.6, 5.2 Hz, 1H), 1.23 (t, *J* = 7.2 Hz, 3H).
(7) Compound **45-9** (900 mg, 3.20 mmol) was dissolved in 15.0 mL of toluene, and compound **E** (1.22 g, 3.84 mmol), cesium carbonate (2.09 g, 6.40 mmol), 1,1'-binaphthyl-2,2'-diphenylphosphine (399 mg, 640 µmol), and tris (dibenzylideneacetone) dipalladium (293 mg, 320 µmol) were added to the solution. The reaction mixture was stirred at 80°C under a nitrogen atmosphere for 10 hours. After drying with anhydrous sodium sulfate, the mixture was filtered, concentrated under reduced pressure, and the crude product was purified by silica gel column chromatography (petroleum ether/ethyl acetate = 1:0 to 3:1) to obtain compound **45-10.** MS-ESI [M+H]⁺, calculated: 564, found: 564. ¹H NMR (400 MHz, CDCl₃) *δ* 7.43 (d, *J* = 8.4 Hz, 2H), 7.33 (s, 1H), 6.96-7.03 (m, 2H), 6.83-6.86 (m, 2H), 6.77-6.81 (m, 2H), 5.20-5.26 (m, 1H), 5.05-5.10 (m, 1H), 4.90 (br d, *J* = 2.4 Hz, 1H), 4.12 (qd, *J* = 7.2, 2.8 Hz, 3H), 3.79 (s, 3H), 3.74-3.78 (m, 1H), 3.61 (br d, *J* = 11.6 Hz, 1H), 2.37-2.47 (m, 1H), 2.26-2.35 (m, 2H), 2.11-2.20 (m, 1H), 2.01-2.10 (m, 1H), 1.72 (dq, *J* = 15.6, 7.2 Hz, 1H), 1.24 (t, *J* = 7.2 Hz, 3H).
(8) Compound **45-10** (950 mg, 1.69 mmol) was dissolved in 6.0 mL of tetrahydrofuran, and 2.0 mL of water and lithium hydroxide monohydrate (707 mg, 16.9 mmol) was added to the solution. The reaction mixture was stirred at 25°C under a nitrogen atmosphere for 12 hours. To the reaction mixture, hydrochloric acid solution (1 M) was added to adjust the pH to less than 7. The mixture was then extracted with 100 mL of dichloromethane. After drying over anhydrous sodium sulfate, the mixture was filtered and concentrated under reduced pressure to obtain compound **45-11.** MS-ESI [M+H]⁺, calculated: 536, found: 536. ¹H NMR (400 MHz, CDCl₃) *δ* 7.28-7.46 (m, 3H), 6.95-7.03 (m, 2H), 6.76-6.86 (m, 4H), 5.22-5.35 (m, 1H), 5.07 (d, *J* = 13.2 Hz, 1H), 4.91 (br d, *J* = 2.4 Hz, 1H), 4.09-4.19 (m, 1H), 3.70-3.85 (m, 4H), 3.61 (br d, *J* = 11.2 Hz, 1H), 2.30-2.47 (m, 3H), 2.12-2.20 (m, 1H), 2.06 (s, 2H).
(9) Compound **45-11** (910 mg, 1.70 mmol) was dissolved in 6.0 mL of dichloromethane, compound **B-4** trifluoroacetate (474 mg, 2.04 mmol), diisopropylethylamine (1.10 g, 8.50 mmol), and a solution of propylphosphonic anhydride in ethyl acetate (3.24 g, 5.10 mmol, 50% purity) were added. The reaction mixture was stirred at 25°C under a nitrogen atmosphere for 4 hours. Water (30.0 mL) was added to the reaction mixture, followed by extraction with 150 mL of dichloromethane. After drying over anhydrous sodium sulfate, the mixture was filtered, and concentrated under reduced pressure. The crude product was purified by silica gel column chromatography (petroleum ether/ethyl acetate = 1:0 to 1:1) to isolate compound **45-12.** MS-ESI [M+H]⁺, calculated: 750, found: 750. ¹H NMR (400 MHz, CDCl₃) *δ* 8.53 (s, 2H), 7.63 (s, 1H), 7.42 (d, *J* = 8.8 Hz, 2H), 6.97-7.02 (m, 2H), 6.77-6.85 (m, 4H), 5.05-5.24 (m, 2H), 4.93 (br d, *J* = 3.2 Hz, 1H), 4.14-4.22 (m, 1H), 3.88-3.97 (m, 4H), 3.78 (s, 3H), 3.63-3.74 (m, 3H), 3.46 (br s, 2H), 2.30-2.45 (m, 3H), 2.18-2.23 (m, 1H), 2.01-2.09 (m, 1H), 1.56-1.68 (m, 2H).
(10) Compound **45-12** (350 mg, 467 µmol) was dissolved in 3.0 mL of trifluoroacetic acid, and the reaction mixture was stirred at 70°C for 2 hours. After filtration and concentration under reduced pressure, the pH was adjusted to greater than 7 with sodium carbonate. The mixture was then extracted with 50.0 mL of dichloromethane. The organic phase was dried over anhydrous sodium sulfate, filtered, and concentrated under reduced pressure. The crude product was purified using silica gel column chromatography (petroleum ether/ethyl acetate = 1:0 to 1:1) to obtain compound **45.** MS-ESI [M+H]⁺, calculated: 630, found: 630. ¹H NMR (400 MHz, MeOD) *δ* 8.60 (s, 2H), 7.83 (s, 1H), 6.97-7.03 (m, 2H), 6.88-6.94 (m, 2H), 5.02-5.07 (m, 1H), 4.14-4.23 (m, 1H), 3.93 (dt, *J* = 18.4, 5.2 Hz, 4H), 3.85 (dd, *J* = 11.6, 4.8 Hz, 1H), 3.60-3.69 (m, 5H), 2.50 (t, *J* = 7.2 Hz, 2H), 2.39-2.46 (m, 1H), 2.15-2.23 (m, 2H), 1.74-1.85 (m, 1H).

### Example 46 Synthesis of Compound 46

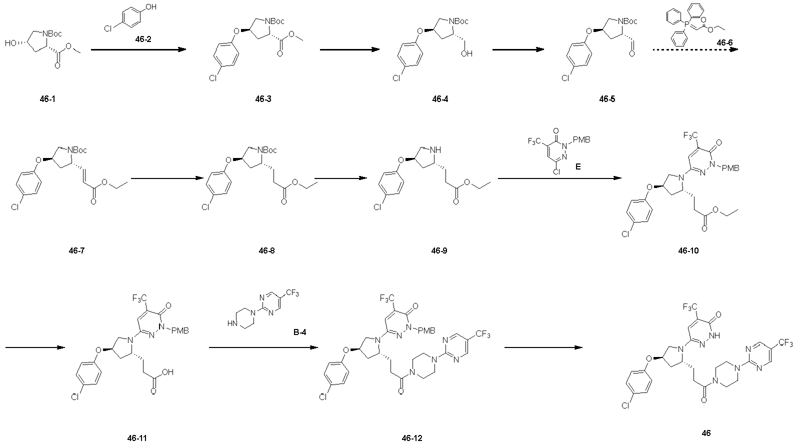

(1) At 0°C, compound **46-1** (5.0 g, 20.4 mmol) was dissolved in 75.0 mL of tetrahydrofuran. To this solution, compound **46-2** (2.62 g, 20.4 mmol), triphenylphosphine (5.88 g, 22.4 mmol), and diisopropyl azodicarboxylate (4.12 g, 20.4 mmol) were added. The reaction mixture was stirred at 25°C for 12 hours. Then, 300 mL of ethyl acetate and 50.0 mL of water were added. After phase separation, the organic phase was dried with anhydrous sodium sulfate, filtered and concentrated under reduced pressure. The crude product was further purified using silica gel column chromatography (petroleum ether/ethyl acetate = 1:0 to 5:1) to isolate compound **46-3.** MS-ESI [M-^{t}Bu+H]⁺, calculated: 256, found: 256.
(2) At 0°C, compound **46-3** (6.5 g, 18.3 mmol) was dissolved in 70.0 mL of tetrahydrofuran. To this solution, lithium aluminum hydride (1.39 g, 36.5 mmol) was added, and the reaction mixture was stirred at 0°C for 1 hour. At 0°C, water (2.0 mL), 15% sodium hydroxide (2.0 mL), and water (6.0 mL) were added to the reaction mixture. The mixture was then extracted with ethyl acetate (100 mL). The organic layer was separated, dried over anhydrous sodium sulfate, filtered, and concentrated under reduced pressure to obtain compound **46-4.** MS-ESI [M-^{t}Bu+H]⁺, calculated: 272, found: 272.
(3) Compound **46-4** (3.0 g, 9.15 mmol) was dissolved in 30.0 mL of dichloromethane, and to this solution, Dess-Martin reagent (4.66 g, 11.0 mmol) was added. The reaction mixture was stirred at 25°C for 12 hours. To the reaction mixture, 80.0 mL of sodium sulfite solution was added, and the mixture was then extracted with 500 mL of dichloromethane. The organic layer was separated, dried over anhydrous sodium sulfate, filtered, and concentrated under reduced pressure. The crude product was purified by silica gel column chromatography (petroleum ether/ethyl acetate = 1:0 to 4:1) to obtain compound **45-5.** MS-ESI [M-Boc+H]⁺, calculated: 226, found: 226.
(4) Compound **46-5** (1.71 g, 5.25 mmol) was dissolved in 20.0 mL of dichloromethane, and to this solution, compound **46-6** (2.01 g, 5.77 mmol) was added. The reaction mixture was stirred at 25°C for 12 hours. The mixture was then filtered, and the solvent was removed under reduced pressure. The crude product was purified by silica gel column chromatography (petroleum ether/ethyl acetate = 1:0 to 4:1) to obtain compound **46-7.** MS-ESI [M-Boc+H]⁺, calculated: 296, found: 296. ¹H NMR (400 MHz, CDCl₃) *δ* 7.22-7.27 (m, 2H), 6.76-6.90 (m, 3H), 5.91 (br d, *J* = 15.6 Hz, 1H), 4.76-4.83 (m, 1H), 4.44-4.73 (m, 1H), 4.21 (q, *J* = 6.8 Hz, 2H), 3.68-3.89 (m, 1H), 3.63 (dd, *J* = 12.4, 4.4 Hz, 1H), 2.37-2.47 (m, 1H), 2.03 (ddd, *J* = 13.2, 8.0, 4.8 Hz, 1H), 1.44 (s, 9H), 1.26-1.33 (m, 3H).
(5) Compound **46-7** (1.0 g, 2.53 mmol) was dissolved in 10.0 mL of methanol, and to this solution, tris(triphenylphosphine)rhodium chloride (70.1 mg, 75.8 µmol) was added. The reaction mixture was stirred at 65°C under a hydrogen atmosphere for 4 hours. The mixture was then filtered, and the solvent was removed under reduced pressure to obtain compound **46-8.** MS-ESI [M-Boc+H]⁺, calculated: 298, found: 298. ¹H NMR (400 MHz, CDCl₃) *δ* 7.21-7.26 (m, 2H), 6.75-6.81 (m, 2H), 4.74-4.81 (m, 1H), 4.14 (q, *J* = 7.2 Hz, 2H), 3.98-4.08 (m, 1H), 3.79 (br s, 1H), 3.49 (dd, *J* = 12.4, 4.4 Hz, 1H), 2.28-2.35 (m, 3H), 2.14 (br d, *J* = 8.0 Hz, 1H), 1.77-1.95 (m, 2H), 1.46 (s, 9H), 1.24-1.28 (m, 3H).
(6) Compound **46-8** (1.25 g, 3.14 mmol) was dissolved in 6.0 mL of dichloromethane, and trifluoroacetic acid (3.08 g, 27.0 mmol) was added to the solution. The reaction mixture was stirred at 25°C for 0.5 hours. After the reaction, the mixture was diluted with 50.0 mL of dichloromethane, and the pH was adjusted to greater than 7 using sodium carbonate. The mixture was extracted with 100.0 mL of dichloromethane. The organic phase was dried over anhydrous sodium sulfate, followed by filtration and concentration under reduced pressure to obtain compound **46-9.** MS-ESI [M+H]⁺, calculated: 300, found: 300. ¹H NMR (400 MHz, CDCl₃) *δ* 7.18-7.26 (m, 2H), 6.75-6.81 (m, 2H), 4.81 (br t, *J* = 5.2 Hz, 1H), 4.14 (q, *J* = 7.2 Hz, 2H), 3.27-3.46 (m, 2H), 3.12 (dt, *J* = 12.4, 1.2 Hz, 1H), 2.39-2.47(m, 2H), 2.17 (dd, *J* = 13.6, 6.4 Hz, 1H), 1.77-1.86 (m, 2H), 1.65 (ddd, *J* = 13.6, 9.2, 6.4 Hz, 1H), 1.26 (t, *J* = 7.2 Hz, 3H).
(7) To the solution of compound **46-9** (720 mg, 2.42 mmol) in 15.0 mL of toluene, compound **E** (1.0 g, 3.14 mmol), cesium carbonate (1.58 g, 4.84 mmol), 1,1'-binaphthyl-2,2'-diphenylphosphine (301 mg, 484 µmol), and tris (dibenzylideneacetone) dipalladium (221 mg, 242 µmol) were added. The reaction mixture was stirred at 80°C for 10 hours under a nitrogen atmosphere. After the reaction, 20.0 mL of water was added, followed by extraction with 200 mL of dichloromethane. The organic phase was separated, dried over anhydrous sodium sulfate, filtered, and then concentrated under reduced pressure. The crude product was purified by silica gel column chromatography ( petroleum ether/ethyl acetate = 1:0 to 3:1) to obtain compound **46-10.**
(8) Compound **46-10** (860 mg, 1.48 mmol) was dissolved in 6.0 mL of tetrahydrofuran, water (2.0 mL) and lithium hydroxide monohydrate (622 mg, 14.8 mmol) was added to the reaction mixture. The reaction was stirred under a nitrogen atmosphere at 25°C for 12 hours. After the reaction, the pH of the mixture was adjusted to below 7 by adding 1 M hydrochloric acid aqueous solution. The mixture was then extracted with 100 mL of ethyl acetate. The organic phase was separated, dried over anhydrous sodium sulfate, filtered, and concentrated under reduced pressure to obtain compound **46-11.** MS-ESI [M+H]⁺, calculated: 552, found: 552. ¹H NMR (400 MHz, CDCl₃) *δ* 7.42 (d, *J* = 8.4 Hz, 2H), 7.35 (s, 1H), 7.22-7.26 (m, 2H), 6.76-6.86 (m, 4H), 5.24 (d, *J* = 13.2 Hz, 1H), 5.07 (d, *J* = 13.6 Hz, 1H), 4.91-4.96 (m, 1H), 3.77-3.80 (m, 4H), 3.61 (br d, *J* = 11.2 Hz, 1H), 2.33-2.45 (m, 3H), 2.06 (s, 3H), 1.71-1.75 (m, 1H).
(9) To the solution of compound **46-11** (825 mg, 1.49 mmol) in 6.0 mL of dichloromethane, compound **B-4** trifluoroacetate (416 mg, 1.79 mmol), diisopropylethylamine (965 mg, 7.47 mmol), and a solution of propylphosphonic anhydride in ethyl acetate (2.85 g, 4.48 mmol, 50% purity) were added. The reaction mixture was stirred at 25°C under a nitrogen atmosphere for 4 hours. Water (20.0 mL) was added to the reaction mixture, and then it was extracted with 200 mL of dichloromethane. The organic phase was separated, dried over anhydrous sodium sulfate, filtered, and concentrated under reduced pressure. The crude product was purified by silica gel column chromatography (petroleum ether/ethyl acetate = 1:0 to 1:1) to obtain compound 46-12. MS-ESI [M+H]⁺, calculated: 766, found: 766.
(10) Compound **46-12** (600 mg, 783 µmol) was dissolved in 3.0 mL of trifluoroacetic acid, and the reaction mixture was stirred at 70°C for 3 hours. After the reaction, it was filtered, concentrated under reduced pressure, and the pH was adjusted to greater than 7 with sodium carbonate. Then, the mixture was extracted with 50.0 mL of dichloromethane. The organic phase was separated, dried over anhydrous sodium sulfate, filtered, and concentrated under reduced pressure. The crude product was purified by silica gel column chromatography (petroleum ether/ethyl acetate = 1:0 to 1:1) to obtain compound **46.** MS-ESI [M+H]⁺, calculated: 646, found: 646. ¹H NMR (400 MHz, MeOD) *δ* 8.60 (s, 2H), 7.83 (s, 1H), 7.22-7.28 (m, 2H), 6.87-6.94 (m, 2H), 5.08 (br s, 1H), 4.13-4.23 (m, 1H), 3.84-3.96 (m, 5H), 3.61-3.69 (m, 5H), 2.50 (t, *J* = 7.2 Hz, 2H), 2.39-2.47 (m, 1H), 2.12-2.25 (m, 2H), 1.72-1.85 (m, 1H).

### Example 47 Synthesis of Compound 47

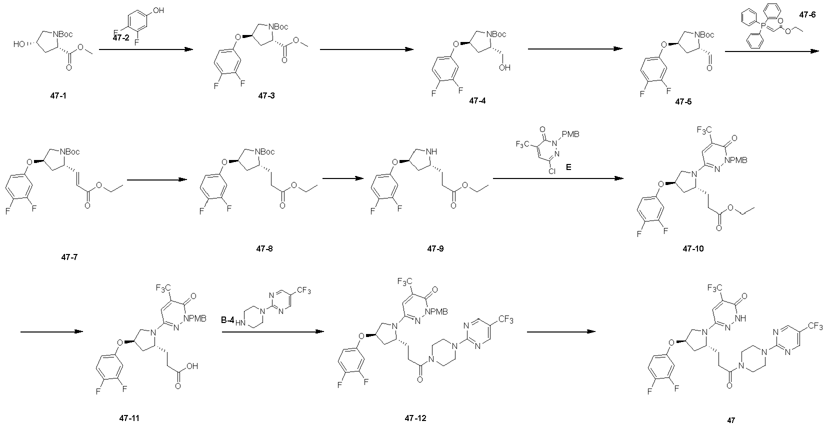

(1) To a solution of compound **47-1** (5.66 g, 23.0 mmol) in tetrahydrofuran (30.0 mL), compound **47-2** (3.00 g, 23.0 mmol), triphenylphosphine (18.1 g, 69.1 mmol), and diisopropyl azodicarboxylate (4.66 g, 23.0 mmol, 4.48 mL) were added. The reaction mixture was stirred at 25°C for 12 hours. Subsequently, ethyl acetate (400 mL) and water (50.0 mL) were added. After phase separation, the organic phase was dried over anhydrous sodium sulfate, filtrated, and concentrated under reduced pressure. The crude product was then purified by silica gel column chromatography (petroleum ether/ethyl acetate = 1:0 to 10:1) to yield compound **47-3.** MS-ESI [M-Boc+H]⁺, calculated: 258, found: 258.
(2) To a solution of compound **47-3** (5.70 g, 15.9 mmol) in tetrahydrofuran (20.0 mL), lithium aluminum hydride (1.21 g, 31.9 mmol) was added. The reaction mixture was stirred at 0°C for 1 hour under a nitrogen atmosphere. At 0°C, water (1.0 mL), 15% sodium hydroxide solution (1.0 mL), and additional water (3.0 mL) were added. The mixture was then dried over anhydrous sodium sulfate, followed by filtration and concentration under reduced pressure to afford compound **47-4.** MS-ESI [M-^{t}Bu+H]⁺, calculated: 274, found: 274.
(3) To a solution of compound **47-4** (500 mg, 1.52 mmol) in dichloromethane (4.0 mL), Dess-Martin reagent (643 mg, 1.52 mmol) was added, and the reaction mixture was stirred at 25°C for 12 hours. Sodium sulfite solution (80.0 mL) was added to the reaction mixture. The solution was extracted with dichloromethane (30.0 mL). The organic phase was dried over anhydrous sodium sulfate, filtered, and concentrated under reduced pressure. The crude product was purified by silica gel column chromatography to obtain compound **47-5.** MS-ESI [M-Boc+H]⁺, calculated: 228, found: 228.
(4) To a solution of compound **47-5** (480 mg, 1.47 mmol) in dichloromethane (5.0 mL), compound **47-6** (613 mg, 1.76 mmol) was added, and the reaction mixture was stirred at 25°C for 12 hours. The reaction mixture was then dried over anhydrous sodium sulfate, filtered, and concentrated under reduced pressure. The crude product was purified by silica gel column chromatography to obtain compound **47-7.** MS-ESI [M-Boc+H]⁺, calculated: 298, found: 298.
(5) To a solution of compound **47-7** (300 mg, 754 µmol) in tetrahydrofuran (5.0 mL), 10% palladium on carbon (300 mg) was added, and the reaction mixture was stirred under a hydrogen atmosphere at 25°C for 1 hour. The reaction mixture was then filtered, and the reaction solvent was removed under reduced pressure to isolate compound **47-8.** MS-ESI [M-Boc+H]⁺, calculated: 300, found: 300.
(6) To a solution of compound **47-8** (380 mg, 951 µmol) in dichloromethane (6.0 mL), trifluoroacetic acid (3.08 g, 27.0 mmol, 2.00 mL) was added, and the reaction mixture was stirred at 25°C for 0.5 hours. The mixture was then adjusted to a pH greater than 7 with sodium carbonate, and extracted with dichloromethane (200 mL). The organic layer was separated, and dried over anhydrous sodium sulfate. After filtration, the solvent was removed under reduced pressure to obtain compound **47-9.**
(7) To a solution of compound **47-9** (165 mg, 551 µmol) in toluene (4.0 mL), compound E (210 mg, 661 µmol), cesium carbonate (359 mg, 1.10 mmol), 1,1'-binaphthyl-2,2'-diphenylphosphine (68.6 mg, 110 µmol), and tris (dibenzylideneacetone) dipalladium (50.4 mg, 55.1 µmol) were added. The reaction mixture was stirred at 80°C under a nitrogen atmosphere for 10 hours. Afterward, the mixture was dried over anhydrous sodium sulfate, filtered, and the solvent was removed under reduced pressure. The crude product was purified by silica gel column chromatography (petroleum ether/ethyl acetate = 1:0 to 3:1) to obtain compound **47-10.** MS-ESI [M+H]⁺, calculated: 582, found: 582.
(8) To a solution of compound **47-10** (390 mg, 670 µmol) in tetrahydrofuran (6.0 mL), water (2.0 mL) and lithium hydroxide monohydrate (281 mg, 6.71 mmol) were added. The reaction mixture was stirred at 25°C for 12 hours under a nitrogen atmosphere. Then, the pH of the reaction mixture was adjusted to less than 7 by adding 1 M hydrochloric acid solution. The mixture was extracted with dichloromethane (100 mL). The organic phase was dried over anhydrous sodium sulfate, filtered, and concentrated under reduced pressure to afford compound **47-11.**
(9) To a solution of compound **47-11** (226 mg, 408 µmol) in dichloromethane (6.0 mL), compound **B-4** trifluoroacetate (113 mg, 489 µmol), diisopropylethylamine (158 mg, 1.22 mmol), and propylphosphonic anhydride solution (779 mg, 1.22 mmol, 728 µL, 50% purity in ethyl acetate) were added. The reaction mixture was stirred at 25°C for 4 hours under a nitrogen atmosphere. Water (30.0 mL) was added to the reaction mixture, followed by extraction with dichloromethane (150 mL). After drying over anhydrous sodium sulfate, the organic phase was filtered and concentrated under reduced pressure. The crude product was purified by silica gel column chromatography (petroleum ether/ethyl acetate = 1:0 to 1:1) to obtain compound **47-12.** MS-ESI [M+H]⁺, calculated: 768, found: 768. ¹H NMR (400 MHz, DMSO-d6) *δ* 8.74 (s, 2H), 7.92 (s, 1H), 7.31-7.39 (m, 1H), 7.27 (d, *J* = 8.8 Hz, 2H), 7.12 (ddd, *J* = 12.4, 6.8, 3.2 Hz, 1H), 6.85 (d, *J* = 8.4 Hz, 2H), 6.75-6.81 (m, 1H), 5.07-5.16 (m, 2H), 4.96 (d, *J* = 13.6 Hz, 1H), 4.07 (br dd, *J* = 7.2, 4.4 Hz, 1H), 3.77-3.87 (m, 5H), 3.70 (s, 3H), 3.50-3.59 (m, 5H), 2.41 (br t, *J* = 6.8 Hz, 2H), 2.15-2.28 (m, 2H), 1.97-2.01 (m, 1H), 1.54-1.66 (m, 1H).
(10) Compound **47-12** (150 mg, 195 µmol) was dissolved in trifluoroacetic acid (10.0 mL) and the reaction mixture was stirred at 70°C for 2 hours. After the reaction, it was filtered, and the solvent was removed under reduced pressure. The pH was adjusted to above 7 using sodium carbonate, and then the mixture was extracted with dichloromethane (50.0 mL). After drying over anhydrous sodium sulfate, the organic phase was filtered, and the solvent was removed under reduced pressure. The crude product was further purified by preparative high-performance liquid chromatography (C18-6 column, 100 mm × 30 mm, 5 µm, A: water (0.225% formic acid) and B: acetonitrile (52% to 82%) over 15 minutes) to obtain compound **47** formate. MS-ESI [M+H]⁺, calculated: 648, found: 648. ¹H NMR (400 MHz, MeOD) *δ* 8.59 (s, 2H), 7.83 (s, 1H), 7.11-7.21 (m, 1H), 6.89 (ddd, *J* = 12.3, 6.4, 3.0 Hz, 1H), 6.68-6.73 (m, 1H), 5.03-5.08 (m, 1H), 4.15-4.22 (m, 1H), 3.94-3.98 (m, 2H), 3.91 (t, *J* = 5.6 Hz, 2H), 3.86 (dd, *J* = 11.6, 4.6 Hz, 1H), 3.62-3.69 (m, 5H), 2.50 (t, *J* = 7.2 Hz, 2H), 2.40-2.46 (m, 1H), 2.14-2.24 (m, 2H), 1.74-1.84 (m, 1H).

### Example 48 Synthesis of Compound 48

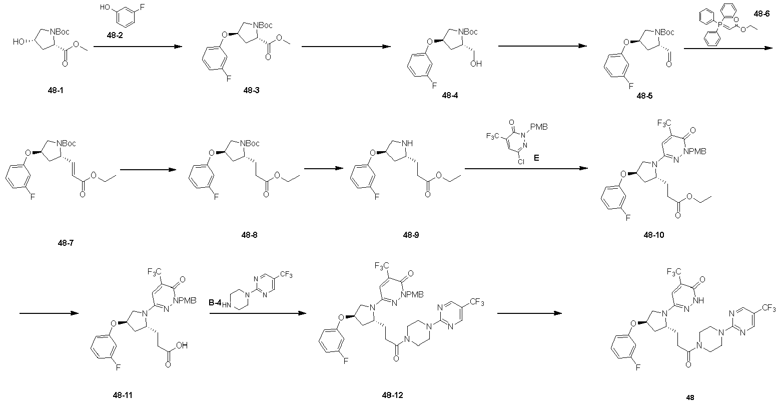

(1) At 0°C, to a solution of compound **48-1** (2.19 g, 8.92 mmol) in tetrahydrofuran (10.0 mL), compound **48-2** (1.0 g, 8.92 mmol), triphenylphosphine (2.57 g, 9.81 mmol), diisopropyl azodicarboxylate (1.8 g, 8.92 mmol) was added. The reaction solution was stirred at 25 °C for 12 hours, ethyl acetate (100 mL) and water (100 mL) was added. After phase separation, the organic phase was washed with saturated sodium chloride solution (100 mL), dried over anhydrous sodium sulfate, filtrated, concentrated under reduced pressure. The crude product was purified by silica gel column chromatography (petroleum ether/ethyl acetate = 1:0 to 10:1) to obtain compound **48-3.** MS-ESI [M-^{t}Bu+H]⁺, calculated: 284, found: 284.¹H NMR (400 MHz, CDCl₃) *δ* 7.20-7.26 (m, 1H), 6.52-6.74 (m, 3H), 4.89 (br s, 1H), 4.36-4.57 (m, 1H), 3.61-3.90 (m, 5H), 2.45-2.61 (m, 1H), 2.16-2.31 (m, 1H), 1.42-1.48 (m, 9H).
(2) At 0°C, to a solution of compound **48-3** (1.8 g, 5.3 mmol) in tetrahydrofuran (20.0 mL), lithium aluminum hydride (402 mg, 10.6 mmol) was added. The reaction solution was stirred under nitrogen atmosphere at 0°C for 1 hour. Then, water (0.4 mL) , 15% sodium hydroxide (0.4 mL) and water (1.2 mL) was added at 0°C. The reaction solution was dried over anhydrous sodium sulfate, filtered, concentrated under reduced pressure and the crude product was purified by silica gel column chromatography (petroleum ether/ethyl acetate = 1:0 to 0:1) to obtain compound **48-4.** MS-ESI [M-^{t}Bu+H]⁺, calculated: 256, found: 256.¹H NMR (400 MHz, CDCl₃) *δ* 7.19-7.26 (m, 1H), 6.62-6.72 (m, 2H), 6.58 (dt, *J* = 10.8, 2.4 Hz, 1H), 4.78 (br s, 1H), 4.04-4.25 (m, 1H), 3.44-3.87 (m, 4H), 2.23-2.37 (m, 1H), 2.02-2.12 (m, 1H), 1.77-1.89 (m, 1H), 1.46 (s, 9H).
(3) To a solution of compound **48-4** (200 mg, 642 µmol) in dichloromethane (5.0 mL), Dess-Martin reagent (272 mg, 642 µmol) was added. The reaction solution was stirred at 25 °C for 12 hours. Sodium thiosulfate solution (10.0 mL) was added, the reaction solution was diluted with dichloromethane (20.0 mL). The organic layer was extracted with water (10.0 mL × 3), washed with saturated sodium chloride solution (10.0 mL × 3) and dried over anhydrous sodium sulfate. The solution was filtered and concentrated under reduced pressure. The crude product was purified by silica gel column chromatography to obtain compound **48-5.** MS-ESI [M-Boc+H]⁺, calculated: 210, found: 210.¹H NMR (400 MHz, CDCl₃) *δ* 9.61 (d, *J* = 2.4 Hz, 0.2H), 9.49 (d, *J* = 3.6 Hz, 0.4H), 7.18-7.26 (m, 1H), 6.54-6.75 (m, 3H), 4.88 (br s, 1H), 4.25-4.50 (m, 1H), 3.82-3.95 (m, 1H), 3.66-3.80 (m, 1H), 2.32-2.50 (m, 1H), 2.04-2.19 (m, 1H), 1.40-1.52 (m, 9H).
(4) To a solution of compound **48-5** (117 mg, 378 µmol) in dichloromethane (3.0 mL), compound **48-6** (158 mg, 453 µmol) was added. The reaction solution was stirred at 25 °C for 12 hours. The reaction solution was dried over anhydrous sodium sulfate, filtered and concentrated under reduced pressure. The crude product was purified by silica gel column chromatography to obtain compound **48-7.** MS-ESI [M-Boc+H]⁺, calculated: 280, found: 280.
(5) To a solution of compound **48-7** (1.7 g, 4.47 mmol) in tetrahydrofuran (20 mL), 10% palladium on carbon (500 mg) was added. The reaction solution was stirred under a hydrogen atmosphere at 25°C for 2 hours. The reaction solution was filtered and concentrated under reduced pressure to obtain compound **48-8.** MS-ESI [M-Boc+H]⁺, calculated: 282, found: 282.¹H NMR (400 MHz, CDCl₃) *δ* 7.19-7.26 (m, 1H), 6.52-6.72 (m, 3H), 4.73-4.83 (m, 1H), 3.96-4.23 (m, 3H), 3.82 (br d, *J* = 9.2 Hz, 1H), 3.51 (dd, *J* = 12.4, 4.4 Hz, 1H), 2.25-2.41 (m, 3H), 2.05 (s, 1H), 1.75-1.98 (m, 2H), 1.46 (s, 9H), 1.22-1.29 (m, 3H).
(6) To a solution of compound **48-8** (1.1 g, 2.88 mmol) in dichloromethane (9.0 mL), trifluoroacetic acid (4.34 g, 38.1 mmol) was added. The reaction solution was stirred at 25°C for 1 hour. The mixture was diluted with water (10.0 mL), neutralized with sodium bicarbonate solution (30.0 mL), and extracted with dichloromethane (10.0 mL). The organic layer was dried over anhydrous sodium sulfate, filtered and concentrated under reduced pressure to obtain compound **48-9.** MS-ESI [M+H]⁺, calculated: 282, found: 282.¹H NMR (400 MHz, CDCl₃) *δ* 7.18-7.26 (m, 1H), 6.52-6.72 (m, 3H), 4.75-4.91 (m, 1H), 4.14 (q, *J* = 7.2 Hz, 2H), 3.31-3.48 (m, 2H), 3.07-3.24 (m, 1H), 2.33-2.53 (m, 3H), 2.20 (br dd, *J* = 13.6, 6.4 Hz, 1H), 1.84 (q, *J* = 7.2 Hz, 2H), 1.61-1.73 (m, 1H), 1.19-1.29 (m, 3H).
(7) To a solution of compound **48-9** (700 mg, 2.49 mmol) in toluene (10.0 mL), compound **E** (951 mg, 2.99 mmol), cesium carbonate (1.62 g, 4.98 mmol), 1,1'-binaphthyl-2,2'-diphenylphosphine (309 mg, 497 µmol) and tris (dibenzylideneacetone) dipalladium (227 mg, 248 µmol) was added. The reaction solution was stirred at 80°C under nitrogen atmosphere for 3 hours. The reaction solution was added with water (10.0 mL), extracted with ethyl acetate (10.0 mL) and the organic layer was combined, dried over anhydrous sodium sulfate, filtered and concentrated under reduced pressure. The crude product was purified by silica gel chromatography (petroleum ether/ethyl acetate = 1:0 to 4:1) to obtain compound **48-10.** MS-ESI [M+H]⁺, calculated: 564, found: 564.
(8) To a solution of compound **48-10** (1.06 g, 1.87 mmol) in tetrahydrofuran (9.0 mL), water (3.0 mL) and lithium hydroxide monohydrate (448 mg, 18.7 mmol) was added. The reaction solution was stirred under nitrogen atmosphere at 25°C for 3 hours. Then the reaction solution was diluted with water (10.0 mL) and the pH was adjusted to 5-6 with 1 mol/L aqueous hydrochloric acid solution. The mixture was extracted with ethyl acetate (15.0 mL × 2), dried over anhydrous sodium sulfate, filtered and concentrated under reduced pressure to obtain compound **48-11.** MS-ESI [M+H]⁺, calculated: 536, found: 536.
(9) To a solution of compound **48-11** (700 mg, 1.31 mmol) in dichloromethane (10.0 mL), compound **B-4** trifluoroacetate (364 mg, 1.57 mmol), diisopropylethylamine (844 mg, 6.54 mmol), propylphosphonic anhydride solution (2.5 g, 3.92 mmol, 50% purity in ethyl acetate) was added. The reaction mixture was stirred under nitrogen atmosphere at 25 °C for 1 hour. Then the reaction solution was diluted with water (10.0 mL), extracted with dichloromethane (10.0 mL) and the organic layer was dried over anhydrous sodium sulfate, filtered and concentrated under reduced pressure. The crude product was purified by silica gel column chromatography (petroleum ether/ethyl acetate = 1:0 to 4:1) to obtain compound **48-12.** MS-ESI [M+H]⁺, calculated: 750, found: 750.¹H NMR (400 MHz, CDCl₃) *δ* 8.53 (s, 2H), 7.63 (s, 1H), 7.42 (d, *J* = 8.8 Hz, 2H), 7.27 (s, 2H), 6.80-6.87 (m, 2H), 6.55-6.74 (m, 3H), 5.16-5.23 (m, 1H), 5.06-5.13 (m, 1H), 4.98 (br s, 1H), 4.09-4.23 (m, 1H), 3.79-3.99 (m, 3H), 3.78 (s, 3H), 3.63-3.76 (m, 3H), 3.38-3.51 (m, 2H), 2.28-2.46 (m, 3H), 2.10-2.25 (m, 2H), 1.67-1.79 (m, 2H).
(10) Compound **48-12** (416 mg, 555 µmol) was dissolved in trifluoroacetic acid (5.0 mL) and the reaction solution was stirred at 70°C for 3 hours. The reaction mixture was filtered and the organic layer was concentrated under reduced pressure. The crude product was purified by preparative high performance liquid chromatography (C18, 100 mm × 30 mm, 5 µm; A: water (0.225% formic acid); B: acetonitrile, 52%-82% in 15 minutes) to obtain compound **48** formate. MS-ESI [M+H]⁺, calculated: 630, found: 630.¹H NMR (400 MHz, MeOD) *δ* 8.58 (s, 2H), 7.83 (s, 1H), 7.20-7.30 (m, 1H), 6.63-6.76 (m, 3H), 5.07-5.12 (m, 1H), 4.12-4.25 (m, 1H), 3.82-4.00 (m, 5H), 3.59-3.72 (m, 5H), 2.39-2.54 (m, 3H), 2.12-2.28 (m, 2H), 1.72-1.84 (m, 1H).

### Example 49 Synthesis of Compound 49

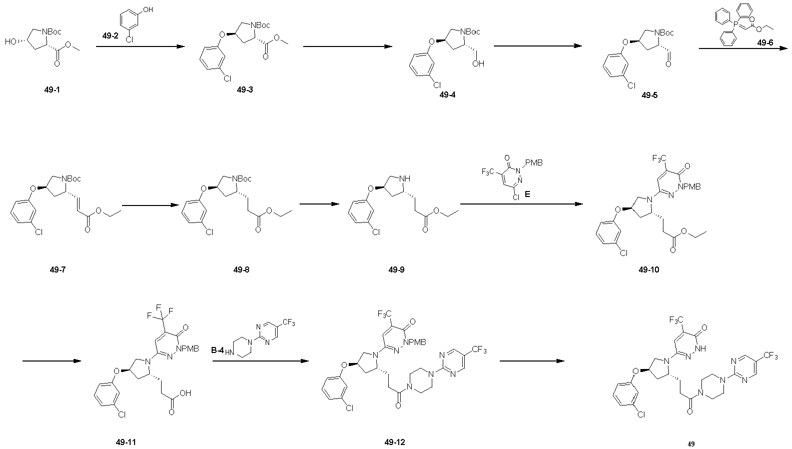

(1) At 0°C, to a solution of compound **49-1** (5.72 g, 23.3 mmol) in tetrahydrofuran (10.0 mL), compound **49-2** (3.0 g, 23.3 mmol), triphenylphosphine (6.73 g, 25.6 mmol), diisopropyl azodicarboxylate (4.72 g, 23.3 mmol) was added. The reaction solution was stirred at 25 °C for 12 hours, ethyl acetate (20.0 mL) and water (20.0 mL) was added. After phase separation, the organic phase was dried over anhydrous sodium sulfate, filtrated and concentrated under reduced pressure. The crude product was purified by silica gel column chromatography (petroleum ether/ethyl acetate = 1:0 to 5:1) to obtain compound **49-3.** MS-ESI [M-Boc+H]⁺, calculated: 256, found: 256.¹H NMR (400 MHz, CDCl₃) *δ* 7.17-7.25 (m, 1H), 6.94-7.00 (m, 1H), 6.87 (s, 1H), 6.75 (br d, *J* = 8.8 Hz, 1H), 4.89 (br d, *J* = 2.4 Hz, 1H), 4.37-4.55 (m, 1H), 3.56-3.84 (m, 5H), 2.46-2.63 (m, 1H), 2.24 (ddd, *J* = 13.6, 8.4, 4.8 Hz, 1H), 1.38-1.49 (m, 9H).
(2) At 0°C, to a solution of compound **49-3** (1.71 g, 4.81 mmol) in tetrahydrofuran (20.0 mL), lithium aluminum hydride (365 mg, 9.61 mmol) was added. The reaction solution was stirred at 0 °C under nitrogen atmosphere for 1 hour. Water (0.4 mL), 15% sodium hydroxide (0.4 mL), and water (1.2 mL) were added at 0 °C. The mixture was dried over anhydrous sodium sulfate, filtrated and concentrated under reduced pressure. The crude product was purified by silica gel column chromatography (petroleum ether/ethyl acetate = 1:0 to 5:1) to obtain compound **49-4.** MS-ESI [M-^{t}Bu+H]⁺, calculated: 272, found: 272.¹H NMR (400 MHz, CDCl₃) *δ* 7.22 (t, *J* = 8.0 Hz, 1H), 6.97 (dd, *J* = 8.0, 1.2 Hz, 1H), 6.87 (t, *J* = 2.4 Hz, 1H), 6.76 (ddd, *J* = 8.4, 2.4, 0.8 Hz, 1H), 4.79 (br s, 1H), 4.02-4.24 (m, 2H), 3.79 (br d, *J* = 12.0 Hz, 2H), 3.52-3.65 (m, 2H), 2.00-2.09 (m, 2H), 1.48 (s, 9H).
(3) To a solution of compound **49-4** (300 mg, 915 µmol) in dichloromethane (5.0 mL), Dess-Martin reagent (465 mg, 1.1 mmol) was added. The reaction solution was stirred at 25 °C for 12 hours. Then sodium thiosulfate (10.0 mL) was added, the reaction solution was diluted with dichloromethane (20.0 mL). The reaction solution was extracted with water (10.0 mL × 3), washed with saturated sodium chloride solution (10.0 mL × 3), dried over anhydrous sodium sulfate, filtered and concentrated under reduced pressure. The crude product was purified by silica gel column chromatography (petroleum ether/ethyl acetate = 1:0 to 5:1) to obtain compound **49-5.** MS-ESI [M-Boc+H]⁺, calculated: 226, found: 226.¹H NMR (400 MHz, CDCl₃) *δ* 9.61 (d, *J* = 2.4 Hz, 0.2H), 9.49 (d, *J* = 3.6 Hz, 0.4H), 7.18-7.26 (m, 1H), 6.54-6.75 (m, 3H), 4.88 (br s, 1H), 4.25-4.50 (m, 1H), 3.82-3.95 (m, 1H), 3.66-3.80 (m, 1H), 2.32-2.50 (m, 1H), 2.04-2.19 (m, 1H), 1.40-1.52 (m, 9H).
(4) To a solution of compound **49-5** (2.0 g, 6.14 mmol) in dichloromethane (20.0 mL), compound **49-6** (2.57 g, 7.37 mmol) was added. The reaction solution was stirred at 25 °C for 12 hours, anhydrous sodium sulfate was added, the reaction solution was filtered and concentrated under reduced pressure. The crude product was purified by silica gel column chromatography to obtain compound **49-7.** MS-ESI [M-Boc+H]⁺, calculated: 296, found: 296.¹H NMR (400 MHz, CDCl₃) *δ* 7.18-7.25 (m, 1H), 6.97 (br d, *J* = 7.6 Hz, 1H), 6.86 (t, *J* = 2.4 Hz, 1H), 6.75 (dd, *J* = 8.4, 2.4 Hz, 1H), 5.91 (br d, *J* = 15.6 Hz, 1H), 5.31 (s, 1H), 4.73-4.93 (m, 1H), 4.47-4.71 (m, 1H), 4.21 (br d, *J* = 7.2 Hz, 2H), 3.59-3.91 (m, 2H), 2.34-2.48 (m, 1H), 1.98-2.10 (m, 1H), 1.44 (s, 9H), 1.26-1.33 (m, 3H).
(5) To a solution of compound **49-7** (1.0 g, 2.53 mmol) in methanol (20.0 mL), tris(triphenylphosphine)rhodium chloride (100 mg, 108 µmol) was added. The reaction solution was stirred under a hydrogen atmosphere at 25 °C for 2 hours. The reaction solution was filtered, concentrated under reduced pressure to obtain compound **49-8.** MS-ESI [M-Boc+H]⁺, calculated: 298, found: 298.
(6) To a solution of compound **49-8** (1.55 g, 3.90 mmol) in dichloromethane (9.0 mL), trifluoroacetic acid (4.62 g, 40.5 mmol) was added. The reaction solution was stirred at 25 °C for 1 hour. Then water (10.0 mL) was added, then the mixture was neutralized with saturated sodium bicarbonate (30.0 mL) and extracted with dichloromethane (10.0 mL). Organic layer was dried over anhydrous sodium sulfate, filtered and concentrated under reduced pressure to obtain compound **49-9.** MS-ESI [M+H]⁺, calculated: 298, found: 298.¹H NMR (400 MHz, CDCl₃) *δ* 7.19 (t, *J* = 8.0 Hz, 1H), 6.94 (dd, *J* = 8.0, 1.2 Hz, 1H), 6.84 (t, *J* = 2.0 Hz, 1H), 6.74 (dd, *J* = 8.4, 1.8 Hz, 1H), 4.86 (br t, *J* = 4.4 Hz, 1H), 4.13 (q, *J* = 7.2 Hz, 2H), 3.90-4.06 (m, 1H), 3.38-3.55 (m, 2H), 3.19 (br d, *J* = 12.8 Hz, 1H), 2.33-2.52 (m, 2H), 2.22 (dd, *J* = 13.6, 6.4 Hz, 1H), 1.87 (q, *J* = 7.2 Hz, 2H), 1.73 (ddd, *J* = 13.6, 9.6, 5.6 Hz, 1H), 1.25 (t, *J* = 7.2 Hz, 3H).
(7) To a solution of compound **49-9** (1.0 g, 3.36 mmol) in toluene (20.0 mL), compound E (1.28 g, 4.03 mmol), cesium carbonate (2.19 g, 6.72 mmol), 1,1'-binaphthyl-2,2'-diphenylphosphine (418 mg, 671 µmol) and tris (dibenzylideneacetone) dipalladium (307 mg, 335 µmol) was added. The reaction solution was stirred at 80°C under nitrogen atmosphere for 3 hours. Water (10.0 mL) was added to the reaction solution, followed by extraction with ethyl acetate (10.0 mL). The combined organic layer was dried with anhydrous sodium sulfate, filtered and concentrated under reduced pressure. The crude product was purified by silica gel column chromatography (petroleum ether/ethyl acetate = 1:0 to 4:1) to obtain compound **49-10.** MS-ESI [M+H]⁺, calculated: 580, found: 580.¹H NMR (400 MHz, CDCl₃) *δ* 7.43 (d, *J* = 8.8 Hz, 2H), 7.34 (s, 1H), 7.22 (t, *J* = 8.0 Hz, 1H), 6.98 (dd, *J* = 8.0, 1.2 Hz, 1H), 6.83-6.87 (m, 3H), 6.74 (dd, *J* = 8.4, 2.0 Hz, 1H), 5.20-5.26 (m, 1H), 5.06-5.12 (m, 1H), 4.92-4.99 (m, 1H), 4.13 (qd, *J* = 7.2, 2.8 Hz, 3H), 3.76-3.83 (m, 4H), 3.62 (br d, *J* = 11.2 Hz, 1H), 2.39-2.47 (m, 1H), 2.28-2.36 (m, 2H), 2.02-2.20 (m, 2H), 1.73 (dq, *J* = 15.6, 6.8 Hz, 1H), 1.21-1.27 (m, 3H).
(8) To a solution of compound **49-10** (617 mg, 1.06 mmol) in tetrahydrofuran (9.0 mL), water (3.0 mL) and lithium hydroxide monohydrate (254 mg, 10.6 mmol) was added. The reaction solution was stirred under nitrogen atmosphere at 25 °C for 3 hours. Then the reaction solution was diluted with water (10.0 mL) and the pH was adjusted to 5-6 with citric acid solution. The mixture was extracted with ethyl acetate (30.0 mL). The organic layer was dried over anhydrous sodium sulfate, filtered and concentrated under reduced pressure to obtain compound **49-11.** MS-ESI [M+H]⁺, calculated: 552, found: 552.
(9) To a solution of compound **49-11** (557 mg, 1.01 mmol) in dichloromethane (5.0 mL), compound **B-4** trifluoroacetate (281 mg, 1.21 mmol), diisopropylethylamine (782 mg, 6.05 mmol), propylphosphonic anhydride solution (1.93 g, 3.03 mmol, 50% purity in ethyl acetate) was added. The reaction mixture was stirred under nitrogen atmosphere at 35 °C for 3 hour. Water (10.0 mL) was added to the reaction solution, followed by extraction with dichloromethane (10.0 mL). The organic layer was dried over anhydrous sodium sulfate, filtered and concentrated under reduced pressure. The crude product was purified by silica gel column chromatography (dichloromethane/methanol = 1:0 to 1:1) to obtain compound **49-12.** MS-ESI [M+H]⁺, calculated: 766, found: 766.
(10) To a solution of compound **49-12** (300 mg, 392 µmol) in trifluoroacetic acid (10.0 mL), the reaction solution was stirred at 70 °C for 2 hours. Then the reaction solution was filtered, concentrated under reduced pressure. The crude product was purified by preparative high performance liquid chromatography (C18, 100 mm × 30 mm × 5 µm, A: water (0.225% formic acid); B: acetonitrile, 55%-85%: 15 minutes) to obtain compound **49** formate. MS-ESI [M+H]⁺, calculated: 646, found: 646.¹H NMR (400 MHz, MeOD) *δ* 8.59 (s, 2H), 7.83 (s, 1H), 7.24 (t, *J* = 8.4 Hz, 1H), 6.91-6.98 (m, 2H), 6.83-6.90 (m, 1H), 5.06-5.16 (m, 1H), 4.11-4.27 (m, 1H), 3.83-3.99 (m, 5H), 3.60-3.74 (m, 5H), 2.39-2.55 (m, 3H), 2.12-2.27 (m, 2H), 1.70-1.86 (m, 1H).

### Example 50 Synthesis of Compound 50

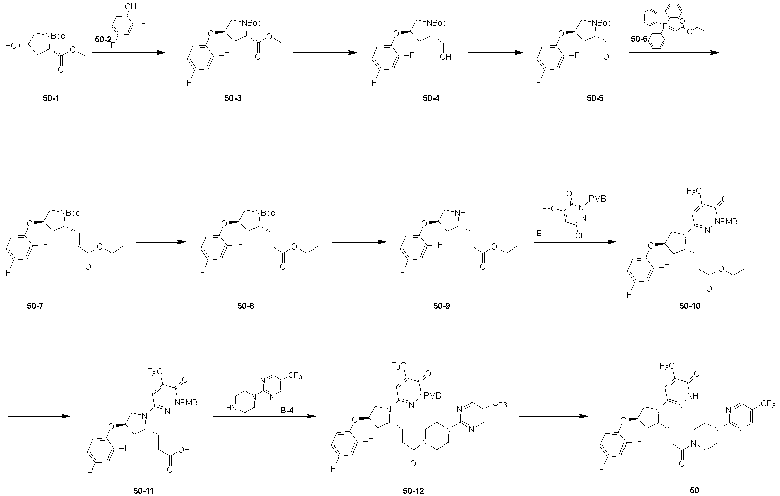

(1) At 0°C, to a solution of compound **50-1** (2.0 g, 8.15 mmol) in tetrahydrofuran (20.0 mL), compound **50-2** (1.06 g, 8.15 mmol), triphenylphosphine (6.42 g, 24.5 mmol) and diisopropyl azodicarboxylate (1.65 g, 8.15 mmol) was added. The reaction mixture was stirred at 25 °C for 12 hours. Water (20.0 mL) was added, extracted with ethyl acetate (20.0 × 3 mL). The organic phase was washed with saturated sodium chloride (20.0 mL × 3), dried over anhydrous sodium sulfate, filtered and concentrated under reduced pressure. The crude product was purified by silica gel column chromatography (petroleum ether/ethyl acetate = 1:0 to 5:1) to obtain compound **50-3.** MS-ESI [M+H]⁺, calculated: 358, found: 358.
(2) At 0°C, to a solution of compound **50-3** (1.75 g, 4.90 mmol) in tetrahydrofuran (20.0 mL), lithium aluminum hydride (372 mg, 9.79 mmol) was added. The reaction mixture was stirred for 1 hour at 0°C under nitrogen atmosphere. Water (1.0 mL), 15% sodium hydroxide (1.0 mL) and water (3.0 mL) was added at 0°C. The mixture was dried over anhydrous sodium sulfate, filtered and concentrated under reduced pressure. The crude product was purified by silica gel column chromatography (petroleum ether/ethyl acetate = 2:1) to obtain compound **50-4.** MS-ESI [M+H]⁺, calculated: 330, found: 330.
(3) To a solution of compound **50-4** (800 mg, 2.43 mmol) in dichloromethane (20.0 mL), Dess-Martin reagent (1.24 g, 2.91 mmol) was added. The reaction mixture was stirred at 25 °C for 12 hours. Then sodium thiosulfate solution (20.0 mL) was added, the reaction solution was diluted with dichloromethane (20.0 mL). The reaction solution was extracted with water (10.0 mL × 3), washed with saturated sodium chloride solution (10.0 mL × 3), dried over anhydrous sodium sulfate, filtered and concentrated under reduced pressure. The crude product was purified by silica gel column chromatography (petroleum ether/ethyl acetate = 1:0 to 0:1) to obtain compound **50-5.** MS-ESI [M-Boc+H]⁺, calculated: 228, found: 228.
(4) To a solution of compound **50-5** (200 mg, 611 µmol) in dichloromethane (10.0 mL), compound **50-6** (426 mg, 1.22 mmol) was added. The mixture was stirred at 25 °C for 8 hours. The reaction mixture was filtrated, concentrated under reduced pressure. The crude product was purified by silica gel chromatography (petroleum ether/ethyl acetate = 1:0 to 1:1) to obtain compound **50-7.**
(5) To a solution of compound **50-7** (200 mg, 503 µmol) in tetrahydrofuran (5.0 mL), palladium on carbon (50.0 mg, 10%) was added. The reaction mixture was stirred at 25 °C under a hydrogen atmosphere for 2 hours. The reaction mixture filtered, concentrated under the reduced pressure to obtain compound **50-8.** MS-ESI [M+H]⁺, calculated: 400, found: 400.
(6) To a solution of compound **50-8** (200 mg, 500 µmol) in dichloromethane (4.0 mL), trifluoroacetic acid (1.54 g, 13.5 mmol) was added. The reaction mixture was stirred at 25 °C for 1 hour. Water (10.0 mL) was added. The mixture was neutralized with sodium bicarbonate (30.0 mL), and extracted with dichloromethane (10.0 mL). The organic phase was dried with anhydrous sodium sulfate, filtered and concentrated under reduced pressure to obtain compound **50-9.**
(7) To a solution of compound **50-9** (80.0 mg, 267 µmol) in toluene (5.0 mL), compound E (127 mg, 400 µmol), cesium carbonate (174 mg, 534 µmol), 1,1'-binaphthyl-2,2'-diphenylphosphine (33.3 mg, 53.4 µmol) and tris (dibenzylideneacetone) dipalladium (24.4 mg, 26.7 µmol) was added. The reaction solution was stirred at 80°C under nitrogen atmosphere for 3 hours. Water (10.0 mL× 2) was added. The reaction solution was extracted with ethyl acetate (20.0 mL). The combined organic layer was dried over anhydrous sodium sulfate, filtered and concentrated under reduced pressure. The crude product was purified by silica gel chromatography (petroleum ether/ethyl acetate = 1:0 to 1:1) to obtain compound **50-10.** MS-ESI [M+H]⁺, calculated: 582, found: 582.
(8) To a solution of compound **50-10** (300 mg, 515 µmol) in tetrahydrofuran (6.0 mL), water (2.0 mL) and lithium hydroxide monohydrate (37.1 mg, 1.55 mmol) was added, the reaction was stirred at 25 °C under nitrogen atmosphere for 12 hours. The mixture was diluted with water (10.0 mL), adjusted the pH to 5-6 with citric acid aqueous solution, and extracted with ethyl acetate (20.0 mL). The organic phase was dried with anhydrous sodium sulfate, filtered and concentrated under reduced pressure to obtain compound **50-11.** MS-ESI [M+H]⁺, calculated: 554, found: 554.
(9) To a solution of compound **50-11** (177 mg, 319 µmol) in dichloromethane (5.0 mL), compound **B-4** trifluoroacetate (89.1 mg, 383 µmol), diisopropylethylamine (247 mg, 1.92 mmol), propylphosphonic anhydride solution (610 mg, 959 µmol, 50% purity in ethyl acetate) was added. The reaction mixture was stirred under nitrogen atmosphere at 25 °C for 1 hour. Water (20.0 mL) was added to the reaction solution, followed by extraction with dichloromethane (20.0 mL). The organic phase was dried with anhydrous sodium sulfate, filtered and concentrated under reduced pressure. The crude product was purified by silica gel column chromatography (dichloromethane/methanol = 1:0 to 10:1) to obtain compound **50-12.** MS-ESI [M+H]⁺, calculated: 768, found: 768.
(10) Compound **50-12** (45.0 mg, 58.6 µmol) was dissolved in trifluoroacetic acid (1.0 mL). The reaction mixture was stirred at 70 °C for 2 hours. To the reaction solution was added water (10.0 mL), followed by extraction with dichloromethane (10.0 mL). The reaction solution was filtrated and concentrated under reduced pressure. The crude product was purified by preparative high performance liquid chromatography (Xtimate C18, 100 mm × 30 mm 10 µm, A: water (0.225% formic acid); B: acetonitrile, 60%-80%: 10 minutes) to obtain compound **50** formate. MS-ESI [M+H]⁺, calculated: 648, found: 648.¹H NMR (400 MHz, DMSO-*d₆*) *δ* 12.63 (s, 1H), 8.74 (s, 2H), 7.91 (s, 1H), 7.24-7.32 (m, 2H), 7.02-7.05 (m, 1H), 5.11-5.12 (m, 1H), 4.08-4.10 (m, 1H), 3.77-3.87 (m, 5H), 3.56-3.58 (m, 5H),2.40-2.41 (m, 2H), 2.25-2.31 (m, 1H), 2.08-2.12 (m, 1H), 1.95-2.02 (m, 1H), 1.55-1.62 (m,1H).

### Example 51 Synthesis of Compound 51

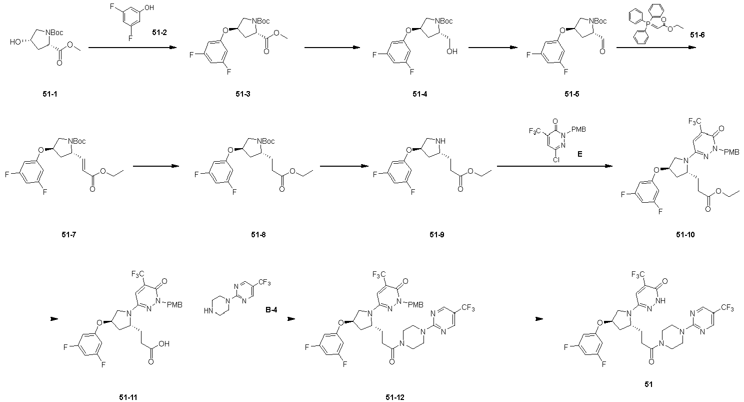

(1) At 0°C, to a solution of compound **51-1** (4.0 g, 16.3 mmol) in tetrahydrofuran (50.0 mL), compound **51-2** (2.33 g, 17.9 mmol), triphenylphosphine (4.71 g, 17.9 mmol) and diisopropyl azodicarboxylate (3.3 g, 16.3 mmol) was added. The reaction mixture was stirred at 25 °C for 12 hours. Water (30.0 mL) was added, followed by extraction with ethyl acetate (200 mL). The organic phase was dried over anhydrous sodium sulfate, filtered and concentrated under reduced pressure. The crude product was purified by silica gel column chromatography (petroleum ether/ethyl acetate = 1:0 to 5:1) to obtain compound **51-3.** MS-ESI [M-Boc+H]⁺, calculated: 258, found: 258.¹H NMR (400 MHz, CDCl₃) *δ* 6.33-6.49 (m, 3H), 4.85 (d, *J* = 2.8 Hz, 1H), 4.36-4.51 (m, 1H), 3.74-3.82 (m, 5H), 2.48-2.61 (m, 1H), 2.20-2.29 (m, 1H), 1.41-1.49 (m, 9H).
(2) At 0°C, to a solution of compound **51-3** (1.45 g, 4.06 mmol) in tetrahydrofuran (15.0 mL), lithium aluminum hydride (169 mg, 4.46 mmol) was added. The reaction mixture was stirred under nitrogen atmosphere at 0 °C for 1 hour. Water (0.2 mL) , 15% sodium hydroxide (0.2 mL) and water (0.6 mL) was added at 0 °C. The mixture was dried over anhydrous sodium sulfate, filtered and concentrated under reduced pressure. The crude product was purified by silica gel column chromatography (petroleum ether/ethyl acetate = 1:0 to 0:1) to obtain compound **51-4.** MS-ESI [M-^{t}Bu+H]⁺, calculated: 274, found: 274.¹H NMR (400 MHz, CDCl₃) *δ* 6.33-6.49 (m, 3H), 4.75 ( s, 1H), 4.10-4.17 (m, 1H), 3.74-3.82 (m, 2H), 3.55-3.64 (m, 2H), 2.30-2.40 (m, 1H), 1.90 (s, 1H), 1.47 (s, 9H).
(3) To a solution of compound **51-4** (1.0 g, 3.04 mmol) in dichloromethane (15.0 mL), Dess-Martin reagent (1.55 g, 3.64 mmol) was added. The reaction mixture was stirred at 25 °C for 2 hours. Then dichloromethane (10.0 mL) and sodium sulfite (20.0 mL) was added. The reaction mixture was extracted with dichloromethane (25.0 mL × 2), dried over anhydrous sodium sulfate, filtered and concentrated under reduced pressure. The crude product was purified by silica gel column chromatography (petroleum ether/ethyl acetate = 1:0 to 0:1) to obtain compound **51-5.** ¹H NMR (400 MHz, CDCl₃) *δ* 9.43-9.67 (m, 1H), 6.34-6.50 (m, 3H), 4.84 (s, 1H), 4.24-4.48 (m, 1H), 3.72-3.90 (m, 1H), 3.71 (d, *J* = 2.8 Hz, 1H), 2.31-2.48 (m, 1H), 2.08-2.20 (m, 1H), 1.44-1.49 (m, 9H).
(4) To a solution of compound **51-5** (430 mg, 1.31 mmol) in dichloromethane (5.0 mL), compound **51-6** (549 mg, 1.58 mmol) was added. The reaction mixture was stirred at 25 °C for 12 hours. Then dichloromethane (10.0 mL) was added, the reaction mixture was extracted with water (25.0 mL × 2). The organic phase was dried over anhydrous sodium sulfate, filtered and concentrated under reduced pressure. The crude product was purified by silica gel column chromatography to obtain compound **51-7.** MS-ESI [M-Boc+H]⁺, calculated: 298, found: 298.
(5) To a solution of compound **51-7** (450 mg, 1.13 mmol) in tetrahydrofuran (5.0 mL), 10% palladium on carbon (100 mg) was added. The reaction mixture was stirred under a hydrogen atmosphere at 25 °C for 2 hours. Then the mixture was filtered, the filtrate was concentrated under reduced pressure to obtain compound **51-8.** MS-ESI [M+H]⁺, calculated: 400, found: 400.¹H NMR (400 MHz, CDCl₃) *δ* 6.32-6.47 (m, 3H), 4.70-4.80 (m, 1H), 4.10-4.16 (m, 2H), 4.04 (s, 1H), 3.69-3.95 (m, 1H), 3.49 (dd, *J* = 12.8, 4.4 Hz, 1H), 2.32 (t, *J* = 7.2 Hz, 3H), 2.13 (d, *J* = 6.4 Hz, 1H), 1.88-1.97 (m, 1H), 1.78-1.87 (m, 1H), 1.46 (s, 9H), 1.25 (t, *J* = 7.2 Hz, 3H).
(6) To a solution of compound **51-8** (410 mg, 1.03 mmol) in dichloromethane (4.0 mL), trifluoroacetic acid (1.54 g, 13.5 mmol) was added. The reaction mixture was stirred at 25 °C for 1 hour. Then sodium bicarbonate was added to adjust the pH > 7. The reaction mixture was extracted with dichloromethane (20.0 mL × 2), dried over anhydrous sodium sulfate, filtered and concentrated under reduced pressure to obtain compound **51-9.** MS-ESI [M+H]⁺, calculated: 300, found: 300.¹H NMR (400 MHz, CDCl₃) *δ* 6.48 (tt, *J* = 8.8, 2.4 Hz, 1H), 6.36-6.43 (m, 2H), 5.00 (t, *J* = 4.4 Hz, 1H), 4.11 (q, *J* = 7.2 Hz, 2H), 3.80-3.97 (m, 2H), 3.50 (d, *J* = 13.2 Hz, 1H), 2.88 (s, 2H), 2.53 (t, *J* = 7.2 Hz, 2H), 2.44 (dd, *J* = 14.0, 5.6 Hz, 1H), 2.04-2.16 (m, 2H), 1.20-1.25 (m, 3H).
(7) To a solution of compound **51-9** (250 mg, 835 µmol) in toluene (8.0 mL), compound E (266 mg, 835 µmol), cesium carbonate (544 mg, 1.67 mmol), 1,1'-binaphthyl-2,2'-diphenylphosphine (104 mg, 167 µmol) and tris (dibenzylideneacetone) dipalladium (76.4 mg, 83.5 µmol) was added. The reaction solution was stirred at 80°C under nitrogen atmosphere for 4 hours. Water (75.0 mL × 2) was added, extracted with ethyl acetate (30.0 mL). The organic layer was combined, dried over anhydrous sodium sulfate, filtered, concentrated under reduced pressure and the crude product was purified by silica gel column chromatography (petroleum ether/ethyl acetate = 1:0 to 0:1) to obtain compound **51-10.** MS-ESI [M+H]⁺, calculated: 582, found: 582.¹H NMR (400 MHz, CDCl₃) *δ* 7.42 (d, *J* = 8.8 Hz, 2H), 7.34 (s, 1H), 6.84 (d, *J* = 8.4 Hz, 2H), 6.45 (tt, *J* = 9.2, 2.0 Hz, 1H), 6.33-6.41 (m, 2H), 5.17-5.24 (m, 1H), 5.05-5.11 (m, 1H), 4.87-4.94 (m, 1H), 4.10-4.13 (m, 2H), 3.75-3.83 (m, 4H), 3.61 (d, *J* = 11.6 Hz, 1H), 2.37-2.47 (m, 1H), 2.26-2.35 (m, 2H), 2.06-2.20 (m, 2H), 1.71 (*J* = 15.6, 6.8 Hz, 2H), 1.22-1.26 (m, 3H).
(8) To a solution of compound **51-10** (190 mg, 326 µmol) in tetrahydrofuran (3.0 mL), water (1.0 mL) and lithium hydroxide monohydrate (137 mg, 3.27 mmol) was added, the reaction was stirred at 30 °C under nitrogen atmosphere for 12 hours. Hydrochloric acid aqueous solution (1 mol/L) was added to adjust the pH < 7, followed by extraction with ethyl acetate (15.0 mL × 2). The organic phase was dried over anhydrous sodium sulfate, filtered and concentrated under reduced pressure to obtain compound 51-11. MS-ESI [M-H]⁻, calculated: 552, found: 552.¹H NMR (400 MHz, CDCl₃) *δ* 7.41 (d, *J* = 8.4 Hz, 2H), 7.35 (s, 1H), 6.83 (d, *J* = 8.4 Hz, 2H), 6.46 (tt, *J* = 6.8, 2.4 Hz, 1H), 6.31-6.41 (m, 2H), 5.19-5.27 (m, 1H), 5.07 (d, *J* = 13.6 Hz, 1H), 4.91 (d, *J* = 2.4 Hz, 1H), 3.72-3.84 (m, 4H), 3.61 (d, *J* = 11.6 Hz, 1H), 2.30-2.49 (m, 3H), 2.07-2.19 (m, 2H), 1.65-1.81 (m, 2H).
(9) To a solution of compound **51-11** (160 mg, 289 µmol) in dichloromethane (5.0 mL), compound **B-4** trifluoroacetate (87.2 mg, 375 µmol), diisopropylethylamine (112 mg, 867 µmol), propylphosphonic anhydride solution (551 mg, 867 µmol, 50% purity in ethyl acetate) was added. The reaction mixture was stirred under nitrogen atmosphere at 25 °C for 1 hour. Water (25.0 mL × 2) was added, the reaction solution was extracted with dichloromethane (10.0 mL). The organic phase was washed with saturated sodium chloride solution (25.0 mL × 2), dried over anhydrous sodium sulfate, filtered and concentred under reduced pressure. The crude product was purified by silica gel column chromatography (petroleum ether/ethyl acetate = 1:0 to 0:1) to obtain compound **51-12.** MS-ESI [M+H]⁺, calculated: 768, found: 768.¹H NMR (400 MHz, CDCl₃) *δ* 8.52 (s, 2H), 7.62 (s, 1H), 7.41 (d, *J* = 8.8 Hz, 2H), 6.83 (d, *J* = 8.8 Hz, 2H), 6.33-6.50 (m, 3H), 5.15-5.24 (m, 1H), 5.04-5.14 (m, 1H), 4.94 (s, 1H), 4.14-4.22 (m, 1H), 3.85-3.98 (m, 4H), 3.77 (s, 3H), 3.66-3.75 (m, 2H), 3.42-3.50 (m, 2H), 2.29-2.46 (m, 3H), 2.08-2.24 (m, 2H), 1.59-1.79 (m, 3H).
(10) A solution of compound **51-12** (75.0 mg, 97.7 µmol) in trifluoroacetic acid (2.25 mL) was stirred at 70 °C for 4 hours. The mixture was filtered and concentrated under reduced pressure. The crude product was purified by preparative high performance liquid chromatography (Phenomenex C18, 75 mm × 30 mm× 3 µm, A: water (10 mmol/L ammonium bicarbonate); B: acetonitrile, gradient elution from 44% to 74% over 10 minutes) to obtain compound **51.** MS-ESI [M+H]⁺, calculated: 648, found: 648.¹H NMR (400 MHz, MeOD) *δ* 8.59 (s, 2H), 7.83 (s, 1H), 6.45-6.63 (m, 3H), 5.05-5.14 (m, 1H), 4.14-4.23 (m, 1H), 3.93-4.00 (m, 2H), 3.84-3.93 (m, 3H), 3.59-3.73 (m, 5H), 2.51 (t, *J* = 7.2 Hz, 2H), 2.40-2.48 (m, 1H), 2.12-2.27 (m, 2H), 1.72-1.87 (m, 1H).

### Example 52 Synthesis of Compound 52

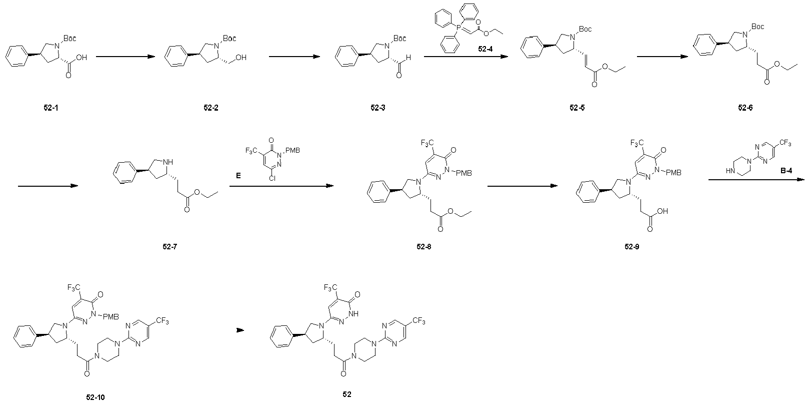

(1) At 0°C, to a solution of compound **52-1** (200 mg, 687 µmol) in tetrahydrofuran (2.0 mL), borane-tetrahydrofuran complex (1.37 mL, 1 mol/L) was added. The reaction mixture was stirred at 0 °C for 2 hours, methanol (10.0 mL), water (100 mL) was added and extracted with ethyl acetate (200 mL× 2). The organic phase was dried over anhydrous sodium sulfate, filtered and concentrated under reduced pressure to obtain compound **52-2.** MS-ESI [M+H]⁺, calculated: 222, found: 222.
(2) To a solution of compound **52-2** (700 mg, 2.52 mmol) in dichloromethane (10.0 mL), Dess-Martin reagent (1.28 g, 3.03 mmol) was added, the reaction mixture was stirred at 25 °C for 12 hours. Water (100 mL) was added to the reaction solution, followed by extraction with ethyl acetate (200 mL × 2). The organic phase was dried over anhydrous sodium sulfate, filtered and concentrated under reduced pressure. The crude product was purified by silica gel column chromatography (petroleum ether/ethyl acetate = 1:0 to 10:1) to obtain compound **52-3.**
(3) To a solution of compound **52-3** (600 mg, 2.18 mmol) in dichloromethane (7.0 mL), compound **52-4** (835 mg, 2.40 mmol) was added, the reaction mixture was stirred at 25 °C for 1 hour. Water (100 mL) was added to the reaction solution, followed by extraction with ethyl acetate (200 mL × 2). The organic phase was dried over anhydrous sodium sulfate, filtered and concentrated under reduced pressure. The crude product was purified by silica gel column chromatography (petroleum ether/ethyl acetate = 1:0 to 10:1) to obtain compound **52-5.**MS-ESI [M+H]⁺, calculated: 346, found: 346.
(4) To a solution of compound **52-5** (500 mg, 1.45 mmol) in tetrahydrofuran (5.0 mL), 10% palladium on carbon (100 mg) was added, the reaction mixture was stirred under a hydrogen atmosphere at 25 °C for 2 hours. The mixture was filtered and concentrated under reduced pressure to obtain compound **52-6.** MS-ESI [M+H]⁺, calculated: 348, found: 348.¹H NMR (400 MHz, CDCl₃) *δ* 7.30-7.36 (m, 2H), 7.22-7.26 (m, 3H), 4.15 (q, *J* = 7.2 Hz, 2H), 3.90-4.09 (m, 1H), 3.70-3.80 (m, 1H), 3.27-3.54 (m, 2H), 2.34-2.45 (m, 2H), 1.98-2.17 (m, 3H), 1.81 (m, *J* = 13.6, 8.0 Hz, 1H), 1.48 (s, 9H), 1.27 (t, *J* = 7.2 Hz, 3H).
(5) To a solution of compound **52-6** (400 mg, 1.15 mmol) in dichloromethane (4.0 mL), trifluoroacetic acid (1.31 g, 11.5 mmol) was added. The reaction mixture was stirred at 25 °C for 1 hour and concentrated under reduced pressure to obtain compound **52-7.** MS-ESI [M+H]⁺, calculated: 248, found: 248.
(6) To a solution of compound **52-7** (110 mg, 445 µmol) in toluene (2.0 mL) compound **E** (156 mg, 489 µmol), cesium carbonate (290 mg, 889 µmol), 1,1'-binaphthyl-2,2'-diphenylphosphine (55.4 mg, 88.5 µmol) and tris (dibenzylideneacetone) dipalladium (81.5 mg, 88.9 µmol) was added. The reaction solution was stirred at 80°C under nitrogen atmosphere for 2 hours. Water (100 mL) was added and extracted with ethyl acetate (200 mL × 2). The organic phase was dried over anhydrous sodium sulfate, filtered and concentrated under reduced pressure to obtain compound **52-8.** MS-ESI [M+H]⁺, calculated: 530, found: 530.
(7) To a solution of compound **52-8** (110 mg, 208 µmol) in tetrahydrofuran (3.0 mL), water (1.0 mL), methanol (1.0 mL) and lithium hydroxide monohydrate (43.6 mg, 1.04 mmol) was added. The reaction mixture was stirred under nitrogen atmosphere at 25 °C for 1 hour. Water (50.0 mL) was added, followed by extraction with ethyl acetate (50.0 mL × 2). The organic phase was dried over anhydrous sodium sulfate, filtered and concentrated under reduced pressure to obtain compound **52-9.** MS-ESI [M+H]⁺, calculated: 502, found: 502.
(8) To a solution of compound **52-9** (70.0 mg, 140 µmol) in dichloromethane (3.0 mL), compound **B-4** trifluoroacetate (35.7 mg, 154 µmol), diisopropylethylamine (126 mg, 977 µmol), propylphosphonic anhydride solution (222 mg, 349 µmol, 50% purity in ethyl acetate) was added. The reaction mixture was stirred under nitrogen atmosphere at 25 °C for 1 hour. Water (100 mL) was added, followed by extraction with ethyl acetate (50.0 mL × 2). The organic phase was dried over anhydrous sodium sulfate, filtered and concentrated under reduced pressure. The crude product was purified by silica gel column chromatography (petroleum ether/ethyl acetate = 1:0 to 3:1) to obtain compound **52-10.** MS-ESI [M+H]⁺, calculated: 716, found: 716.
(9) To a solution of compound **52-10** (60.0 mg, 83.8 µmol) in dichloromethane (2.0 mL), trifluoromethanesulfonic acid (340 mg, 2.27 mmol) was added. The reaction mixture was stirred at 25 °C for 1 hour. The reaction mixture was concentrated under reduced pressure. The crude product was purified by preparative high performance liquid chromatography (Xtimate C18, 100 mm × 30 mm 10 µm, A: water (0.225% formic acid); B: acetonitrile, 55%-85% over 10 minutes) to obtain compound **52** formate. MS-ESI [M+H]⁺, calculated: 596, found: 596.¹H NMR (400 MHz, MeOD) *δ* 8.60 (s, 2H), 8.00 (br s, 1H), 7.30-7.37 (m, 4H), 7.21-7.26 (m, 1H), 4.16 (br s, 1H), 3.91-4.02 (m, 5H), 3.63-3.77 (m, 5H), 3.34-3.42 (m, 1H), 2.59 (t, *J* = 6.8 Hz, 2H), 2.22-2.31 (m, 2H), 2.07-2.16 (m, 1H), 1.79-1.90 (m, 1H).

### Example 53 Synthesis of Compound 53

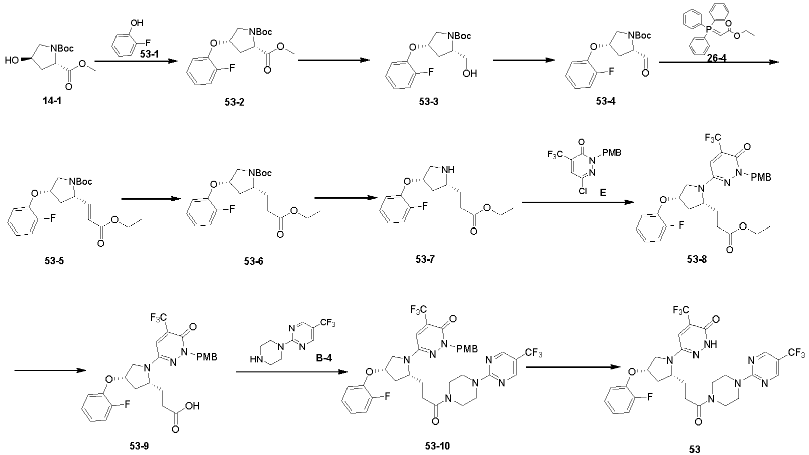

(1) A solution of compound **14-1** (3.00 g, 12.2 mmol) and **53-1** (1.51g, 13.4 mmol) in tetrahydrofuran (30.0 mL) was cooled to 0°C, triphenylphosphine (3.53 g, 13.4 mmol) and diisopropyl azodicarboxylate (2.47 g, 12.2 mmol) was added. The reaction was stirred at 25°C for 12 hours. Water (500 mL) was added, followed by extraction with ethyl acetate (500 mL × 2). The organic layer was washed with saturated sodium chloride (200.0 mL), dried over anhydrous sodium sulfate, filtered and concentrated under reduced pressure. The crude product was purified by silica gel column chromatography (petroleum ether/ethyl acetate = 1 : 0 to 0 : 1) to obtain compound **53-2.** MS-ESI [M-Boc+H]⁺, calculated: 240, found: 240.¹H NMR (400 MHz, CDCl₃) *δ* 7.00-7.12 (m, 2H), 6.85-6.99 (m, 2H), 4.98 (dq, *J* = 12.4, 6.0 Hz, 3H), 4.42-4.60 (m, 1H), 3.76 (s, 3H), 2.39-2.57 (m, 2H), 1.44-1.51 (m, 9H).
(2) At 0°C, to a solution of compound **53-2** (1.40 g, 4.13 mmol) in tetrahydrofuran (30.0 mL), lithium aluminum hydride (172 mg, 4.54 mmol) was added. The reaction was stirred at 0°C for 1 hour. Then water (1.0 mL), 15% sodium hydroxide solution (1.0 mL) and water (3.0 mL) was added. The mixture was stirred for 10 minutes, extracted with ethyl acetate (100 mL). The organic layer was dried over anhydrous sodium sulfate, filtered and concentrated under reduced pressure to obtain compound **53-3.** MS-ESI [M-^{t}Bu+H]⁺, calculated: 256, found: 256.
(3) To a solution of compound **53-3** (1.11 g, 3.57 mmol) in dichloromethane (20.0 mL), Dess-Martin reagent (1.51 g, 3.57 mmol) was added. The reaction solution was stirred at 25°C for 3 hours. Then saturated sodium sulfite solution (50.0 mL) was added. The mixture was stirred for 10 minutes, diluted with water (30.0 mL), and extracted with dichloromethane (50.0 mL × 2). The organic layer was combined, washed with saturated sodium bicarbonate (50.0 mL), dried over anhydrous sodium sulfate, filtered and concentrated under reduced pressure. The crude product was purified by silica gel column chromatography (petroleum ether/ethyl acetate = 1 : 0 to 0 : 1) to obtain compound **53-4.** ¹H NMR (400 MHz, CDCl₃) *δ* 9.63-9.81 (m, 1H), 7.02-7.14 (m, 2H), 6.87-7.01 (m, 2H), 4.94 (br d, *J* = 2.8 Hz, 1H), 4.15-4.35 (m, 1H), 3.77-3.97 (m, 1H), 3.59-3.69 (m, 1H), 2.28-2.53 (m, 2H), 1.46-1.53 (m, 9H).
(4) To a solution of compound **53-4** (760 mg, 2.46 mmol) in dichloromethane (15.0 mL), compound **26-4** (1.03 g, 2.95 mmol) was added. The reaction solution was stirred at 25°C for 12 hours. It was concentrated under reduced pressure. The crude product was purified by silica gel column chromatography (petroleum ether/ethyl acetate = 1 : 0 to 0 : 1) to obtain compound **53-5.** MS-ESI [M+Na]⁺, calculated: 402, found: 402.¹H NMR (400 MHz, CDCl₃)*δ* 6.92-7.05 (m, 3H), 6.81-6.90 (m, 2H), 5.73-5.92 (m, 1H), 4.91 (br d, *J* = 3.2 Hz, 1H), 4.34-4.61 (m, 1H), 4.14 (br d, *J* = 6.8 Hz, 2H), 3.65-3.70 (m, 2H), 2.26-2.41 (m, 1H), 2.14 (br d, *J* = 12.4 Hz, 1H), 1.37 (br s, 9H), 1.23 (br t, *J* = 6.8 Hz, 3H).
(5) To a solution of compound **53-5** (800 mg, 2.11 mmol) in tetrahydrofuran (15.0 mL), 10% palladium on carbon (200 mg) was added. The reaction solution was stirred under a hydrogen atmosphere (15 psi) at 25°C for 2 hours and filtered, and the filtrate was concentrated under reduced pressure to obtain compound **53-6.** MS-ESI [M+H]⁺, calculated: 382, found: 382.
(6) To a solution of compound **53-6** (400 mg, 1.05 mmol) in dichloromethane (4.0 mL), trifluoroacetic acid (1.54 g, 13.5 mmol) was added. The reaction mixture was stirred at 25 °C for 2 hour. Then sodium bicarbonate was added to adjust the pH > 7. The reaction mixture was diluted with water (20.0 mL), and extracted with dichloromethane (30.0 mL × 2). The organic layer was combined, washed with saturated sodium chloride (30.0 mL), dried over anhydrous sodium sulfate, filtered and concentrated under reduced pressure to obtain compound **53-7.** MS-ESI [M+H]⁺, calculated: 282, found: 282.
(7) To a solution of compound **E** (203 mg, 639 µmol) and compound **53-7** (150 mg, 533 µmol) in toluene (5.0 mL), cesium carbonate (521 mg, 1.60 mmol), 1,1'-binaphthyl-2,2'-diphenylphosphine (66.4 mg, 106 µmol) and tris (dibenzylideneacetone) dipalladium (48.3 mg, 53.3 µmol) was added. The reaction solution was stirred at 80°C under nitrogen atmosphere for 3 hours. Water (10.0 mL) was added to the reaction solution, followed by extraction with ethyl acetate (20.0 mL × 2). The combined organic layer was washed with saturated sodium chloride (10.0 mL), dried over anhydrous sodium sulfate, filtered and concentrated under reduced pressure. The crude product was purified by silica gel chromatography (petroleum ether/ethyl acetate = 2:1) to obtain compound **53-8.** MS-ESI [M+H]⁺, calculated: 564, found: 564.
(8) Compound **53-8** (100 mg, 177 µmol) was dissolved in tetrahydrofuran (3.0 mL) and water (1.0 mL), lithium hydroxide monohydrate (44.7 mg, 1.06 mmol) was added. The reaction was stirred at 25 °C for 6 hours. 1 mol/L hydrochloric acid solution was added to the reaction solution to adjust the pH < 7. The mixture was diluted with water (20.0 mL) and extracted with dichloromethane (30.0 mL × 2). The combined organic layer was washed with saturated sodium chloride (30.0 mL), dried over anhydrous sodium sulfate, filtered and concentrated under reduced pressure to give compound **53-9.** MS-ESI [M+H]⁺, calculated: 536, found: 536.
(9) To a solution of compound **53-9** (45.0 mg, 84.0 µmol), **B-4** (39.0 mg, 168 µmol) in dichloromethane (1.0 mL), diisopropylethylamine (54.3 mg, 420 µmol) and 50% propylphosphonic anhydride (213 mg, 336 µmol) was added. The reaction solution was stirred for 1 hour at 25°C. Water (10.0 mL) was added to the reaction solution, followed by extraction with dichloromethane (20.0 mL × 2). The combined organic layer was washed with saturated sodium chloride (10.0 mL), dried over anhydrous sodium sulfate, filtered and concentrated under reduced pressure. The crude product was purified by silica gel column chromatography (petroleum ether/ethyl acetate = 2:1) to obtain compound **53-10.** MS-ESI [M+H]⁺, calculated: 750, found: 750.
(10) A solution of compound **53-10** (30.0 mg, 40.2 µmol) in trifluoroacetic acid (3.0 mL) was stirred at 70°C for 3 hours. The reaction solution was cooled to room temperature and adjusted to pH > 8 with saturated sodium bicarbonate. The reaction solution was extracted with ethyl acetate (10.0 mL × 2). The combined organic layer was washed with saturated sodium chloride (10.0 mL), dried over anhydrous sodium sulfate, filtered, concentrated under reduced pressure. The crude product was purified by preparative high performance liquid chromatography (Xtimate C18, 100 mm × 30 mm 10 µm, mobile phase A: water (0.225% formic acid); B: acetonitrile, 55% to 85% gradient over 10 minutes) to obtain compound 53 formate. ¹H NMR (400 MHz, MeOD) *δ* 8.60 (s, 2H), 7.96 (s, 1H), 7.08-7.18 (m, 3H), 6.92-7.00 (m, 1H), 5.18 (br d, *J* = 2.8 Hz, 1H), 4.13-4.18 (m, 1H), 3.88-3.95 (m, 4H), 3.79-3.81 (m, 2H), 3.64-3.70 (m, 2H), 3.58-3.63 (m, 2H), 2.49-2.62 (m, 2H), 2.39-2.47 (m, 1H), 2.27-2.33 (m, 1H), 2.08-2.19 (m, 2H).

### Example 54 Synthesis of Compound 54

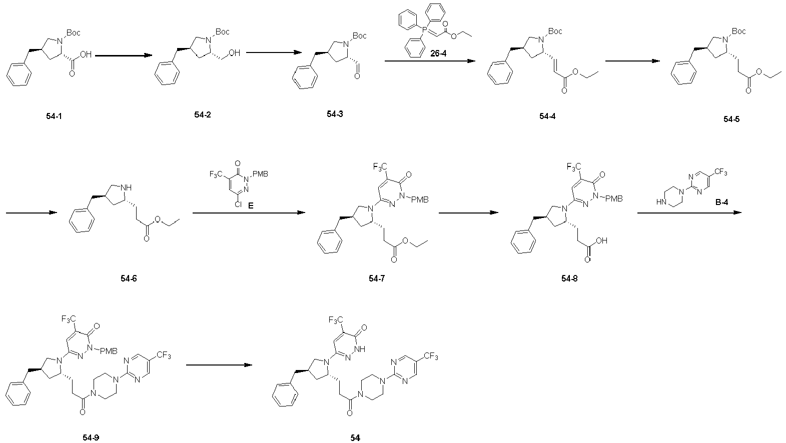

(1) A solution of compound **54-1** (1.80 g, 5.89 mmol) in tetrahydrofuran (18.0 mL) was cooled to 0-5°C, 1 mol/L borane tetrahydrofuran solution (11.8 mL) was slowly added. The reaction was stirred at 20°C for 2 hours. The reaction solution was cooled to 5°C, quenched with methanol (4.5 mL), diluted with water (90.0 mL), and extracted with ethyl acetate (50.0 mL × 3). The combined organic layer was washed with saturated sodium chloride (50.0 mL), dried over anhydrous sodium sulfate, filtered and concentrated under reduced pressure. The crude product was purified by silica gel chromatography (dichloromethane /methane = 20:1) to obtain compound **54-2.** MS-ESI [M+H]⁺, calculated: 292, found: 292.¹H NMR (400 MHz, CDCl₃) *δ* 7.28-7.34 (m, 2H), 7.13-7.26 (m, 3H), 4.01-4.12 (m, 1H), 3.57-3.64 (m, 2H), 3.42 (dd, *J* = 10.8, 6.8 Hz, 1H), 3.16 (dd, *J* = 10.8, 7.6 Hz, 1H), 2.64 (dd, *J* = 7.6, 3.6 Hz, 2H), 2.41-2.52 (m, 1H), 1.65-1.84 (m, 2H), 1.48 (s, 9H).
(2) Compound **54-2** (800 mg, 2.75 mmol) was dissolved in dichloromethane (8.0 mL), Dess-Martin reagent (1.40 g, 3.29 mmol) was added. The reaction solution was stirred at 20°C for 12 hours. The reaction solution was quenched with saturated sodium sulfite solution (30.0 mL), extracted with dichloromethane (10.0 mL × 2). The combined organic layer was dried over anhydrous sodium sulfate, filtered and concentrated under reduced pressure. The crude product was purified by silica gel column chromatography (petroleum ether/ethyl acetate = 100:0 to 15:1) to obtain compound **54-3.** MS-ESI [M-Boc+H]⁺, calculated: 190, found: 190.
(3) To a solution of compound **54-3** (580 mg, 2.00 mmol) in dichloromethane (6.0 mL), compound **26-4** (768 mg, 2.20 mmol) was added. The reaction solution was stirred at 20°C for 12 hours. Water (20.0 mL) was added and the reaction solution was extracted with dichloromethane (10.0 mL × 3). The combined organic phase was washed with saturated sodium chloride (10.0 mL), dried over anhydrous sodium sulfate, filtered and concentrated under reduced pressure. The crude product was purified by silica gel column chromatography (petroleum ether/ethyl acetate = 100:0 to 10:1) to obtain compound **54-4.** MS-ESI [M-^{t}Bu+H]⁺, calculated: 304, found: 304.¹H NMR (400 MHz, CDCl₃) *δ* 7.27-7.34 (m, 2H), 7.12-7.25 (m, 3H), 6.74-6.86 (m, 1H), 5.80 (dd, *J* = 15.2, 8.0 Hz, 1H), 4.37-4.61 (m, 1H), 4.10-4.28 (m, 2H), 3.44-3.61 (m, 1H), 3.01-3.20 (m, 1H), 2.60-2.76 (m, 2H), 2.39-2.54 (m, 1H), 1.77-1.94 (m, 2H), 1.44 (d, *J* = 18.4 Hz, 9H), 1.24-1.32 (m, 3H).
(4) To a solution of compound **54-4** (610 mg, 1.70 mmol) in tetrahydrofuran (10.0 mL), 10% palladium on carbon (120 mg) was added. The reaction solution was stirred under a hydrogen atmosphere (15 psi) at 25°C for 1 hour. The reaction solution was filtered and the filtrate was concentrated under reduced pressure to obtain compound **54-5.** MS-ESI [M+H]⁺, calculated: 362, found: 362.
(5) To a solution of compound **54-5** (500 mg, 1.38 mmol) in dichloromethane (1.0 mL), trifluoroacetic acid (0.5 mL, 6.75 mmol) was added. The reaction solution was stirred at 25°C for 3 hours and concentrated under reduced pressure, then water (5.0 mL) was added and the pH was adjusted to 9-10 with solid sodium carbonate. The mixture was extracted with dichloromethane (5.0 mL × 3). The combined organic layer was dried over anhydrous magnesium sulfate, filtered and concentrated under reduced pressure to obtain compound **54-6.** MS-ESI [M+H]⁺, calculated: 262, found: 262.
(6) To a mixture of compound **E** (488 mg, 1.53 mmol), **54-6** (400 mg, 1.53 mmol), cesium carbonate (997 mg, 3.06 mmol), (2-dicyclohexylphosphino-2',6'-diisopropoxy-1,1'-biphenyl)[(2'-amino-1,1'-biphenyl-2-yl)]palla dium methanesulfonate (256 mg, 306 µmol) was added dioxane (4.0 mL). The reaction solution was stirred at 60°C under nitrogen atmosphere for 6 hours. Then water (40.0 mL) was added and extracted with ethyl acetate (20.0 mL × 3). The combined organic layer was washed with saturated sodium chloride (20.0 mL), dried over anhydrous sodium sulfate, filtered and concentrated under reduced pressure. The crude product was purified by silica gel column chromatography (dichloromethane/methanol = 100:0 to 50:1) to obtain compound **54-7.** MS-ESI [M+H]⁺, calculated: 544, found: 544.
(7) Compound **54-7** (600 mg, 1.10 mmol) was dissolved in tetrahydrofuran (10.0 mL) and water (2.5 mL), lithium hydroxide monohydrate (463 mg, 11.0 mmol) was added. The reaction was allowed to proceed at 35 °C for 12 hours. Water (15.0 mL) was added to the reaction solution, followed by extraction with methyl tert-butyl ether (10.0 mL). The aqueous layer was adjusted to pH 7-8 with 1 mol/L hydrochloric acid solution and extracted with ethyl acetate (15.0 mL × 2). The combined organic layer was dried over anhydrous sodium sulfate, filtered and concentrated under reduced pressure to obtain compound **54-8.** MS-ESI [M+H]⁺, calculated: 516, found: 516.
(8) To a solution of compound **54-8** (500 mg, 970 µmol and compound **B-4** (261 mg, 970 µmol) in dichloromethane (7.0 mL), diisopropylethylamine (627 mg, 4.85 mmol, 845 µL) and 50% propylphosphonic anhydride (926 mg, 1.45 mmol, 865 µL) was added. The reaction solution was stirred at 25°C for 12 hours. Water (10.0 mL) was added to the reaction solution, followed by extraction with dichloromethane (20.0 mL). The organic layer was washed with water (10.0 mL) and saturated sodium chloride (10.0 mL), dried over anhydrous sodium sulfate, filtered and concentrated under reduced pressure. The crude product was purified by silica gel chromatography (dichloromethane/methanol = 100:0 to 50:1) to obtain compound **54-9.** MS-ESI [M+H]⁺, calculated: 730, found: 730.
(9) To a solution of compound **54-9** (460 mg, 630.38 µmol) in dichloromethane (5.0 mL), trifluoromethanesulfonic acid (4.53 mmol, 0.4 mL) was added. The reaction solution was stirred at 25°C for 1 hour. The reaction solution was concentrated under reduced pressure. The residue was dissolved in dichloromethane (30.0 mL), the pH was adjusted to 8-9 with saturated sodium bicarbonate solution. After phase separation, the organic phase was washed with water (10.0 mL × 2) and saturated sodium chloride (10.0 mL), dried over anhydrous sodium sulfate, filtered and concentrated under reduced pressure. The crude product was purified by preparative high performance liquid chromatography (Boston Green ODS, 150 mm × 30 mm, 5 µm; A: water (10 mmol/L ammonium bicarbonate); B: methanol, gradient from 20% to 80% over 35 minutes) to obtain compound **54.** MS-ESI [M+H]⁺, calculated: 610, found: 610.¹H NMR (400 MHz, MeOD) *δ* 8.59 (s, 2H), 7.84 (s, 1H), 7.14-7.31 (m, 5H), 3.81-4.07 (m, 5H), 3.65 (t, *J* = 5.2 Hz, 2H), 3.59 (t, *J* = 5.2 Hz, 2H), 3.56 (d, *J* = 6.4 Hz, 1H), 3.04 (t, *J* = 9.2 Hz, 1H), 2.67-2.81 (m, 3H), 2.46 (t, *J=* 7.2 Hz, 2H), 1.87-2.04 (m, 2H), 1.61-1.86 (m, 2H).

### Example 55 Synthesis of Compound 55

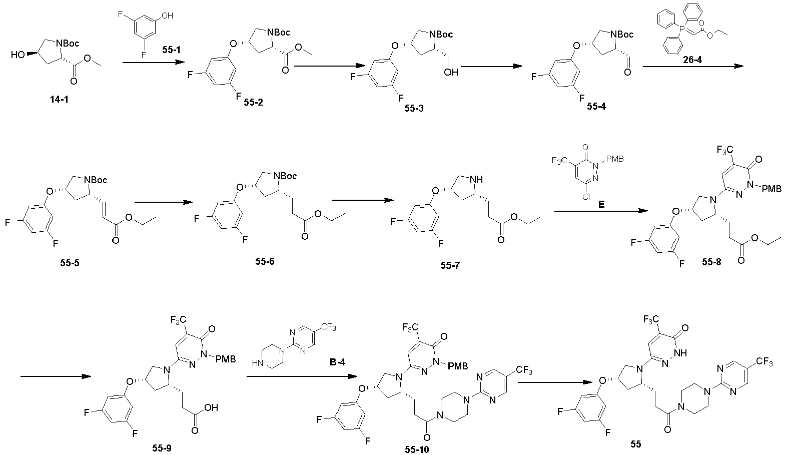

(1) A solution of compound 14-1 (4.00 g, 16.3 mmol), triphenylphosphine (4.71 g, 17.9 mmol), compound 55-1 (2.33 g, 17.9 mmol) in tetrahydrofuran (60.0 mL) was cooled to 0°C, diisopropyl azodicarboxylate (3.30 g, 16.3 mmol, 3.17 mL) was added. The reaction mixture was stirred at 25°C for 12 hours. The reaction solution was diluted with ethyl acetate (20.0 mL), washed with saturated sodium chloride (20.0 mL × 2). The organic layer was dried over anhydrous sodium sulfate, filtered and concentrated under reduced pressure. The crude product was purified by silica gel column chromatography (petroleum ether/ethyl acetate = 1:0 to 3:1) to obtain compound **55-2.** MS-ESI [M-Boc+H]⁺, calculated: 258, found: 258.¹H NMR (400 MHz, CDCl₃) *δ* 6.41-6.52 (m, 1H), 6.32 (br d, *J* = 8.4 Hz, 2H), 4.84 (br s, 1H), 4.41-4.59 (m, 1H), 3.77-3.84 (m, 1H), 3.72-3.77 (m, 4H), 2.44-2.54 (m, 2H), 1.44-1.50 (m, 9H).
(2) A solution of compound **55-2** (2.00 g, 5.60 mmol) in tetrahydrofuran (20.0 mL) was cooled to 0°C, lithium aluminum hydride (234 mg, 6.16 mmol) was added. The reaction mixture was stirred at 0°C for 1 hour. Water (0.3 mL), 15% sodium hydroxide solution (0.3 mL), water (0.9 mL) and sodium sulfate was added to the reaction solution. The mixture was stirred for 10 minutes, filtered and concentrated under reduced pressure. The crude product was purified by silica gel column chromatography (petroleum ether/ethyl acetate = 1:0 to 2:1) to obtain compound **55-3.** MS-ESI [M-^{t}Bu+H]⁺, calculated: 274, found: 274.¹H NMR (400 MHz, CDCl₃) *δ* 6.46 (tt, *J* = 8.8, 2.4 Hz, 1H), 6.38 (dd, *J* = 8.8, 2.0 Hz, 2H), 4.75-4.86 (m, 1H), 4.12-4.26 (m, 1H), 3.83-3.93 (m, 1H), 3.69-3.80 (m, 1H), 3.62 (br t, *J* = 13.2 Hz, 2H), 2.36-2.45 (m, 1H), 1.93-1.99 (m, 1H), 1.49 (s, 9H).
(3) To a solution of compound **55-3** (1.50 g, 4.55 mmol) in dichloromethane (25.0 mL), Dess-Martin reagent (2.32 g, 5.47 mmol) was added. The reaction mixture was stirred at 25°C for 6 hours. Saturated sodium sulfite solution (20.0 mL) was added, the mixture was stirred for 10 minutes. The reaction solution was extracted with ethyl acetate (20.0 mL × 2). The organic layer was combined, dried over anhydrous sodium sulfate, filtered and concentrated under reduced pressure. The crude product was purified by silica gel column chromatography (petroleum ether/ethyl acetate = 1:0 to 2:1) to obtain compound **55-4.** ¹H NMR (400 MHz, CDCl₃) *δ* 9.58-9.69 (m, 1H), 6.46 (br t, *J* = 8.8 Hz, 1H), 6.30-6.37 (m, 2H), 4.85 (br s, 1H), 4.18-4.38 (m, 1H), 3.68-3.90 (m, 2H), 2.37-2.52 (m, 2H), 1.46-1.51 (m, 9H).
(4) To a solution of compound **55-4** (1.21 g, 3.70 mmol) in dichloromethane (18.0 mL), compound **26-4** (1.55 g, 4.44 mmol) was added. The reaction mixture was stirred at 25°C for 12 hours. The reaction solution was concentrated under reduced pressure. The crude product was purified by silica gel column chromatography (petroleum ether/ethyl acetate = 1:0 to 2:1) to obtain compound **55-5.** MS-ESI [M-Boc+H]⁺, calculated: 298, found: 298.¹H NMR (400 MHz, CDCl₃) *δ* 6.94 (br dd, *J* = 15.6, 6.8 Hz, 1H), 6.44 (tt, *J* = 8.8, 2.0 Hz, 1H), 6.33-6.39 (m, 2H), 5.80-5.95 (m, 1H), 4.85-4.91 (m, 1H), 4.46-4.69 (m, 1H), 4.22 (br d, *J* = 6.8 Hz, 2H), 3.67-3.84 (m, 2H), 2.43 (br d, *J* = 2.0 Hz, 1H), 2.13-2.24 (m, 1H), 1.45 (br s, 9H), 1.31 (br t, *J* = 7.2 Hz, 3H).
(5) To a solution of compound **55-5** (1.20 g, 3.02 mmol) in tetrahydrofuran (12.0 mL), 10% palladium on carbon (1.20 g) was added. The reaction mixture was stirred under a hydrogen atmosphere (15 psi) at 25°C for 1 hour. The reaction solution was filtered and the filtrate was concentrated under reduced pressure to obtain compound **55-6.** MS-ESI [M+H]⁺, calculated: 400, found: 400.¹H NMR (400 MHz, CDCl₃) *δ* 6.35-6.48 (m, 3H), 4.82 (tt, *J* = 5.6, 2.0 Hz, 1H), 4.13 (qd, *J* = 7.2, 2.4 Hz, 2H), 3.83-4.06 (m, 2H), 3.53 (br d, *J* = 13.2 Hz, 1H), 2.27-2.39 (m, 3H), 2.04 (br s, 1H), 1.77-1.95 (m, 2H), 1.46-1.49 (m, 9H), 1.23-1.28 (m, 3H).
(6) To a solution of compound **55-6** (1.20 g, 3.00 mmol) in dichloromethane (9.0 mL), trifluoroacetic acid (4.62 g, 40.5 mmol, 3.0mL) was added. The reaction solution was stirred at 25°C for 1 hours. The reaction solution was concentrated under reduced pressure to obtain compound **55-7.** MS-ESI [M+H]⁺, calculated: 300, found: 300.¹H NMR (400 MHz, CDCl₃) *δ* 6.46-6.50 (m, 1H), 6.41 (d, *J* = 2.0 Hz, 1H), 6.39 (d, *J* = 2.0 Hz, 1H), 4.15 (s, 2H), 3.70 (br s, 1H), 3.45-3.51 (m, 2H), 2.56-2.60 (m, 3H), 2.37 (br d, *J* = 6.8 Hz, 1H), 2.13-2.18 (m, 1H), 1.83-1.94 (m, 2H), 1.26-1.28 (m, 3H)
(7) To a solution of compound **55-7** (450 mg, 1.50 mmol) and compound **E** (575 mg, 1.80 mmol) in toluene (9.0 mL), cesium carbonate (187 mg, 301 µmol), 1,1'-binaphthyl-2,2'-diphenylphosphine (125 mg, 200 µmol) and tris (dibenzylideneacetone) dipalladium (138 mg, 150 µmol) was added. The reaction solution was stirred at 80°C under nitrogen atmosphere for 6 hours. The reaction solution was diluted with ethyl acetate (10.0 mL), washed with saturated sodium chloride (10.0 mL × 2), dried over anhydrous sodium sulfate, filtered and concentrated under reduced pressure. The crude product was purified by silica gel column chromatography (petroleum ether/ethyl acetate = 1:0 to 3:1) to obtain compound **55-8.** MS-ESI [M+H]⁺, calculated: 582, found: 582.
(8) Compound **55-8** (100 mg, 172 µmol) was dissolved in tetrahydrofuran (3.0 mL) and water (1.0 mL). Lithium hydroxide monohydrate (72.2 mg, 1.72 mmol) was added. The reaction solution was stirred at 25°C for 12 hours. It was adjusted the pH to 5 with 1 mol/L hydrochloric acid solution, diluted with ethyl acetate (10.0 mL), and washed with saturated sodium chloride (10.0 mL × 2). The organic phase was dried over anhydrous sodium sulfate, filtered and concentrated under reduced pressure to obtain compound **55-9.** MS-ESI [M+H]⁺, calculated: 554, found: 554.¹H NMR (400 MHz, CDCl₃) *δ* 7.51-7.58 (m, 1H), 7.44 (d, *J* = 8.8 Hz, 2H), 6.85 (d, *J* = 8.8 Hz, 2H), 6.46-6.51 (m, 1H), 6.42 (dd, *J* = 8.8, 2.0 Hz, 2H), 5.09-5.23 (m, 2H), 4.98 (br s, 1H), 4.00-4.07 (m, 1H), 3.80 (s, 3H), 3.70-3.74 (m, 2H), 2.46-2.53 (m, 1H), 2.34-2.38 (m, 1H), 2.15-2.24 (m, 2H), 1.62-1.67 (m, 2H).
(9) To a solution of compound **55-9** (70.0 mg, 126.6 µmol), **B-4** (35.2 mg, 152 µmol) in dichloromethane (6.0 mL), diisopropylethylamine (81.7 mg, 632 µmol, 110 µL) and 50% propylphosphonic anhydride (241 mg, 379 µmol, 226 µL) was added and stirred for 5 hours at 25°C. The reaction solution was diluted with ethyl acetate (10.0 mL), washed with saturated sodium chloride (10.0 mL × 2), dried with anhydrous sodium sulfate, filtered and concentrated under reduced pressure. The crude product was purified by silica gel column chromatography (petroleum ether/ethyl acetate = 1:0 to 1:1) to obtain compound **55-10.** MS-ESI [M+H]⁺, calculated: 768, found: 768.¹H NMR (400 MHz, CDCl₃) *δ* 8.53 (s, 2H), 7.97 (s, 1H), 7.45 (d, *J* = 8.8 Hz, 2H), 6.86 (d, *J* = 8.4 Hz, 2H), 6.38-6.45 (m, 3H), 5.16 (d, *J* = 15.2 Hz, 2H), 4.99 (br s, 1H), 4.12 (s, 1H), 3.92 (br s, 4H), 3.79 (s, 3H), 3.74 (br d, *J* = 1.6 Hz, 2H), 3.49 (br d, *J* = 6.8 Hz, 2H), 2.42-2.51 (m, 1H), 2.30-2.37 (m, 2H), 2.20-2.23 (m, 1H), 2.16-2.19 (m, 1H), 2.12-2.16 (m, 1H), 1.92-1.98 (m, 1H), 1.29 (br d, *J* = 1.6 Hz, 1H).
(10) Compound **55-10** (80.0 mg, 104 µmol) was dissolved in trifluoroacetic acid (5.0 mL) and stirred at 70 °C for 3 hours. The reaction solution was cooled to room temperature, adjusted to pH>8 with saturated sodium bicarbonate solution, extracted with ethyl acetate (10.0 mL× 2). The combined organic layer was washed with saturated sodium chloride (10.0 mL), dried over anhydrous sodium sulfate, filtered and concentrated under reduced pressure. The crude product was purified by preparative high performance liquid chromatography (C18-6, 100 mm × 30 mm 5 µm, A: water (0.225% formic acid); B: acetonitrile, 55%-85%: 8 minutes) to obtain compound **55** formate. MS-ESI [M+H]⁺, calculated: 648, found: 648.¹H NMR (400 MHz, MeOD) *δ* 12.6 (s, 1H), 8.73 (s, 2H), 8.10 (s, 1H), 6.75-6.83 (m, 3H), 5.17 (br t, *J* = 4.8 Hz, 1H), 3.99 (br t, *J* = 8.8 Hz, 1H), 3.82 (br dd, *J* = 12.4, 6.0 Hz, 4H), 3.69-3.74 (m, 1H), 3.52-3.61 (m, 5H), 2.32-2.44 (m, 3H), 2.11 (br d, *J* = 14.0 Hz, 1H), 1.94-2.02 (m, 1H), 1.72-1.80 (m, 1H).

### Example 56 Synthesis of Compound 56

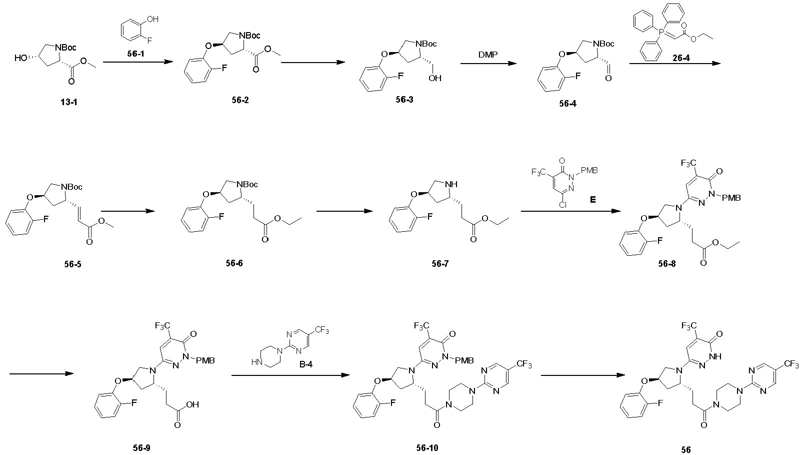

(1) A solution of compound **13-1** (3.00 g, 12.2 mmol), triphenylphosphine (3.53 g, 13.5 mmol) and compound **56-1** (1.51 g, 13.5 mmol, 1.25 mL) in tetrahydrofuran (50.0 mL) was cooled to 0°C, diisopropyl azodicarboxylate (2.72 g, 13.5 mmol, 2.62 mL) was added and stirred at 25°C for 12 hours. The reaction solution was diluted with ethyl acetate (30.0 mL), washed with saturated sodium chloride (30.0 mL × 2). The organic layer was dried over anhydrous sodium sulfate, filtered and concentrated under reduced pressure. The crude product was purified by silica gel chromatography (petroleum ether/ethyl acetate = 1:0 to 0:1) to obtain compound **56-2.** MS-ESI [M-Boc+H]⁺, calculated: 240, found: 240.¹H NMR (400 MHz, CDCl₃) *δ* 7.01-7.14 (m, 2H), 6.89-7.01 (m, 2H), 4.91 (br d, *J* = 2.8 Hz, 1H), 4.44-4.59 (m, 1H), 3.73-3.87 (m, 5H), 2.50-2.65 (m, 1H), 2.14-2.27 (m, 1H), 1.41-1.47 (m, 9H).
(2) A solution of compound **56-2** (1.69 g, 4.98 mmol) in tetrahydrofuran (20.0 mL) was cooled to 0°C, lithium aluminum hydride (2.21 g, 5.48 mmol) was added. The reaction solution was stirred at 0°C for 1 hour. Water (0.25 mL), 15% NaOH solution (0.25 mL), water (0.75 mL), sodium sulfate was added to the reaction solution, stirred for 10 minutes, filtered and concentrated. The crude product was purified by silica gel chromatography (petroleum ether/ethyl acetate = 1:0 to 0:1) to obtain compound **56-3.** ¹H NMR (400 MHz, CDCl₃) *δ* 7.02-7.13 (m, 2H), 6.90-7.02 (m, 2H), 4.82 (br s, 1H), 4.17-4.28 (m, 1H), 3.73-3.88 (m, 2H), 3.60 (dd, *J* = 11.6, 6.8 Hz, 1H), 3.51 (dd, *J* = 12.8, 4.0 Hz, 1H), 2.34 (ddt, *J* = 13.6, 7.2, 2.0 Hz, 1H), 1.75-1.91 (m, 1H), 1.47 (s, 9H).
(3) To a solution of compound **56-3** (1.03 g, 3.46 mmol) in dichloromethane (15.0 mL), Dess-Martin reagent (1.62 g, 3.81 mmol, 1.18 mL) was added. The reaction solution was stirred at 25°C for 3 hours. Dichloromethane (10.0 mL) and saturated sodium sulfite solution (20.0 mL) was added, stirred for 10 minutes and extracted with dichloromethane (30.0 mL × 2). The organic layer was dried over anhydrous sodium sulfate, filtered and concentrated under reduced pressure. The crude product was purified by silica gel chromatography (petroleum ether/ethyl acetate = 1:0 to 0:1) to obtain compound **56-4.** ¹H NMR (400 MHz, CDCl₃) *δ* 9.44-9.64 (m, 1H), 7.03-7.14 (m, 2H), 6.90-7.02 (m, 2H), 4.91 (br s, 1H), 4.30-4.54 (m, 1H), 3.75-3.97 (m, 1H), 3.67 (dd, *J* = 12.4, 4.0 Hz, 1H), 2.31-2.49 (m, 1H), 2.05-2.13 (m, 1H), 1.42-1.50 (m, 9H).
(4) To a solution of compound **56-4** (700 mg, 2.26 mmol) in dichloromethane (10.0 mL), compound **26-4** (946 mg, 2.72 mmol) was added. The reaction solution was stirred at 25°C for 12 hours. The reaction solution was concentrated under reduced pressure. The crude product was purified by silica gel chromatography (petroleum ether/ethyl acetate = 1:0 to 2:1) to obtain compound **56-5.** MS-ESI [M-Boc+H]⁺, calculated: 280, found: 280.¹H NMR (400 MHz, CDCl₃)*δ* 7.02-7.15 (m, 2H), 6.91-7.01 (m, 2H), 6.85 (br s, 1H), 5.92 (br d, *J* = 15.2 Hz, 1H), 4.82-4.87 (m, 1H), 4.56-4.76 (m, 1H), 4.21 (br d, *J* = 6.0 Hz, 2H), 3.74-3.96 (m, 1H), 3.59 (dd, *J* = 12.4, 4.4 Hz, 1H), 2.48 (br d, *J* = 1.6 Hz, 1H), 1.99-2.05 (m, 1H), 1.44 (s, 9H), 1.28-1.32 (m, 3H).
(5) To a solution of compound **56-5** (1.00 g, 2.64 mmol) in tetrahydrofuran (12.0 mL), 10% palladium on carbon (0.5 g) was added. The reaction solution was stirred under a hydrogen atmosphere (15 psi) at 25°C for 1 hour. The reaction solution was filtered, and the filtrate was concentrated under reduced pressure to obtain compound **56-6.** MS-ESI [M-Boc+H]⁺, calculated: 282, found: 282.¹H NMR (400 MHz, CDCl₃) *δ* 7.03-7.11 (m, 2H), 6.92-6.99 (m, 2H), 4.84 (br s, 1H), 4.09-4.19 (m, 3H), 3.80-3.95 (m, 1H), 3.45 (br dd, *J* = 12.4, 4.4 Hz, 1H), 2.28-2.38 (m, 3H), 2.12-2.20 (m, 1H), 1.83-1.94 (m, 2H), 1.47 (s, 9H), 1.26 (t, *J* = 6.4 Hz, 3H).
(6) To a solution of compound **56-6** (800 mg, 2.10 mmol) in dichloromethane (6.0 mL), trifluoroacetic acid (3.08 g, 27.0 mmol, 2.0 mL) was added. The reaction solution was stirred at 25°C for 1 hour. The reaction solution was adjusted to pH 7 with saturated sodium bicarbonate solution, diluted with ethyl acetate (10.0 mL), and washed with saturated sodium chloride (10.0 mL × 2). The organic layer was dried over anhydrous sodium sulfate, filtered and concentrated under reduced pressure to obtain compound **56-7.** ¹H NMR (400 MHz, CDCl₃) *δ* 7.05-7.15 (m, 2H), 6.95-7.05 (m, 2H), 4.08-4.18 (m, 2H), 4.00 (br dd, *J* = 13.2, 5.6 Hz, 1H), 3.75-3.86 (m, 1H), 3.58 (br d, *J* = 12.8 Hz, 1H), 2.53-2.59 (m, 2H), 2.45-2.50 (m, 2H), 2.21-2.29 (m, 1H), 2.10-2.20 (m, 1H), 1.98-2.08 (m, 1H), 1.44 (s, 1H), 1.21-1.29 (m, 3H).
(7) To a solution of compound **56-7** (300 mg, 1.07 mmol) and compound **E** (408 mg, 1.28 mmol) in toluene (6.0 mL), cesium carbonate (695 mg, 2.13 mmol), 1,1'-binaphthyl-2,2'-diphenylphosphine (133 mg, 213 µmol) and tris (dibenzylideneacetone) dipalladium (97.7 mg, 107 µmol). The reaction solution was stirred at 80°C under nitrogen atmosphere for 6 hours. The reaction solution was diluted with ethyl acetate (10.0 mL), washed with saturated sodium chloride (10.0 mL × 2), dried over anhydrous sodium sulfate, filtered and concentrated under reduced pressure. The crude product was purified by silica gel column chromatography (petroleum ether/ethyl acetate = 1:0 to 3:1) to obtain compound **56-8.** MS-ESI [M+H]⁺, calculated: 564, found: 564.
(8) Compound **56-8** (100 mg, 177 µmol) was dissolved in tetrahydrofuran (3.0 mL) and water (1.0 mL), lithium hydroxide monohydrate (74.5 mg, 1.77 mmol) was added. The reaction solution was stirred at 25°C for 12 hours. It was adjusted pH to 5 with 1 mol/L hydrochloric acid solution, diluted with ethyl acetate (10.0 mL), and washed with saturated sodium chloride (10.0 mL × 2). The organic phase was dried with anhydrous sodium sulfate, filtered and concentrated under reduced pressure to obtain compound **56-9.** MS-ESI [M+H]⁺, calculated: 536, found: 536.¹H NMR (400 MHz, CDCl₃) *δ*7.42 (d, *J* = 8.4 Hz, 2H), 7.34 (s, 1H), 7.06-7.10 (m, 2H), 6.99-7.01 (m, 1H), 6.92 (d, *J* = 1.6 Hz, 1H), 6.85 (d, *J* = 8.4 Hz, 2H), 5.27 (d, *J* = 13.2 Hz, 1H), 4.99-5.11 (m, 2H), 4.13 (d, *J* = 7.2 Hz, 1H), 3.79 (s, 3H), 3.76 (d, *J* = 4.4 Hz, 1H), 3.65-3.69 (m, 1H), 2.37 (d, *J* = 7.2 Hz, 2H), 2.11 (s, 1H), 2.06 (s, 2H), 1.46-1.48 (m, 1H).
(9) To a solution of compound **56-9** (70.0 mg, 131 µmol) and compound **B-4** trifluoroacetate (36.4 mg, 157 µmol) in dichloromethane (5.0 mL), diisopropylethylamine (84.5 mg, 654 µmol, 114 µL) and propylphosphonic anhydride solution (125 mg, 392 µmol, 117 µL, 50% purity) was added. The reaction was stirred at 25°Cfor 5 hours. The reaction was diluted with dichloromethane (10.0 mL), washed with water (5.0 mL × 2) and saturated sodium chloride (5.0 mL × 2), dried over anhydrous sodium sulfate, filtered and concentrated under reduced pressure. The crude product was purified by silica gel column chromatography (petroleum ether/ethyl acetate = 1:0 to 1:1) to obtain compound **56-10.** MS-ESI [M+H]⁺, calculated: 750, found: 750.
(10) A solution of compound **56-10** (30.0 mg, 40.0 µmol) in trifluoroacetic acid (3.0 mL) was stirred at 70°C for 4 hours. The reaction solution was concentrated under reduced pressure. The crude product was purified by preparative high performance liquid chromatography (C18-6, 100 mm × 30 mm 5 µm, A: water (0.225% formic acid); B: acetonitrile, 55%-85%: 8 minutes) to obtain compound **56** formate. MS-ESI [M+H]⁺, calculated: 630, found: 630.¹H NMR (400 MHz, MeOD) *δ* 12.62 (br s, 1H), 8.73 (s, 2H), 7.91 (s, 1H), 7.12-7.25 (m, 3H), 6.94-6.99 (m, 1H), 5.16 (br s, 1H), 4.10 (br d, *J* = 6.4 Hz, 1H), 3.79-3.88 (m, 5H), 3.56 (br d, *J* = 3.6 Hz, 5H), 2.42 (br t, *J* = 6.8 Hz, 2H), 2.28-2.34 (m, 1H), 2.18-2.25 (m, 1H), 2.00 (br d, *J* = 7.6 Hz, 1H), 1.56-1.65 (m, 1H).

### Example 57 Synthesis of Compound 57

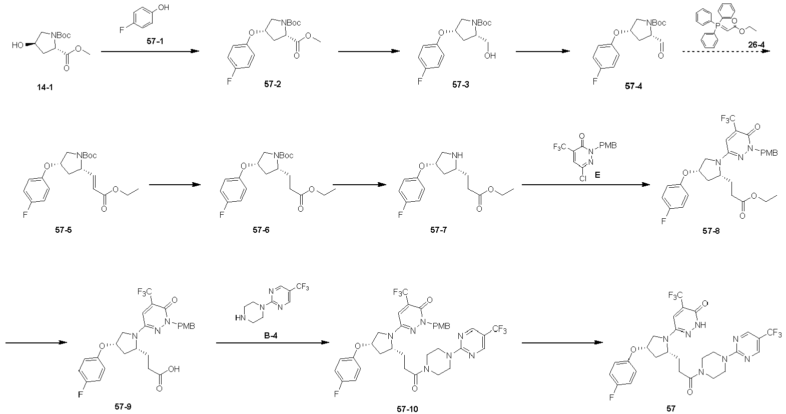

(1) A solution of compound **14-1** (3.00 g, 12.2 mmol), triphenylphosphine (3.53 g, 13.5 mmol) and compound **57-1** (1.51 g, 13.5 mmol, 1.25 mL) in tetrahydrofuran (80.0 mL) was cooled to 0°C, diisopropyl azodicarboxylate (2.47 g, 12.2 mmol, 2.38 mL) was added, and stirred at 25°C for 12 hours. Water (50.0 mL) was added to the reaction solution, followed by extraction with ethyl acetate (400.0 mL). The organic layer was dried over anhydrous sodium sulfate, filtered and concentrated under reduced pressure. The crude product was purified by silica gel chromatography (petroleum ether/ethyl acetate = 1:0 to 4:1) to obtain compound **57-2.** MS-ESI [M-^{t}Bu+H]⁺, calculated: 284, found: 284.¹H NMR (400 MHz, CDCl₃) *δ* 6.93-7.03 (m, 2H), 6.71-6.80 (m, 2H), 4.84 (br dd, *J* = 4.8, 1.6 Hz, 1H), 4.40-4.59 (m, 1H), 3.63-3.79 (m, 5H), 2.37-2.50 (m, 2H), 1.43-1.49 (m, 9H).
(2) A solution of compound **57-2** (2.00 g, 5.89 mmol) in tetrahydrofuran (20.0 mL) was cooled to 0°C, lithium aluminum hydride (447 mg, 11.8 mmol) was added. The reaction solution was reacted at 0°C for 1 hour. Water (0.5 mL), 15% sodium hydroxide solution (0.5 mL), water (1.5 mL), sodium sulfate was added, stirred for 10 minutes, filtered and concentrated to obtain compound **57-3.** MS-ESI [M-^{t}Bu+H]⁺, calculated: 256, found: 256.¹H NMR (400 MHz, CDCl₃) *δ* 6.95-7.03 (m, 2H), 6.76-6.84 (m, 2H), 4.76-4.83 (m, 1H), 4.16 (br s, 1H), 3.90 (br dd, *J* = 10.8, 8.0 Hz, 1H), 3.56-3.75 (m, 3H), 2.36 (ddd, *J* = 14.4, 9.2, 5.2 Hz, 1H), 2.01 (br s, 1H), 1.49 (s, 9H).
(3) To a solution of compound **57-3** (1.65 g, 5.30 mmol) in dichloromethane (18.0 mL), Dess-Martin reagent (2.70 g, 6.36 mmol) was added. The reaction solution was stirred at 25°C for 12 hours. Saturated sodium sulfite solution (80.0 mL) was added, stirred for 10 minutes, extracted with dichloromethane (300 mL). The organic layer was combined, dried over anhydrous sodium sulfate, filtered and concentrated under reduced pressure. The crude product was purified by silica gel column chromatography (petroleum ether/ethyl acetate = 1:0 to 78:22) to obtain compound **57-4.** MS-ESI [M-^{t}Bu+H]⁺, calculated: 254, found: 254.¹H NMR (400 MHz, CDCl₃) *δ* 9.62-9.71 (m, 1H), 6.92-7.03 (m, 2H), 6.72-6.81 (m, 2H), 4.84 (t, *J* = 3.2 Hz, 1H), 4.15-4.36 (m, 1H), 3.87 (d, *J* = 12.4 Hz, 1H), 3.61-3.70 (m, 1H), 2.26-2.48 (m, 2H), 1.44-1.54 (m, 9H).
(4) To a solution of compound **57-4** (1.56 g, 5.04 mmol) in dichloromethane (12.0 mL), compound **26-4** (2.11 g, 6.05 mmol) was added. The reaction solution was stirred at 25°C for 12 hours. The reaction solution was concentrated under reduced pressure. The crude product was purified by silica gel column chromatography (petroleum ether/ethyl acetate = 1:0 to 3:1) to obtain compound **57-5.** MS-ESI [M-Boc+H]⁺, calculated: 280, found: 280.¹H NMR (400 MHz, CDCl₃)*δ* 6.92-7.06 (m, 3H), 6.74-6.86 (m, 2H), 5.80-5.97 (m, 1H), 4.82-4.94 (m, 1H), 4.44-4.71 (m, 1H), 4.22 (br d, *J* = 5.6 Hz, 2H), 3.60-3.84 (m, 2H), 2.41 (br s, 1H), 2.16 (br d, *J* = 12.8 Hz, 1H), 1.45 (br s, 9H), 1.27-1.35 (m, 3H).
(5) 10% palladium on carbon (0.20 g) was added to a solution of compound **57-5** (1.58 g, 4.16 mmol) in tetrahydrofuran (20.0 mL). The reaction solution was stirred under a hydrogen atmosphere (15 psi) at 25°C for 1 hour, and filtered. The filtrate was concentrated under reduced pressure to obtain compound **57-6.** MS-ESI [M+H]⁺, calculated: 382, found: 382.¹H NMR (400 MHz, CDCl₃) *δ* 6.98 (t, *J* = 8.0 Hz, 2H), 6.77-6.84 (m, 2H), 4.76-4.86 (m, 1H), 4.12 (q, *J* = 7.2 Hz, 2H), 3.73-4.04 (m, 2H), 3.53 (br d, *J* = 12.4 Hz, 1H), 2.17-2.40 (m, 4H), 1.88-2.05 (m, 2H), 1.48 (s, 9H), 1.25 (t, *J* = 7.2 Hz, 3H).
(6) To a solution of compound **57-6** (1.50 g, 3.93 mmol) in dichloromethane (15.0 mL), trifluoroacetic acid (7.70 g, 67.5 mmol, 5.0 mL) was added. The reaction solution was stirred at 25°C for 0.5 hour. The pH of the reaction solution was adjusted to >7 with saturated sodium carbonate solution. The reaction solution was extracted with dichloromethane (200.0 mL). The organic layer was dried over anhydrous sodium sulfate, filtered and concentrated under reduced pressure to obtain compound **57-7.** MS-ESI [M+H]⁺, calculated: 282, found: 282.¹H NMR (400 MHz, CDCl₃) *δ* 6.98-7.08 (m, 2H), 6.77-6.90 (m, 2H), 5.01 (br t, *J* = 4.8 Hz, 1H), 4.13-4.23 (m, 2H), 4.04 (br dd, *J* = 5.6, 2.4 Hz, 1H), 3.59-3.66 (m, 1H), 2.62-2.70 (m, 2H), 2.24-2.40 (m, 2H), 2.12-2.19 (m, 3H), 1.28 (t, *J* = 7.2 Hz, 3H).
(7) To a solution of compound **57-7** (1.00 g, 3.55 mmol) and compound **E** (1.70 g, 5.33 mmol) in toluene (20.0 mL), cesium carbonate (326 mg, 355 µmol), 1,1'-binaphthyl-2,2'-diphenylphosphine (443 mg, 711 µmol) and tris (dibenzylideneacetone) dipalladium (326 mg, 355 µmol) was added. The reaction solution was stirred at 80°C under nitrogen atmosphere for 5 hours. It was concentrated under reduced pressure. The crude product was purified by silica gel column chromatography (petroleum ether/ethyl acetate = 1:0 to 4:1) to obtain compound **57-8.** MS-ESI [M+H]⁺, calculated: 564, found: 564.¹H NMR (400 MHz, CDCl₃) *δ* 7.72-7.90 (m, 1H), 7.51 (s, 1H), 7.45 (d, *J* = 8.4 Hz, 2H), 6.98-7.07 (m, 2H), 6.83-6.87 (m,3H), 5.09-5.21 (m, 2H), 4.97 (br t, *J* = 4.8 Hz, 1H), 4.15 (q, *J* = 7.2 Hz, 2H), 3.98-4.07 (m, 1H), 3.79 (s, 3H), 3.66-3.75 (m, 2H), 2.36-2.44 (m, 1H), 2.23-2.34 (m, 2H), 2.08-2.22 (m, 2H), 1.94-2.03 (m, 1H), 1.24-1.28 (m, 3H).
(8) To a solution of compound **57-8** (490 mg, 870 µmol) in tetrahydrofuran (3.0 mL) and water (1.0 mL), lithium hydroxide monohydrate (292 mg, 6.96 mmol) was added to the solution. The reaction was stirred at 25 °C for 12 hours. Hydrochloric acid aqueous solution (1 mol/L) was added to the reaction solution to adjust the pH < 7, followed by extraction with dichloromethane (100.0 mL). The organic phase was dried with anhydrous sodium sulfate, filtered and concentrated under reduced pressure to obtain compound **57-9.** MS-ESI [M+H]⁺, calculated: 536, found: 536.
(9) To a solution of compound **57-9** (350 mg, 654 µmol), **B-4** (182 mg, 784 µmol) in dichloromethane (5.0 mL), diisopropylethylamine (422 mg, 3.27 mmol, 569 µL) and 50% propylphosphonic anhydride (624 mg, 1.96 mmol, 583 µL) was added. The reaction solution was stirred at 25°C for 4 hours, diluted with water (20.0 mL) and extracted with dichloromethane (10.0 mL). The organic layer was dried over anhydrous sodium sulfate, filtered and concentrated under reduced pressure. The crude product was purified by silica gel chromatography (petroleum ether/ethyl acetate = 1:0 to 7:3) to obtain compound **57-10.** MS-ESI [M+H]⁺, calculated: 750, found: 750.¹H NMR (400 MHz, CDCl₃) *δ* 8.53 (s, 2H), 7.90 (s, 1H), 7.45 (d, *J* = 8.8 Hz, 2H), 7.00-7.05 (m, 2H), 6.81-6.87 (m, 4H), 5.10-5.21 (m, 2H), 4.98 (br d, *J* = 2.4 Hz, 1H), 4.12 (br s, 1H), 3.88-3.94 (m, 4H), 3.79 (s, 3H), 3.73 (br s, 4H), 3.45-3.51 (m, 2H), 2.41-2.49 (m, 1H), 2.27-2.36 (m, 2H), 2.20 (br d, *J* = 14.4 Hz, 2H), 2.02 (br d, *J* = 2.4 Hz, 1H).
(10) Compound **57-10** (200 mg, 267 µmol) was dissolved in trifluoroacetic acid (3.0 mL) and stirred at 70 °C for 2 hours. The reaction solution was cooled to room temperature, adjusted pH > 7 with saturated sodium carbonate, and extracted with dichloromethane (50.0 mL). The organic layer was dried over anhydrous sodium sulfate, filtered and concentrated under reduced pressure. The crude product was purified by preparative high performance liquid chromatography (Xtimate C18, 100 mm × 30 mm, 10 µm, A: water (0.225% formic acid); B: acetonitrile, 60%-90%: 10 minutes) to obtain compound **57** formate. MS-ESI [M+H]⁺, calculated: 630, found: 630.¹H NMR (400 MHz, MeOD) *δ* 8.60 (s, 2H), 7.95 (s, 1H), 7.00-7.06 (m, 2H), 6.92-6.97 (m, 2H), 5.07-5.10 (m, 1H), 4.10-4.16 (m, 1H), 3.89-3.94 (m, 4H), 3.72-3.78 (m, 2H), 3.67 (br d, *J* = 4.4 Hz, 2H), 3.56-3.60 (m, 2H), 2.48-2.54 (m, 2H), 2.36-2.43 (m, 1H), 2.22-2.27 (m, 1H), 2.12-2.19 (m, 1H), 1.99-2.06 (m, 1H).

### Example 58 Synthesis of Compound 58

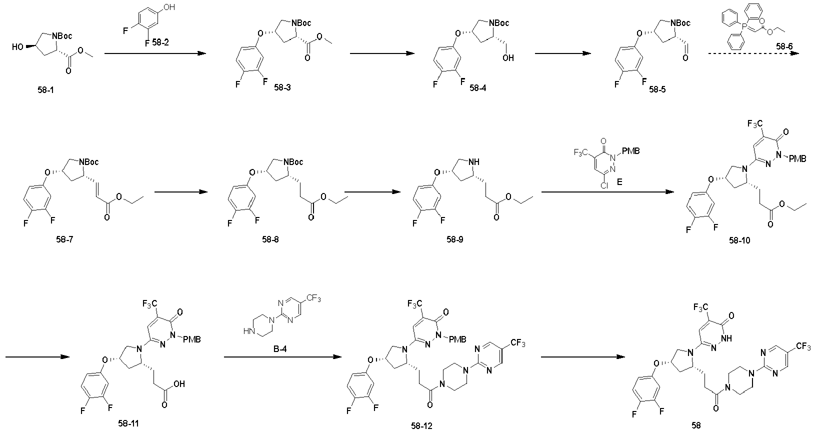

(1) At 0°C, to a solution of compound **58-1** (5.66 g, 23.0 mmol) in tetrahydrofuran (20.0 mL), compound **58-2** (3.00 g, 23.0 mmol), triphenylphosphine (18.1 g, 69.1 mmol), diisopropyl azodicarboxylate (4.66 g, 23.0 mmol, 4.48 mL) was added. The reaction solution was stirred at 25 °C for 12 hours. Water (50.0 mL) was added, followed by extraction with ethyl acetate (400 mL). The organic layer was dried with anhydrous sodium sulfate, filtered and concentrated under reduced pressure. The crude product was purified by silica gel column chromatography (petroleum ether/ethyl acetate = 1:0 to 10:1) to obtain compound **58-3.** MS-ESI [M-Boc+H]⁺, calculated: 258, found: 258.¹H NMR (400 MHz, CDCl₃) *δ* 7.06 (qd, *J* = 9.2, 4.8 Hz, 1H), 6.59-6.67 (m, 1H), 6.47-6.54 (m, 1H), 4.78-4.85 (m, 1H), 4.56 (dd, *J* = 8.8, 2.4 Hz, 0.5H), 4.43 (dd, *J* = 7.6, 4.0 Hz, 0.5H), 3.66-3.81 (m, 5H), 2.40-2.52 (m, 2H), 1.43-1.50 (m, 9H).
(2) To a solution of compound **58-3** (8.00 g, 22.4 mmol) in tetrahydrofuran (40.0 mL), lithium aluminum hydride (1.70 g, 44.7 mmol) was added at 0°C. The reaction solution was stirred under nitrogen atmosphere at 0°C for 1 hour. Water (1.0 mL), 15% sodium hydroxide (1.0 mL) and water (3.0 mL) was added at 0°C. The mixture was dried over anhydrous sodium sulfate, filtered, and concentrated under reduced pressure to obtain compound **58-4.** MS-ESI [M-Boc+H]⁺, calculated: 230, found: 230.
(3) To a solution of compound **58-4** (3.50 g, 10.6 mmol) in dichloromethane (70.0 mL), Dess-Martin reagent (4.51 g, 10.6 mmol) was added. The reaction solution was stirred at 25°C for 12 hours. The reaction solution was quenched with sodium sulfite solution (80.0 mL) and extracted with dichloromethane (30.0 mL). The organic layer was dried over anhydrous sodium sulfate, filtered, and concentrated under reduced pressure. The crude product was purified by silica gel column chromatography (petroleum ether/ethyl acetate = 1:0 to 5:1) to obtain compound **58-5.** MS-ESI [M-^{t}Bu+H]⁺, calculated: 272, found: 272.
(4) To a solution of compound **58-5** (1.15 g, 3.51 mmol) in dichloromethane (12.0 mL), compound **58-6** (1.47 g, 4.22 mmol) was added. The reaction solution was stirred at 25°C for 12 hours. The solution was dried over anhydrous sodium sulfate, filtered, concentrated under reduced pressure, and purified by silica gel column chromatography (petroleum ether/ethyl acetate = 1:0 to 7:1) to obtain compound **58-7.** MS-ESI [M-Boc+H]⁺, calculated: 298, found: 298.
(5) To a solution of compound **58-7** (1.14 g, 2.87 mmol) in tetrahydrofuran (11.0 mL), 10% palladium on carbon (1.14 g) was added. The reaction mixture was stirred under a hydrogen atmosphere at 25 °C for 1 hour, and filtered. The filtrate was concentrated under reduced pressure to obtain compound **58-8.** MS-ESI [M-Boc+H]⁺, calculated: 300, found: 300.¹H NMR (400 MHz, CDCl₃) *δ* 7.06 (q, *J* = 9.2 Hz, 1H), 6.68 (ddd, *J* = 11.6, 6.4, 3.2 Hz, 1H), 6.52-6.59 (m, 1H), 4.75-4.82 (m, 1H), 4.07-4.18 (m, 2H), 3.98 (br s, 1H), 3.71-3.90 (m, 1H), 3.51 (d, *J* = 12.4 Hz, 1H), 2.32 (br d, *J* = 7.6 Hz, 2H), 2.25-2.29 (m, 1H), 2.18 (br s, 1H), 2.00 (br d, *J* = 14.0 Hz, 1H), 1.85-1.95 (m, 1H), 1.47 (s, 9H), 1.27 (s, 3H).
(6) To a solution of compound **58-8** (900 mg, 2.25 mmol) in dichloromethane (12.0 mL), trifluoroacetic acid (4.86 g, 42.6 mmol, 3.16 mL) was added. The reaction solution was stirred at 25°C for 0.5 hours. It was adjusted to pH > 7 with sodium carbonate, extracted with dichloromethane (200 mL). The organic phase was dried over anhydrous sodium sulfate, filtered and concentrated under reduced pressure to obtain compound **58-9.** MS-ESI [M+H]⁺, calculated: 300, found: 300.
(7) To a solution of compound **58-9** (447 mg, 1.40 mmol) in toluene (7.0 mL) compound E (210 mg, 661 µmol), cesium carbonate (761 mg, 2.34 mmol), 1,1'-binaphthyl-2,2'-diphenylphosphine (145 mg, 233 µmol) and tris (dibenzylideneacetone) dipalladium (107 mg, 116.3 µmol) was added. The reaction solution was stirred at 80°C under nitrogen atmosphere for 10 hours. The reaction solution was dried over anhydrous sodium sulfate, filtered and concentrated under reduced pressure. The crude product was purified by silica gel column chromatography (petroleum ether/ethyl acetate = 1:0 to 3:1) to obtain compound **58-10.** MS-ESI [M+H]⁺, calculated: 582, found: 582.
(8) To the solution of compound **58-10** (188 mg, 323 µmol) in tetrahydrofuran (6.0 mL), lithium hydroxide monohydrate (135 mg, 3.23 mmol) and water (2.0 mL) was added. The reaction was stirred at 25 °C under nitrogen atmosphere for 12 hours. Hydrochloric acid aqueous solution (1 mol/L) was added to the reaction solution to adjust the pH < 7, followed by extraction with dichloromethane (100 mL). The organic phase was dried with anhydrous sodium sulfate, filtered and concentrated under reduced pressure to obtain compound **58-11.** MS-ESI [M+H]⁺, calculated: 554, found: 554.
(9) To a solution of compound **58-11** (85.5 mg, 368 µmol) in dichloromethane (10.0 mL), compound **B-4** trifluoroacetate (170 mg, 307 µmol), diisopropylethylamine (119 mg, 921 µmol) and a solution of propylphosphonic anhydride in ethyl acetate (586 mg, 921 µmol, 50% purity) was added. The reaction was stirred at 25°C under nitrogen atmosphere for 4 hours. The reaction was then quenched with water (30.0 mL), extracted with dichloromethane (150 mL), dried over anhydrous sodium sulfate, filtered and concentrated under reduced pressure. The crude product was purified by silica gel column chromatography (petroleum ether/ethyl acetate = 1:0 to 2:1) to obtain compound **58-12.**
(10) Compound **58-12** (64.0 mg, 83.3 µmol) was dissolved in trifluoroacetic acid (10.0 mL), and stirred at 70°C under nitrogen atmosphere for 2 hours. The reaction solution was filtered and concentrated under vacuum. And the pH was adjusted to > 7 with sodium carbonate, followed by extraction with dichloromethane (50.0 mL). The organic phase was dried over anhydrous sodium sulfate, filtered, and concentrated under reduced pressure. The crude product was purified by preparative high performance liquid chromatography (Xtimate C18, 100 mm × 30 mm × 5 µm, A: water (0.225% formic acid); B: acetonitrile, 52%-82%: 15 minutes) to obtain compound **58** formate. MS-ESI [M+H]⁺, calculated: 648, found: 648.¹H NMR (400 MHz, MeOD) *δ* 8.59 (s, 2H), 7.96 (s, 1H), 7.14-7.24 (m, 1H), 6.92 (ddd, *J* = 12.4, 6.4, 3.2 Hz, 1H), 6.72-6.78 (m, 1H), 5.09 (t, *J* = 4.8 Hz, 1H), 4.10-4.16 (m, 1H), 3.92 (dt, *J* = 10.0, 5.2 Hz, 4H), 3.76-3.81 (m, 1H), 3.71-3.75 (m, 1H), 3.67 (t, *J* = 5.6 Hz, 2H), 3.59 (t, *J* = 5.2 Hz, 2H), 2.51 (td, *J* = 7.2, 3.6 Hz, 2H), 2.41 (ddd, *J* = 14.0, 8.4, 5.6 Hz, 1H), 2.24 (br d, *J* = 13.6 Hz, 1H), 2.12-2.20 (m, 1H), 1.95-2.03 (m, 1H).

### Example 59 Synthesis of Compound 59

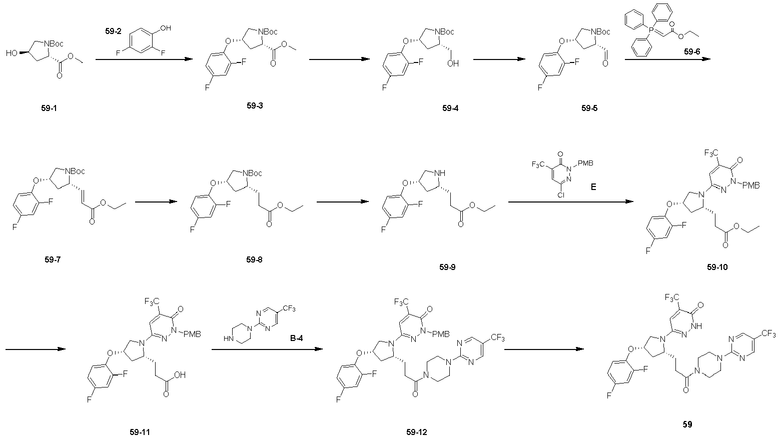

(1) At 0°C, to a solution of compound **59-1** (5.0 g, 20.4 mmol) in tetrahydrofuran (50.0 mL), compound **59-2** (2.65 g, 20.4 mmol), triphenylphosphine (8.02 g, 30.6 mmol), diisopropyl azodicarboxylate (4.12 g, 20.4 mmol) was added. The reaction solution was stirred at 25 °C for 12 hours. Then water (20.0 mL) was added and the reaction solution was extracted with ethyl acetate (20.0 mL × 3). The organic layer was washed with saturated sodium chloride solution (20.0 mL × 3), dried with anhydrous sodium sulfate, filtered and concentrated under reduced pressure. The crude product was purified by silica gel column chromatography (petroleum ether/ethyl acetate = 5:1) to obtain compound **59-3.** MS-ESI [M-^{t}Bu+H]⁺, calculated: 302, found: 302.
(2) At 0°C, to a solution of compound **59-3** (2.5 g, 7.0 mmol) in tetrahydrofuran (25.0 mL), lithium aluminum hydride (531 mg, 14.0 mmol) was added. The reaction solution was stirred at 0 °C for 1 hour under nitrogen atmosphere. Water (1.0 mL) , 15% sodium hydroxide solution (1.0 mL) and water (3.0 mL) was added at 0 °C. The reaction solution was filtered, concentrated under reduced pressure and purified by silica gel column chromatography (petroleum ether/ethyl acetate = 3:1) to obtain compound **59-4.** MS-ESI [M+H]⁺, calculated: 330, found: 330.
(3) To a solution of compound **59-4** (2.3 g, 6.98 mmol) in dichloromethane (60.0 mL), Dess-Martin reagent (3.55 g, 8.38 mmol) was added. The reaction solution was stirred at 25 °C for 12 hours. Water (30.0 mL) was added to the reaction solution, followed by extraction with dichloromethane (30.0 mL × 2). The organic layer was washed with saturated sodium chloride solution (30.0 mL × 2), dried over anhydrous sodium sulfate, filtered and concentrated under reduced pressure to obtain compound **59-5.** MS-ESI [M+H]⁺, calculated: 272, found: 272.
(4) To a solution of compound **59-5** (2.7 g, 8.25 mmol) in dichloromethane (30.0 mL), compound **59-6** (3.45 g, 9.90 mmol) was added. The reaction solution was stirred at 25°C for 12 hours, filtered and concentrated under reduced pressure. The crude product was purified by silica gel column chromatography (petroleum ether/ethyl acetate = 1:0 to 6:1) to obtain compound **59-7.** MS-ESI [M-Boc+H]⁺, calculated: 298, found: 298.¹H NMR (400 MHz, CDCl₃) *δ* 7.04 (dd, *J* = 15.6, 7.2 Hz, 1H), 6.82-6.92 (m, 2H), 6.75-6.82 (m, 1H), 5.87 (br d, *J* = 16.0 Hz, 1H), 4.91 (br d, *J* = 2.4 Hz, 1H), 4.43-4.65 (m, 1H), 4.21 (br d, *J* = 6.8 Hz, 2H), 3.62-3.81 (m, 2H), 2.30-2.49 (m, 1H), 2.20 (br d, *J* = 13.6 Hz, 1H), 1.44 (br s, 9H), 1.24-1.34 (m, 3H).
(5) To a solution of compound **59-7** (2.81 g, 7.07 mmol) in tetrahydrofuran (30.0 mL), palladium on carbon (500 mg, 7.07 mmol) was added. The reaction solution was stirred under a hydrogen atmosphere at 25°C for 2 hours, filtered and concentrated under reduced pressure to obtain compound **59-8.** MS-ESI [M-Boc+H]⁺, calculated: 300, found: 300.
(6) To a solution of compound **59-8** (2.60 g, 6.51 mmol) in dichloromethane (18.0 mL), trifluoroacetic acid (8.93 g, 78.3 mmol) was added. The reaction solution was stirred at 25°C for 1 hour. The pH of the reaction solution was adjusted to pH = 9 with sodium bicarbonate. Water (10.0 mL) was added and the reaction solution was extracted with dichloromethane (10.0 mL). The organic phase was dried over anhydrous sodium sulfate, filtered and concentrated under vacuum to obtain compound **59-9.** MS-ESI [M+H]⁺, calculated: 300, found: 300.
(7) To a solution of compound **59-9** (60.0 mg, 200 µmol) in toluene (3.0 mL), compound **E** (76.6 mg, 240 µmol), cesium carbonate (130 mg, 400 µmol), 1,1'-binaphthyl-2,2'-diphenylphosphine (24.9 mg, 40.1 µmol) and tris (dibenzylideneacetone) dipalladium (18.4 mg, 20.1 µmol) was added. The reaction solution was stirred at 80°C under nitrogen atmosphere for 3 hours. Water (10.0 mL) was added to the reaction solution, followed by extraction with ethyl acetate (10.0 mL). The organic phase was dried over anhydrous sodium sulfate, filtered and concentrated under reduced pressure. The crude product was purified by silica gel column chromatography (petroleum ether/ethyl acetate = 1:0 to 1:1) to obtain compound **59-10.** MS-ESI [M+H]⁺, calculated: 582, found: 582.
(8) Compound **59-10** (83.0 mg, 142 µmol) was dissolved in tetrahydrofuran (3.0 mL), lithium hydroxide monohydrate (34.1 mg, 1.43 mmol) and water (1.0 mL) was added to the solution. The reaction mixture was stirred under nitrogen atmosphere at 35°C for 12 hours. The pH of the reaction solution was then adjusted to 5-6 with a solution of citric acid, water (10.0 mL) was added. The solution was extracted with ethyl acetate (30.0 mL). The organic phase was dried over anhydrous sodium sulfate and filtered, concentrated under reduced pressure to obtain compound **59-11.** MS-ESI [M+H]⁺, calculated: 554, found: 554.
(9) To a solution of compound **59-11** (120 mg, 216 µmol) in dichloromethane (3.0 mL), compound **B-4** trifluoroacetate (60.4 mg, 260 µmol), diisopropylethylamine (140 mg, 1.08 mmol) and a solution of propylphosphonic anhydride in ethyl acetate (344 mg, 650 µmol, 50% purity) was added. The reaction solution was stirred under nitrogen atmosphere at 25 °C for 1 hours. Then water (10.0 mL) was added, the reaction solution was extracted with dichloromethane (10.0 mL). The organic layer was dried over anhydrous sodium sulfate, filtered and concentrated under reduced pressure. The crude product was purified by silica gel column chromatography (petroleum ether/ethyl acetate = 1:0 to 1:1) to obtain compound **59-12.** MS-ESI [M+H]⁺, calculated: 768, found: 768.
(10) Compound **59-12** (37.0 mg, 48.2 µmol) was dissolved in trifluoroacetic acid (3.0 mL) and stirred at 70°C for 2 hours. The reaction solution was filtered and concentrated under reduced pressure. The crude product was then purified by preparative high performance liquid chromatography (C18, 100 mm × 30 mm × 5 µm, A: water (0.225% formic acid); B: acetonitrile, gradient elution from 53% to 83% over 8 minutes) to obtain compound **59** formate. MS-ESI [M+H]⁺, calculated: 648, found: 648.¹H NMR (400 MHz, MeOD) *δ* 8.59 (s, 2H), 7.97 (s, 1H), 7.16 (td, *J* = 9.2, 5.2 Hz, 1H), 7.01 (ddd, *J* = 11.2, 8.4, 2.8 Hz, 1H), 6.87-6.95 (m, 1H), 5.12 (br s, 1H), 4.15 (td, *J* = 8.8, 3.2 Hz, 1H), 3.93 (dt, *J* = 11.6, 5.6 Hz, 4H), 3.73-3.78 (m, 2H), 3.58-3.71 (m, 4H), 2.36-2.62 (m, 3H), 2.23-2.34 (m, 1H), 2.01-2.22 (m, 2H).

### Example 60 Synthesis of Compound 60

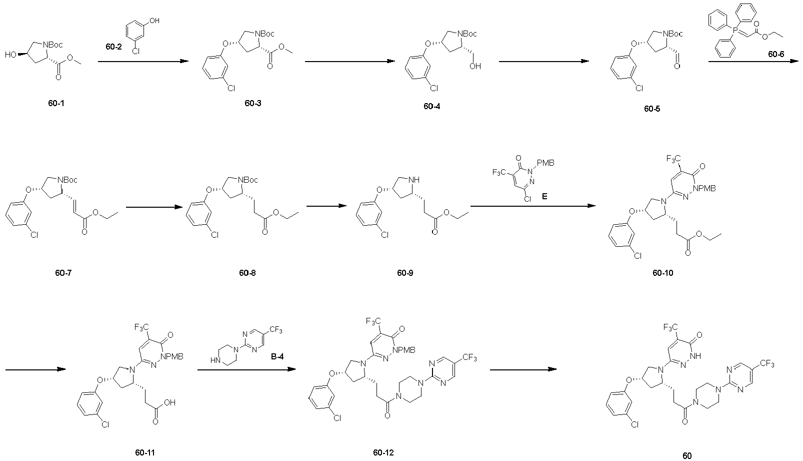

(1) At 0°C, to a solution of compound **60-1** (3.98 g, 16.2 mmol) in tetrahydrofuran (40.0 mL), compound **60-2** (2.00 g, 15.6 mmol), triphenylphosphine (4.49 g, 17.1 mmol), diethyl azodicarboxylate (2.71 g, 15.6 mmol) was added. The reaction solution was stirred at 25 °C for 12 hours. Water (20.0 mL) was added, followed by extraction with ethyl acetate (20.0 mL). The organic phase was dried over anhydrous sodium sulfate, filtered and concentrated under reduced pressure. The crude product was purified by silica gel chromatography (petroleum ether/ethyl acetate = 1:0 to 5:1) to obtain compound **60-3.** MS-ESI [M-Boc+H]⁺, calculated: 256, found: 256.¹H NMR (400 MHz, CDCl₃) *δ* 7.16-7.23 (m, 1H), 6.96 (br t, *J* = 6.0 Hz, 1H), 6.78-6.84 (m, 1H), 6.68 (br dd, *J* = 7.6, 2.8 Hz, 1H), 4.88 (dt, *J* = 4.4, 2.0 Hz, 1H), 4.56 (dd, *J* = 8.4, 2.8 Hz, 0.5H), 4.44 (dd, *J* = 8.4, 3.6 Hz, 0.5H), 3.64-3.83 (m, 5H), 2.32-2.55 (m, 2H), 1.42-1.51 (m, 9H).
(2) To a solution of compound **60-3** (3.85 g, 10.8 mmol) in tetrahydrofuran (30.0 mL) at 0 °C, lithium aluminum hydride (821 mg, 21.6 mmol) was added. The reaction solution was stirred at 0 °C under nitrogen atmosphere for 1 hour. Water (0.8 mL) , 15% sodium hydroxide (0.8 mL) and water (2.4 mL) was added at 0 °C. The solution was filtered and concentrated under reduced pressure. The crude product was purified to obtain compound **60-4.** MS-ESI [M-^{t}Bu+H]⁺, calculated: 272, found: 272. ¹H NMR (400 MHz, CDCl₃) *δ* 7.22 (t, *J* = 8.0 Hz, 1H), 6.95-6.99 (m, 1H), 6.86 (t, *J* = 2.0 Hz, 1H), 6.75 (ddd, *J* = 8.4, 2.4, 0.8 Hz, 1H), 4.84 (br s, 1H), 4.11-4.23 (m, 1H), 3.83-3.93 (m, 1H), 3.55-3.78 (m, 3H), 2.39 (ddd, *J* = 14.4, 9.2, 5.2 Hz, 1H), 1.88-2.08 (m, 1H), 1.49 (s, 9H).
(3) To a solution of compound **60-4** (3.02 g, 9.21 mmol) in dichloromethane (20.0 mL), Dess-Martin reagent (3.91 g, 9.21 mmol) was added. The reaction solution was stirred at 25 °C for 12 hours. Sodium thiosulfate (10.0 mL) was added, and the reaction solution was extracted with dichloromethane (20.0 mL). The organic layer was washed with water (30.0 mL) and saturated sodium chloride solution (30.0 mL), dried over anhydrous sodium sulfate, filtered and concentrated under reduced pressure to obtain compound **60-5.** MS-ESI [M-^{t}Bu+H]⁺, calculated: 270, found: 270.
(4) To a solution of compound **60-5** (2.90 g, 8.90 mmol) in dichloromethane (30.0 mL), compound **60-6** (3.72 g, 10.6 mmol) was added. The reaction solution was stirred at 25 °C for 12 hours. The solution was filtered and concentrated under reduced pressure. The crude product was purified by silica gel column chromatography to obtain compound **60-7.** MS-ESI [M-Boc+H]⁺, calculated: 296, found: 296.
(5) To a solution of compound **60-7** (1.80 g, 4.55 mmol) in methanol (10.0 mL), tris(triphenylphosphine)rhodium chloride (900 mg, 972 µmol) was added. The reaction mixture was stirred under a hydrogen atmosphere at 65°C for 4 hours. The solution was filtered, concentrated under reduced pressure to obtain compound **60-8.** MS-ESI [M+H]⁺, calculated: 398, found: 398.¹H NMR (400 MHz, CDCl₃) *δ* 7.17-7.24 (m, 1H), 6.95 (br d, *J* = 8.0 Hz, 1H), 6.86 (t, *J* = 2.0 Hz, 1H), 6.75 (dd, *J* = 8.4, 2.0 Hz, 1H), 4.86 (br t, *J* = 5.6 Hz, 1H), 3.66-4.28 (m, 4H), 3.53 (br d, *J* = 13.2 Hz, 1H), 2.13-2.41 (m, 4H), 1.86-2.07 (m, 2H), 1.48 (s, 9H), 1.25 (t, *J* = 7.2 Hz, 3H).
(6) To a solution of compound **60-8** (2.00 g, 5.03 mmol) in dichloromethane (30.0 mL), trifluoroacetic acid (573 mg, 5.03 mmol) was added. The reaction solution was stirred at 25 °C for 1 hour. Then sodium bicarbonate (30.0 mL) and water (10.0 mL) was added, the reaction solution was extracted with dichloromethane (10.0 mL). The organic phase was dried over anhydrous Na₂SO₄, filtered and concentrated under reduced pressure to obtain compound **60-9.** MS-ESI [M+H]⁺, calculated: 298, found: 298.
(7) To a solution of compound **60-9** (1.00 g, 3.36 mmol) in toluene (10.0 mL) compound **E** (1.28 g, 4.03 mmol), cesium carbonate (2.19 g, 6.72 mmol), 1,1'-binaphthyl-2,2'-diphenylphosphine (418 mg, 671 µmol) and tris (dibenzylideneacetone) dipalladium (307 mg, 335 µmol) was added. The reaction solution was stirred at 80°C under nitrogen atmosphere for 12 hours. Water (10.0 mL) was added, and the solution was extracted with ethyl acetate (10.0 mL). The organic phase was dried over anhydrous sodium sulfate, filtered and concentrated under reduced pressure. The crude product was purified by silica gel column chromatography (petroleum ether/ethyl acetate = 1:0 to 1:1) to obtain compound **60-10.** MS-ESI [M+H]⁺, calculated: 580, found: 580.
(8) To a solution of compound **60-10** (790 mg, 1.36mmol) in tetrahydrofuran (6.0 mL), water (2.0 mL) and lithium hydroxide monohydrate (326 mg, 13.6 mmol) was added. The reaction solution was stirred under nitrogen atmosphere at 35 °C for 12 hours. The pH was adjusted to 5-6 with 1 mol/L citric acid solution. Water (10.0 mL) was added, and the solution was extracted with ethyl acetate (30.0 mL). The organic phase was dried over anhydrous sodium sulfate, filtered and concentrated under reduced pressure to obtain compound **60-11.** MS-ESI [M+H]⁺, calculated: 552, found: 552.
(9) To a solution of compound **60-11** (603 mg, 1.09 mmol) in dichloromethane (10.0 mL), compound **B-4** trifluoroacetate (304 mg, 1.31 mmol), diisopropylethylamine (706 mg, 5.46 mmol) and a solution of propylphosphonic anhydride in ethyl acetate (1.74 g, 3.28 mmol, 50% purity) was added. The reaction solution was stirred under nitrogen atmosphere at 25 °C for 2 hours. Then water (10.0 mL) was added, the reaction solution was extracted with dichloromethane (10.0 mL). The organic layer was dried over anhydrous sodium sulfate, filtered and concentrated under reduced pressure. The crude product was purified by silica gel column chromatography (petroleum ether/ethyl acetate = 1:0 to 1:1) to obtain compound **60-12.** MS-ESI [M+H]⁺, calculated: 766, found: 766.
(10) Compound **60-12** (280 mg, 366 µmol) was dissolved in trifluoroacetic acid (5.0 mL) and stirred at 70 °C for 3 hours. The reaction solution was filtered and concentrated under reduced pressure. The crude product was purified by preparative high performance liquid chromatography (C18, 100 mm × 30 mm × 5 µm, A: water (0.225% formic acid); B: acetonitrile, gradient elution from 57% to 87% over 8 minutes) to obtain compound **60** formate. MS-ESI [M+H]⁺, calculated: 646, found: 646.¹H NMR (400 MHz, MeOD) *δ* 8.59 (s, 2H), 7.95 (s, 1H), 7.27 (t, *J* = 8.4 Hz, 1H), 6.86-7.01 (m, 3H), 5.14 (t, *J* = 4.8 Hz, 1H), 4.08-4.18 (m, 1H), 3.85-3.95 (m, 4H), 3.71-3.81 (m, 2H), 3.61-3.68 (m, 2H), 3.52-3.59 (m, 2H), 2.37-2.53 (m, 3H), 2.24 (br d, *J* = 13.6 Hz, 1H), 2.09-2.19 (m, 1H), 1.92-2.04 (m, 1H)

### Example 61 Synthesis of Compound 61

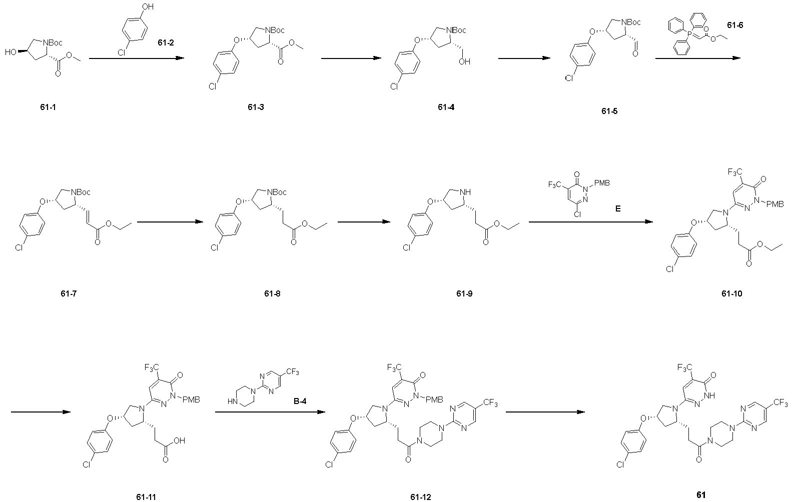

(1) At 0°C, to a solution of compound **61-1** (3.0 g, 12.2 mmol) in tetrahydrofuran (30.0 mL), compound **61-2** (1.73 g, 13.4 mmol) , triphenylphosphine (3.53 g, 13.4 mmol) and diisopropyl azodicarboxylate (2.47 g, 12.4 mmol) was added. The reaction mixture was stirred at 25°C for 12 hours. Ethyl acetate (1000 mL) was added, the solution was washed with water (500 mL). The organic layer was dried over anhydrous sodium sulfate, filtered and concentrated under reduced pressure. The crude product was purified by silica gel column chromatography ( petroleum ether/ethyl acetate = 1:0 to 0:1) to obtain compound **61-3.** MS-ESI [M-^{t}Bu+H]⁺, calculated: 300, found: 300.¹H NMR (400 MHz, CDCl₃) *δ* 7.23 (br dd, *J* = 8.8, 5.2 Hz, 2H), 6.74 (br dd, *J* = 8.8, 3.6 Hz, 2H), 4.99 (dt, *J* = 12.4, 6.4 Hz, 2H), 4.86 (br dd, *J* = 4.4, 2.8 Hz, 1H), 4.36-4.62 (m, 1H), 3.72-3.74 (m, 3H), 2.40-2.51 (m, 2H), 1.46 (d, *J* = 18.4 Hz, 9H).
(2) To a solution of compound **61-3** (2.1 g, 5.90 mmol) in tetrahydrofuran (30.0 mL), lithium aluminum hydride (264 mg, 6.49 mmol) was added at 0°C. The reaction mixture was stirred at 0°C under nitrogen atmosphere for 1 hour. Water (1.0 mL), 15% sodium hydroxide (1.0 mL), and water (3.0 mL) were added at 0°C. The solution was dried over anhydrous sodium sulfate, filtered, concentrated under reduced pressure, purified to obtain compound **61-4.** MS-ESI [M+H]⁺, calculated: 328, found: 328.¹H NMR (400 MHz, CDCl₃) *δ* 7.22-7.27 (m, 2H), 6.76-6.82 (m, 2H), 4.76-4.87 (m, 1H), 4.11-4.16 (m, 1H), 3.89 (br dd, *J* = 10.8, 8.0 Hz, 1H), 3.57-3.78 (m, 3H), 2.93 (br s, 1H), 2.37 (ddd, *J* = 14.4, 9.2, 5.2 Hz, 1H), 1.48 (s, 9H).
(3) To a solution of compound **61-4** (1.9 g, 5.80 mmol) in dichloromethane (20.0 mL), Dess-Martin reagent (2.70 g, 6.38 mmol) was added. The reaction mixture was stirred at 25°C for 3 hours. Sodium sulfite solution (50.0 mL) was added, and the reaction mixture was extracted with dichloromethane (50.0 mL × 2). The organic phase was washed with sodium bicarbonate (50.0 mL), dried over anhydrous sodium sulfate, filtered, and concentrated under reduced pressure. The crude product was purified by silica gel column chromatography ( petroleum ether/ethyl acetate = 1:0 to 0:1) to obtain compound **61-5.** ¹H NMR (400 MHz, CDCl₃) *δ* 9.61-9.69 (m, 1H), 7.20-7.26 (m, 2H), 6.71-6.77 (m, 2H), 4.87 (br s, 1H), 4.15-4.36 (m, 1H), 3.61-3.91 (m, 2H), 2.26-2.50 (m, 2H), 1.44-1.51 (m, 9H).
(4) To a solution of compound **61-5** (1.20 g, 3.68 mmol) in dichloromethane (15.0 mL), compound **61-6** (1.54 g, 4.42 mmol) was added. The reaction mixture was stirred at 25°C for 12 hours. The solution was filtered, concentrated under reduced pressure and purified by silica gel column chromatography ( petroleum ether/ethyl acetate = 1:0 to 0:1) to obtain compound **61-7.** MS-ESI [M+H]⁺, calculated: 296, found: 296.¹H NMR (400 MHz, CDCl₃) *δ* 7.24 (d, *J* = 8.8 Hz, 2H), 6.97 (dd, *J* = 15.6, 7.2 Hz, 1H), 6.74-6.80 (m, 2H), 5.77-5.97 (m, 1H), 4.90 (br t, *J* = 4.8 Hz, 1H), 4.42-4.70 (m, 1H), 4.11-4.29 (m, 2H), 3.61-3.84 (m, 2H), 2.32-2.49 (m, 1H), 2.15 (br d, *J* = 14.0 Hz, 1H), 1.44 (br s, 9H), 1.30 (br t, *J* = 6.8 Hz, 3H).
(5) To a solution of compound **61-7** (600 mg, 1.52 mmol) in ethanol (8.0 mL), tris(triphenylphosphine)rhodium chloride (60.0 mg, 64.8 µmol) was added. The reaction mixture was stirred under a hydrogen atmosphere at 65°C for 4 hours. The solution was filtered, concentrated under reduced pressure and purified by silica gel column chromatography (petroleum ether/ethyl acetate = 1:0 to 0:1) to obtain compound **61-8.** MS-ESI [M+H]⁺, calculated: 398, found: 398.
(6) To a solution of compound **61-8** (330 mg, 829 µmol) in dichloromethane (4.0 mL), trifluoroacetic acid (1.54 g, 13.5 mmol) was added. The reaction mixture was stirred at 25°C for 2 hours. The solution was adjusted to pH > 8 with sodium bicarbonate, water (20.0 mL) was added. The reaction mixture was extracted with dichloromethane (30.0 mL × 2). The organic phase was washed with saturated sodium chloride solution (30.0 mL), dried over anhydrous sodium sulfate, filtered, and concentrated under reduced pressure, and purified to give compound **61-9.** MS-ESI [M+H]⁺, calculated: 298, found: 298.
(7) To a solution of compound **61-9** (250 mg, 839 µmol) in toluene (5.0 mL) compound **E** (321 mg, 1.01 mmol), cesium carbonate (820 mg, 2.52 mmol), 1,1'-binaphthyl-2,2'-diphenylphosphine (104 mg, 167 µmol) and tris (dibenzylideneacetone) dipalladium (76.8 mg, 83.9 µmol) was added. The reaction solution was stirred at 80°C under nitrogen atmosphere for 3 hours. The reaction solution was added with water (10.0 mL), extracted with ethyl acetate (20.0 mL × 2). The organic phase was washed with saturated sodium chloride solution (10.0 mL), dried over anhydrous sodium sulfate, filtered, and concentrated under reduced pressure. The crude product was purified by silica gel column chromatography (petroleum ether/ethyl acetate = 2:1) to give compound **61-10.** MS-ESI [M+H]⁺, calculated: 580, found: 580.
(8) To a solution of compound **61-10** (50.0 mg, 86.2 µmol) in tetrahydrofuran (3.0 mL), water (1.0 mL) and lithium hydroxide monohydrate (21.7 mg, 517 µmol) were added. The reaction was stirred at 25°C for 6 hours under nitrogen atmosphere. Hydrochloric acid aqueous solution (1 mol/L) was added to the reaction solution to adjust the pH value < 7. The reaction solution was added with water (20.0 mL), and extracted with dichloromethane (30.0 mL × 2). The organic phase was washed with saturated sodium chloride solution (30.0 mL), dried over anhydrous sodium sulfate, filtered, concentrated under reduced pressure to obtain compound **61-11.** MS-ESI [M+H]⁺, calculated: 552, found: 552.
(9) To a solution of compound **61-11** (20.0 mg, 36.2 µmol) in dichloromethane (1.0 mL), compound **B-4** trifluoroacetate (16.8 mg, 72.4 µmol), diisopropylethylamine (23.4 mg, 181 µmol), and propylphosphonic anhydride solution (92.2 mg, 144 µmol, 50% purity in ethyl acetate solution) were added. The reaction was stirred at 25°C under nitrogen atmosphere for 1 hour. The reaction mixture was added with water (10.0 mL), extracted with dichloromethane (20.0 mL × 2). The organic phase was washed with saturated sodium chloride solution (10.0 mL), dried over anhydrous sodium sulfate, filtered and concentrated under reduced pressure. The crude product was purified by silica gel column chromatography (petroleum ether/ethyl acetate = 1:0 to 1:1) to obtain compound **61-12.** MS-ESI [M+H]⁺, calculated: 766, found: 766.
(10) Compound **61-12** (25.0 mg, 32.6 µmol) was dissolved in trifluoroacetic acid (3.0 mL), and stirred at 70°C for 3 hours. It was filtered and concentrated under reduced pressure. Sodium carbonate was added to adjust pH > 8, followed by extraction with ethyl acetate (10.0 mL × 2). The organic phase was washed with saturated sodium chloride solution (10.0 mL), dried with anhydrous sodium sulfate, filtered and concentrated under reduced pressure. The crude product was purified by preparative high performance liquid chromatography (Xtimate C18, 100 mm × 30 mm, 10 µm column; A: water (0.225% formic acid); B: acetonitrile: 55%-85% B over 10 minutes) to obtain compound **61** formate. MS-ESI [M+H]⁺, calculated: 646, found: 646.¹H NMR (400 MHz, MeOD) *δ* 8.60 (s, 2H), 7.95 (s, 1H), 7.24-7.31 (m, 2H), 6.93-6.98 (m, 2H), 5.11 (br t, *J* = 4.8 Hz, 1H), 4.10-4.18 (m, 1H), 3.88-3.94 (m, 4H), 3.74-3.81 (m, 2H), 3.66 (br t, *J* = 5.2 Hz, 2H), 3.55-3.59 (m, 2H), 2.50 (td, *J* = 7.2, 2.8 Hz, 2H), 2.36-2.44 (m, 1H), 2.25 (br d, *J* = 14.4 Hz, 1H), 2.10-2.20 (m, 1H), 1.95-2.05 (m, 1H).

### Example 62 Synthesis of Compound 62

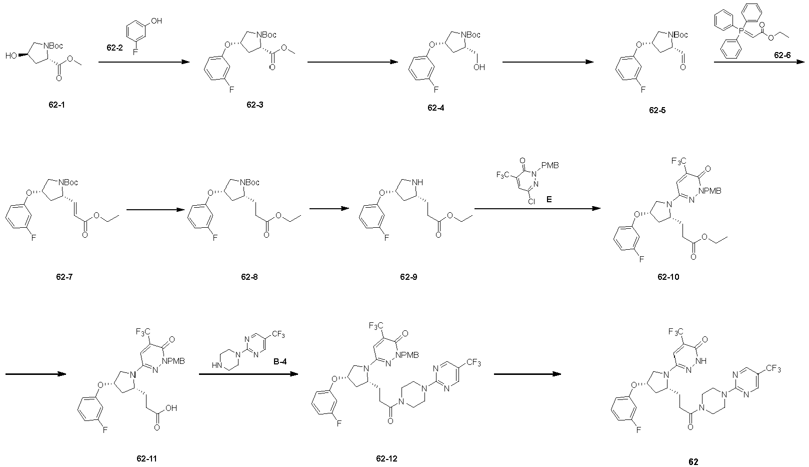

(1) At 0°C, to a solution of compound **62-1** (3.00 g, 12.2 mmol) in tetrahydrofuran (50.0 mL), compound **62-2** (1.65 g, 14.7 mmol, 1.35 mL), triphenylphosphine (3.53 g, 13.5 mmol) and diisopropyl azodicarboxylate (2.47 g, 12.2 mmol, 2.38 mL) were added. The reaction solution was stirred at 25°C for 12 hours. Water (50.0 mL) was added, and extracted with ethyl acetate (400 mL). The organic phase was dried over anhydrous sodium sulfate, filtered and concentrated under reduced pressure. The crude product was purified by silica gel column chromatography (petroleum ether/ethyl acetate = 1:0 to 1:1) to give compound **62-3.** MS-ESI [M-^{t}Bu+H]⁺, calculated: 284, found: 284.¹H NMR (400 MHz, CDCl₃) *δ* 7.12-7.20 (m, 1H), 6.59-6.62 (m, 1H), 6.48-6.52 (m, 1H), 6.42-6.48 (m, 1H), 4.78-4.82 (m, 1H), 4.35-4.50 (m, 1H), 3.60-3.72 (m, 5H), 2.32-2.44 (m, 2H), 1.20-1.42 (m, 9H).
(2) To a solution of compound **62-3** (2.50 g, 7.37 mmol) in tetrahydrofuran (30.0 mL), lithium aluminum hydride (336 mg, 8.85 mmol) was added at 0°C. The reaction solution was stirred at 0°C under nitrogen atmosphere for 1 hour. Water (1.0 mL) , 15% sodium hydroxide (1.0 mL) and water (3.0 mL) were added at 0°C. The mixture was dried over anhydrous sodium sulfate, filtered and concentrated under reduced pressure. The crude product was purified by silica gel column chromatography (petroleum ether/ethyl acetate = 1:0 to 1:1) to give compound **62-4.** MS-ESI [M-^{t}Bu+H]⁺, calculated: 256, found: 256.¹H NMR (400 MHz, CDCl₃) *δ* 7.20-7.26 (m, 1H), 6.61-6.71 (m, 2H), 6.54-6.59 (m, 1H), 4.80-4.83 (m, 1H), 4.11-4.16 (m, 1H), 3.82-3.98 (m, 1H), 3.59-3.79 (m, 3H), 2.31-2.46 (m, 1H), 1.89-2.12 (m, 1H), 1.48 (s, 9H).
(3) To a solution of compound **62-4** (1.00 g, 3.21 mmol) in dichloromethane (15.0 mL), Dess-Martin reagent (1.63 g, 3.85 mmol) was added. The reaction solution was stirred at 25°C for 12 hours. Sodium sulfite solution (80.0 mL) was added to the reaction solution, followed by extraction with dichloromethane (30.0 mL). The organic phase was dried over anhydrous sodium sulfate, filtered and concentrated under reduced pressure. The crude product was purified by silica gel column chromatography (petroleum ether/ethyl acetate = 1:0 to 1:1) to give compound **62-5.** MS-ESI [M-^{t}Bu+H]⁺, calculated: 254, found: 254.
(4) Compound **62-5** (970 mg, 3.14 mmol) was dissolved in dichloromethane (20.0 mL), and compound **62-6** (1.31 g, 3.76 mmol) was added. The reaction mixture was stirred at 25°C for 12 hours. The reaction solution was dried with anhydrous sodium sulfate, filtered and concentrated under reduced pressure. The crude product was purified by silica gel column chromatography (petroleum ether/ethyl acetate = 1:0 to 1:1) to obtain compound **62-7**. MS-ESI [M-Boc+H]⁺, calculated: 280, found: 280.
(5) To a solution of compound **62-7** (1.07 g, 2.82 mmol) in tetrahydrofuran (20.0 mL), 10% palladium on carbon (400 mg) was added. The reaction mixture was stirred under a hydrogen atmosphere at 25°C for 1 hour. The reaction solution was filtered, concentrated under reduced pressure to obtain compound **62-8.** MS-ESI [M+H]⁺, calculated: 382, found: 382.¹H NMR (400 MHz, CDCl₃) *δ* 7.18-7.26 (m, 1H), 6.55-6.68 (m, 3H), 4.82-4.86 (m, 1H), 4.06-4.14 (m, 2H), 3.72-4.05 (m, 2H), 3.48-3.60 (m, 1H), 2.13-2.39 (m, 4H), 1.82-2.08 (m, 2H), 1.43-1.50 (m, 9H), 1.22-1.26 (m, 3H).
(6) To a solution of compound **62-8** (500 mg, 1.31 mmol) in dichloromethane (3.0 mL), trifluoroacetic acid (1.54 g, 13.51 mmol, 1.00 mL) was added. The reaction mixture was stirred at 25°C for 0.5 hours. The mixture was adjusted pH > 7 with sodium carbonate, extracted with dichloromethane (200 mL). The organic phase was dried with anhydrous sodium sulfate, filtered, and concentrated under reduced pressure to obtain compound **62-9**. MS-ESI [M+H]⁺, calculated: 282, found: 282.
(7) To a solution of compound **62-9** (400 mg, 1.42 mmol) in toluene (10.0 mL) compound **E** (543 mg, 1.71 mmol), cesium carbonate (926 mg, 2.84 mmol), 1,1'-binaphthyl-2,2'-diphenylphosphine (177 mg, 284 µmol) and tris (dibenzylideneacetone) dipalladium (130 mg, 142 µmol) were added. The reaction solution was stirred at 80°C under nitrogen atmosphere for 10 hours. The reaction solution was dried over anhydrous sodium sulfate, filtered and concentrated under reduced pressure. The crude product was purified by silica gel column chromatography (petroleum ether/ethyl acetate = 1:0 to 2:1) to obtain compound **62-10**. MS-ESI [M+H]⁺, calculated: 564, found: 564.
(8) To a solution of compound **62-10** (130 mg, 230 µmol) in tetrahydrofuran (6.0 mL), water (2.0 mL) and lithium hydroxide monohydrate (29.0 mg, 692 mmol) were added. The reaction was stirred at 25 °C under nitrogen atmosphere for 12 hours. Hydrochloric acid (1 mol/L) was added to the reaction solution to adjust the pH < 7, followed by extraction with dichloromethane (100 mL). The organic phase was dried with anhydrous sodium sulfate, filtered and concentrated under reduced pressure to obtain compound **62-11**. MS-ESI [M+H]⁺, calculated: 536, found: 536.
(9) To a solution of compound **62-11** (160 mg, 298 µmol) in dichloromethane (5.0 mL), compound **B-4** trifluoroacetate (83.2 mg, 358 µmol), diisopropylethylamine (115 mg, 896 µmol) and a solution of propylphosphonic anhydride in ethyl acetate (570 mg, 896 µmol, 50% purity) were added. The mixture was stirred at 25 °C for 4 hours. It was diluted with water (30.0 mL), and extracted with dichloromethane (150 mL). The organic layer was dried over anhydrous sodium sulfate, filtered and concentrated under reduced pressure. The crude product was purified by silica gel column chromatography (petroleum ether/ethyl acetate = 1:0 to 2:1) to obtain compound **62-12**. MS-ESI [M+H]⁺, calculated: 750, found: 750.
(10) Compound **62-12** (70 mg, 93.4 µmol) was dissolved in trifluoroacetic acid (5.0 mL) and stirred at 70°C for 2 hours. The mixture was filtered, concentrated under reduced pressure, adjusted to pH>7 with saturated sodium carbonate, and extracted with dichloromethane (50.0 mL). The organic layer was dried over anhydrous sodium sulfate, filtered and concentrated under reduced pressure. The crude product was purified by preparative high-performance liquid chromatography (Xtimate C18 column, 100 mm × 30 mm, 10 µm; A: water (0.225% formic acid); B: acetonitrile: 52%-82% over 15 minutes) to obtain compound **62** formate. MS-ESI [M+H]⁺, calculated: 630, found: 630.¹H NMR (400 MHz, MeOD) *δ*8.60 (s, 2H), 7.11-7.49 (m, 2H), 6.67-6.80 (m, 3H), 5.14 (s, 1H), 4.10-4.17 (m, 1H), 3.88-3.95 (m, 4H), 3.75-3.83 (m, 2H), 3.64-3.70 (m, 2H), 3.56-3.61 (m, 2H), 2.40-2.52 (m, 3H), 2.23-2.28 (m, 1H), 2.10-2.19 (m, 1H), 1.95-2.05 (m, 1H).

### Example 63 Synthesis of Compound 63

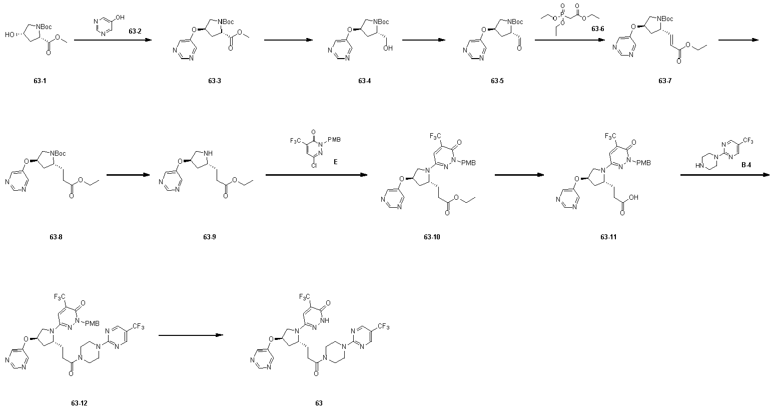

(1) To a solution of compound **63-1** (7.0 g, 28.5 mmol) in tetrahydrofuran (100 mL) at 0°C, compound **63-2** (3.02 g, 31.3 mmol), triphenylphosphine (8.23 g, 31.3 mmol), diisopropyl azodicarboxylate (6.35 g, 36.4 mmol) were added. The reaction solution was stirred at 25°C for 4 hours. Water (125 mL) was added, followed by extraction with ethyl acetate (100 mL). The organic phase was washed with saturated sodium chloride (150 mL), dried over anhydrous sodium sulfate, filtered and concentrated under reduced pressure. The crude product was purified by silica gel column chromatography (petroleum ether/ethyl acetate = 1:1) to obtain compound **63-3**. MS-ESI [M-^{t}Bu+H]⁺, calculated: 268, found: 268.
(2) To a solution of compound **63-3** (5.0 g, 15.4 mmol) in tetrahydrofuran (50.0 mL), lithium aluminum hydride (645 mg, 17.0 mmol) was added at 0 °C. The reaction solution was stirred at 0 °C under nitrogen for 1 hour. Water (0.7 mL), 15% sodium hydroxide (0.7 mL) and water (3.5 mL) were added at 0 °C. The solution was dried over anhydrous sodium sulfate, filtered and concentrated under reduced pressure. The crude product was purified by silica gel column chromatography (dichloromethane /methanol = 5:1) to obtain compound **63-4**. ¹H NMR (400 MHz, CDCl₃) *δ* 8.89 (s, 1H), 8.42 (s, 2H), 4.92 (s, 1H), 4.17 (s, 1H), 3.79 (t, *J* = 10.4 Hz, 2H), 3.59-3.67 (m, 2H), 2.33 (dd, *J* = 13.2, 7.2 Hz, 1H), 1.94 (s, 1H), 1.45-1.48 (m, 9H).
(3) To a solution of compound **63-4** (1.0 g, 3.55 mmol) in dichloromethane (10.0 mL), Dess-Martin reagent (1.81 g, 4.27 mmol) was added. The mixture was stirred at 25 °C for 3 hours. The reaction solution was filtered and concentrated under reduced pressure. The crude product was purified by silica gel column chromatography (tetrahydrofuran/ethyl acetate =1:1) to obtain compound **63-5**. ¹H NMR (400 MHz, CDCl₃) *δ* 9.47-9.64 (m, 1H), 8.85-8.92 (m, 1H), 8.35-8.41 (m, 2H), 4.99 (s, 1H), 4.28-4.50 (m, 1H), 3.73-3.97 (m, 2H), 2.35-2.51 (m, 1H), 2.22 (m, 1H), 1.44-1.47 (m, 9H).
(4) To a solution of sodium hydride (122 mg, 3.07 mmol) in tetrahydrofuran (5.0 mL), compound **63-6** (550 mg, 2.45 mmol) was added. The reaction solution was stirred at 0 °C for 0.5 hours, and compound **63-5** (600 mg, 2.05 mmol) was added. The reaction solution was stirred at 0 °C for 0.5 hours. The reaction solution was added to ice water (20.0 mL), extracted with ethyl acetate (30.0 mL). The organic layer was washed with water (30.0 mL) and saturated sodium chloride solution (30.0 mL), dried over anhydrous anhydrous sodium sulfate, filtered and concentrated under reduced pressure. The crude product was purified by silica gel column chromatography (tetrahydrofuran /ethyl acetate = 1:1) to obtain compound **63-7**. ¹H NMR (400 MHz, CDCl₃) *δ* 8.84-8.96 (m, 1H), 8.31-8.47 (m, 2H), 6.83 (d, *J* = 6.4 Hz, 1H), 5.92 (d, *J* = 15.2 Hz, 1H), 4.82-5.02 (m, 1H), 4.48-4.79 (m, 1H), 4.16-4.26 (m, 2H), 3.70-3.98 (m, 2H), 2.42-2.52 (m, 1H), 2.06-2.17 (m, 1H), 1.39-1.49 (m, 9H), 1.23-1.31 (m, 3H).
(5) To a solution of compound **63-7** (370 mg, 1.02 mmol) in tetrahydrofuran (5.0 mL), 10% palladium on carbon (100 mg) was added. The reaction solution was stirred at 25°C under hydrogen atmosphere for 6 hours. The reaction solution was filtered, concentrated under reduced pressure to obtain compound **63-8**. MS-ESI [M-Boc+H]⁺, calculated: 266, found: 266.
(6) To a solution of compound **63-8** (250 mg, 684 µmol) in dichloromethane (2.0 mL), trifluoroacetic acid (1.93 g, 16.8 mmol) was added. The mixture was stirred at 25 °C for 2 hours. The reaction solution was adjusted to pH > 7 with sodium bicarbonate. The crude product was purified by preparative high performance liquid chromatography (Phenomenex C18, 75 mm × 30 mm, 3 µm; A: water (10 mmol/L ammonium acetate); B: acetonitrile; 0%-30%, over 10 minutes) to obtain compound **63-9**. MS-ESI [M+H]⁺, calculated: 266, found: 266.¹H NMR (400 MHz, CDCl₃) *δ* 8.82-8.92 (m, 1H), 8.37 (d, *J* = 5.2 Hz, 2H), 4.07-4.19 (m, 2H), 3.42-3.48 (m, 1H), 3.15-3.29 (m, 1H), 2.78 (dt, *J* = 16.8, 10.0 Hz, 1H), 2.35-2.49 (m, 3H), 2.21 (dd, *J* = 14.0, 6.2 Hz, 1H), 1.76-1.90 (m, 2H), 1.60-1.76 (m, 1H), 1.22-1.30 (m, 3H).
(7) To a solution of compound **63-9** (30.0 mg, 113.3 µmol) in toluene (3.0 mL) compound **E** (36.0 mg, 113 µmol), cesium carbonate (92.1 mg, 282 µmol), 1,1'-binaphthyl-2,2'-diphenylphosphine (14.0 mg, 22.6 µmol) and tris (dibenzylideneacetone) dipalladium (10.3 mg, 11.3 µmol) were added. The reaction solution was stirred at 80°C under nitrogen atmosphere for 6 hours. Water (15.0 mL) was added, followed by extraction with ethyl acetate (5.00 mL). The combined organic layer was washed with saturated sodium chloride solution (15.0 mL), dried over anhydrous sodium sulfate, filtered, and concentrated under reduced pressure. The crude product was purified by silica gel column chromatography (petroleum ether/ethyl acetate = 1:1) to obtain compound **63-10**. MS-ESI [M+H]⁺, calculated: 548, found: 548.
(8) To a solution of compound **63-10** (20.0 mg, 36.5 µmol) in tetrahydrofuran (1.50 mL), water (0.50 mL) and lithium hydroxide monohydrate (15.3 mg, 365 µmol) were added. The reaction solution was stirred at 30°C under nitrogen atmosphere for 12 hours. The reaction solution was added with hydrochloric acid solution (1 mol/L) to adjust the pH < 7, and extracted with ethyl acetate (10.0 mL). The organic phase was dried over anhydrous sodium sulfate, filtered and concentrated under reduced pressure to obtain compound **63-11**.
(9) To a solution of compound **63-11** (18.0 mg, 34.6 µmol) in dichloromethane (2.0 mL), **B-4** trifluoroacetate (10.4 mg, 45.0 µmol), diisopropylethylamine (13.4 mg, 103 µmol) and a solution of propylphosphonic anhydride in ethyl acetate (66.1 mg, 103 µmol, 50% purity) were added. The mixture was stirred at 25 °C for 1 hours. It was diluted with water (5.0 mL), and extracted with dichloromethane (3.0 mL). The organic layer was dried over anhydrous sodium sulfate, filtered and concentrated under reduced pressure. The crude product was purified by silica gel column chromatography (petroleum ether/ethyl acetate = 1:1) to obtain compound **63-12**. MS-ESI [M+H]⁺, calculated: 734, found: 734.
(10) Compound **63-12** (10.0 mg, 13.6 µmol) was dissolved in trifluoroacetic acid (1.0 mL) and stirred at 70°C for 4 hours. The solution was adjusted to pH >7 with saturated sodium bicarbonate. The crude product was purified by preparative high-performance liquid chromatography (Phenomenex C18, 75 mm × 30 mm, 3 µm; A: water (10 mmol/L ammonium acetate); B: acetonitrile: 23%-53% over 10 minutes) to obtain compound **63**. MS-ESI [M+H]⁺, calculated: 614, found: 614.¹H NMR (400 MHz, MeOD) *δ* 8.78 (s, 1H), 8.60 (s, 2H), 8.53 (s, 2H), 7.86 (s, 1H), 4.23 (d, *J* = 6.4 Hz, 1H), 3.88-4.00 (m, 6H), 3.78 (d, *J* = 10.8 Hz, 1H), 3.62-3.69 (m, 4H), 2.52 (t, *J* = 7.2 Hz, 3H), 2.25-2.33 (m, 1H), 2.15-2.21 (m, 1H), 1.76-1.87 (m, 1H).

### Example 64 Synthesis of Compound 64

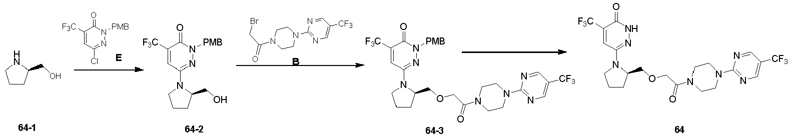

(1) To a solution of compound **64-1** (1 g, 9.89 mmol) and compound **E** (3.78 g, 11.86 mmol) in toluene (40.0 mL), cesium carbonate (6.44 g, 19.77 mmol), 1,1'-binaphthyl-2,2'-diphenylphosphine (1.23 g, 1.98 mmol), tris (dibenzylideneacetone) dipalladium (905.34 mg, 988 µmol) were added. The mixture was stirred at 80 °C under nitrogen for 6 hours. The reaction solution was filtered through celite, and the filter cake was washed with ethyl acetate (20.0 mL). The filtrate was washed with saturated sodium chloride solution (20.0 mL), dried over anhydrous sodium sulfate, filtered and concentrated under reduced pressure. The crude product was purified by silica gel column chromatography (petroleum ether/ethyl acetate = 1:0 to 2:1) to obtain compound **64-2**. MS-ESI [M+H]⁺, calculated: 384, found: 384.
(2) To a solution of compound **64-2** (250 mg, 652 µmol) and compound **B** (191.91 mg, 543 µmol) in dichloromethane (4.0 mL), sodium hydroxide (250 mg, 6.25 mmol), tetrabutylammonium bisulfate (92 mg, 271 µmol) and water (0.5 mL) were added. The mixture was stirred at 25 °C for 2 hours. It was diluted with dichloromethane (5.0 mL), and washed with saturated sodium chloride solution (5.0 mL). The organic layer was dried over anhydrous sodium sulfate, filtered and concentrated under reduced pressure. The crude product was purified by silica gel column chromatography (petroleum ether/ethyl acetate = 1:0 to 20:1) to obtain compound **64-3**. MS-ESI [M+H]⁺, calculated: 656, found: 656.
(3) Compound **64-3** (100 mg, 152 µmol) was dissolved in trifluoroacetic acid (3.0 mL), and stirred at 70 °C for 3 hours. The reaction solution was concentrated under reduced pressure. The crude product was purified by preparative high-performance liquid chromatography (Phenomenex C18, 75 mm × 30 mm, 3 µm; A: water (10 mmol/L ammonium bicarbonate); B: acetonitrile: 24%-64% over 36 minutes) to obtain compound **64**. MS-ESI [M+H]⁺, calculated: 536, found: 536.¹H NMR (400 MHz, MeOD) *δ* 8.60 (s, 2H), 7.75 (s, 1H), 4.19-4.32 (m, 3H), 3.87-3.97 (m, 4H), 3.65 (br t, *J* = 4.8 Hz, 2H), 3.52-3.60 (m, 5H), 3.34-3.40 (m, 1H), 1.98-2.10 (m, 4H).

### Example 65 Synthesis of Compound 65

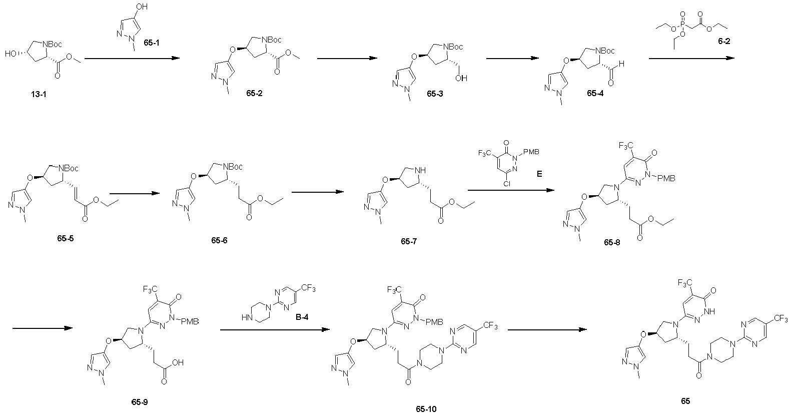

(1) Compound **13-1** (2.50 g, 10.1 mmol) and compound **65-1** (1.00 g, 10.1 mmol) were dissolved in tetrahydrofuran (30.0 mL) and cooled to 0°C. Triphenylphosphine (2.94 g, 11.2 mmol) and diethyl azodicarboxylate (1.78 g, 10.1 mmol, 1.85 mL) were added to the solution. The reaction mixture was stirred at 25°C for 12 hours. Water (20.0 mL) was added to the reaction solution, followed by extraction with ethyl acetate (20.0 mL). The organic layer was dried over anhydrous sodium sulfate, filtered and concentrated under reduced pressure. The crude product was purified by silica gel column chromatography (dichloromethane/methanol = 1:0 to 49:1) to obtain compound **65-2**. MS-ESI [M+H]⁺, calculated: 326, found: 326.
(2) Compound **65-2** (2.50 g, 7.68 mmol) was dissolved in tetrahydrofuran (30.0 mL) and cooled to 0°C. Lithium aluminum hydride (583 mg, 15.3 mmol) was added, and the reaction mixture was stirred at 0°C for 1 hour. Water (0.6 mL), 15% sodium hydroxide solution (0.6 mL) and water (1.8 mL) were added to the reaction solution at 0°C. The mixture was stirred for 30 minutes, filtered and concentrated under reduced pressure. The crude product was purified by silica gel column chromatography (dichloromethane/ethyl acetate = 0:1 to 1:0) to obtain compound **65-3**. MS-ESI [M-^{t}Bu+H]⁺, calculated: 242, found: 242.
(3) Compound **65-3** (597 mg, 2.01 mmol) was dissolved in dichloromethane (3.0 mL), and Dess-Martin reagent (851 mg, 2.01 mmol, 621 µL) was added. The reaction mixture was stirred at 25°C for 12 hours. Saturated sodium sulfite solution (20.0 mL) was added. The reaction solution was stirred for 10 minutes, and extracted with dichloromethane (30.0 mL). The organic layer was dried over anhydrous sodium sulfate, filtered, and concentrated under reduced pressure. The crude product was purified by silica gel column chromatography (dichloromethane/ethyl acetate = 0:1 to 1:4) to obtain compound **65-4**. ¹H NMR (400 MHz, CDCl₃) *δ* 9.64 (m, 1H), 7.16 (s, 1H), 7.01 (s, 1H), 4.55 (br s, 1H), 4.16-4.28 (m, 1H), 3.84-3.92 (m, 1H), 3.81 (s, 3H), 3.52-3.63 (m, 1H), 2.40-2.53 (m, 1H), 2.21-2.34 (m, 1H), 1.46-1.50 (m, 9H).
(4) To a solution of 60% sodium hydride (30.47 mg, 761.85 µmol) in tetrahydrofuran (5.0 mL), compound **6-2** (136 mg, 609 µmol, 120 µL) was added at 0°C. The reaction mixture was stirred at 0°C for 30 minutes, a solution of compound **65-4** (150 mg, 507.90 µmol) in tetrahydrofuran (10.0 mL) was added dropwise at 0°C. The reaction was stirred at 0°C for 1 hour. Water (15.0 mL) was slowly added to the reaction solution to quench the reaction at 0°C. The reaction mixture was extracted with ethyl acetate (10.0 mL). The organic layer was dried over anhydrous sodium sulfate, filtered, and concentrated under reduced pressure. The crude product was purified by silica gel column chromatography (dichloromethane/petroleum ether = 0:1 to 1:4) to obtain compound **65-5**. MS-ESI [M-^{t}Bu+H]⁺, calculated: 310, found: 310.
(5) To a solution of compound **65-5** (100 mg, 273 µmol) in tetrahydrofuran (5.0 mL), 10% palladium on carbon (100 mg) was added. The reaction mixture was stirred at 25°C for 2 hours under a hydrogen atmosphere (15 psi). The reaction solution was filtered and the filtrate was concentrated under reduced pressure to obtain compound **65-6**. MS-ESI [M-^{t}Bu+H]⁺, calculated: 268, found: 268.
(6) To a solution of compound **65-6** (100 mg, 272 µmol) in dichloromethane (3.0 mL), trifluoroacetic acid (1.54 g, 13.5 mmol, 1.0 mL) was added. The reaction mixture was stirred at 25°C for 1 hour. The reaction solution was adjusted to pH > 7 with saturated sodium bicarbonate solution. It was extracted with dichloromethane (10.0 mL). The organic layer was dried over anhydrous sodium sulfate, filtered, and concentrated under reduced pressure to obtain compound **65-7**. MS-ESI [M+H]⁺, calculated: 268, found: 268.
(7) To a mixture of compound **65-7** (80 mg, 299 µmol), compound **E** (95.3 mg, 299 µmol), cesium carbonate (195 mg, 598µmol), tris (dibenzylideneacetone) dipalladium (27.4 mg, 29.9 µmol) and 1,1'-binaphthyl-2,2'-diphenylphosphine (37.2 mg, 59.8µmol), toluene (3.0 mL) was added. The mixture was stirred at 80 °C under nitrogen for 10 hours. Water (10.0 mL) was added to the reaction solution, followed by extraction with ethyl acetate (10.0 mL). The organic layer was dried over anhydrous sodium sulfate, filtered, and concentrated under reduced pressure. The crude product was purified by silica gel column chromatography (petroleum ether/ethyl acetate = 1:0 to 1:1) to obtain compound **65-8**. MS-ESI [M+H]⁺, calculated: 550, found: 550.
(8) Compound **65-8** (38 mg, 69.1 µmol) was dissolved in tetrahydrofuran (3.0 mL) and water (1.0 mL), and lithium hydroxide monohydrate (29.0 mg, 691 µmol) was added. The reaction was stirred at 25 °C for 12 hours. Water (10.0 mL) was added, and the pH was adjusted to 5-6 with saturated citric acid solution. Ethyl acetate (30.0 mL) was added for extraction. The organic layer was dried over anhydrous sodium sulfate, filtered, and concentrated under reduced pressure to obtain compound **65-9**. MS-ESI [M+H]⁺, calculated: 522, found: 522.
(9) To a solution of compound **65-9** (55 mg, 105 µmol) and **B-4** (24 mg, 105 µmol) in dichloromethane (5.0 mL), diisopropylethylamine (68 mg, 527 µmol) and 50% propylphosphonic anhydride (201mg, 316 µmol) was added. The mixture was stirred at 25 °C for 2 hours. The reaction mixture was diluted with water (10.0 mL) and extracted with ethyl acetate (10.0 mL). The organic layer was dried over anhydrous sodium sulfate, filtered and concentrated under reduced pressure. The crude product was purified by silica gel column chromatography (dichloromethane/ethyl acetate = 0:1 to 3:7) to obtain compound **65-10**. MS-ESI [M+H]⁺, calculated: 736, found: 736.
(10) Compound **65-10** (20 mg, 27.1 µmol) was dissolved in trifluoroacetic acid (3.0 mL), and stirred at 70 °C for 2 hours. The reaction solution was cooled to room temperature, adjusted to pH >7 with saturated sodium bicarbonate solution, and extracted with dichloromethane (10.0 mL). The organic layer was dried over anhydrous sodium sulfate, filtered and concentrated under reduced pressure. The crude product was purified by preparative high-performance liquid chromatography (C18-6, 100 mm × 30 mm, 5 µm; A: water (0.225% formic acid); B: acetonitrile (10%-70% B for 15 minutes)) to obtain compound **65** formate. MS-ESI [M+H]⁺, calculated: 616, found: 616.¹H NMR (400 MHz, MeOD) *δ* 8.60 (s, 2H), 7.94 (s, 1H), 7.40 (s, 1H), 7.24 (s, 1H), 4.80 (br t, *J* = 4.6 Hz, 1H), 4.10 (br t, *J* = 8.5 Hz, 1H), 3.89-3.99 (m, 4H), 3.81 (s, 3H), 3.73-3.78 (m, 1H), 3.65-3.72 (m, 3H), 3.59-3.64 (m, 2H), 2.47-2.57 (m, 2H), 2.24-2.37 (m, 2H), 2.09-2.19 (m, 1H), 1.95-2.04 (m, 1H).

### Example 66 Synthesis of Compound 66

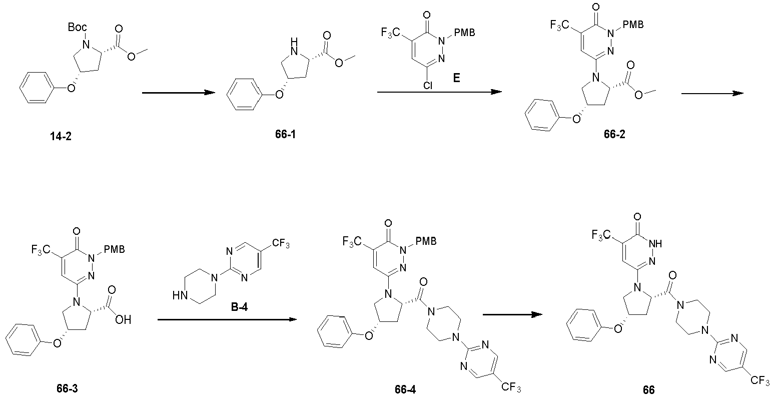

(1) To a solution of compound **14-2** (1.29 g, 4.00 mmol) in dichloromethane (9.0 mL), trifluoroacetic acid (4.62 g, 40.5 mmol, 3.0 mL) was added. The reaction mixture was stirred at 25°C for 0.5 hours. The reaction solution was diluted with water (10.0 mL) and extracted with dichloromethane (10.0 mL). The organic layer was washed with saturated sodium carbonate solution (30.0 mL), dried over anhydrous sodium sulfate, filtered, and concentrated under reduced pressure to obtain compound **66-1**. MS-ESI [M+H]⁺, calculated: 222, found: 222. ¹H NMR (400 MHz, DMSO-*d*6) *δ* 7.13-7.35 (m, 2H), 6.76-6.98 (m, 3H), 4.79-4.89 (m, 1H), 3.71-3.81 (m, 1H), 3.61 (s, 3H), 2.99-3.09 (m, 2H), 2.63-2.98 (m, 1H), 2.35-2.45 (m, 1H), 1.94-2.04 (m, 1H).
(2) To a solution of compound **E** (433 mg, 1.36 mmol) and compound **66-1** (300 mg, 1.36 mmol) in toluene (5.0 mL), cesium carbonate (886 mg, 2.72 mmol), 1,1'-binaphthyl-2,2'-diphenylphosphine (169 mg, 272 µmol) and tris (dibenzylideneacetone) dipalladium (125 mg, 136 µmol) were added. The solution was stirred for 4 hours at 80°C under nitrogen atmosphere. The reaction solution was concentrated under reduced pressure. The residue was dissolved in ethyl acetate (40.0 mL), and washed with water (40.0 mL). The organic layer was dried over anhydrous sodium sulfate, filtered, concentrated under reduced pressure. The crude product was purified by silica gel column chromatography (petroleum ether/ethyl acetate = 1:0 to 3:2) to obtain compound **66-2**. MS-ESI [M+H]⁺, calculated: 504, found: 504
(3) Compound **66-2** (565 mg, 1.12 mmol) was dissolved in tetrahydrofuran (9.0 mL) and water (3.0 mL), lithium hydroxide monohydrate (471 mg, 11.2 mmol) was added. The reaction solution was stirred at 35°C for 12 hours. To the reaction mixture was added 1.0 mol/L hydrochloric acid solution to adjust pH to 1. The mixture was extracted with ethyl acetate (45.0 mL). The organic layer was washed with water (45.0 mL), dried over anhydrous sodium sulfate, filtered, and concentrated under reduced pressure to obtain compound **66-3**. MS-ESI [M+H]⁺, calculated: 490, found: 490.
(4) To a solution of compound **66-3** (333 mg, 680 µmol), **B-4** (158 mg, 680 µmol) in dichloromethane (5.0 mL), diisopropylethylamine (439 mg, 3.40 mmol, 592 µL) and 50% propylphosphonic anhydride (1.30 g, 2.04 mmol, 1.21 mL) were added. The reaction solution was stirred at 25 °C for 2 hours, diluted with water (35.0 mL) and extracted with dichloromethane (35.0 mL). The organic layer was dried over anhydrous sodium sulfate, filtered, and concentrated under reduced pressure. The crude product was purified by silica gel chromatography (petroleum ether/ethyl acetate = 1:0 to 0:1) to obtain compound **66-4**. MS-ESI [M+H]⁺, calculated: 704, found: 704.
(5) Compound **66-4** (91 mg, 129 µmol) was dissolved in trifluoroacetic acid (3.9 mL) and trifluoromethanesulfonic acid (390 µL). The mixture was stirred at 25°C for 1 hour. The reaction solution was adjusted to pH 11 with saturated sodium carbonate solution, and extracted with dichloromethane (30.0 mL). The organic layer was washed with water (30.0 mL), dried over anhydrous sodium sulfate, filtered, and concentrated under reduced pressure. The crude product was purified by preparative high performance liquid chromatography (C18-6, 100 mm × 30 mm, 5 µm, A: water (0.225% formic acid); B: acetonitrile, 45%-75%: 15 minutes) to obtain compound **66** formate. MS-ESI [M+H]⁺, calculated: 584, found: 584.¹H NMR (400 MHz, MeOD) *δ* 8.60 (s, 2H), 7.57 (s, 1H), 7.22-7.32 (m, 2H), 6.89-6.97 (m, 3H), 5.17 (tt, *J* = 5.6, 2.8 Hz, 1H), 5.02 (dd, *J* = 9.6, 3.6 Hz, 1H), 4.02 (br dd, *J* = 10.8, 5.6 Hz, 2H), 3.85 (br dd, *J* = 10.8, 2.8 Hz, 4H), 3.59-3.75 (m, 4H), 2.78-2.90 (m, 1H), 2.32 (dt, *J* = 13.6, 3.2 Hz, 1H).

### Example 67 Synthesis of Compound 67

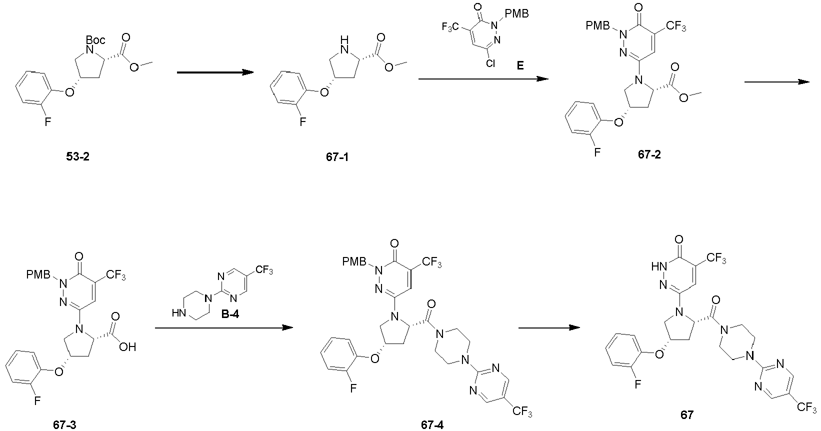

(1) To a solution of compound **53-2** (3.31 g, 9.75 mmol) in dichloromethane (30.0 mL), trifluoroacetic acid (15.40 g, 135.06 mmol, 10.0 mL) was added. The reaction mixture was stirred at 25°C for 1 hour. The reaction solution was diluted with water (10.0 mL) and extracted with dichloromethane (10.0 mL). The organic layer was washed with saturated sodium bicarbonate solution (15.0 mL × 2), dried over anhydrous sodium sulfate, filtered and concentrated under reduced pressure to obtain compound **67-1**. MS-ESI [M+H]⁺, calculated: 240, found: 240.
(2) To a solution of compound **E** (1.60 g, 5.02 mmol) and compound **67-1** (1.00 g, 4.18 mmol) in toluene (10.0 mL), cesium carbonate (2.72 g, 8.36 mmol), 1,1'-binaphthyl-2,2'-diphenylphosphine (520 mg, 835 µmol) and tris (dibenzylideneacetone) dipalladium (382 mg, 417 µmol) were added. The reaction solution was stirred under nitrogen atmosphere at 70°C for 4 hours. The reaction solution was diluted with ethyl acetate (10.0 mL), and washed with water (10.0 mL). The organic layer was dried over anhydrous sodium sulfate, filtered, and concentrated under reduced pressure. The crude product was purified by silica gel column chromatography (petroleum ether/ethyl acetate = 1:0 to 3:2) to obtain compound **67-2**. MS-ESI [M+H]⁺, calculated: 522, found: 522.
(3) Compound **67-2** (560 mg, 1.07 mmol) was dissolved in tetrahydrofuran (9.0 mL) and water (3.0 mL). Lithium hydroxide monohydrate (450 mg, 10.7 mmol) was added. The reaction mixture was stirred at 35°C for 12 hours. Water (10.0 mL) was added to the reaction mixture, and pH was adjusted to 5-6 with saturated citric acid solution. The reaction mixture was extracted with ethyl acetate (15.0 mL × 2). The combined organic layer was dried over anhydrous sodium sulfate, filtered and concentrated under reduced pressure to obtain compound **67-3**. MS-ESI [M+H]⁺, calculated: 508, found: 508.
(4) To a solution of compound **67-3** (300 mg, 591 µmol) and compound **B-4** (164 mg, 709 µmol) in dichloromethane (5.0 mL), diisopropylethylamine (382 mg, 2.96 mmol, 514 µL) and 50% propylphosphonic anhydride (1.13 g, 1.77 mmol, 1.05 mL) were added. The reaction was stirred at 25°C for 2 hours. The reaction solution was diluted with water (10.0 mL), and extracted with dichloromethane (10.0 mL). The organic layer was dried with anhydrous sodium sulfate, filtered and concentrated under reduced pressure. The crude product was purified by silica gel column chromatography (petroleum ether/ethyl acetate = 1 : 0 to 1 : 1) to obtain compound **67-4**. MS-ESI [M+H]⁺, calculated: 722, found: 722.
(5) Compound **67-4** (60 mg, 83.1 µmol) was dissolved in trifluoroacetic acid (3.0 mL) and trifluoromethanesulfonic acid (0.3 mL). The mixture was stirred at 25°C for 2 hours. The reaction solution was diluted with water (10.0 mL) and extracted with dichloromethane (10.0 mL). The organic layer was washed with saturated sodium bicarbonate (15.0 mL × 2), dried with anhydrous sodium sulfate, filtered and concentrated under reduced pressure. The crude product was purified by preparative high performance liquid chromatography (C18-6, 100 mm × 30 mm, 5 µm, A: water (0.225% formic acid); B: acetonitrile, 55%-85%, 15 minutes) to obtain compound **67** formate. MS-ESI [M+H]⁺, calculated: 602, found: 602.¹H NMR (400 MHz, MeOD) δ 8.60 (s, 2H), 7.57 (s, 1H), 7.05-7.15 (m, 3H), 6.92-7.00 (m, 1H), 5.14-5.23 (m, 1H), 5.04 (dd, *J* = 9.4, 3.8 Hz, 1H), 4.03 (br dd, *J* = 10.8, 5.8 Hz, 2H), 3.84-3.96 (m, 4H), 3.62-3.81 (m, 4H), 2.80-2.90 (m, 1H), 2.33 (dt, *J* = 13.8, 3 Hz, 1H).

### Example 68 Synthesis of Compound 68

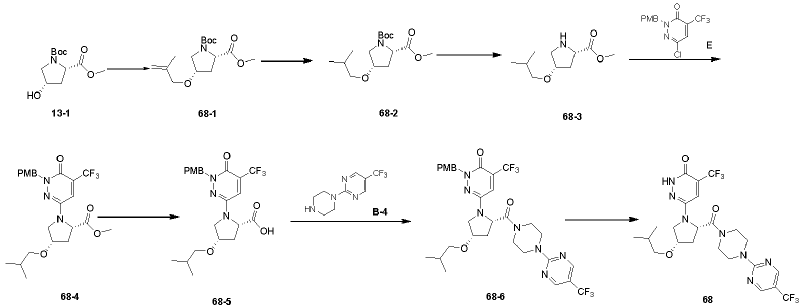

(1) To a solution of compound **13-1** (5.00 g, 20.3 mmol) in tetrahydrofuran (30.0 mL), sodium hydride (1.63 g, 40.7 mmol, 60% purity) was added at 0°C. The reaction mixture was stirred under nitrogen atmosphere for 1 hour at 0°C, 3-bromo-2-methylpropene (8.26 g, 61.1 mmol, 6.16 mL) was added. The reaction was stirred at 25°C for 4 hours. The reaction mixture was slowly poured into a solution of 0.5 mol/L hydrochloric acid (50.0 mL) at 0°C. The reaction solution was extracted with dichloromethane (20.0 mL × 3). The organic layer was dried over anhydrous sodium sulfate, filtered and concentrated under reduced pressure. The crude product was purified by silica gel column chromatography (petroleum ether/ethyl acetate = 1:0 to 21:4) to obtain compound **68-1**. MS-ESI [M+H]⁺, calculated: 300, found: 300.¹H NMR (400 MHz, CDCl₃) *δ* 4.82-4.95 (m, 2H), 4.25-4.43 (m, 1H), 3.99-4.12 (m, 1H), 3.78-3.87 (m, 2H), 3.66-3.73 (m, 3H), 3.46-3.64 (m, 2H), 1.99-2.35 (m, 2H), 1.66-1.72 (m, 3H), 1.37-1.45 (m, 9H).
(2) Compound **68-1** (1.0 g, 3.34 mmol) was dissolved in tetrahydrofuran (10.0 mL), and 10% palladium on carbon (300 mg) was added. The reaction mixture was stirred under a hydrogen atmosphere (15 psi) at 25°C for 10 hours. The reaction mixture was filtered, concentrated under reduced pressure to obtain compound **68-2**. ¹H NMR (400 MHz, CDCl₃) *δ* 4.25-4.48 (m, 1H), 3.91-4.10 (m, 1H), 3.70-3.76 (m, 3H), 3.40-3.68 (m, 2H), 3.02-3.26 (m, 2H), 1.95-2.38 (m, 2H), 1.69-1.87 (m, 1H), 1.39-1.50 (m, 9H), 0.83-0.91 (m, 6H).
(3) To a solution of compound **68-2** (906 mg, 3.01 mmol) in dichloromethane (9.0 mL), trifluoroacetic acid (4.62 g, 40.5 mmol, 3.0 mL) was added. The reaction mixture was stirred at 25°C for 1 hour. The reaction solution was diluted with water (10.0 mL) and extracted with dichloromethane (10.0 mL). The organic layer was washed with saturated sodium bicarbonate solution (15.0 mL × 2), dried over anhydrous sodium sulfate, filtered and concentrated under reduced pressure to obtain compound **68-3**.
(4) To a solution of compound **E** (653 mg, 2.05 mmol) and compound **68-3** (413 mg, 2.05 mmol) in toluene (5.0 mL), cesium carbonate (1.34 g, 4.10 mmol), 1,1'-binaphthyl-2,2'-diphenylphosphine (255 mg, 410 µmol) and tris (dibenzylideneacetone) dipalladium (187 mg, 205 µmol) were added. The reaction solution was stirred under nitrogen atmosphere at 70°C for 4 hours. The reaction solution was diluted with ethyl acetate (20.0 mL) and washed with water (10.0 mL). The organic layer was dried over anhydrous sodium sulfate, filtered and concentrated by reduced pressure. The crude product was purified by silica gel column chromatography (petroleum ether/ethyl acetate = 1:0 to 1:1) to obtain compound **68-4**. MS-ESI [M+H]⁺, calculated: 484, found: 484.
(5) Compound **68-4** (323 mg, 668.07 µmol) was dissolved in tetrahydrofuran (9.0 mL) and water (3.0 mL), lithium hydroxide monohydrate (280 mg, 6.68 mmol) was added. The reaction mixture was stirred at 35°C for 10 hours. The reaction solution was diluted with water (10.0 mL), adjusted pH to 5-6 with citric acid. The reaction mixture was extracted with ethyl acetate (30.0 mL). The organic layer was dried over anhydrous sodium sulfate, filtered and concentrated under reduced pressure to obtain compound **68-5**. MS-ESI [M+H]⁺, calculated: 470, found: 470.
(6) To a solution of compound **68-5** (244 mg, 519 µmol), **B-4** (120 mg, 519 µmol) in dichloromethane (5.0 mL), diisopropylethylamine (335mg, 2.60 mmol, 452 µL) and 50% propylphosphonic anhydride (992 mg, 1.56 mmol, 927 µL) was added. The mixture was stirred at 25°C for 1 hours. The reaction solution was diluted with water (10.0 mL), extracted with dichloromethane (10.0 mL). The organic layer was dried over anhydrous sodium sulfate, filtered and concentrated under reduced pressure. The crude product was purified by silica gel column chromatography (petroleum ether/ethyl acetate = 1:0 to 1:1) to obtain compound **68-6**. MS-ESI [M+H]⁺, calculated: 684, found: 684.¹H NMR (400 MHz, CDCl₃) *δ* (ppm) 8.41-8.50 (m, 2H), 7.12-7.17 (m, 3H), 6.66-6.72 (m, 2H), 4.94-5.04 (m, 2H), 4.73-4.81 (m, 1H), 4.16-4.26 (m, 1H), 3.93-4.02 (m, 1H), 3.72-3.86 (m, 4H), 3.65-3.70 (m, 3H), 3.42-3.64 (m, 5H), 3.10-3.16 (m, 2H), 2.24-2.35 (m, 1H), 2.04-2.14 (m, 1H), 1.70-1.82 (m, 1H), 0.79-0.87 (m, 6H).
(7) Compound **68-6** (60 mg, 87.7 umol) was dissolved in trifluoroacetic acid (2.0 mL) and trifluoromethanesulfonic acid (200 µL). The reaction solution was stirred at 25°C for 1 hour. It was diluted with water (10.0 mL) and extracted with dichloromethane (10.0 mL). The organic layer was washed with saturated sodium bicarbonate (30.0 mL), dried over anhydrous sodium sulfate, filtered and concentrated under reduced pressure. The crude product was purified by preparative high performance liquid chromatography (C18-6, 100 mm × 30 mm 5 µm, A: water (0.225% formic acid); B: acetonitrile, 15%-85%: 15 minutes) to obtain compound **68** formate. MS-ESI [M+H]⁺, calculated: 564, found: 564.¹H NMR (400 MHz, MeOD) *δ* 8.62 (s, 2H), 7.59 (s, 1H), 4.99 (br t, *J* = 7.6 Hz, 1H), 4.24-4.31 (m, 1H), 4.06-4.20 (m, 2H), 3.97-4.04 (m, 1H), 3.88-3.96 (m, 1H), 3.77-3.87 (m, 3H), 3.72 (ddd, *J* = 13.2, 8.0, 3.2 Hz, 1H), 3.65 (dd, *J* = 10.4, 1.6 Hz, 1H), 3.51-3.61 (m, 1H), 3.23-3.29 (m, 2H), 2.43-2.53 (m, 1H), 2.14 (ddd, *J* = 13.2, 7.2, 5.2 Hz, 1H), 1.71-1.91 (m, 1H), 0.91 (dd, *J* = 6.8, 5.2 Hz, 6H).

### Example 69 Synthesis of Compound 69

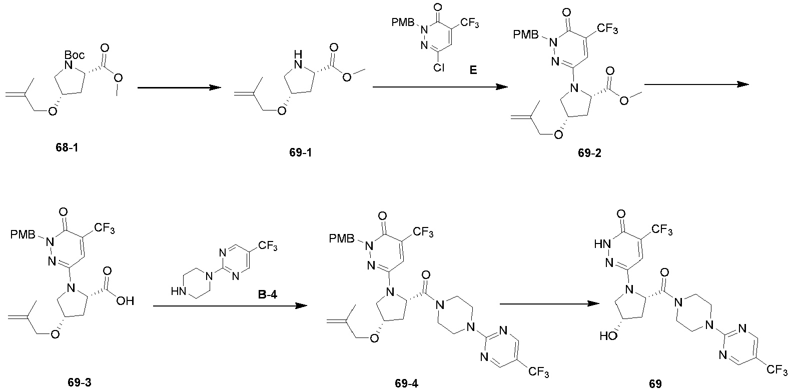

(1) To a solution of compound **68-1** (2.70 g, 9.02 mmol) in dichloromethane (48.0 mL), trifluoroacetic acid (18.4 g, 162 mmol, 12.0 mL) was added. The reaction solution was stirred at 25°C for 1 hour. The mixture was adjusted to pH 7-8 using saturated sodium carbonate solution, extracted with dichloromethane (20.0 mL × 3). The organic layer was combined and dried over anhydrous sodium sulfate, filtered and concentrated under reduced pressure to obtain compound **69-1**.
(2) To a solution of compound **E** (1.25 g, 3.91 mmol) and compound **69-1** (649 mg, 3.26 mmol) in toluene (10.0 mL), cesium carbonate (2.12 g, 6.51 mmol), 1,1'-binaphthyl-2,2'-diphenylphosphine (405 mg, 651 µmol) and tris (dibenzylideneacetone) dipalladium (298 mg, 325 µmol) was added. The reaction solution was stirred under nitrogen atmosphere at 80°C for 15 hours. The reaction mixture was concentrated by reduced pressure. The crude product was purified by silica gel column chromatography (petroleum ether/ethyl acetate = 1:0 to 11:9) to obtain compound **69-2**. MS-ESI [M+H]⁺, calculated: 482, found: 482.
(3) Compound **69-2** (380 mg, 789 µmol) was dissolved in tetrahydrofuran (18.0 mL) and water (6.0 mL). Lithium hydroxide monohydrate (331 mg, 7.89 mmol) was added and the reaction mixture was stirred at 30°C for 17 hours. The reaction solution was adjusted to pH 3-4 with 1 mol/L hydrochloric acid solution, and extracted with dichloromethane (20.0 mL × 3). The organic layers were combined, dried over anhydrous sodium sulfate, filtered, and concentrated under reduced pressure to obtain compound **69-3**.
(4) To a solution of compound **69-3** (360 mg, 770 µmol), **B-4** (214 mg, 924 µmol) in dichloromethane (10.0 mL), diisopropylethylamine (298 mg, 2.31 mmol, 402 µL) and 50% propylphosphonic anhydride (1.47 g, 2.31 mmol, 1.37 mL) were added. The mixture was stirred at 30°C for 2 hours. The reaction solution was diluted with water (5.0 mL), extracted with dichloromethane (10.0 mL × 3). The organic layer was dried over anhydrous sodium sulfate, filtered, and concentrated under reduced pressure to obtain compound **69-4**. MS-ESI [M+H]⁺, calculated: 682, found: 682.
(5) Compound **69-4** (120 mg, 176 µmol) was dissolved in trifluoroacetic acid (10.0 mL) and trifluoromethanesulfonic acid (6.0 mL). The mixture was stirred at 70°C for 1 hour. The reaction solution was concentrated under reduced pressure. The crude product was purified by preparative high-performance liquid chromatography (Welch Xtimate C18, 150 mm × 30 mm 5 µm, A: water (0.225% formic acid); B: acetonitrile, 0%-90%: 36 minutes) to obtain compound **69** formate. MS-ESI [M+H]⁺, calculated: 508, found: 508.¹H NMR (400 MHz, MeOD) *δ* 8.61 (s, 2H), 7.56 (s, 1H), 5.03 (t, *J* = 7.6 Hz, 1H), 4.55-4.59 (m, 1H), 4.03-4.19 (m, 3H), 3.99-4.02 (m, 1H), 3.89-3.96 (m, 1H), 3.84 (br dd, *J* = 10.4, 4.8 Hz, 3H), 3.69-3.76 (m, 1H), 3.58 (dt, *J* = 8.4, 4.4 Hz, 1H), 3.52 (br d, *J* = 10.4 Hz, 1H), 2.34-2.41 (m, 1H), 2.12 (ddd, *J* = 13.2, 7.8, 5.2 Hz, 1H).

### Example 70 Synthesis of Compound 70

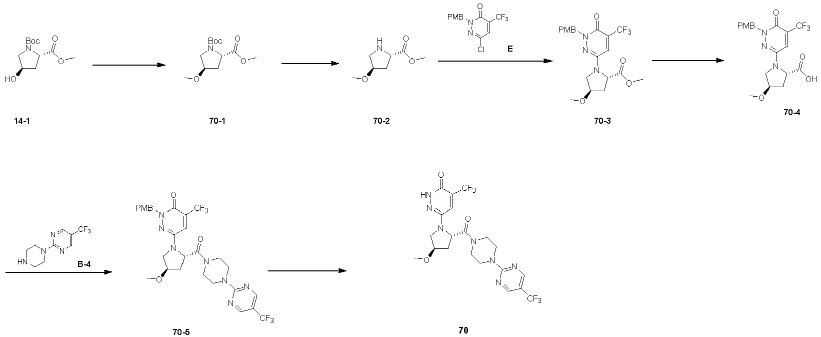

(1) To a solution of compound **14-1** (5.00 g, 20.3 mmol) in tetrahydrofuran (50.0 mL), sodium hydride (1.63 g, 40.7 mmol, 60% purity) was added at 0°C. The mixture was stirred for 1 hour under nitrogen atmosphere at 0°C, and a solution of methyl iodide (14.4 g, 101 mmol) in tetrahydrofuran (10.0 mL) was added dropwise. The mixture was stirred for 1 hour at 25°C. The reaction solution was quenched with water (15.0 mL) and then extracted with ethyl acetate (20.0 mL) and water (20.0 mL). The organic layer was dried over anhydrous sodium sulfate, filtered and concentrated under reduced pressure. The crude product was purified by silica gel chromatography (petroleum ether/ethyl acetate = 4:1) to obtain compound **70-1**. ¹H NMR (400 MHz, CDCl₃) *δ* 4.26-4.47 (m, 1H), 3.89-4.02 (m, 1H), 3.69-3.79 (m, 3H), 3.45-3.68 (m, 2H), 3.24-3.35 (m, 3H), 2.19-2.43 (m, 1H), 2.04 (ddd, *J* = 13.2, 7.6, 5.2 Hz, 1H), 1.40-1.49 (m, 9H).
(2) To a solution of compound **70-1** (3.50 g, 13.5 mmol) in dichloromethane (30.0 mL), trifluoroacetic acid (15.4 g, 135 mmol) was added. The mixture was stirred at 25°C for 1 hour, and concentrated under reduced pressure. Saturated sodium bicarbonate solution (15.0 mL) and water (10.0 mL) was added, followed by extraction with dichloromethane (10.0 mL). The organic phase was dried over anhydrous sodium sulfate, filtered, and concentrated under reduced pressure to obtain compound **70-2**.
(3) To a mixture of compound **70-2** (830 mg, 5.21 mmol), compound **E** (1.66 g, 5.21 mmol), cesium carbonate (3.40 g, 10.4 mmol), tris (dibenzylideneacetone) dipalladium (477 mg, 521 µmol), and 1,1'-binaphthyl-2,2'-diphenylphosphine (649 mg, 1.04 mmol) was added toluene (20.0 mL). The mixture was stirred at 80°C under nitrogen atmosphere for 12 hours. It was extracted with ethyl acetate (10.0 mL) and water (10.0 mL). The organic phase was dried over anhydrous sodium sulfate, filtered and concentrated under reduced pressure. The crude product was purified by silica gel chromatography (petroleum ether/ethyl acetate = 4:1) to obtain compound **70-3**. MS-ESI [M+H]⁺, calculated: 442, found: 442.
(4) Compound **70-3** (680 mg, 1.54 mmol) was dissolved in tetrahydrofuran (9.00 mL) and water (3.0 mL). Lithium hydroxide monohydrate (646 mg, 15.4 mmol) was added, and the mixture was stirred at 35°C for 12 hours. Water (10.0 mL) was added to the solution and citric acid solution was added to adjust pH to 5-6. The mixture was extracted with ethyl acetate (15.0 mL × 2). The organic layer was dried over anhydrous sodium sulfate, filtered and concentrated under reduced pressure to obtain compound **70-4**. MS-ESI [M+H]⁺, calculated: 428, found: 428.
(5) To a solution of intermediate **70-4** (292 mg, 683 µmol) in dichloromethane (10.0 mL), compound **B-4** trifluoroacetate (158 mg, 683µmol), diisopropylethylamine (441 mg, 3.42 mmol), and propylphosphonic anhydride solution (1.30 g, 2.05 mmol, purity 50%) were added. The mixture was stirred at 25°C under nitrogen atmosphere for 2 hours. The mixture was extracted with dichloromethane (10.0 mL) and water (10.0 mL). The combined organic layer was dried over anhydrous sodium sulfate, filtered, and concentrated under reduced pressure. The crude product was purified by silica gel column chromatography (petroleum ether/ethyl acetate = 2:1) to obtain compound **70-5**. MS-ESI [M+H]⁺, calculated: 642, found: 642.
(6) Compound **70-5** (150 mg, 233 µmol) was dissolved in trifluoroacetic acid (5.00 mL), and trifluoromethanesulfonic acid (0.50 mL) was added. The mixture was stirred at 25°C for 2 hours. Saturated sodium bicarbonate solution (30.0 mL) was added to the mixture. The mixture was extracted with dichloromethane (10.0 mL) and water (10.0 mL). The organic layer was concentrated under reduced pressure. The crude product was purified by preparative high performance liquid chromatography (Waters Xbridge BEH C18, 100 mm × 30 mm 10 µm; A: water (0.225% formic acid); B: acetonitrile, gradient from 14% to 54% over 30 minutes) to obtain compound 70 formate. MS-ESI [M+H]⁺, calculated: 522, found: 522.¹H NMR (400 MHz, MeOD) *δ* 8.62 (s, 2H), 7.59 (s, 1H), 4.62 (s, 1H), 4.06-4.22 (m, 3H), 3.97-4.05 (m, 1H), 3.92 (ddd, *J* = 13.4, 6.4, 3.2 Hz, 1H), 3.78-3.87 (m, 3H), 3.65-3.76 (m, 2H), 3.53-3.60 (m, 1H), 3.37 (s, 3H), 2.45-2.55 (m, 1H), 2.12 (ddd, *J* = 13.4, 7.6, 5.6 Hz, 1H).

### Example 71 Synthesis of Compound 71

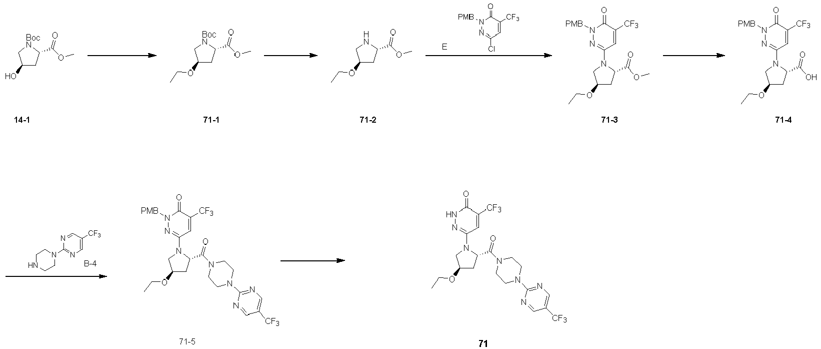

(1) At 0°C, to a solution of compound **14-1** (5.00 g, 20.3 mmol) in tetrahydrofuran (50.0 mL) was added sodium hydride (1.63 g, 40.7 mmol). The mixture was stirred at 0°C for 1 hour. A solution of iodoethane (15.9 g, 101 mmol) in tetrahydrofuran (10.0 mL) was added and the reaction mixture was stirred at 25°C for 1 hour. Water (15.0 mL) was added. The reaction mixture was extracted with ethyl acetate (20.0 mL) and water (20.0 mL). The organic layer was dried with anhydrous sodium sulfate, filtered, and concentrated under reduced pressure. The crude product was purified by silica gel column chromatography (petroleum ether/ethyl acetate = 7:1) to obtain compound **71-1**. ¹H NMR (400 MHz, CDCl₃) *δ* 4.27-4.35 (m, 1H), 4.07 (dt, *J* = 7.6, 4.0 Hz, 1H), 3.69-3.78 (m, 3H), 3.38-3.52 (m, 4H), 2.19-2.38 (m, 2H), 1.41 (br d, *J* = 4.8 Hz, 9H), 1.19 (t, *J* = 7.2 Hz, 3H).
(2) Compound **71-1** (4.00 g, 14.6 mmol) was dissolved in dichloromethane (40.0 mL), trifluoroacetic acid (15.4 g, 135 mmol) was added. The mixture was stirred at 25°C for 1 hour. The reaction mixture was added with saturated sodium bicarbonate solution (30.0 mL) and water (10.0 mL). It was extracted with dichloromethane (10.0 mL). The organic layer was dried with anhydrous sodium sulfate, filtered and concentrated under reduced pressure to obtain compound **71-2**.
(3) To a mixture of compound **71-2** (1.20 g, 6.93 mmol), compound **E** (2.21 g, 6.93 mmol), cesium carbonate (4.51 g, 13.8 mmol), tris (dibenzylideneacetone) dipalladium (634 mg, 692 µmol), and 1,1'-binaphthyl-2,2'-diphenylphosphine (862 mg, 1.39 mmol) were added toluene (10.0 mL). And the mixture was stirred at 80°C under nitrogen atmosphere for 12 hours. Water (20.0 mL) was added, followed by extraction with ethyl acetate (20.0 mL). The organic phase was dried over anhydrous sodium sulfate, filtered and concentrated under reduced pressure. The crude product was purified by silica gel chromatography (petroleum ether/ethyl acetate = 3:1) to obtain compound **71-3**. MS-ESI [M+H]⁺, calculated: 456, found: 456.
(4) Compound **71-3** (1.43 g, 3.14 mmol) was dissolved in tetrahydrofuran (9.00 mL) and water (3.00 mL). Lithium hydroxide monohydrate (1.32 g, 31.4 mmol) was added and the mixture was stirred at 35°C for 12 hours. Citric acid solution was added to adjust pH to 5-6. Water (10.0 mL) was added, followed by extraction with ethyl acetate (30.0 mL). The organic layer was dried over anhydrous sodium sulfate, filtered and concentrated under reduced pressure to obtain compound **71-4**. MS-ESI [M+H]⁺, calculated: 442, found: 442.
(5) To a solution of intermediate **71-4** (1.10 g, 2.49 mmol) in dichloromethane (20.0 mL), compound **B-4** trifluoroacetate (578 mg, 2.49 mmol), diisopropylethylamine (1.61 g, 12.4 mmol) and propylphosphonic anhydride solution (4.76 g, 7.48 mmol, 50% purity in ethyl acetate) were added. The mixture was stirred at 25°C for 2 hours. The reaction solution was extracted with dichloromethane (10.0 mL) and water (10.0 mL). The organic layer was dried over anhydrous sodium sulfate, filtered and concentrated under reduced pressure to give compound **71-5**. MS-ESI [M+H]⁺, calculated: 656, found: 656.
(6) Compound **71-5** (200 mg, 305 µmol) was dissolved in trifluoroacetic acid (3.00 mL), trifluoromethanesulfonic acid (510 mg, 3.40 mmol) was added. The solution was stirred at 25°C for 2 hours. The reaction solution was added with saturated sodium bicarbonate solution (30.0 mL). It was extracted with dichloromethane (10.0 mL) and water (10.0 mL). The organic phase was concentrated under reduced pressure. The crude product was purified by preparative high performance liquid chromatography (Xtimate C18, 100 mm × 30 mm 10 µ m; Water (0.225% formic acid); B: acetonitrile, 50%-80%: 10 minutes) to obtain compound **71** formate. MS-ESI [M+H]⁺, calculated: 536, found: 536.¹H NMR (400 MHz, MeOD) *δ* 8.58-8.65 (m, 2H), 7.58 (s, 1H), 4.62 (s, 1H), 4.26-4.35 (m, 1H), 3.97-4.20 (m, 3H), 3.89-3.96 (m, 1H), 3.79-3.86 (m, 3H), 3.62-3.76 (m, 2H), 3.51-3.61 (m, 3H), 2.40-2.54 (m, 1H), 2.13 (ddd, *J* = 13.2, 7.2, 5.6 Hz, 1H), 1.19 (t, *J* = 7.2 Hz, 3H).

### Example 72 Synthesis of Compound 72

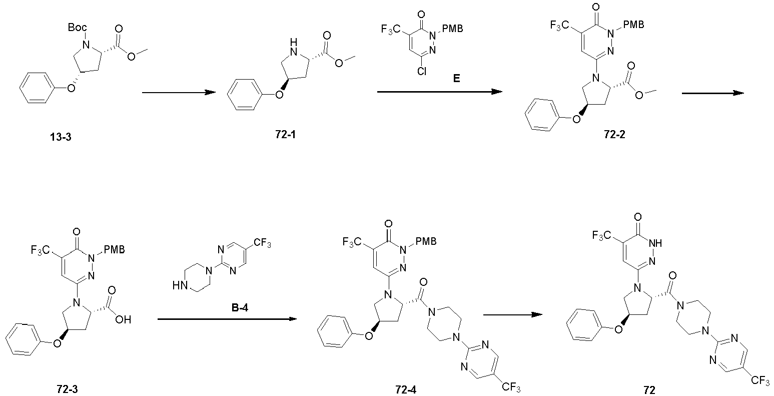

(1) To a solution of compound **13-3** (4.40 g, 13.7 mmol) in dichloromethane (40.0 mL), trifluoroacetic acid (15.4 g, 135 mmol, 10.0 mL) was added. The reaction mixture was stirred at 25°C for 0.5 hours. The reaction mixture was diluted with water (10.0 mL) and extracted with dichloromethane (10.0 mL). The organic layer was washed with saturated sodium carbonate solution (30.0 mL), dried over anhydrous sodium sulfate, filtered, and concentrated under reduced pressure to give compound **72-1**. MS-ESI [M+H]+, calculated: 222, found: 222.
(2) To a solution of compound **E** (2.16 g, 6.78 mmol) and **72-1** (1.50 g, 6.78 mmol) in toluene (30.0 mL), cesium carbonate (4.42 g, 13.6 mmol), 1,1'-binaphthyl-2,2'-diphenylphosphine (844 mg, 1.36 mmol) and tris(dibenzylideneacetone)dipalladium (621 mg, 678 µmol) were added. The reaction mixture was stirred at 80°C under nitrogen atmosphere for 4 hours. The reaction mixture was concentrated by reduced pressure. The residue was dissolved in ethyl acetate (40.0 mL), washed with water (40.0 mL). The organic layer was dried over anhydrous sodium sulfate, filtered and concentrated under reduced pressure. The crude product was purified by silica gel column chromatography (petroleum ether/ethyl acetate = 1:0 to 1:2) to give compound **72-2**. MS-ESI [M+H]+, calculated: 504, found: 504.
(3) Compound **72-2** (1.40 g, 2.78 mmol) was dissolved in tetrahydrofuran (27.0 mL) and water (9.0 mL). Lithium hydroxide monohydrate (1.17 g, 27.8 mmol) was added and the reaction mixture was stirred at 35°C for 12 hours. 1 mol/L hydrochloric acid solution was added to adjust the pH to 1. Ethyl acetate (45.0 mL) was added for extraction. The organic layer was washed with water (45.0 mL), dried over anhydrous sodium sulfate, filtered and concentrated under reduced pressure to give compound **72-3**. MS-ESI [M+H]+, calculated: 490, found: 490.
(4) To a solution of compound **72-3** (500 mg, 1.02 mmol) and **B-4** (356 mg, 1.53 mmol) in dichloromethane (5.0 mL), diisopropylethylamine (660 mg, 5.11 mmol, 890 µL) and HATU (1.17 g, 3.06 mmol) were added. The reaction mixture was stirred at 25°C for 2 hours. The reaction solution was diluted with water (10.0 mL), followed by extraction with ethyl acetate (10.0 mL). The organic layer was dried over anhydrous sodium sulfate, filtered and concentrated under reduced pressure. The crude product was purified by silica gel column chromatography (petroleum ether/ethyl acetate = 1:0 to 1:1) to obtain compound **72-4**. MS-ESI [M+H]+, calculated: 704, found: 704.
(5) Compound **72-4** (122 mg, 173 µmol) was dissolved in trifluoroacetic acid (3.4 mL) and trifluoromethanesulfonic acid (343 µL). The reaction solution was stirred at 25°C for 1 hour. The pH was adjusted to 11 with saturated sodium carbonate, followed by extraction with dichloromethane (30.0 mL). The organic phase was washed with water (30.0 mL), dried over anhydrous sodium sulfate, filtered and concentrated under reduced pressure. The crude product was purified by preparative high performance liquid chromatography (Xtimate C18, 100 mm × 30 mm 10 µm, A: water (0.225% formic acid); B: acetonitrile, 65% to 95% over 10 minutes) to obtain compound **72** formate. MS-ESI [M+H]⁺, calculated: 584, found: 584. ¹H NMR (400 MHz, MeOD) *δ* 8.61 (s, 2H), 7.58 (s, 1H), 7.23-7.37 (m, 2H), 6.89-7.05 (m, 3H), 5.17-5.22 (m, 1H), 5.13 (t, *J* = 7.6 Hz, 1H), 4.05-4.17 (m, 2H), 3.96-4.05 (m, 2H), 3.92 (ddd, *J* = 13.2, 6.4, 3.2 Hz, 1H), 3.76-3.88 (m, 3H), 3.66-3.75 (m, 1H), 3.51-3.62 (m, 1H), 2.61-2.72 (m, 1H), 2.35 (ddd, *J* = 13.2, 7.6, 5.2 Hz, 1H).

### Test Example 1

### Inhibitory effect of compounds on the proliferation of NCI-H1373 cells

1. Experimental Principle: NCI-H1373 cells are a type of lung adenocarcinoma cells that overexpress PARP7. The compounds disclosed in this invention inhibit NCI-H1373 cell proliferation by suppressing PARP7.
2. Experimental Materials: NCI-H1373 cells were obtained from Nanjing Cobioer Biosciences Co., Ltd. CellTiter-Glo^{®} (cell viability chemiluminescent assay reagent) was purchased from Promega (Catalog No. G7571). RPMI-1640 medium was obtained from ATCC (Catalog No. 30-2001). RPMI-1640 medium, penicillin/streptomycin antibiotics were purchased from WISENT. Fetal bovine serum was obtained from Biosera. The Nivo multimode reader was procured from PerkinElmer.
3. Experimental Method: NCI-H1373 cells were seeded in a white 96-well plate with 80 µL of cell suspension per well, containing 3000 NCI-H1373 cells. The cell plate was incubated overnight in a carbon dioxide (CO₂) incubator.

The test compounds were serially diluted 5-fold to the 9th concentration, ranging from 2 mmol/L to 5.12 nmol/L, for a duplicate assay. 78 µL of culture medium was added to the intermediate plate, and then, according to the corresponding positions, 2 µL of each gradient-diluted compound was transferred to the intermediate plate. After thorough mixing, 20 µL of the compound solution was transferred to the cell plate. The compound concentration range in the cell plate ranged from 10 µmol/L to 0.0256 nmol/L. The cell plate was incubated in a CO₂ incubator for 6 days.

Another cell plate was prepared, and the signal values were read on the day of dosing to obtain the maximum value referred to as Max in the following equation for data analysis. 25 µL of cell viability chemiluminescent assay reagent was added to each well of this cell plate, and it was incubated at room temperature for 10 minutes to stabilize the luminescent signal. Readings were taken using a multimode reader.

After 6 days of treatment, 25 µL of cell viability chemiluminescent assay reagent was added to each well of the cell plate, and it was incubated at room temperature for 10 minutes to stabilize the luminescent signal. Readings were taken using a multimode reader.

The original data were converted to inhibition rates using the equation (Sample-Min)/(Max-Min)*100%. The IC₅₀ value was determined by four-parameter curve fitting (obtained by using the "log(inhibitor) vs. response -- Variable slope" model in GraphPad Prism).

Specific test results are shown in Table 1.

**Table 1.**

| Compound | H1373 IC₅₀(nM) |
|---|---|
| Formate of 1 | 38 |
| Formate of 6 | 10 |
| Formate of 20 | 12.4 |
| Formate of 21 | 73.6 |
| 51 | 69.2 |
| Formate of 52 | 95.9 |
| 63 | 12.4 |
| Formate of 70 | 58.2 |
| Formate of 71 | 44.6 |

Conclusion: The test data from Table 1 indicates that the compounds as described in formula I in this invention exhibit significant inhibitory effects on the growth of NCI-H1373 cells, showing the potential for the development of drugs for the treatment and prevention of PARP7-related diseases.

### Test Example 2

### Inhibition of Compounds on PARP1/7 Enzyme Activity

1. Experimental Principle: The inhibitory effect of the test substance on the enzymatic activity of PARP1/7 is assessed by using an Enzyme-Linked Immunosorbent Assay (ELISA).
2. Experimental Method:
   (1) Pre-coating: Add 100 µL of a PBS buffer (10 mM NaH₂PO₄, 10 mM Na₂HPO₄, 150 mM NaCl, pH 7.4) containing 20 µg/mL of histone to each well of a 96-well plate, and incubate at 4°C overnight.
   (2) Add 30 µL of reaction buffer (50 mM Tris, 2 mM MgCl₂, pH 8.0) containing 100 µM NAD⁺, 25 µM biotinylated NAD⁺, and 200 nM slDNA to each well.
   (3) Add 5 µL of the test substance or solvent to each well.
   (4) Add 20 µL of PARP1 or PARP7 (50 ng/well), and incubate at 30°C for 1 hour.
   (5) Add 50 µL of streptavidin-HRP to the reaction mixture, and incubate at 30°C for 30 minutes.
   (6) Finally, add 100 µL of a citrate buffer containing H₂O₂ and luminol (0.1 M, pH 5.4), and measure the luminescent signal using a microplate reader (Molecular Devices SpectraMax M5).
   (7) Calculate the inhibition rate of PARP1 or PARP7 enzyme activity as [(control group - treatment group) / control group] × 100%. Fit the dose-response data with standard dilutions using Prism GraphPad software and calculate the concentration required to achieve 50% inhibition of PARP1 or PARP7 enzyme activity (IC₅₀).

Specific test results are shown in Table 2.

**Table 2.**

| Compound | PARP7 IC₅₀(nM) | PARP1 IC₅₀(nM) | The ratio of PARP1 IC₅₀ to PARP7 IC₅₀. |
|---|---|---|---|
| Formate of 1 | 4 | 293 | 73.25 |
| Formate 6 | 2.9 | 1.3 | 0.45 |
| 18 | 2.2 | 2.5 | 1.14 |
| 19 | 4.4 | 2015 | 457.95 |
| Formate of 20 | 3.2 | 49 | 15.31 |
| Formate of 21 | 3.4 | 816 | 240.00 |
| Formate of 22 | 4.6 | 31.6 | 6.87 |
| Formate of 23 | 3.2 | 9.7 | 3.03 |
| Formate of 24 | 2.1 | 4.1 | 1.95 |
| Formate of 27 | 4.4 | 12.5 | 2.84 |
| Formate of 29 | 25.6 | 18.3 | 0.71 |
| Formate of 30 | 5.1 | 7.6 | 1.49 |
| 31 | 4.4 | 25.9 | 5.89 |
| 32 | 10.1 | 14.6 | 1.45 |
| Formate of 33 | 22.2 | 46.4 | 2.09 |
| Formate of 34 | 3.3 | 13.6 | 4.12 |
| 35 | 4.3 | 47.0 | 10.93 |
| Formate of 36 | 3.8 | 14.4 | 3.79 |
| Formate of 37 | 7 | 49 | 7.00 |
| 38 | 5 | 36 | 7.20 |
| Formate of 39 | 8 | 60 | 7.50 |
| 40 | 4 | 7 | 1.75 |
| 41 | 4 | 4 | 1.00 |
| 42 | 37 | 6 | 0.16 |
| Formate of 43 | 4 | 4 | 1.00 |
| 44 | 7 | 18 | 2.57 |
| Formate of 49 | 26 | 79 | 3.04 |
| Formate of 50 | 13 | 15 | 1.15 |
| Formate of 55 | 82 | 15558 | 189.73 |
| Formate of 58 | 54 | 3187 | 59.02 |
| 63 | 3 | 179 | 59.67 |
| Formate of 66 | 4 | 427 | 106.75 |
| Formate of 67 | 43 | 11994 | 278.93 |
| RBN-2397 | 4.6 | 122.5 | 26.63 |

Conclusion:The experimental results indicate that some compounds of the invention are selective inhibitors of PARP7 with high activity, and the selectivity is significantly higher than RBN-2397. Additionally, some compounds are highly active dual inhibitors of PARP1 and PARP7, with PARP1 activity significantly superior to RBN-2397.

### Test Example 3

Determination of the metabolic stability in liver microsomes of compounds
1. Experiment purpose: This study aims to determine the metabolic stability of selected compounds from the embodiments of the invention in liver microsomes of humans, dogs, monkeys, rats, and mice using the LC-MS/MS method.
2. Experimental materials:
   Test compounds: The test drug is self-made from the example compounds of the present invention;
   Positive control compound RBN2397 purchased from Shanghai BioChemPartner Biotechnology Co., Ltd.
   Liver microsomes obtained from Xenotech.
3. Experimental Method:
   Each incubation system had a total volume of 45 µL and was composed of 100 mM phosphate buffer (PBS, pH 7.4) containing 0.5 mg/mL of liver microsomes protein, 1.00 µM of the compound, and 2.00 mM NADPH. The incubation was carried out at 37°C in a CO₂ incubator. The reactions were terminated by adding 150 µL of cold acetonitrile at different time points (0, 5, 15, 30, and 45 minutes). The 96-well plate was shaken at 600 rpm for 10 minutes, followed by centrifugation at 6000 rpm for 15 minutes. The supernatant (80 µL) was mixed with 140 µL of pure water, and the remaining amount of the compound was determined using LC-MS/MS. The intrinsic clearance (Clᵢₙₜ) was calculated using the formula Clᵢₙₜ = k/ liver microsomes protein concentration, where "k" is the linear regression slope of log compound remaining percentage against incubation time.

Specific test results are presented in Table 3.

**Table 3**

| Compound | intrinsic clearance, Clᵢₙₜ (mL/min/kg) | | |
|---|---|---|---|
| | Human | Dog | Rat |
| RBN2397 | 87.98 | 40.80 | 144.14 |
| Formate of 6 | 64.59 | 31.64 | 136.71 |
| Formate of 20 | 46.61 | 28.68 | 74.41 |

The experimental results indicate that some compounds from the present invention exhibit good stability in liver microsomes.

### Test Example 4

### Determination of pharmacokinetic properties of the compound in rats

1. Test purpose: to determine the pharmacokinetic characteristics of some of the example compound of the present invention in SD rats after single intravenous injection(IV) and oral administration(PO) by LC-MS/MS method.
2. Test material: the test drug is self-made from the example compounds of the present invention; Positive control compound RBN2397 purchased from Shanghai BioChemPartner Biotechnology Co., Ltd. SD male rats were obtained from the Laboratory Animal Management Department of Shanghai Family Planning Research Institute. Animal production license number (SCXK (Shanghai) 2018-0006).
3. Test method:
   Preparation of 0.5% methyl cellulose: 5g of methyl cellulose were weighed and dissolved in 1000 mL of purified water.

Preparation of oral formulation: A required amount of test compound was weighed and dissolved in 0.5% methyl cellulose solution to obtain a suspension or solution of 0.5 mg/mL or 1.0 mg/mL.

Preparation of intravenous injection formulation: A required amount of test compound was weighed, and dissolved in 0.5 mL of dimethyl sulfoxide to prepare a solution of 4 mg/mL. 0.25 mL of the above solution was added into 0.5 mL of polyethylene glycol 15 hydroxystearate (solutol) and 4.25 mL of physiological saline to obtain a solution of 0.2 mg/mL.

Administration: SD male rats were orally administered (PO) after fasting overnight, and fed 4 hours after administration. The single intravenous injection (IV) group did not need fasting.

Sample Collection: blood was collected through jugular vein or other suitable vein, and 0.2 mL of blood was collected at each time point. Samples were placed into tubes containing ethylenediamine tetraacetic acid dipotassium salt and kept on ice before centrifugation. Within 1 hour after blood collection, the blood samples were centrifuged at 2-8°C and 6800 g for 6 minutes and stored at -80°C. The time point for blood collection was 0.083, 0.25, 0.5, 1, 2, 4, 8 and 24 hours after intravenous administration, and 0.25, 0.5, 1, 2, 4, 6, 8 and 24 hours after oral administration. The test results are shown in Table 4.

**Table 4**

| **Compound** | Intravenous injection | | | | Oral gavage | | |
|---|---|---|---|---|---|---|---|
| | t_{1/2}, h | Cₘₐₓ, ng/mL | AUC_{(0-∞)}, h*ng/mL | CL, mL/min/kg | Cₘₐₓ, ng/mL | AUC_{(0-∞)}, h*ng/mL | F, % |
| RBN2397 | 0.34 | 633.57 | 288.23 | 58.11 | 5.55 | 11.49 | 3.76 |
| Formate of 1 | 0.58 | 719.56 | 359.55 | 46.90 | 53.52 | 89.23 | 21.97 |
| Formate of 6 | 1.43 | 1946.95 | 1383.54 | 12.22 | 30.39 | 248.05 | 18.1 |
| 19 | 0.68 | 476.53 | 396.98 | 42.17 | 15.14 | 60.67 | 14.34 |
| Formate of 20 | 0.82 | 733.64 | 522.7 | 32.00 | 14.99 | 94.41 | 15.53 |
| Formate of 21 | 0.51 | 1210.32 | 481.84 | 36.89 | 40.21 | 110.69 | 21.18 |

The intravenous dosage was 1 mg/kg, and the pharmacokinetic parameters for oral administration were standardized to a 1 mg/kg dose. The data in Table 4 demonstrate that, compared to the positive compound RBN-2397, certain compounds from the embodiments of the present invention exhibit excellent pharmacokinetic properties in rats, particularly in terms of exposure and oral bioavailability, offering a significant advantage.

All references mentioned in the present invention were cited in this application as each reference was individually cited. Furthermore, it should be understood that, after reading the above description of the present invention, those skilled in the art may make various changes or modifications to the invention, and such equivalent forms are also within the scope defined by the claims appended to this application.

## Claims

1. A compound of formula I, or pharmaceutically acceptable salts thereof, enantiomers, diastereomers, tautomers, cis-trans isomers, solvates, polymorphs, deuterated compounds, or combinations thereof, 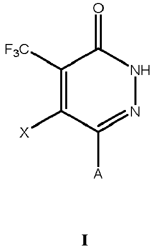 wherein,
X is selected from hydrogen, halogen, or cyano;
A is 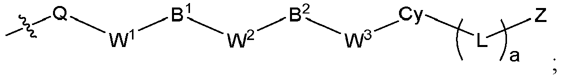 wherein,
Q is absent, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to 6-10 membered aryl, optionally substituted saturated or unsaturated 3-8 membered carbocyclyl fused to 5-10 membered heteroaryl, optionally substituted saturated or unsaturated 3-8 membered heterocyclyl fused to 6-10 membered aryl, optionally substituted saturated or unsaturated 3-8 membered heterocyclyl fused to 5-10 membered heteroaryl, optionally substituted 6-10 membered aryl, or optionally substituted 5-10 membered heteroaryl; wherein, the substitutions are defined as being replaced by one or more R groups;
W¹ is absent, -NR^{w}-, -O-, or -S-, wherein the R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups; at most one of Q and W1 is absent;
Each W² and W³ is independently absent, optionally substituted methylene group, -O-, -S-, -NR^{w'}-, -(C=O)-, -C(=O)O-, -C(=O)NR^{w'}-, -(S=O)-, -S(=O)₂-, -S(=O)₂NR^{w'}-, -S(=O)NR^{w'}-, or -NR^{w'}C(=O)NR^{w'}-, wherein, each R^{w'} is independently H, optionally substituted C1-C6 alkyl, optionally substituted saturated or unsaturated 3-8 membered carbocyclyl, or optionally substituted saturated or unsaturated 4-12 membered heterocyclyl; wherein, the substitutions are defined as being replaced by one or more R groups;
Each B¹ and B² is independently absent, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused with 6-10 membered aryl, optionally substituted saturated or unsaturated 3-8 membered carbocyclyl fused with 5-10 membered heteroaryl, optionally substituted saturated or unsaturated 3-8 membered heterocyclyl fused with 6-10 membered aryl, optionally substituted saturated or unsaturated 3-8 membered heterocyclyl fused with 5-10 membered heteroaryl, optionally substituted 6-10 membered aryl, optionally substituted 5-10 membered heteroaryl, optionally substituted C1-C4 alkyl, optionally substituted C2-C4 alkenyl, or optionally substituted C2-C4 alkynyl, and at most one of B1 and B2 is absent; wherein, the substitutions are defined as being replaced by one or more R groups;
Cy is selected from optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl ring, optionally substituted 6-10 membered aryl, and optionally substituted 5-10 membered heteroaryl; wherein, the substitutions are defined as being replaced by one or more R groups;
L is optionally substituted C1-C3 alkyl, -O-, -S-, -NR^{L}-, -(C=O)-, -C(=O)O-, -C(=O)NR^{L}-, -(S=O)-, -S(=O)₂-, -S(=O)₂NR^{L}-, -S(=O)NR^{L}-, or -NR^{L}C(=O)NR^{L}-, wherein, each R^{L} is independently H or optionally substituted C1-C4 alkyl; wherein, the substitutions are defined as being replaced by one or more R groups;
a is 0 or 1;
Z is selected from hydrogen, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, optionally substituted 5-10 membered heteroaryl, optionally substituted C1-C6 alkyl, optionally substituted C2-C6 alkenyl and optionally substituted C2-C6 alkynyl; wherein, the substitutions are defined as being replaced by one or more R groups;
Each R is independently selected from: D, halogen, -OH, oxo, thiol, cyano, -CD₃, C1-C6 alkyl, C2-C6 alkenyl, C2-C6 alkynyl, C3-C8 cycloalkyl, 6-10 membered aryl, 4-12 membered heterocyclyl, 5-10 membered heteroaryl, 6-10 membered aryl-C1-C6 alkyl, 5-10 membered heteroaryl-C1-C6 alkyl, C1-C6 haloalkyl, -OC1-C6 alkyl, -OC2-C6 alkenyl, C3-C8 cycloalkyl-O-, 4-12 membered heterocyclyl-O-, 6-10 membered aryl-O-, 5-10 membered heteroaryl-O-, -O-C1-C6 alkylphenyl, -C1-C6 alkyl-OH, -C1-C6 alkyl-SH, -C1-C6 alkyl-O-C1-C6 alkyl, -OC1-C6 haloalkyl, -NH₂, -C1-C6 alkyl-NH₂, -N(C1-C6 alkyl)₂, -NH(C1-C6 alkyl), -N(C1-C6 alkyl)(C1-C6 alkylphenyl), -NH(C1-C6 alkylphenyl), -N(C1-C6 alkyl)(6-10 membered aryl), -NH(6-10 membered aryl), nitro, -C(O)-OH, -C(O)OC1-C6 alkyl, -CONRⁱRⁱⁱ, -NHC(O)(C1-C6 alkyl), -NHC(O)(phenyl), -N(C1-C6 alkyl)C(O)(C1-C6 alkyl), -N(C1-C6 alkyl)C(O)(phenyl), -C(O)C1-C6 alkyl, 5-10 membered heteroaryl C(O), -C(O)C1-C6 alkylphenyl, -C(O)C1-C6 haloalkyl, -OC(O)C1-C6 alkyl, -S(O)₂-C1-C6 alkyl, -S(O)-C1-C6 alkyl, -S(O)₂-phenyl, -S(O)₂-C1-C6 haloalkyl, -S(O)₂NH₂, -S(O)₂NH(C1-C6 alkyl), -S(O)₂NH(phenyl), -NHS(O)₂(C1-C6 alkyl), -NHS(O)₂(phenyl), and -NHS(O)₂(C1-C6 haloalkyl); wherein each of the alkyl, alkenyl, alkynyl, cycloalkyl, phenyl, aryl, heterocyclyl, and heteroaryl is optionally further substituted by one or more substituents selected from halogen, -OH, oxo (=O), -NH₂, C3-C8 cycloalkyl, 3-8 membered heterocyclyl, C1-C4 alkyl, C1-C4 haloalkyl, -OC1-C4 alkyl, -C1-C4 alkyl-OH, -C1-C4 alkyl-O-C1-C4 alkyl, -OC1-C4 haloalkyl, cyano, nitro, -C(O)-OH, -C(O)OC1-C6 alkyl, -CON(C1-C6 alkyl)₂, -CONH(C1-C6 alkyl), -CONH₂, -NHC(O)(C1-C6 alkyl), -NH(C1-C6 alkyl)C(O)(C1-C6 alkyl), -SO₂(C1-C6 alkyl), -SO₂(phenyl), -SO₂(C1-C6 haloalkyl), -SO₂NH₂, -SO₂NH(C1-C6 alkyl), -SO₂NH(phenyl), -NHSO₂(C1-C6 alkyl), -NHSO₂(phenyl), and -NHSO₂(C1-C6 haloalkyl); each Rⁱ and Rⁱⁱ is independently H, D, or C1-6 alkyl.

2. The compound according to claim 1, or pharmaceutically acceptable salts thereof, enantiomers, diastereomers, tautomers, cis-trans isomers, solvates, polymorphs, deuterated compounds, or combinations thereof, wherein;
X is H or halogen;
A is 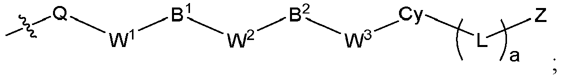
wherein,
Q is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, or optionally substituted 5-8 membered heteroaryl; wherein, the substitutions are defined as being replaced by one or more R groups;
W¹ is absent, -NR^{w}-, or -O-, wherein R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups; at most one of Q and W¹ is absent;
Each W² and W³ is independently absent, optionally substituted methylene, -O-, -S-, -NR^{w'}-, -(C=O)-, -C(=O)NR^{w'}-, -(S=O)-, -S(=O)₂-, -S(=O)₂NR^{w'}-, -S(=O)NR^{w'}-, or -NR^{w'}C(=O)NR^{w'}-, wherein each R^{w'} is independently H or optionally substituted C1-C6 alkyl; wherein, the substitutions are defined as being replaced by one or more R groups;
Each B¹ and B² is independently absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl; wherein, the substitutions are defined as being replaced by one or more R groups; at most one of B¹ and B² is absent;
Cy is optionally substituted saturated or unsaturated 3-8 membered carbocyclyl or optionally substituted saturated or unsaturated 4-12 membered heterocyclyl; wherein, the substitutions are defined as being replaced by one or more R groups;
L is optionally substituted C1-C3 alkyl, -O-, -S-, -NR^{L}-, -(C=O)-, -C(=O)NR^{L}-, -(S=O)-, -S(=O)₂-, -S(=O)₂NR^{L}-, -S(=O)NR^{L}-, or -NR^{L}C(=O)NR^{L}-, wherein, each R^{L} is independently H or optionally substituted C1-C4 alkyl; wherein, the substitutions are defined as being replaced by one or more R groups.
a is 0 or 1;
Z is optionally substituted 6-10 membered aryl, or optionally substituted 5-10 membered heteroaryl; wherein, the substitutions are defined as being replaced by one or more R groups;
The definition of R is as described in claim 1.

3. The compound according to claim 1 or 2, or pharmaceutically acceptable salts thereof, enantiomers, diastereomers, tautomers, cis-trans isomers, solvates, polymorphs, deuterated compounds, or combinations thereof, wherein;
A is either of formula A-1 or formula A-2, 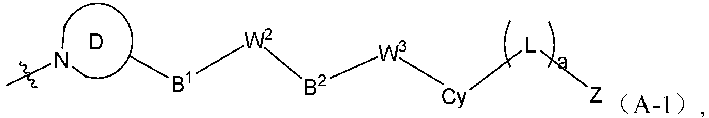 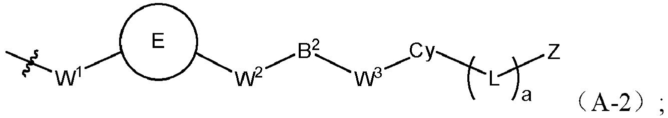 wherein,
The D ring is optionally substituted saturated or unsaturated 4-10 membered heterocyclyl, optionally substituted saturated or unsaturated 3-8 membered heterocyclyl fused to a 6-8 membered aryl, or optionally substituted saturated or unsaturated 3-8 membered heterocyclyl fused to 5-8 membered heteroaryl; wherein, the substitutions are defined as being replaced by one or more R groups;
The E ring is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to 6-8 membered aryl, optionally substituted saturated or unsaturated 3-8 membered heterocyclyl fused to 5-8 membered heteroaryl, optionally substituted 6-8 membered aryl, or optionally substituted 5-8 membered heteroaryl; wherein, the substitutions are defined as being replaced by one or more R groups;
B¹ is absent, optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl; wherein, the substitutions are defined as being replaced by one or more R groups;
W¹ is -NR^{w}- or -O-, wherein, R^{w} is H, optionally substituted C1-C6 alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused to a 6-10 membered aryl, optionally substituted saturated or unsaturated 3-8 membered carbocyclyl fused to a 5-10 membered heteroaryl, optionally substituted saturated or unsaturated 3-8 membered heterocyclyl fused to a 6-10 membered aryl, optionally substituted saturated or unsaturated 3-8 membered heterocyclyl fused to a 5-10 membered heteroaryl, optionally substituted 6-10 membered aryl, or optionally substituted 5-10 membered heteroaryl; wherein, the substitutions are defined as being replaced by one or more R groups;
Each W² and W³ is independently absent, optionally substituted methylene, -O-, -S-, -NR^{w'}-, -(C=O)-, -C(=O)NR^{w'}-, -(S=O)-, -S(=O)₂-, -S(=O)₂NR^{w'}-, -S(=O)NR^{w'}-, or -NR^{w'}C(=O)NR^{w'}-; wherein, each R^{w'} is independently H, or optionally substituted C1-C6 alkyl; wherein, the substitutions are defined as being replaced by one or more R groups;
B² is optionally substituted saturated or unsaturated 3-8 membered carbocyclyl, optionally substituted saturated or unsaturated 4-10 membered heterocyclyl, optionally substituted saturated or unsaturated 3-8 membered carbocyclyl fused to a 6-8 membered aryl, optionally substituted saturated or unsaturated 3-8 membered carbocyclyl fused to a 5-8 membered heteroaryl, optionally substituted saturated or unsaturated 3-8 membered heterocyclyl fused to a 6-8 membered aryl, optionally substituted saturated or unsaturated 3-8 membered heterocyclyl fused to a 5-8 membered heteroaryl, optionally substituted 6-8 membered aryl, optionally substituted 5-8 membered heteroaryl, or optionally substituted C1-C2 alkyl group; wherein, the substitutions are defined as being replaced by one or more R groups;
Cy is optionally substituted saturated or unsaturated 3-8 membered carbocyclyl or optionally substituted saturated or unsaturated 4-12 membered heterocyclyl; wherein, the substitutions are defined as being replaced by one or more R groups;
L is optionally substituted C1-C3 alkyl group, -O-, -S-, -NR^{L}-, -(C=O)-, -C(=O)NR^{L}-, -(S=O)-, -S(=O)₂-, -S(=O)₂NR^{L}-, -S(=O)NR^{L}-, or -NR^{L}C(=O)NR^{L}-; wherein, each R^{L} independently is H or optionally substituted C1-C4 alkyl group; wherein, the substitutions are defined as being replaced by one or more R groups;
a is 0 or 1;
Z is optionally substituted 6-10 membered aryl or optionally substituted 5-10 membered heteroaryl; wherein, the substitutions are defined as being replaced by one or more R groups;
The definition of the R group is as described in claim 1.

4. The compound according to any of claims 1-3, or pharmaceutically acceptable salts thereof, enantiomers, diastereomers, tautomers, cis-trans isomers, solvates, polymorphs, deuterated compounds, or combinations thereof, wherein;
Z is selected from: 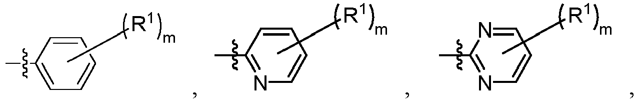 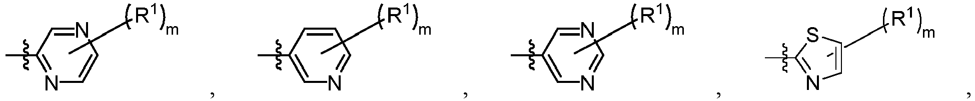 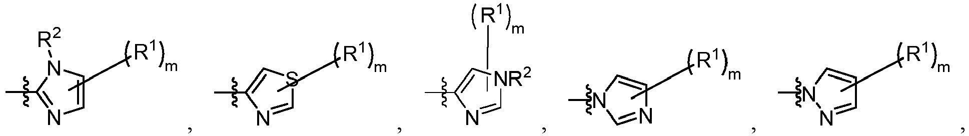 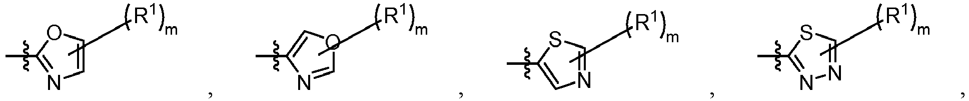 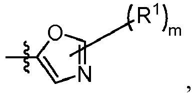 wherein, each of R¹ and R² is independently selected from: halogen, optionally substituted C1-C6 alkyl, cyano, optionally substituted C3-C16 cycloalkyl, optionally substituted 4-16 membered heterocyclyl, 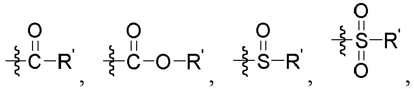 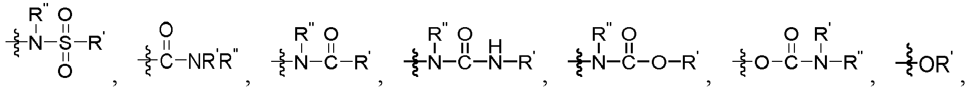 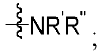 or two R¹ groups located on any adjacent atoms of ring together with their adjacent atoms form optionally substituted C3-C16 carbocyclyl, optionally substituted 4-16 membered heterocyclyl; or R² with R¹ on the adjacent atom of ring forms an optionally substituted 4-16 membered heterocyclyl; wherein, R' and R" are each independently selected from: H, optionally substituted C1-C6 alkyl, optionally substituted C3-C8 cycloalkyl, optionally substituted 4-16 membered heterocyclyl; or R' and R" together with their adjacent atoms form an optionally substituted 4-16 membered heterocyclyl; wherein, the heterocyclyl formed by R' and R" with the N atom includes 1-3 heteroatoms selected from N, O, S, P; wherein, the substitutions are defined as being replaced by one or more R groups;
m is 0, 1, 2, or 3; The definition of the R group is as described in claim 1.

5. The compound according to any of claims 1-4, or pharmaceutically acceptable salts thereof, enantiomers, diastereomers, tautomers, cis-trans isomers, solvates, polymorphs, deuterated compounds, or combinations thereof, wherein;
Cy is selected from the following groups: 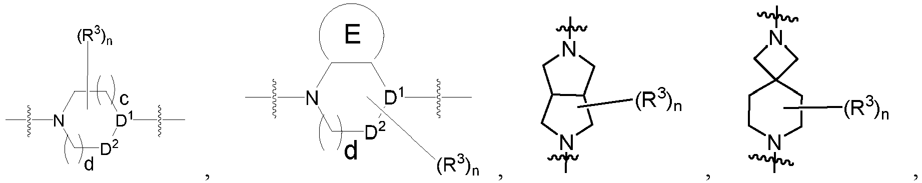 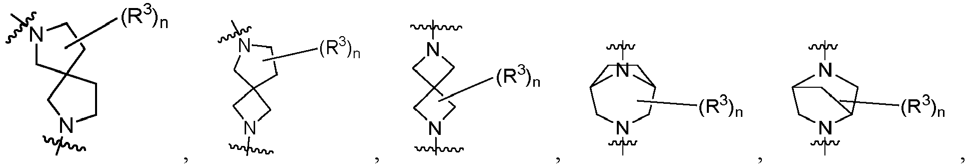 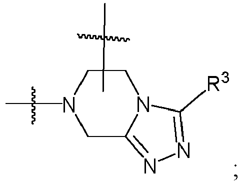
D¹ is N or CR^{D1}, wherein, R^{D1} is H, optionally substituted C1-C3 alkyl, halogen, hydroxy, optionally substituted C1-C3 alkoxy, or optionally substituted amino; wherein, the substitutions are defined as being replaced by one or more R groups;
D2 is NR^{D2}, optionally substituted methylene, O, or S; wherein, R^{D2} is H or optionally substituted C1-C4 alkyl; wherein, the substitutions are defined as being replaced by one or more R groups;
Each R³ is independently selected from: H, halogen, optionally substituted C1-C6 alkyl, optionally substituted C3-C16 cycloalkyl, optionally substituted 4-16 membered heterocyclyl, 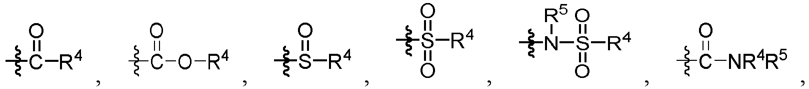 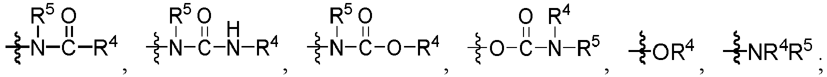 wherein, R⁴ and R⁵ are independently selected from: H, optionally substituted C1-C6 alkyl, optionally substituted C3-C8 cycloalkyl, optionally substituted 4-16 membered heterocyclyl; or R⁴ and R⁵ together with the adjacent atoms form optionally substituted 4-16 membered heterocyclyl, wherein the heterocyclyl formed by R⁴ and R⁵ with the N atom contains 1-3 heteroatoms selected from N, O, S, P, 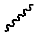 indicating the attachment position of the group; or two R³ groups located on any adjacent atoms of ring and their adjacent atoms together form optionally substituted C3-C16 cycloalkyl or optionally substituted 4-16 membered heterocyclyl; wherein, the substitutions are defined as being replaced by one or more R groups;
E-ring is optionally substituted 6-8 membered aryl or optionally substituted 5-8 membered heteroaryl; wherein, the substitutions are defined as being replaced by one or more R groups;
n represents the number of substituents R³, and n is 0, 1, 2, or 3;
c and d are independently 0, 1, 2, or 3;
The definition of R is as described in claim 1.

6. The compound according to any of claims 1-5, or pharmaceutically acceptable salts thereof, enantiomers, diastereomers, tautomers, cis-trans isomers, solvates, polymorphs, deuterated compounds, or combinations thereof, wherein;
Cy is selected from the following groups: 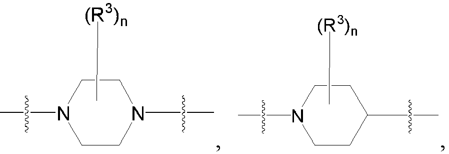 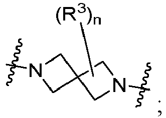
The definition of R³ and n is as described in claim 5;

7. The compound according to claim 1, or pharmaceutically acceptable salts thereof, enantiomers, diastereomers, tautomers, cis-trans isomers, solvates, polymorphs, deuterated compounds, or combinations thereof, wherein;
Q is absent, optionally substituted saturated or unsaturated C3-C8 cycloalkyl, optionally substituted saturated or unsaturated 4-10 membered heterocyclyl, optionally substituted saturated or unsaturated 3-8 membered heterocyclyl fused to a phenyl, optionally substituted saturated or unsaturated 3-8 membered heterocyclyl fused to a 5-6 membered heteroaryl; preferably, Q is optionally substituted C3-C6 cycloalkyl or optionally substituted 4-10 membered heterocyclyl; wherein, the substitutions are defined as being replaced by one or more R groups; The definition of R is as described in claim 1.

8. The compound according to claim 1, or pharmaceutically acceptable salts thereof, enantiomers, diastereomers, tautomers, cis-trans isomers, solvates, polymorphs, deuterated compounds, or combinations thereof, wherein; 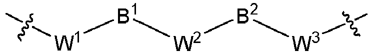 is selected from:

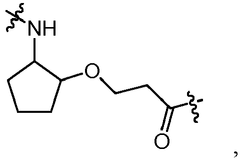

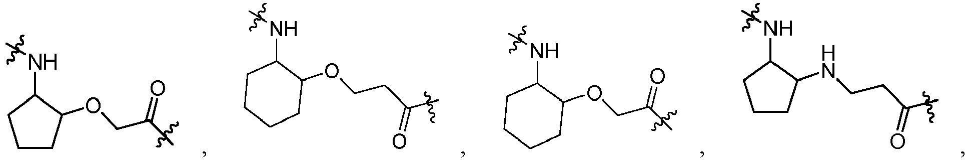

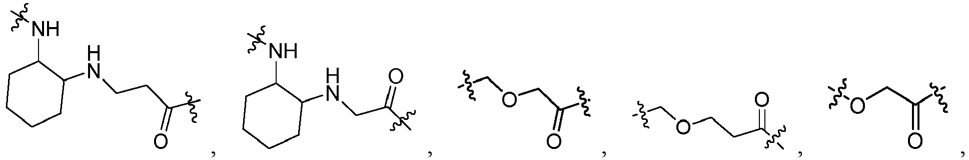

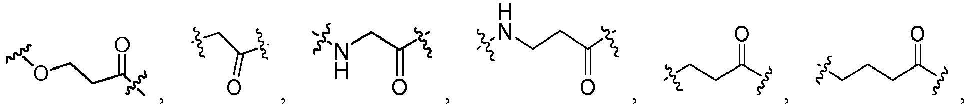

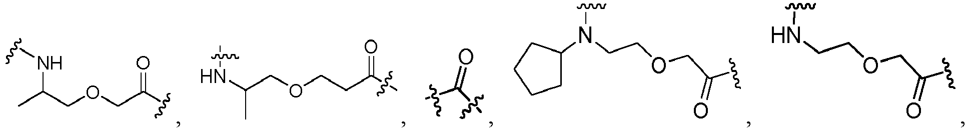

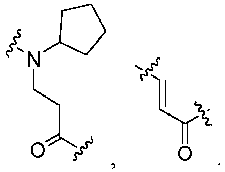

9. The compound according to any of claims 1-8, or pharmaceutically acceptable salts thereof, enantiomers, diastereomers, tautomers, cis-trans isomers, solvates, polymorphs, deuterated compounds, or combinations thereof, wherein; 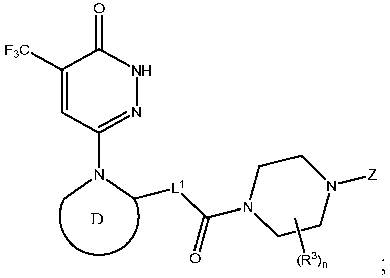
Wherein, L¹ is selected from the following groups: absent, 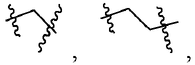 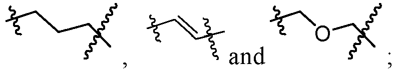 and L¹ may be optionally substituted by one or more R;
D ring is optionally substituted saturated or unsaturated 4-10 membered heterocyclyl, optionally substituted saturated or unsaturated 3-8 membered heterocyclyl fused to a 6-8 membered aryl, or optionally substituted saturated or unsaturated 3-8 membered heterocyclyl fused to a 5-8 membered heteroaryl, preferably, D ring is optionally substituted 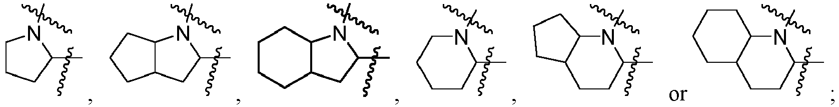 wherein, the substitutions are defined as being replaced by one or more R groups;
The definitions of Z, R³, n, and R are as previously described.

10. The compound according to claim 1, or pharmaceutically acceptable salts thereof, enantiomers, diastereomers, tautomers, cis-trans isomers, solvates, polymorphs, deuterated compounds, or combinations thereof, wherein, the compound is selected from the following compounds:

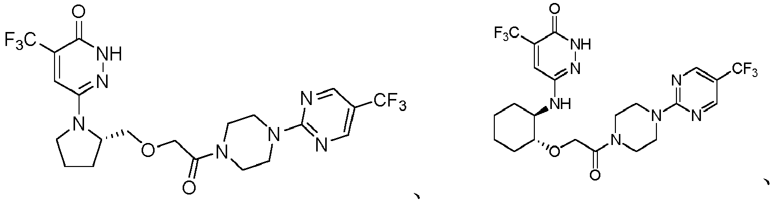

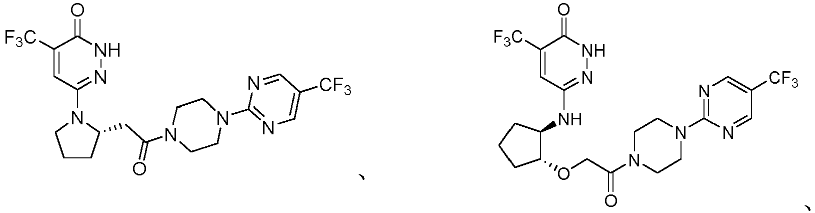

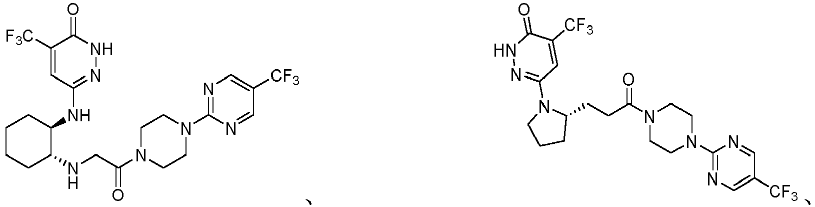

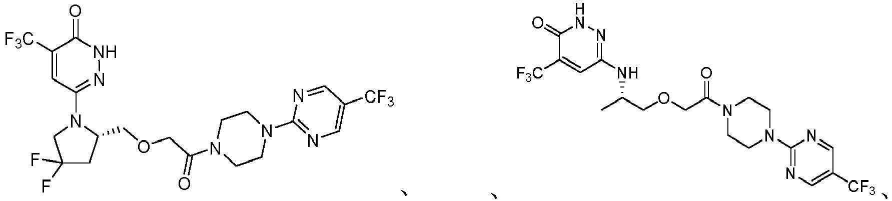

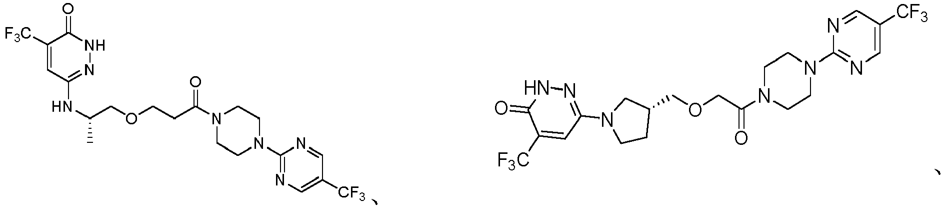

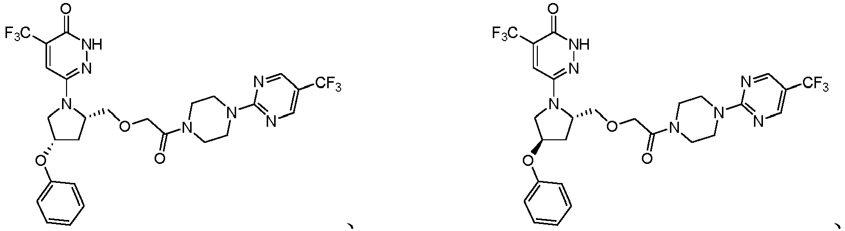

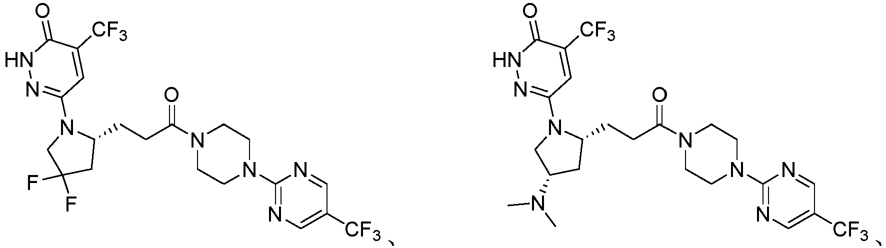

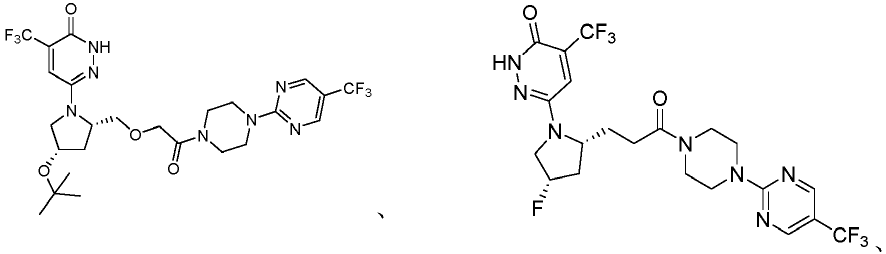

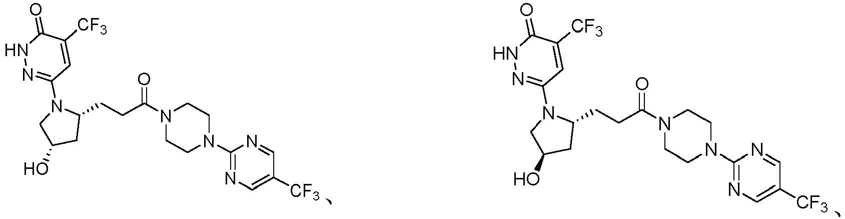

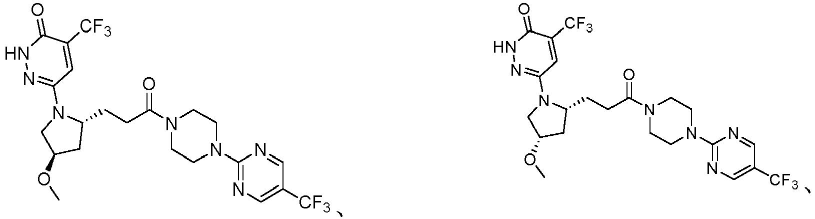

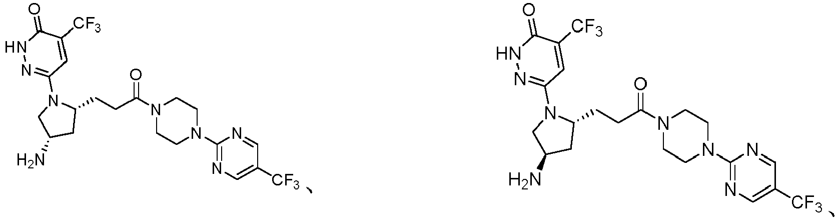

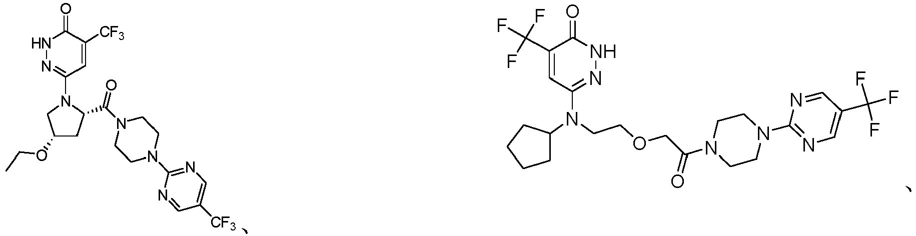

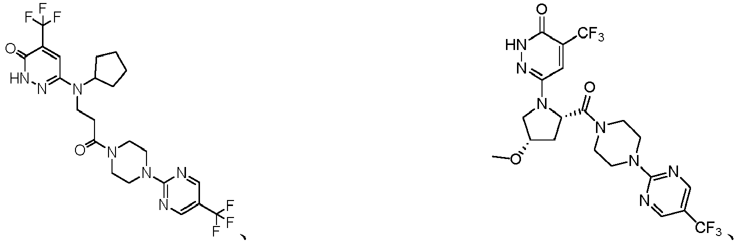

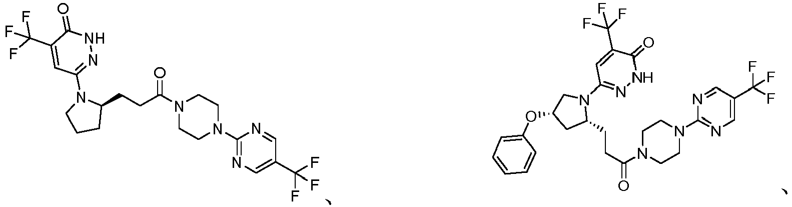

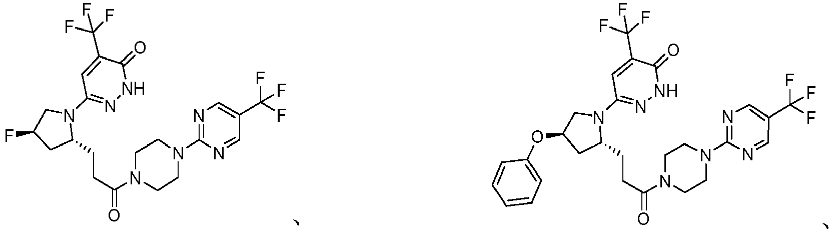

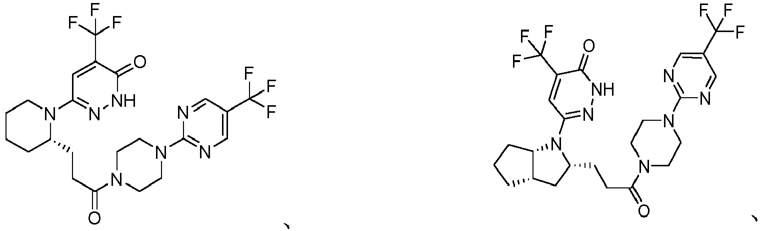

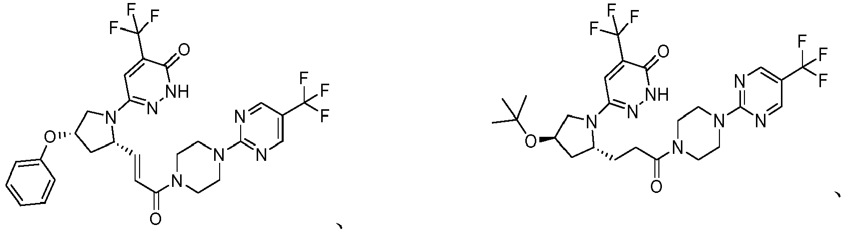

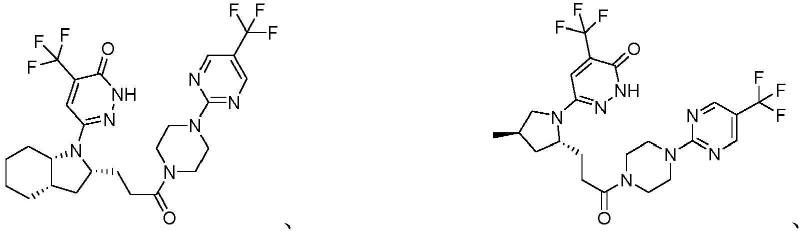

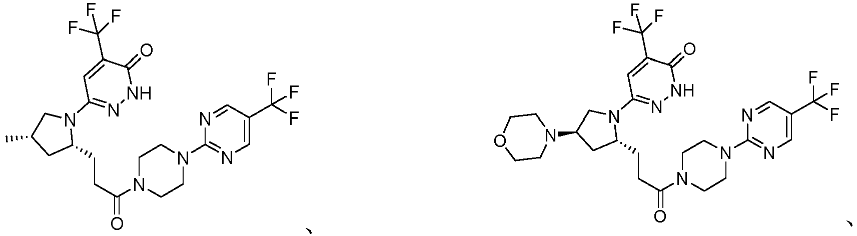

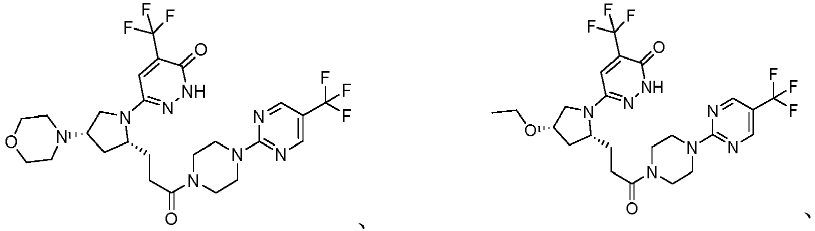

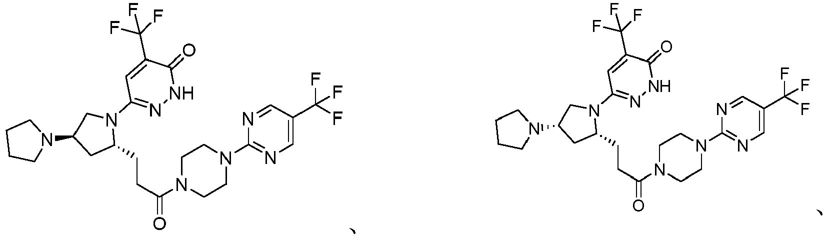

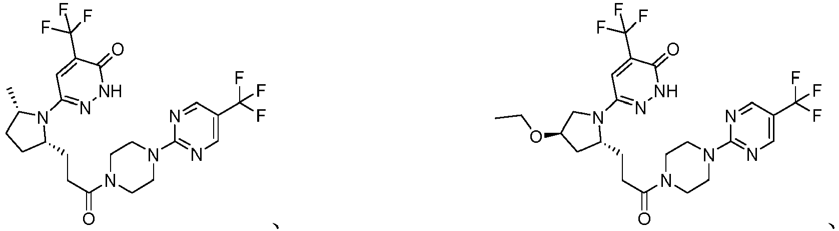

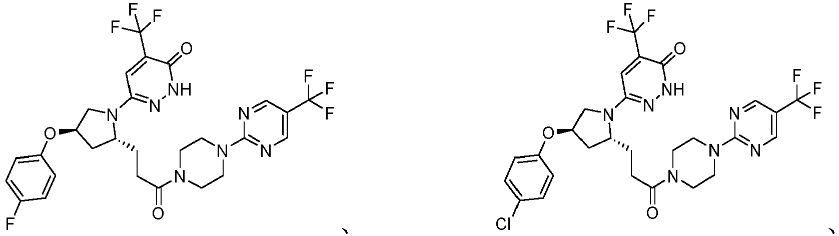

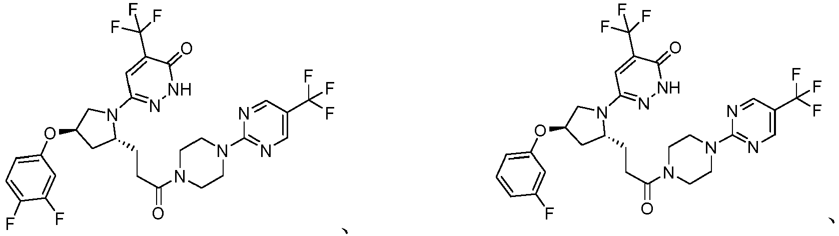

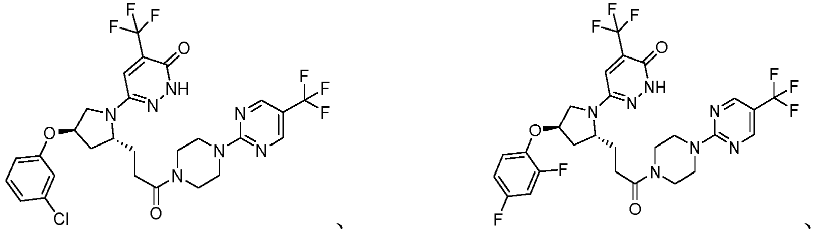

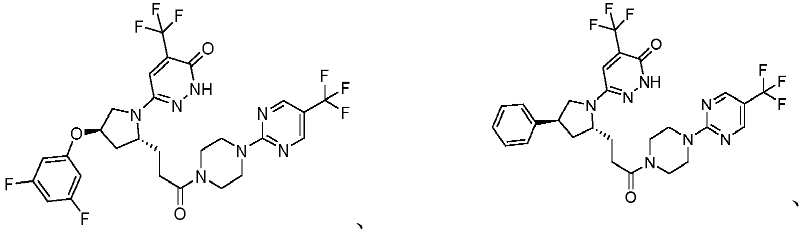

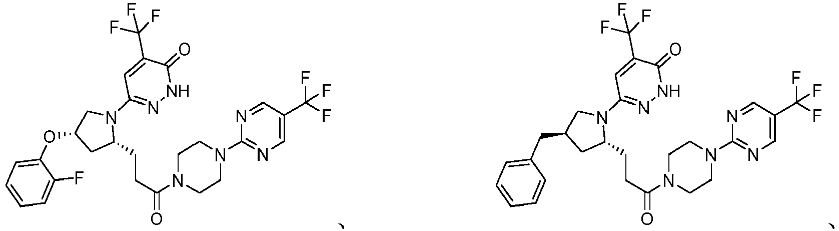

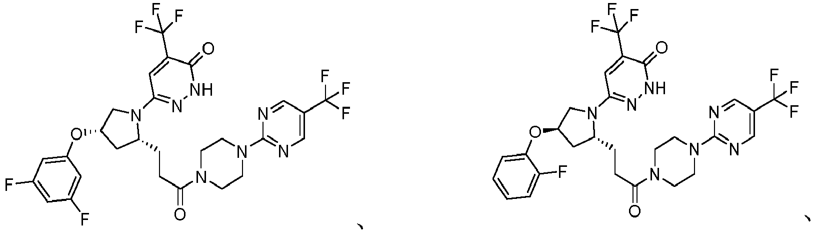

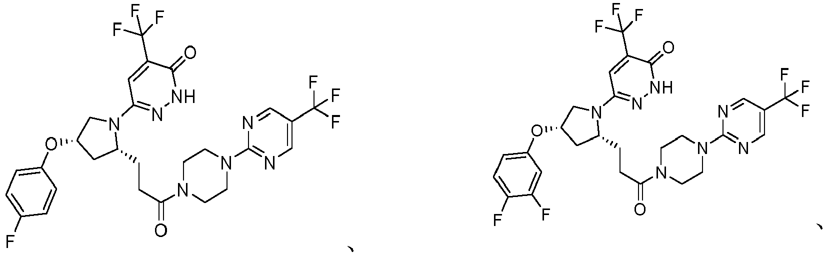

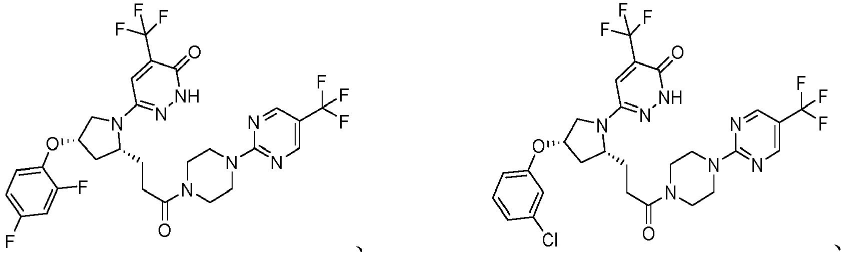

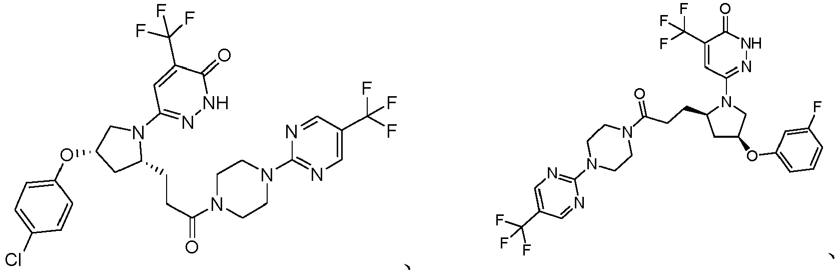

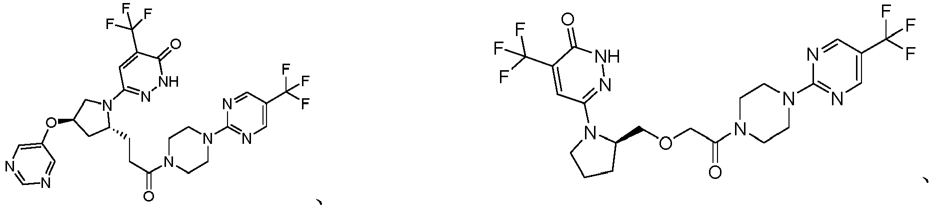

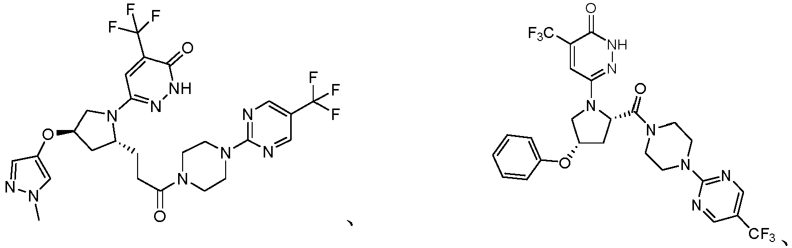

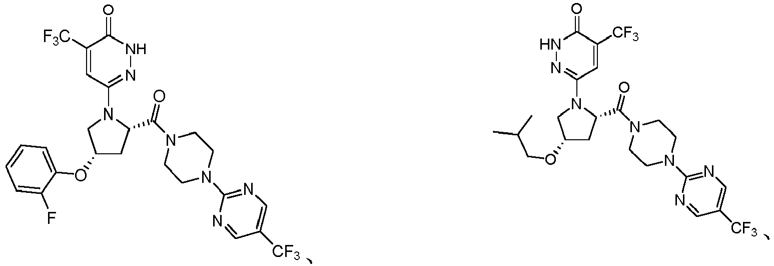

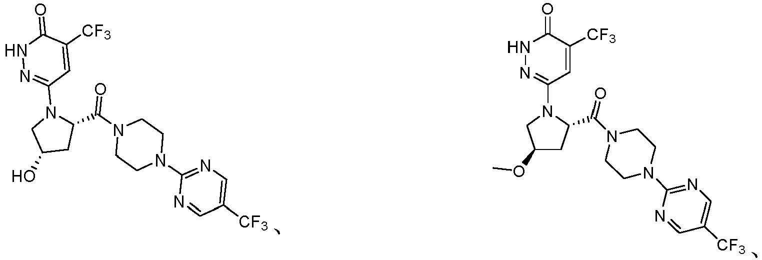

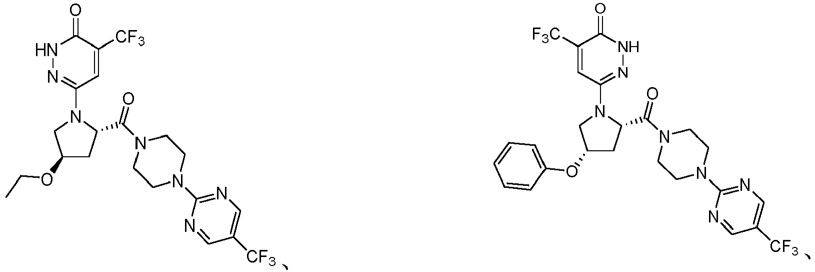

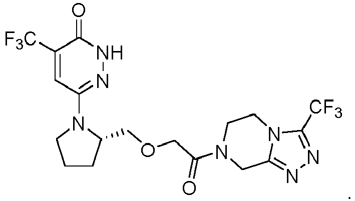

11. A compound of Formula II-1 or II-2, or their pharmaceutically acceptable salts thereof, isomers, solvates, polymorphs, or deuterated forms, 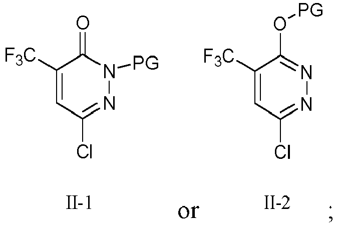
Wherein, PG is a protecting group other than a methyl group; preferably, the protecting group is 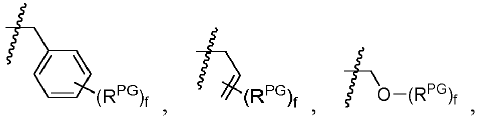 tert-butoxycarbonyl, benzyloxycarbonyl, 9-fluorenylmethyloxycarbonyl, 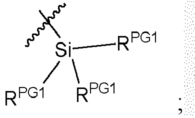 Each R^{PG} is independently C1-C4 alkyl, C1-C4 alkoxy, halogen, nitro, phenyl, benzyl, p-methoxybenzyl, trifluoromethyl, or 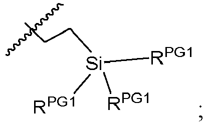 each R^{PG1} is independently C1-C4 alkyl or phenyl.
More preferably, the protecting group is 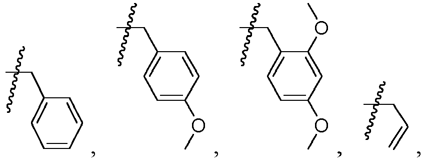 methoxymethyl, or 2-(trimethylsilyl)ethoxymethyl.

12. A pharmaceutical composition, comprising:
(1) a therapeutically effective amount of one or more of the compounds according to any of claims 1-10, or pharmaceutically acceptable salts thereof, enantiomers, diastereomers, tautomers, cis-trans isomers, solvates, polymorphs, and deuterated compounds thereof as active ingredients; and
(2) optionally, pharmaceutically acceptable excipients.

13. A use of the compound according to any one of claims 1 to 10, or pharmaceutically acceptable salts thereof, enantiomers, diastereomers, tautomers, cis-trans isomers, solvates, polymorphs or deuterated compounds thereof, or the pharmaceutical composition according to claim 12 in the preparation of medicaments for prevention or treatment of diseases related to PARP7.

14. The use according to claim 13, wherein, the diseases are selected from the following diseases: cancer, infections, immune diseases, cardiovascular diseases, central nervous system diseases, and metabolic diseases.

15. The use according to claim 14, wherein, cancer is selected from the following types: lung cancer, pancreatic cancer, colorectal cancer, leukemia, Ewing's sarcoma, breast cancer, prostate cancer, T-cell lymphoma, B-cell lymphoma, malignant rhabdomyosarcoma, synovial sarcoma, uterine fibroids, gastric cancer, liver cancer, kidney cancer, melanoma, ovarian cancer, brain glioma, bile duct cancer, nasopharyngeal cancer, cervical cancer, head and neck cancer, esophageal cancer, thyroid cancer, and bladder cancer.
